(19) **Europäisches Patentamt / European Patent Office / Office européen des brevets**

(11) **EP 2 489 726 A2**

(12) # EUROPEAN PATENT APPLICATION

(43) Date of publication:
**22.08.2012 Bulletin 2012/34**

(21) Application number: **12152296.5**

(22) Date of filing: **12.01.2006**

(51) Int Cl.:
*C12N 5/04* (2006.01)      *C12N 15/82* (2006.01)
*C12N 15/90* (2006.01)      *A01H 5/00* (2006.01)
*C12N 9/00* (2006.01)

(84) Designated Contracting States:
**AT BE BG CH CY CZ DE DK EE ES FI FR GB GR HU IE IS IT LI LT LU LV MC NL PL PT RO SE SI SK TR**

(30) Priority: **12.01.2005 US 643717 P**

(62) Document number(s) of the earlier application(s) in accordance with Art. 76 EPC:
**06718079.4 / 1 850 656**

(71) Applicant: **Monsanto Technology LLC**
**St. Louis, MO 63167 (US)**

(72) Inventor: **Abad, Mark**
**St. Louis, MO Missouri 63167 (US)**

(74) Representative: **von Kreisler Selting Werner**
**Deichmannhaus am Dom**
**Bahnhofsvorplatz 1**
**50667 Köln (DE)**

Remarks:
•The complete document including Reference Tables and the Sequence Listing can be downloaded from the EPO website
•This application was filed on 24-01-2012 as a divisional application to the application mentioned under INID code 62.
•Claims filed after the date of filing of the application /after the date of receipt of the divisional application (Rule 68(4) EPC).

(54) **Genes and uses for plant improvement**

(57)     This invention provides transgenic plant cells with recombinant DNA for expression of proteins that are useful for imparting enhanced agronomic trait(s) to transgenic crop plants. This invention also provides transgenic plants and progeny seed comprising the transgenic plant cells where the plants are selected for having an enhanced trait selected from the group of traits consisting of enhanced water use efficiency, enhanced cold tolerance, increased yield, enhanced nitrogen use efficiency, enhanced seed protein and enhanced seed oil. Also disclosed are methods for manufacturing transgenic seed and plants with enhanced traits.

EP 2 489 726 A2

Description

## CROSS REFERENCE TO RELATED APPLICATIONS

This application claims benefit under 35USC § 119(e) of United States provisional application Serial No. 60/642,717, filed 01/12/2005, herein incorporated by reference.

## INCOPORATION OF SEQUENCE LISTING

Two copies of the sequence listing (Copy 1 and Copy 2) and a computer readable form (CRF) of the sequence listing, all on CD-ROMs, each containing the file named 38-21(53629)B_seqListing.txt, which is 59,207,680 bytes (measured in MS-WINDOWS) and was created on January 11, 2006, are herein incorporated by reference.

## INCORPORATION OF COMPUTER PROGRAM LISTING

Computer Program Listing folders hmmer-2.3.2 and 158pfamDir are contained on a compact disc and is hereby incorporated herein by reference in their entirety. Folder hmmer-2.3.2 contains the source code and other associated file for implementing the HMMer software for Pfam analysis. Folder 158pfamDir contains 158 Pfam Hidden Markov Models. Both folders were created on the disk on January 11, 2006, having a total size of 16,027,648 bytes (measured in MS-WINDOWS).

## FIELD OF THE INVENTION

Disclosed herein are inventions in the field of plant genetics and developmental biology. More specifically, the present inventions provide transgenic seeds for crops, wherein the genome of said seed comprises recombinant DNA, the expression of which results in the production of transgenic plants that have improved trait(s).

## BACKGROUND OF THE INVENTION

Transgenic plants with improved traits such as improved yield, environmental stress tolerance, pest resistance, herbicide tolerance, modified seed compositions, and the like are desired by both farmers and consumers. Although considerable efforts in plant breeding have provided significant gains in desired traits, the ability to introduce specific DNA into plant genomes provides further opportunities for generation of plants with improved and/or unique traits. The ability to develop transgenic plants with improved traits depends in part on the identification of genes that are useful in recombinant DNA constructs for production of transformed plants with improved properties.

## SUMMARY OF THE INVENTION (NEW)

This invention provides recombinant DNA for expression of proteins that impart enhanced agronomic traits in transgenic plants. Recombinant DNA in this invention is provided in a construct comprising a promoter that is functional in plant cells and that is operably linked to DNA that encodes a protein having at least one amino acid domain in a sequence that exceeds the Pfam gathering cutoff for amino acid sequence alignment with a protein domain family identified by a Pfam name in the group of Pfam domain names identified in Table 17. In more specific embodiments of the invention plant cells are provided which express a protein having amino acid sequence with at least 90% identity to a consensus amino acid sequence in the group of consensus amino acid sequences consisting of the consensus amino acid sequence constructed for SEQ ID NO: 205 and homologs thereof listed in Table 2 through the consensus amino acid sequence constructed for SEQ ID NO:408 and homologs thereof listed in Table 2. Amino acid sequences of homologs are SEQ ID NO:409 through 19247. In even more specific embodiments of the invention the protein expressed in plant cells is a protein selected from the group of proteins identified in Table 1 by annotation to a related protein in Genbank and alternatively identified in Table 16 by identification of protein domain family. An exemplary plant cell of this invention has recombinant DNA that encodes a protein identified by the Pdam name "RNA_pol_L".

Other aspects of the invention are specifically directed to transgenic plant cells comprising the recombinant DNA of the invention, transgenic plants comprising a plurality of such plant cells, progeny transgenic seed, embryo and transgenic pollen from such plants. Such plant cells are selected from a population of transgenic plants regenerated from plant cells transformed with recombinant DNA and that express the protein by screening transgenic plants in the population for an enhanced trait as compared to control plants that do not have said recombinant DNA, where the enhanced trait is enhanced water use efficiency, enhanced cold tolerance, enhanced heat tolerance, enhanced shade tolerance, enhanced tolerance to salt exposure, increased yield, enhanced nitrogen use efficiency, enhanced seed protein and enhanced seed oil. Thus, this invention provides transgenic plants and seeds with cells having recombinant DNA that impart at least one of those enhanced traits to the plants or seeds.

In yet another aspect of the invention the plant cells, plants, seeds, embryo and pollen further comprise DNA expressing a protein that provides tolerance from exposure to an herbicide applied at levels that are lethal to a wild type of said plant cell. Such tolerance is especially useful not only as an advantageous trait in such plants but is also useful in a selection step in the methods of the invention. In aspects of the invention the agent of such herbicide is a glyphosate, dicamba, or glufosinate compound.

Yet other aspects of the invention provide transgenic plants which are homozygous for the recombinant DNA and transgenic seed of the invention from corn, soybean, cotton, canola, alfalfa, wheat or rice plants. In other important embodiments for practice of various aspects of the invention in Argentina the recombinant DNA is provided in plant cells derived from corn lines that that are and maintain resistance to the Mal de Rio Cuarto virus or the *Puccina sorghi* fungus or both.

This invention also provides methods for manufacturing non-natural, transgenic seed that can be used to produce a crop of transgenic plants with an enhanced trait resulting from expression of stably-integrated, recombinant DNA for expressing a protein having at least one domain of amino acids in a sequence that exceeds the Pfam gathering cutoff for amino acid sequence alignment with a protein domain family identified by a Pfam name in the group of Pfam names identified in Table 17. More specifically the method comprises (a) screening a population of plants for an enhanced trait and recombinant DNA, where individual plants in the population can exhibit the trait at a level less than, essentially the same as or greater than the level that the trait is exhibited in control plants which do not express the recombinant DNA, (b) selecting from the population one or more plants that exhibit the trait at a level greater than the level that said trait is exhibited in control plants, (c) verifying that the recombinant DNA is stably integrated in said selected plants, (d) analyzing tissue of a selected plant to determine the production of a protein having the function of a protein encoded by nucleotides in a sequence of one of SEQ ID NO:1-204; and (e) collecting seed from a selected plant. In one aspect of the invention the plants in the population further comprise DNA expressing a protein that provides tolerance to exposure to an herbicide applied at levels that are lethal to wild type plant cells and the selecting is effected by treating the population with the herbicide, e.g. a glyphosate, dicamba, or glufosinate compound. In another aspect of the invention the plants are selected by identifying plants with the enhanced trait. The methods are especially useful for manufacturing corn, soybean, cotton, alfalfa, wheat or rice seed selected as having one of the enhanced traits described above.

Another aspect of the invention provides a method of producing hybrid corn seed comprising acquiring hybrid corn seed from a herbicide tolerant corn plant which also has stably-integrated, recombinant DNA comprising a promoter that is (a) functional in plant cells and (b) is operably linked to DNA that encodes a protein having at least one domain of amino acids in a sequence that exceeds the Pfam gathering cutoff for amino acid sequence alignment with a protein domain family identified by a Pfam name in the group of Pfam names identified in Table 17. The methods further comprise producing corn plants from said hybrid corn seed, where a fraction of the plants produced from said hybrid corn seed is homozygous for said recombinant DNA, a fraction of the plants produced from said hybrid corn seed is hemizygous for said recombinant DNA, and a fraction of the plants produced from said hybrid corn seed has none of said recombinant DNA; selecting corn plants which are homozygous and hemizygous for said recombinant DNA by treating with an herbicide; collecting seed from herbicide-treated-surviving corn plants and planting said seed to produce further progeny corn plants; repeating the selecting and collecting steps at least once to produce an inbred corn line; and crossing the inbred corn line with a second corn line to produce hybrid seed.

Another aspect of the invention provides a method of selecting a plant comprising plant cells of the invention by using an immunoreactive antibody to detect the presence of protein expressed by recombinant DNA in seed or plant tissue. Yet another aspect of the invention provides anti-counterfeit milled seed having, as an indication of origin, a plant cells of this invention with unique recombinant DNA.

Another aspect of the invention provides plant cells having recombinant DNA for suppressing the expression of DNA identified in Table 1 and Table 16. More specific aspects of the invention provide plant cells having recombinant DNA for suppressing the expression of a protein having the function in a plant of the protein with amino acid sequence of SEQ ID NO: 213, 215, 218, 222, 258, 269, 275, 334, 361, 368, and 407 or the corresponding Pfam identified in Table 16, i.e. Catalase, Bromdomain, FTCD_N, MatE, DPBB_1, tRNA-synt_2b, Sugar_tr and MFS_1, DUF6 and DUF250, LEA_4, MIP and DUF231, respectively. Such suppression can be effected by any of a number of ways known in the art, e.g. anti-sense suppression, sense co-suppression, RNAi or knockout.

Still other specific aspects of this invention relate to growing transgenic plants with enhanced water use efficiency or enhanced nitrogen use efficiency. For instance, this invention provides methods of growing a corn, cotton or soybean crop without irrigation water comprising planting seed having plant cells of the invention which are selected for enhanced water use efficiency. Alternatively methods comprise applying reduced irrigation water, e.g. providing up to 300 millimeters of ground water during the production of a corn crop. This invention also provides methods of growing a corn, cotton or soybean crop without added nitrogen fertilizer comprising planting seed having plant cells of the invention which are selected for enhanced nitrogen use efficiency.

The various aspects of this invention are especially useful for transgenic plant cells in seeds and transgenic plants having any of the above-described enhanced traits in crop plants such as corn (maize), soybean, cotton, canola (rape), wheat, sunflower, sorghum, alfalfa, barley, millet, rice, tobacco, fruit and vegetable crops, and turfgrass.

The invention also comprises recombinant DNA constructs of the DNA useful for imparting enhanced traits in plants having thee cells of this invention.

## BRIEF DESCRIPTION OF THE DRAWINGS

Figure 1 is an alignment of amino acid sequences.
Figures 2 and 3 are plasmid maps.

## DETAILED DESCRIPTION OF THE INVENTION

In the attached sequence listing:

SEQ ID NO: 1-204 are DNA sequence of "genes" used in the recombinant DNA imparting an enhanced trait in plant cells;
SEQ ID NO:205-408 are amino acid sequence of the cognate protein of those "genes";
SEQ ID NO:409-19247 are amino acid sequence of homologous proteins;
SEQ ID NO:19248 is a consensus amino acid sequence.
SEQ ID NO:19249 is a DNA sequence of a plasmid vector useful for corn transformation; and
SEQ ID NO:19250 is a DNA sequence of a plasmid vector useful for soybean transformation.

As used herein, "gene" means DNA including chromosomal DNA, plasmid DNA, cDNA, synthetic DNA, or other DNA that is transcribed to RNA, e.g. mRNA that encodes a protein or a protein fragment or anti-sense RNA or dsRNA for suppression of expression of a target gene and its cognate protein.

"Transgenic plant cell" means a plant cell produced as an original transformation event, cells in plants regenerated from the original transformation, cells in progeny plants and seeds, and cells in plants and seed from later generations or crosses of progeny plants and seeds, where such plant cells have recombinant DNA in their genome resulting from the original transformation.

"Recombinant DNA" means genetically engineered polynucleotide produced from endogenous and/or exogenous elements generally arranged as a transcription unit. Recombinant DNA may comprise DNA segments obtained from different sources, or DNA segments obtained from the same source, but which have been manipulated to join DNA segments which do not naturally exist in the joined form. A recombinant polynucleotide may exist outside of the cell, for example as a PCR fragment, or integrated into a genome, such as a plant genome.

"Trait" means to a physiological, morphological, biochemical, or physical characteristic of a plant or particular plant material or cell. In some instances, this characteristic is visible to the human eye, such as seed or plant size, or can be measured by biochemical techniques, such as detecting the protein, starch, or oil content of seed or leaves, or by observation of a metabolic or physiological process, e.g. by measuring uptake of carbon dioxide, or by the observation of the expression level of a gene or genes, e.g., by employing Northern analysis, RT-PCR, microarray gene expression assays, or reporter gene expression systems, or by agricultural observations such as stress tolerance, yield, or pathogen tolerance. An "enhanced trait" as used in describing the aspects of this invention includes enhanced water use efficiency, enhanced cold tolerance, enhanced heat tolerance, enhanced shade tolerance, enhanced tolerance to salt exposure, increased yield, enhanced nitrogen use efficiency, enhanced seed protein and enhanced seed oil.

As used herein, "control plant" is a plant without trait-improving recombinant DNA. A control plant is used to measure and compare trait improvement in a transgenic plant with such trait-improving recombinant DNA. A suitable control plant may be a non-transgenic plant of the parental line used to generate a transgenic plant herein. Alternatively, a control plant may be a transgenic plant that comprises an empty vector or marker gene, but does not contain the recombinant DNA that produces the trait improvement. A control plant may also be a negative segregant progeny of hemizygous transgenic plant. In certain demonstrations of trait improvement, the use of a limited number of control plants can cause a wide variation in the control dataset. In analyzing trait data during screening to discover DNA useful in the plant cells of this invention it is useful to minimize the effect of the variation within the control dataset, i.e. a "reference" which is a trimmed mean of all data from both transgenic and control plants grown under the same conditions and at the same developmental stage. The trimmed mean is calculated by eliminating a specific percentage, i.e. 20%, of the smallest and largest observation from the data set and then calculating the average of the remaining observation. Many transgenic plants comprising transgenic plant cells containing the recombinant DNA identified herein as imparting an enhanced trait will not exhibit an enhanced agronomic trait. The transgenic plants and seeds comprising the transgenic plant cells and having enhanced agronomic traits of this invention are identified by screening a population of transgenic plants and/or seeds for the members of the population having the enhanced trait. Screens for transgenic plant cells in crop plants are described more particularly in the examples below. In some cases, the trait enhancement can be measured quantitatively. For example, the trait enhancement can be at least a 2% desirable difference in an observed trait, at least a 5% desirable difference, at least about a 10% desirable difference, at least about a 20% desirable difference, at least about a 30% desirable difference, at least about a 50% desirable difference, at least about a 70% desirable difference, or at least about a 100% difference, or an even greater desirable difference. In other cases, the trait enhancement is measured qualitatively.

Many agronomic traits can affect "yield", including without limitation, plant height, pod number, pod position on the plant, number of internodes, incidence of pod shatter, grain size, efficiency of nodulation and nitrogen fixation, efficiency of nutrient assimilation, resistance to biotic and abiotic stress, carbon assimilation, plant architecture, resistance to lodging, percent seed germination, seedling vigor, and juvenile traits. Other traits that can affect yield include, efficiency of germination (including germination in stressed conditions), growth rate (including growth rate in stressed conditions), ear number, seed number per ear, seed size, composition of seed (starch, oil, protein) and characteristics of seed fill. Also of interest is the generation of transgenic plants that demonstrate desirable phenotypic properties that may or may not confer an increase in overall plant yield. Such properties include enhanced plant morphology, plant physiology or improved components of the mature seed harvested from the transgenic plant.

"Stress condition" refers to the condition unfavorable for a plant, which adversely affect plant metabolism, growth and/or development. A plant under the stress condition typically shows reduced germination rate, retarded growth and development, reduced photosynthesis rate, and eventually leading to reduction in yield. Specifically, "water deficit stress" used herein preferably refers to the sub-optimal conditions for water and humidity needed for normal growth of natural plants. Relative water content (RWC) can be used as a physiological measure of plant water deficit. It measures the effect of osmotic adjustment in plant water status, when a plant is under stressed conditions. Conditions which may result in water deficit stress include heat, drought, high salinity and PEG induced osmotic stress. "Cold stress" used herein preferably refers to the exposure of a plant to a temperatures below (two or more degrees Celsius below) those normal for a particular species or particular strain of plant. "Low nitrogen availability stress" used herein preferably refers to a plant growth condition with 50% of the conventional nitrogen inputs. "Shade stress" used herein preferably refers to limited light availability that triggers the shade avoidance response in plant. Plants are subject to shade stress when localized at lower part of the canopy, or in close proximity of neighboring vegetation. Shade stress may become exacerbated when the planting density exceeds the average prevailing density for a particular plant species. The average prevailing densities per acre of a few examples of crop plants in the USA in the year 2000 were: wheat 1,000,000-1; 500,000; rice 650,000-900,000; soybean 150,000-200,000, canola 260,000-350,000, sunflower 17,000-23,000 and cotton 28,000-55,000 plants per acre (Cheikh, et al., (2003) U.S. Patent Application No. .20030101479).

"Increased yield" of a transgenic plant of the present invention may be evidenced and measured in a number of ways, including test weight, seed number per plant, seed weight, seed number per unit area (i.e. seeds, or weight of seeds, per acre), bushels per acre, tons per acre, tons per acre, kilo per hectare. For example, maize yield may be measured as production of shelled corn kernels per unit of production area, e.g. in bushels per acre or metric tons per hectare, often reported on a moisture adjusted basis, e.g. at 15.5 % moisture. Increased yield may result from improved utilization of key biochemical compounds, such as nitrogen, phosphorous and carbohydrate, or from improved tolerance to environmental stresses, such as cold, heat, drought, salt, and attack by pests or pathogens. Trait-enhancing recombinant DNA may also be used to provide transgenic plants having improved growth and development, and ultimately increased yield, as the result of modified expression of plant growth regulators or modification of cell cycle or photosynthesis pathways.

"Expression" means transcription of DNA to produce RNA. The resulting RNA may be without limitation mRNA encoding a protein, antisense RNA that is complementary to an mRNA encoding a protein, or an RNA transcript comprising a combination of sense and antisense gene regions, such as for use in RNAi technology. Expression as used herein may also refer to production of encoded protein from mRNA.

"Promoter" means a region of DNA that is upstream from the start of transcription and is involved in recognition and binding of RNA polymerase and other proteins to initiate transcription. A "plant promoter" is a promoter capable of initiating transcription in plant cells whether or not its origin is a plant cell. Exemplary plant promoters include, but are not limited to, those that are obtained from plants, plant viruses, and bacteria which comprise genes expressed in plant cells such Agrobacterium or Rhizobium. Examples of promoters under developmental control include promoters that preferentially initiate transcription in certain tissues, such as leaves, roots, or seeds. Such promoters are referred to as "tissue preferred". Promoters which initiate transcription only in certain tissues are referred to as "tissue specific". A "cell type" specific promoter primarily drives expression in certain cell types in one or more organs, for example, vascular cells in roots or leaves. An "inducible" or "repressible" promoter is a promoter which is under environmental control. Examples of environmental conditions that may effect transcription by inducible promoters include anaerobic conditions, or certain chemicals, or the presence of light. Tissue specific, tissue preferred, cell type specific, and inducible promoters constitute the class of "non-constitutive" promoters. A "constitutive" promoter is a promoter which is active under most conditions. As used herein, "antisense orientation" includes reference to a polynucleotide sequence that is operably linked to a promoter in an orientation where the antisense strand is transcribed. The antisense strand is sufficiently complementary to an endogenous transcription product such that translation of the endogenous transcription product is often inhibited. "Operably linked" refers to the association of two or more nucleic acid fragments on a single nucleic acid fragment so that the function of one is affected by the other. For example, a promoter is operably linked with a coding sequence when it is capable of affecting the expression of that coding sequence (i.e., that the coding sequence is under the transcriptional control of the promoter). Coding sequences can be operably linked to regulatory sequences in sense or antisense orientation.

"Consensus amino acid sequence" means an artificial, amino acid sequence of conserved parts of the proteins encoded by homologous genes, e.g. as determined by a CLUSTALW alignment of amino acid sequence of homolog proteins or a group of proteins having identified by the gathering cutoff for a Pfam protein domain family.

Homologous genes are genes which encode homologous proteins with the same or similar biological function or having the same Pfam protein domain family. Homologous genes may be generated by the event of speciation (see ortholog) or by the event of genetic duplication (see paralog). "Orthologs" refer to a set of homologous genes in different species that evolved from a common ancestral gene by specification. Normally, orthologs retain the same function in the course of evolution; and "paralogs" refer to a set of homologous genes in the same species that have diverged from each other as a consequence of genetic duplication. Thus, homologous genes can be from the same or a different organism. As used herein, "homolog" means a protein that performs the same biological function as a second protein including those identified by sequence identity search.

"Percent identity" refers to the extent to which two optimally aligned DNA or protein segments are invariant throughout a window of alignment of components, e.g. nucleotide sequence or amino acid sequence. An "identity fraction" for aligned segments of a test sequence and a reference sequence is the number of identical components which are shared by sequences of the two aligned segments divided by the total number of sequence components in the reference segment over a window of alignment which is the smaller of the full test sequence or the full reference sequence. "Percent identity" ("% identity") is the identity fraction times 100. "% identity to a consensus amino acid sequence" is 100 times the identity fraction in a window of alignment of an amino acid sequence of a test protein optimally aligned to consensus amino acid sequence of this invention.

"Arabidopsis" means plants of Arabidopsis thaliana.

As used herein "Pfam" refers to a large collection of multiple sequence alignments and hidden Markov models covering many common protein families, e.g. Pfam version 18.0 (August 2005) contains alignments and models for 7973 protein families and is based on the Swissprot 47.0 and SP-TrEMBL 30.0 protein sequence databases. See S.R. Eddy, "Profile Hidden Markov Models", Bioinformatics 14:755-763, 1998. Pfam is currently maintained and updated by a Pfam Consortium. The alignments represent some evolutionary conserved structure that has implications for the protein's function. Profile hidden Markov models (profile HMMs) built from the Pfam alignments are useful for automatically recognizing that a new protein belongs to an existing protein family even if the homology by alignment appears to be low. Once one DNA is identified as encoding a protein which imparts an enhanced trait when expressed in transgenic plants, other DNA encoding proteins in the same protein family are identified by querying the amino acid sequence of protein encoded by candidate DNA against the Hidden Markov Model which characterizes the Pfam domain using HMMER software, a current version of which is provided in the appended computer listing. Candidate proteins meeting the gathering cutoff for the alignment of a particular Pfam are in the protein family and have cognate DNA that is useful in constructing recombinant DNA for the use in the plant cells of this invention. Hidden Markov Model databases for use with HMMER software in identifying DNA expressing protein in a common Pfam for recombinant DNA in the plant cells of this invention are also included in the appended computer listing. The HMMER software and Pfam databases are version 18.0 and were used to identify known domains in the proteins corresponding to amino acid sequence of SEQ ID NO:205 through SEQ ID NO:408. All DNA encoding proteins that have scores higher than the gathering cutoff disclosed in Table 27 by Pfam analysis disclosed herein can be used in recombinant DNA of the plant cells of this invention, e.g. for selecting transgenic plants having enhanced agronomic traits. The relevant Pfams for use in this invention, as more specifically disclosed below, are 2-oxoacid_dh, ADH_N, ADH_zinc_N, AP2, AUX_IAA, Aa_trans, Abhydrolase_1, Acyl_transf_1, Aldedh, Aldo_ket_red, Alpha-amylase, Aminotran_1_2, Aminotran_3, Ammonium_transp, Arm, Asn_synthase, BAG, BSD, Beta_elim_lyase, Biotin_lipoyl, Brix, Bromodomain, C1_4, CTP_transf_2, Catalase, CcmH, Chal_sti_synt_C, Cyclin_C, Cyclin_N, Cys_Met_Meta_PP, DAO, DIM1, DPBB_1, DRMBL, DUF167, DUF231, DUF250, DUF6, DUF783, DUF962, E2F_TDP, E3_binding, EBP, Enolase_C, Enolase_N, F420_oxidored, FAD_binding_2, FA_desaturase, FKBP_C, FTCD_N, Fe_bilin_red, Fer4, GAF, GATase_2, GIDA, GSHPx, Gpi16, HGTP_anticodon, HI0933_like, HLH, HMG_CoA_synt, HWE_HK, Hamlp_like, HhH-GPD, Homeobox, Hpt, Iso_dh, K-box, LEA_4, LRRNT_2, LRR_1, Ldh_1_C, Ldh_1_N, Lectin_legA, Lectin_legB, Lipase_GDSL, MFS_1, MIP, MatE, Metalloenzyme, Methyltransf_11, Methyltransf_12, Molybdop_Fe4S4, Molybdopterin, Molydop_binding, Mov34, MtN3_slv, Myb_DNA-binding, NAD_Gly3P_dh_N, NAD_binding_2, NIR_SIR, NIR_SIR_ferr, NPH3, NTP_transferase, Nuc_sug_transp, PA, PAR1, PFK, PGI, PGK, PGM_PMM_I, PGM_PMM_II, PGM_PMM_III, PGM_PMM_IV, PP2C, PTR2, Peptidase_C26, Phi_1, Phytochrome, Pkinase, Pkinase_Tyr, Pollen_allerg_1, Pribosyltran, Proteasome, Pyr_redox, Pyr_redox_2, Pyr_redox_dim, RNA_pol_L, RNA_pol_Rpb6, RRM_1, RRN3, Radical_SAM, Ras, Response_reg, Rhodanese, Ribosomal_S8e, Rieske, SAC3_GANP, SBDS, SET, SRF-TF, SURF5, Skp1, Skp1_POZ, Ssl1, Sterol_desat, Sugar_tr, TCP, ThiF, Transaldolase, UQ_con, Ubie_methyltran, WD40, WRKY, adh_short, bZIP_1, bZIP_2, cNMP_binding, iPGM_N, p450, tRNA-synt_2b, ubiquitin, zf-A20, zf-AN1, zf-B_box, zf-C2H2, zf-C3HC4, zf-CCCH, the databases for which are included in the appended computer program listing.

## Recombinant DNA constructs

This invention provides recombinant DNA constructs comprising one or more of the genes disclosed herein for imparting one or more enhanced traits to transgenic plants and seeds. Such constructs also typically comprise a promoter operatively linked to said polynucleotide to provide for expression in a target plant. Other construct components may include additional regulatory elements, such as 5' or 3' untranslated regions (such as polyadenylation sites), intron regions, and transit or signal peptides. Such recombinant DNA constructs can be assembled using methods known to those of ordinary skill in the art.

Recombinant constructs prepared in accordance with this invention generally includes a 3' untranslated DNA region (UTR) that typically contains a polyadenylation sequence following the polynucleotide coding region. Examples of useful 3' UTRs include those from the nopaline synthase gene of *Agrobacterium tumefaciens* (*nos*), a gene encoding the small subunit of a ribulose-1,5-bisphosphate carboxylase-oxygenase (rbcS), and the T7 transcript of *Agrobacterium tumefaciens* and those 3' UTR elements disclosed in the following examples. Constructs and vectors may also include a transit peptide for targeting of a gene target to a plant organelle, particularly to a chloroplast, leucoplast or other plastid organelle. For descriptions of the use of chloroplast transit peptides, see U.S. Patent 5, 188,642 and U.S. Patent No. 5,728,925, incorporated herein by reference.

Table 1 provides a list of genes that can be used in recombinant DNA for imparting an enhanced trait in the transgenic plant cells, plants and seeds of this invention. In screens of recombinant DNA expressed in a model plant the recombinant DNA was shown to be associated with enhanced traits. The cognate protein was used to identify homologs for constructing a consensus amino acid sequence for each cognate protein and for identifying the characterizing Pfams. With reference to Table 1:

"NUC SEQ ID" refers to a SEQ ID NO. for particular DNA sequence in the Sequence Listing;

"PEP SEQ ID" refers to a SEQ ID NO. in the Sequence Listing for the amino acid sequence of a protein cognate to a particular DNA;

"construct_id" refers to an arbitrary number used to identify a particular recombinant DNA construct comprising the particular DNA;

"gene" refers to an arbitrary name used to identify the particular DNA;

"orientation" refers to the orientation of the particular DNA in a recombinant DNA construct relative to the promoter; and

"species name" refers to the organism from which the particular DNA was derived.

Table 1

| NUC SEQ ID | PEP SEQ ID | construct id | Gene | orientation | Species Name |
|---|---|---|---|---|---|
| 1 | 205 | 12360 | CGPG1018 | SENSE | Arabidopsis thaliana |
| 2 | 206 | 12030 | CGPG1063 | SENSE | Arabidopsis thaliana |
| 3 | 207 | 15210 | CGPG 1124 | SENSE | Arabidopsis thaliana |
| 4 | 208 | 17465 | CGPG1178 | SENSE | Arabidopsis thaliana |
| 5 | 209 | 13222 | CGPG1259 | SENSE | Arabidopsis thaliana |
| 6 | 210 | 12919 | CGPG1290 | SENSE | Arabidopsis thaliana |
| 7 | 211 | 12927 | CGPG1300 | SENSE | Arabidopsis thaliana |
| 8 | 212 | 14841 | CGPG1572 | SENSE | Arabidopsis thaliana |
| 9 | 213 | 13478 | CGPG1616 | ANTI-SENSE | Arabidopsis thaliana |

(continued)

| NUC SEQ ID | PEP SEQ ID | construct id | Gene | orientation | Species Name |
|---|---|---|---|---|---|
| 10 | 214 | 17309 | CGPG1840 | SENSE | Arabidopsis thaliana |
| 11 | 215 | 19116 | CGPG1861 | ANTI-SENSE | Arabidopsis thaliana |
| 12 | 216 | 14837 | CGPG1882 | SENSE | Arabidopsis thaliana |
| 13 | 217 | 71253 | CGPG195 | SENSE | Arabidopsis thaliana |
| 14 | 218 | 16004 | CGPG2061 | ANTI-SENSE | Arabidopsis thaliana |
| 15 | 219 | 72712 | CGPG2256 | SENSE | Arabidopsis thaliana |
| 16 | 220 | 71546 | CGPG2305 | SENSE | Arabidopsis thaliana |
| 17 | 221 | 70109 | CGPG2368 | SENSE | Saccharomyces cerevisiae |
| 18 | 222 | 10335 | CGPG246 | ANTI-SENSE | Arabidopsis thaliana |
| 19 | 223 | 17919 | CGPG2780 | SENSE | Arabidopsis thaliana |
| 20 | 224 | 71148 | CGPG3225 | SENSE | Arabidopsis thaliana |
| 21 | 225 | 19647 | CGPG3229 | SENSE | Arabidopsis thaliana |
| 22 | 226 | 18844 | CGPG3371 | SENSE | Arabidopsis thaliana |
| 23 | 227 | 19525 | CGPG3548 | SENSE | Arabidopsis thaliana |
| 24 | 228 | 19617 | CGPG3573 | SENSE | Arabidopsis thaliana |
| 25 | 229 | 19750 | CGPG3913 | SENSE | Saccharomyces cerevisiae |
| 26 | 230 | 19964 | CGPG3922 | SENSE | Glycine max |
| 27 | 231 | 19814 | CGPG3934 | SENSE | Glycine max |
| 28 | 232 | 19720 | CGPG3954 | SENSE | Glycine max |
| 29 | 233 | 19845 | CGPG3956 | SENSE | Glycine max |
| 30 | 234 | 71072 | CGPG3963 | SENSE | Glycine max |
| 31 | 235 | 19949 | CGPG4010 | SENSE | Glycine max |
| 32 | 236 | 19902 | CGPG4016 | SENSE | Glycine max |
| 33 | 237 | 19981 | CGPG4019 | SENSE | Glycine max |
| 34 | 238 | 19757 | CGPG4037 | SENSE | Glycine max |
| 35 | 239 | 19850 | CGPG4056 | SENSE | Glycine max |
| 36 | 240 | 19942 | CGPG4115 | SENSE | Glycine max |
| 37 | 241 | 19787 | CGPG4124 | SENSE | Glycine max |
| 38 | 242 | 19801 | CGPG4137 | SENSE | Glycine max |
| 39 | 243 | 19705 | CGPG4156 | SENSE | Glycine max |
| 40 | 244 | 19737 | CGPG4175 | SENSE | Glycine max |
| 41 | 245 | 19812 | CGPG4184 | SENSE | Glycine max |
| 42 | 246 | 71556 | CGPG4298 | SENSE | Arabidopsis thaliana |
| 43 | 247 | 71330 | CGPG4457 | SENSE | Arabidopsis thaliana |
| 44 | 248 | 71332 | CGPG4460 | SENSE | Arabidopsis thaliana |
| 45 | 249 | 71571 | CGPG4493 | SENSE | Arabidopsis thaliana |
| 46 | 250 | 70677 | CGPG4558 | SENSE | Arabidopsis thaliana |
| 47 | 251 | 71689 | CGPG4661 | SENSE | Glycine max |

(continued)

| NUC SEQ ID | PEP SEQ ID | construct id | Gene | orientation | Species Name |
|---|---|---|---|---|---|
| 48 | 252 | 73685 | CGPG4807 | SENSE | Arabidopsis thaliana |
| 49 | 253 | 72636 | CGPG4858 | SENSE | Arabidopsis thaliana |
| 50 | 254 | 73651 | CGPG4869 | SENSE | Arabidopsis thaliana |
| 51 | 255 | 73342 | CGPG4876 | SENSE | Arabidopsis thaliana |
| 52 | 256 | 73271 | CGPG5011 | SENSE | Arabidopsis thaliana |
| 53 | 257 | 73282 | CGPG5071 | SENSE | Arabidopsis thaliana |
| 54 | 258 | 10903 | CGPG514 | ANTI-SENSE | Arabidopsis thaliana |
| 55 | 259 | 73913 | CGPG5337 | SENSE | Glycine max |
| 56 | 260 | 74305 | CGPG5400 | SENSE | Arabidopsis thaliana |
| 57 | 261 | 74306 | CGPG5402 | SENSE | Arabidopsis thaliana |
| 58 | 262 | 73769 | CGPG5430 | SENSE | Arabidopsis thaliana |
| 59 | 263 | 74237 | CGPG5448 | SENSE | Arabidopsis thaliana |
| 60 | 264 | 74244 | CGPG5466 | SENSE | Arabidopsis thaliana |
| 61 | 265 | 74256 | CGPG5491 | SENSE | Arabidopsis thaliana |
| 62 | 266 | 72714 | CGPG5513 | SENSE | Saccharomyces cerevisiae |
| 63 | 267 | 16014 | CGPG552 | SENSE | Arabidopsis thaliana |
| 64 | 268 | 72751 | CGPG5524 | SENSE | Saccharomyces cerevisiae |
| 65 | 269 | 10814 | CGPG554 | ANTI-SENSE | Arabidopsis thaliana |
| 66 | 270 | 72743 | CGPG5555 | SENSE | Saccharomyces cerevisiae |
| 67 | 271 | 72721 | CGPG5569 | SENSE | Saccharomyces cerevisiae |
| 68 | 272 | 72959 | CGPG5608 | SENSE | Glycine max |
| 69 | 273 | 72984 | CGPG5620 | SENSE | Arabidopsis thaliana |
| 70 | 274 | 73061 | CGPG5675 | SENSE | Rhodopseudomonas palustris |
| 71 | 275 | 10180 | CGPG57 | ANTI-SENSE | Arabidopsis thaliana |
| 72 | 276 | 73029 | CGPG5737 | SENSE | Saccharomyces cerevisiae |
| 73 | 277 | 11145 | CGPG574 | SENSE | Arabidopsis thaliana |
| 74 | 278 | 72920 | CGPG5763 | SENSE | Saccharomyces cerevisiae |
| 75 | 279 | 72957 | CGPG5788 | SENSE | Saccharomyces cerevisiae |
| 76 | 280 | 73044 | CGPG5808 | SENSE | Glycine max |
| 77 | 281 | 74323 | CGPG5873 | SENSE | Arabidopsis thaliana |
| 78 | 282 | 74327 | CGPG5901 | SENSE | Arabidopsis thaliana |
| 79 | 283 | 74329 | CGPG5903 | SENSE | Arabidopsis thaliana |
| 80 | 284 | 74340 | CGPG5907 | SENSE | Arabidopsis thaliana |
| 81 | 285 | 74341 | CGPG5911 | SENSE | Arabidopsis thaliana |
| 82 | 286 | 74345 | CGPG5932 | SENSE | Arabidopsis thaliana |
| 83 | 287 | 74616 | CGPG6017 | SENSE | Arabidopsis thaliana |
| 84 | 288 | 74604 | CGPG6019 | SENSE | Arabidopsis thaliana |
| 85 | 289 | 74608 | CGPG6043 | SENSE | Arabidopsis thaliana |

(continued)

| NUC SEQ ID | PEP SEQ ID | construct id | Gene | orientation | Species Name |
|---|---|---|---|---|---|
| 86 | 290 | 74609 | CGPG6044 | SENSE | Arabidopsis thaliana |
| 87 | 291 | 74366 | CGPG6073 | SENSE | Arabidopsis thaliana |
| 88 | 292 | 74383 | CGPG6098 | SENSE | Arabidopsis thaliana |
| 89 | 293 | 74374 | CGPG6100 | SENSE | Arabidopsis thaliana |
| 90 | 294 | 74618 | CGPG6117 | SENSE | Arabidopsis thaliana |
| 91 | 295 | 74619 | CGPG6118 | SENSE | Arabidopsis thaliana |
| 92 | 296 | 74622 | CGPG6124 | SENSE | Arabidopsis thaliana |
| 93 | 297 | 74628 | CGPG6131 | SENSE | Arabidopsis thaliana |
| 94 | 298 | 74631 | CGPG6139 | SENSE | Arabidopsis thaliana |
| 95 | 299 | 74669 | CGPG6140 | SENSE | Arabidopsis thaliana |
| 96 | 300 | 74670 | CGPG6145 | SENSE | Arabidopsis thaliana |
| 97 | 301 | 74647 | CGPG6163 | SENSE | Arabidopsis thaliana |
| 98 | 302 | 74385 | CGPG6310 | SENSE | Arabidopsis thaliana |
| 99 | 303 | 74386 | CGPG6330 | SENSE | Arabidopsis thaliana |
| 100 | 304 | 74387 | CGPG6331 | SENSE | Arabidopsis thaliana |
| 101 | 305 | 74685 | CGPG6362 | SENSE | Arabidopsis thaliana |
| 102 | 306 | 73487 | CGPG6386 | SENSE | Sinorhizobium meliloti |
| 103 | 307 | 73476 | CGPG6393 | SENSE | Bacillus subtilis subsp. subtilis str. 168 |
| 104 | 308 | 73465 | CGPG6400 | SENSE | Escherichia coli K12 |
| 105 | 309 | 73418 | CGPG6404 | SENSE | Nostoc punctiforme PCC 73102 |
| 106 | 310 | 73480 | CGPG6425 | SENSE | Sinorhizobium meliloti |
| 107 | 311 | 73482 | CGPG6438 | SENSE | Agrobacterium tumefaciens str. C58 |
| 108 | 312 | 73513 | CGPG6457 | SENSE | Bacillus subtilis subsp. subtilis str. 168 |
| 109 | 313 | 73550 | CGPG6468 | SENSE | Bacillus halodurans |
| 110 | 314 | 73527 | CGPG6474 | SENSE | Desulfitobacterium hafniense |
| 111 | 315 | 73516 | CGPG6481 | SENSE | Xenorhabdus nematophila |
| 112 | 316 | 73530 | CGPG6498 | SENSE | Escherichia coli K12 |
| 113 | 317 | 73534 | CGPG6527 | SENSE | Bacillus subtilis subsp. subtilis str. 168 |
| 114 | 318 | 74125 | CGPG6544 | SENSE | Pseudomonas fluorescens PfO-1 |
| 115 | 319 | 74161 | CGPG6547 | SENSE | Escherichia coli K12 |
| 116 | 320 | 74126 | CGPG6552 | SENSE | Escherichia coli K12 |
| 117 | 321 | 74115 | CGPG6559 | SENSE | Escherichia coli K12 |
| 118 | 322 | 74127 | CGPG6560 | SENSE | Escherichia coli K12 |
| 119 | 323 | 74128 | CGPG6568 | SENSE | Escherichia coli K12 |
| 120 | 324 | 74165 | CGPG6579 | SENSE | Escherichia coli K12 |
| 121 | 325 | 74106 | CGPG6582 | SENSE | Pseudomonas fluorescens PfO-1 |

(continued)

| NUC SEQ ID | PEP SEQ ID | construct id | Gene | orientation | Species Name |
|---|---|---|---|---|---|
| 122 | 326 | 74130 | CGPG6584 | SENSE | Pseudomonas syringae pv. tomato str. DC3000 |
| 123 | 327 | 74132 | CGPG6600 | SENSE | Xenorhabdus nematophila |
| 124 | 328 | 74144 | CGPG6601 | SENSE | Xenorhabdus nematophila |
| 125 | 329 | 74402 | CGPG6663 | SENSE | Synechocystis sp. |
| 126 | 330 | 74476 | CGPG6685 | SENSE | Bacillus halodurans |
| 127 | 331 | 74417 | CGPG6688 | SENSE | Bacillus halodurans |
| 128 | 332 | 74453 | CGPG6691 | SENSE | Bacillus subtilis subsp. subtilis str. 168 |
| 129 | 333 | 74418 | CGPG6696 | SENSE | Escherichia coli K12 |
| 130 | 334 | 10150 | CGPG67 | ANTI-SENSE | Arabidopsis thaliana |
| 131 | 335 | 74503 | CGPG6767 | SENSE | Escherichia coli K12 |
| 132 | 336 | 74504 | CGPG6775 | SENSE | Agrobacterium tumefaciens |
| 133 | 337 | 74528 | CGPG6777 | SENSE | Bacillus halodurans |
| 134 | 338 | 74588 | CGPG6782 | SENSE | Escherichia coli K12 |
| 135 | 339 | 74541 | CGPG6786 | SENSE | Escherichia coli K12 |
| 136 | 340 | 74553 | CGPG6787 | SENSE | Nostoc punctiforme PCC 73102 |
| 137 | 341 | 74554 | CGPG6795 | SENSE | Pseudomonas syringae pv.tomato str. DC3000 |
| 138 | 342 | 74578 | CGPG6797 | SENSE | Synechocystis sp. PCC 6803 |
| 139 | 343 | 74590 | CGPG6798 | SENSE | Xenorhabdus nematophila |
| 140 | 344 | 75834 | CGPG6820 | SENSE | Arabidopsis thaliana |
| 141 | 345 | 75835 | CGPG6822 | SENSE | Arabidopsis thaliana |
| 142 | 346 | 18020 | CGPG684 | SENSE | Arabidopsis thaliana |
| 143 | 347 | 75850 | CGPG6893 | SENSE | Arabidopsis thaliana |
| 144 | 348 | 75861 | CGPG6937 | SENSE | Arabidopsis thaliana |
| 145 | 349 | 75875 | CGPG6992 | SENSE | Arabidopsis thaliana |
| 146 | 350 | 74548 | CGPG712 | SENSE | Arabidopsis thaliana |
| 147 | 351 | 74880 | CGPG7357 | SENSE | Glycine max |
| 148 | 352 | 74903 | CGPG7407 | SENSE | Zea mays |
| 149 | 353 | 74915 | CGPG7408 | SENSE | Zea mays |
| 150 | 354 | 74927 | CGPG7409 | SENSE | Zea mays |
| 151 | 355 | 74951 | CGPG7411 | SENSE | Glycine max |
| 152 | 356 | 74940 | CGPG7418 | SENSE | Zea mays |
| 153 | 357 | 74977 | CGPG7429 | SENSE | Synechocystis sp. |
| 154 | 358 | 74954 | CGPG7435 | SENSE | Xanthomonas campestris |
| 155 | 359 | 74907 | CGPG7439 | SENSE | Synechocystis sp. |
| 156 | 360 | 74919 | CGPG7440 | SENSE | Synechocystis sp. |
| 157 | 361 | 11735 | CGPG745 | ANTI-SENSE | Arabidopsis thaliana |

(continued)

| NUC SEQ ID | PEP SEQ ID | construct id | Gene | orientation | Species Name |
|---|---|---|---|---|---|
| 158 | 362 | 74980 | CGPG7453 | SENSE | Synechocystis sp. |
| 159 | 363 | 74993 | CGPG7462 | SENSE | Bacillus halodurans |
| 160 | 364 | 75337 | CGPG7469 | SENSE | Saccharomyces cerevisiae |
| 161 | 365 | 75339 | CGPG7485 | SENSE | Zea mays |
| 162 | 366 | 75316 | CGPG7491 | SENSE | Glycine max |
| 163 | 367 | 75352 | CGPG7494 | SENSE | Zea mays |
| 164 | 368 | 12189 | CGPG752 | ANTI-SENSE | Arabidopsis thaliana |
| 165 | 369 | 75321 | CGPG7531 | SENSE | Zea mays |
| 166 | 370 | 75358 | CGPG7542 | SENSE | Zea mays |
| 167 | 371 | 75312 | CGPG7554 | SENSE | Zea mays |
| 168 | 372 | 75463 | CGPG7583 | SENSE | Zea mays |
| 169 | 373 | 75475 | CGPG7584 | SENSE | Zea mays |
| 170 | 374 | 75440 | CGPG7589 | SENSE | Glycine max |
| 171 | 375 | 75488 | CGPG7593 | SENSE | Glycine max |
| 172 | 376 | 75418 | CGPG7603 | SENSE | Glycine max |
| 173 | 377 | 75419 | CGPG7611 | SENSE | Zea mays |
| 174 | 378 | 75431 | CGPG7612 | SENSE | Glycine max |
| 175 | 379 | 75455 | CGPG7614 | SENSE | Glycine max |
| 176 | 380 | 75491 | CGPG7617 | SENSE | Zea mays |
| 177 | 381 | 75456 | CGPG7622 | SENSE | Glycine max |
| 178 | 382 | 75480 | CGPG7624 | SENSE | Glycine max |
| 179 | 383 | 75492 | CGPG7625 | SENSE | Zea mays |
| 180 | 384 | 75409 | CGPG7626 | SENSE | Zea mays |
| 181 | 385 | 75424 | CGPG7651 | SENSE | Zea mays |
| 182 | 386 | 75550 | CGPG7676 | SENSE | Glycine max |
| 183 | 387 | 75575 | CGPG7686 | SENSE | Glycine max |
| 184 | 388 | 75528 | CGPG7690 | SENSE | Glycine max |
| 185 | 389 | 75564 | CGPG7693 | SENSE | Glycine max |
| 186 | 390 | 75553 | CGPG7700 | SENSE | Glycine max |
| 187 | 391 | 75506 | CGPG7704 | SENSE | Glycine max |
| 188 | 392 | 75554 | CGPG7708 | SENSE | Glycine max |
| 189 | 393 | 75590 | CGPG7711 | SENSE | Glycine max |
| 190 | 394 | 75543 | CGPG7715 | SENSE | Glycine max |
| 191 | 395 | 75567 | CGPG7717 | SENSE | Glycine max |
| 192 | 396 | 75544 | CGPG7723 | SENSE | Glycine max |
| 193 | 397 | 75556 | CGPG7724 | SENSE | Glycine max |
| 194 | 398 | 75546 | CGPG7739 | SENSE | Glycine max |
| 195 | 399 | 75558 | CGPG7740 | SENSE | Glycine max |

(continued)

| NUC SEQ ID | PEP SEQ ID | construct id | Gene | orientation | Species Name |
|---|---|---|---|---|---|
| 196 | 400 | 75571 | CGPG7749 | SENSE | Glycine max |
| 197 | 401 | 75583 | CGPG7750 | SENSE | Glycine max |
| 198 | 402 | 75536 | CGPG7754 | SENSE | Zea mays |
| 199 | 403 | 75991 | CGPG8266 | SENSE | Chloroflexus aurantiacus |
| 200 | 404 | 75909 | CGPG8275 | SENSE | Chloroflexus aurantiacus |
| 201 | 405 | 12824 | CGPG885 | SENSE | Arabidopsis thaliana |
| 202 | 406 | 18026 | CGPG894 | SENSE | Arabidopsis thaliana |
| 203 | 407 | 11810 | CGPG899 | ANTI-SENSE | Arabidopsis thaliana |
| 204 | 408 | 72418 | CGPG968 | SENSE | Arabidopsis thaliana |

Recombinant DNA

Exemplary DNA for use in the present invention to improve traits in plants are provided herein as SEQ ID NO: through SEQ ID NO:204, as well as the homologs of such DNA molecules. A subset of the exemplary DNA includes fragments of the disclosed full polynucleotides consisting of oligonucleotides of at least 15, preferably at least 16 or 17, more preferably at least 18 or 19, and even more preferably at least 20 or more, consecutive nucleotides. Such oligonucleotides are fragments of the larger molecules having a sequence selected from the group consisting of SEQ ID NO: 1 through SEQ ID NO:204, and find use, for example as probes and primers for detection of the polynucleotides of the present invention.

Also of interest in the present invention are variants of the DNA provided herein. Such variants may be naturally occurring, including DNA from homologous genes from the same or a different species, or may be non-natural variants, for example DNA synthesized using chemical synthesis methods, or generated using recombinant DNA techniques. Degeneracy of the genetic code provides the possibility to substitute at least one base of the protein encoding sequence of a gene with a different base without causing the amino acid sequence of the polypeptide produced from the gene to be changed. Hence, a DNA useful in the present invention may have any base sequence that has been changed from the sequences provided herein by substitution in accordance with degeneracy of the genetic code.

Homologs of the genes providing DNA of demonstrated as useful in improving traits in model plants disclosed herein will generally demonstrate significant identity with the DNA provided herein. DNA is substantially identical to a reference DNA if, when the sequences of the polynucleotides are optimally aligned there is about 60% nucleotide equivalence; more preferably 70%; more preferably 80% equivalence; more preferably 85% equivalence; more preferably 90%; more preferably 95%; and/or more preferably 98% or 99% equivalence over a comparison window. A comparison window is preferably at least 50-100 nucleotides, and more preferably is the entire length of the polynucleotide provided herein. Optimal alignment of sequences for aligning a comparison window may be conducted by algorithms; preferably by computerized implementations of these algorithms (for example, the Wisconsin Genetics Software Package Release 7.0-10.0, Genetics Computer Group, 575 Science Dr., Madison, WI). The reference polynucleotide may be a full-length molecule or a portion of a longer molecule. Preferentially, the window of comparison for determining polynucleotide identity of protein encoding sequences is the entire coding region.

Proteins useful for imparting improved traits are entire proteins or at least a sufficient portion of the entire protein to impart the relevant biological activity of the protein. The term "protein" also includes molecules consisting of one or more polypeptide chains. Thus, a protein useful in the present invention may constitute an entire protein having the desired biological activity, or may constitute a portion of an oligomeric protein having multiple polypeptide chains. Proteins useful for generation of transgenic plants having improved traits include the proteins with an amino acid sequence provided herein as SEQ ID NO: 205 through SEQ ID NO: 408, as well as homologs of such proteins.

Homologs of the proteins useful in the present invention may be identified by comparison of the amino acid sequence of the protein to amino acid sequences of proteins from the same or different plant sources, e.g. manually or by using known homology-based search algorithms such as those commonly known and referred to as BLAST, FASTA, and Smith-Waterman. As used herein, a homolog is a protein from the same or a different organism that performs the same biological function as the polypeptide to which it is compared. An orthologous relation between two organisms is not necessarily manifest as a one-to-one correspondence between two genes, because a gene can be duplicated or deleted after organism phylogenetic separation, such as speciation. For a given protein, there may be no ortholog or more than one ortholog. Other complicating factors include alternatively spliced transcripts from the same gene, limited gene identification, redundant copies of the same gene with different sequence lengths or corrected sequence. A local sequence alignment program, e.g. BLAST, can be used to search a database of sequences to find similar sequences, and the summary Expectation value (E-value) used to measure the sequence base similarity. As a protein hit with the best E-value for a particular organism may not necessarily be an ortholog or the only ortholog, a reciprocal BLAST search is used in the present invention to filter hit sequences with significant E-values for ortholog identification. The reciprocal BLAST entails search of the significant hits against a database of amino acid sequences from the base organism that are similar to the sequence of the query protein. A hit is a likely ortholog, when the reciprocal BLAST's best hit is the query protein itself or a protein encoded by a duplicated gene after speciation. Thus, homolog is used herein to described proteins that are assumed to have functional similarity by inference from sequence base similarity. The relationship of homologs with amino acid sequences of SEQ ID NO:409 to 19247 to the proteins with amino acid sequences of SEQ ID NO:206 to 408 is found in the listing of Table 2.

A further aspect of the invention comprises functional homolog proteins which differ in one or more amino acids from those of a trait-improving protein disclosed herein as the result of one or more of the well-known conservative amino acid substitutions, e.g. valine is a conservative substitute for alanine and threonine is a conservative substitute for serine. Conservative substitutions for an amino acid within the native sequence can be selected from other members of a class to which the naturally occurring amino acid belongs. Representative amino acids within these various classes include, but are not limited to: (1) acidic (negatively charged) amino acids such as aspartic acid and glutamic acid; (2) basic (positively charged) amino acids such as arginine, histidine, and lysine; (3) neutral polar amino acids such as glycine, serine, threonine, cysteine, tyrosine, asparagine, and glutamine; and (4) neutral nonpolar (hydrophobic) amino acids such as alanine, leucine, isoleucine, valine, proline, phenylalanine, tryptophan, and methionine. Conserved substitutes for an amino acid within a native amino acid sequence can be selected from other members of the group to which the naturally occurring amino acid belongs. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulfur-containing side chains is cysteine and methionine. Naturally conservative amino acids substitution groups are: valine-leucine, valine-isoleucine, phenylalanine-tyrosine, lysine-arginine, alanine-valine, aspartic acid-glutamic acid, and asparagine-glutamine. A further aspect of the invention comprises proteins that differ in one or more amino acids from those of a described protein sequence as the result of deletion or insertion of one or more amino acids in a native sequence.

Homologs of the trait-improving proteins disclosed provided herein will generally demonstrate significant sequence identity. Of particular interest are proteins having at least 50% sequence identity, more preferably at least about 70% sequence identity or higher, e.g. at least about 80% sequence identity with an amino acid sequence of SEQ ID NO: 205 through SEQ ID NO: 408. Of course useful proteins also include those with higher identity, e.g. 90% to 99% identity. Identity of protein homologs is determined by optimally aligning the amino acid sequence of a putative protein homolog with a defined amino acid sequence and by calculating the percentage of identical and conservatively substituted amino acids over the window of comparison. The window of comparison for determining identity can be the entire amino acid sequence disclosed herein, e.g. the full sequence of any of SEQ ID NO:205 through SEQ ID NO:408.

Genes that are homologous to each other can be grouped into families and included in multiple sequence alignments. Then a consensus sequence for each group can be derived. This analysis enables the derivation of conserved and class- (family) specific residues or motifs that are functionally important. These conserved residues and motifs can be further validated with 3D protein structure if available. The consensus sequence can be used to define the full scope of the invention, e.g. to identify proteins with a homolog relationship. Thus, the present invention contemplates that protein homologs include proteins with an amino acid sequence that has at least 90% identity to such a consensus amino acid sequence sequences.

In particular embodiments, the inventors contemplate the use of antibodies, either monoclonal or polyclonal which bind to the proteins disclosed herein. Means for preparing and characterizing antibodies are well known in the art (See, e.g., Antibodies: A Laboratory Manual, Cold Spring Harbor Laboratory, 1988; incorporated herein by reference). The methods for generating monoclonal antibodies (mAbs) generally begin along the same lines as those for preparing polyclonal antibodies. Briefly, a polyclonal antibody is prepared by immunizing an animal with an immunogenic composition in accordance with the present invention and collecting antisera from that immunized animal. A wide range of animal species can be used for the production of antisera. Typically the animal used for production of anti-antisera is a rabbit, a mouse, a rat, a hamster, a guinea pig or a *goat.* Because of the relatively large blood volume of rabbits, a rabbit is a preferred choice for production of polyclonal antibodies.

mAbs may be readily prepared through use of well-known techniques, such as those exemplified in U.S. Pat. No. 4,196,265, incorporated herein by reference. Typically, this technique involves immunizing a suitable animal with a selected immunogen composition, e.g., a purified or partially purified antifungal protein, polypeptide or peptide. The immunizing composition is administered in a manner effective to stimulate antibody producing cells. Rodents such as mice and rats are preferred animals, however, the use of rabbit, sheep, or frog cells is also possible. The use of rats may provide certain advantages (Goding, 1986, pp. 60-61), but mice are preferred, with the BALB/c mouse being most preferred as this is most routinely used and generally gives a higher percentage of stable fusions.

Following immunization, somatic cells with the potential for producing antibodies, specifically B lymphocytes (B cells), are selected for use in the mAb generating protocol. The antibody-producing B lymphocytes from the immunized animal are then fused with cells of an immortal myeloma cell, generally one of the same species as the animal that was immunized to establish a population of hybridomas from which specific hybridomas are selected. The selected hybridomas would then be serially diluted and cloned into individual antibody-producing cell lines, which clones can then be propagated indefinitely to provide mAbs.

Promoters

Numerous promoters that are active in plant cells have been described in the literature. These include promoters present in plant genomes as well as promoters from other sources, including nopaline synthase (NOS) promoter and octopine synthase (OCS) promoters carried on tumor-inducing plasmids of *Agrobacterium tumefaciens,* caulimovirus promoters such as the cauliflower mosaic virus or figwort mosaic virus promoters. For instance, see U.S. Patents No. 5,858,742 and 5,322,938 which disclose versions of the constitutive promoter derived from cauliflower mosaic virus (CaMV35S), US Patent No. 5,378,619 which discloses a Figwort Mosaic Virus (FMV) 35S promoter, U.S. Patent 6,437,217 which discloses a maize RS81 promoter, U.S. Patent 5,641,876 which discloses a rice actin promoter, U.S. Patent 6,426,446 which discloses a maize RS324 promoter, U.S. Patent 6,429,362 which discloses a maize PR-1 promoter, U.S. Patent 6,232,526 which discloses a maize A3 promoter, U.S. Patent 6,177,611 which discloses constitutive maize promoters, U.S. Patent 6,433,252 which discloses a maize L3 oleosin promoter, U.S. Patent 6,429,357 which discloses a rice actin 2 promoter and intron, U.S. Patent 5,837,848 which discloses a root specific promoter, U.S. Patent 6,084,089 which discloses cold inducible promoters, U.S. Patent 6,294,714 which discloses light inducible promoters, U.S. Patent 6,140,078 which discloses salt inducible promoters, U.S. Patent 6,252,138 which discloses pathogen inducible promoters, U.S. Patent 6,175,060 which discloses phosphorus deficiency inducible promoters, U.S. Patent Application Publication 2002/0192813A1 which discloses 5', 3' and intron elements useful in the design of effective plant expression vectors, U.S. patent application Serial No. 09/078,972 which discloses a coixin promoter, U.S. patent application Serial No. 09/757,089 which discloses a maize chloroplast aldolase promoter, and U.S. patent application Serial No. 10/739,565 which discloses water-deficit inducible promoters, all of which are incorporated herein by reference. These and numerous other promoters that function in plant cells are known to those skilled in the art and available for use in recombinant polynucleotides of the present invention to provide for expression of desired genes in transgenic plant cells.

Furthermore, the promoters may be altered to contain multiple "enhancer sequences" to assist in elevating gene expression. Such enhancers are known in the art. By including an enhancer sequence with such constructs, the expression of the selected protein may be enhanced. These enhancers often are found 5' to the start of transcription in a promoter that functions in eukaryotic cells, but can often be inserted in the forward or reverse orientation 5' or 3' to the coding sequence. In some instances, these 5' enhancing elements are introns. Deemed to be particularly useful as enhancers are the 5' introns of the rice actin 1 and rice actin 2 genes. Examples of other enhancers that can be used in accordance with the invention include elements from the CaMV 35S promoter, octopine synthase genes, the maize alcohol dehydrogenase gene, the maize shrunken 1 gene and promoters from non-plant eukaryotes.

In some aspects of the invention it is preferred that the promoter element in the DNA construct be capable of causing sufficient expression to result in the production of an effective amount of a polypeptide in water deficit conditions. Such promoters can be identified and isolated from the regulatory region of plant genes that are over expressed in water deficit conditions. Specific water-deficit-inducible promoters for use in this invention are derived from the 5' regulatory region of genes identified as a heat shock protein 17.5 gene (*HSP17.5*), an HVA22 gene (*HVA22*), a Rab 17 gene and a cinnamic acid 4-hydroxylase (CA4H) gene (*CA4H*) of *Zea maize.* Such water-deficit-inducible promoters are disclosed in U.S. application Serial No.10/739,565, incorporated herein by reference.

In other aspects of the invention, sufficient expression in plant seed tissues is desired to effect improvements in seed composition. Exemplary promoters for use for seed composition modification include promoters from seed genes such as napin (U.S. Patent 5,420,034), maize L3 oleosin (U.S. Patent 6,433,252), zein Z27 (Russell et al. (1997) Transgenic Res. 6(2):157-166), globulin 1 (Belanger et al (1991) Genetics 129:863-872), glutelin 1 (Russell (1997) *supra*), and peroxiredoxin antioxidant (Per1) (Stacy et al. (1996) Plant Mol Biol. 31(6):1205-1216).

In still other aspects of the invention, preferential expression in plant green tissues is desired. Promoters of interest for such uses include those from genes such as SSU (Fischhoff et al. (1992) Plant Mol Biol. 20:81-93), aldolase and pyruvate orthophosphate dikinase (PPDK) (Taniguchi et al. (2000) Plant Cell Physiol. 41(1):42-48).

Gene overexpression

"Gene overexpression" used herein in reference to a polynucleotide or polypeptide indicates that the expression level of a target protein, in a transgenic plant or in a host cell of the transgenic plant, exceeds levels of expression in a non-transgenic plant. In a preferred embodiment of the present invention, a recombinant DNA construct comprises the polynucleotide of interest in the sense orientation relative to the promoter to achieve gene overexpression, which is identified as such in Table 1.

Gene suppression

Gene suppression includes any of the well-known methods for suppressing transcription of a gene or the accumulation of the mRNA corresponding to that gene thereby preventing translation of the transcript into protein. Posttranscriptional gene suppression is mediated by transcription of integrated recombinant DNA to form double-stranded RNA (dsRNA) having homology to a gene targeted for suppression. This formation of dsRNA most commonly results from transcription of an integrated inverted repeat of the target gene, and is a common feature of gene suppression methods known as anti-sense suppression, co-suppression, RNA interference (RNAi) and knockout, e.g. by mutagenesis. Transcriptional suppression can be mediated by a transcribed dsRNA having homology to a promoter DNA sequence to effect what is called promoter *trans* suppression.

More particularly, posttranscriptional gene suppression by inserting a recombinant DNA construct with anti-sense oriented DNA to regulate gene expression in plant cells is disclosed in U.S. Patent 5,107,065 (Shewmaker et al.) and US Patent 5,759,829 (Shewmaker et al.*). Transgenic plants transformed using such anti-sense oriented DNA constructs for gene suppression can comprise integrated DNA arranged as an inverted repeats that result from insertion of the DNA construct into plants by *Agrobacterium*-mediated transformation, as disclosed by Redenbaugh *et al.* in "Safety Assessment of Genetically Engineered Flavr Savr™ Tomato, CRC Press, Inc. (1992). Inverted repeat insertions can comprises a part or all of the T-DNA construct, e.g. an inverted repeat of a complete transcription unit or an invetred repeat of transcription terminator sequence. Screening for inserted DNA comprising inverted repeat elements can improve the efficiency of identifying transformation events effective for gene silencing whether the transformation construct is a simple anti-sense DNA construct which must be inserted in multiple copies or a complex inverted repeat DNA construct (e.g. an RNAi construct) which can be inserted as a single copy.

Posttranscriptional gene suppression by inserting a recombinant DNA construct with sense-oriented DNA to regulate gene expression in plants is disclosed in U.S. Patent 5,283,184 (Jorgensen et al.) and U.S. Patent 5,231,020 (Jorgensen et al.). Inserted T-DNA providing gene suppression in plants transformed with such sense constructs by *Agrobacterium* is organized predominately in inverted repeat structures, as disclosed by Jorgensen et al., Mol. Gen. Genet., 207:471-477 (1987). See also Stam et al., The Plant Journal" 12(1), 63-82 (1997) who used segregation studies to support Jorgensen's finding that gene silencing is mediated by multimeric transgene T-DNA loci in which the T-DNAs are arranged in inverted repeats. Screening for inserted DNA comprising inverted repeat elements can improve the gene silencing efficiency when transforming with simple sense-orientated DNA constructs. Gene silencing efficiency can also be improved by screening for single insertion events when transforming with an RNAi construct containing inverted repeat elements

As disclosed by Redenbaugh *et al.* gene suppression can be achieved by inserting into a plant genome recombinant DNA that transcribes dsRNA. Such a DNA insert can be transcribed to an RNA element having the 3' region as a double stranded RNA. RNAi constructs are also disclosed in EP 0426195 A1 (Goldbach *et al.* - 1991) where recombinant DNA constructs for transcription into hairpin dsRNA for providing transgenic plants with resistance to tobacco spotted wilt virus. Double-stranded RNAs were also disclosed in WO 94/01550 (Agrawal et al.) where anti-sense RNA was stabilized with a self-complementary 3' segment. Agrawal *et al.* referred to U.S. Patent 5,107,065 for using such self-stablized anti-sense RNAs for regulating gene expression in plant cells; see International Publication No. 94/01550. Other double-stranded hairpin-forming elements in transcribed RNA are disclosed in International Publication No. 98/05770 (Werner *et al.*) where the anti-sense RNA is stabilized by hairpin forming repeats of poly(CG) nucleotides. See also U.S. Patent Application Publication No. 2003/0175965 A1 (Lowe *et al.*) which discloses gene suppression using and RNAi construct comprising a gene coding sequence preceded by inverted repeats of 5'UTR. See also U.S. Patent Application Publication No. 2002/0048814 A1 (Oeller) where RNAi constructs are transcribed to sense or anti-sense RNA which is stabilized by a poly(T)-poly(A) tail. See also U.S. Patent Application Publication No. 2003/0018993 A1 (Gutterson *et al.)* where sense or anti-sense RNA is stabilized by an inverted repeat of a of the 3' untranslated region of the NOS gene. See also U.S. Patent Application Publication No. 2003/0036197 A1 (Glassman *et al.*) where RNA having homology to a target is stabilized by two complementary RNA regions.

Gene silencing can also be effected by transcribing RNA from both a sense and an anti-sense oriented DNA, e.g. as disclosed by Shewmaker *et al.* in U.S. Patent 5,107,065 where in Example 1 a binary vector was prepared with both sense and anti-sense *aroA* genes. See also U.S. Patent 6,326,193 where gene targeted DNA is operably linked to opposing promoters.

Gene silencing can also be affected by transcribing from contiguous sense and anti-sense DNA. In this regard see Sijen et al., The Plant Cell, Vol. 8, 2277-2294 (1996) discloses the use of constructs carrying inverted repeats of a cowpea mosaic virus gene in transgenic plants to mediate virus resistance. Such constructs for posttranscriptional gene suppression in plants by double-stranded RNA are also disclosed in International Publication No. WO 99/53050 (Waterhouse et al.), International Publication No. WO 99/49029 (Graham et al.), U.S. Patent Application No.10/465,800 (Fillatti), U.S. Patent 6,506,559 (Fire et al.). See also U.S. Application Serial No. 10/393,347 (Shewmaker et al.) that discloses constructs and methods for simultaneously expressing one or more recombinant genes while simultaneously suppressing one or more native genes in a transgenic plant. See also U.S. Patent 6,448,473 (Mitsky et al.) that discloses multi-gene suppression vectors for use in plants. All of the above-described patents, applications and international publications disclosing materials and methods for posttranscriptional gene suppression in plants are incorporated herein by reference. Transcriptional suppression such as promoter *trans* suppression can be affected by a expressing a DNA construct comprising a promoter operably linked to inverted repeats of promoter DNA for a target gene. Constructs useful for such gene suppression mediated by promoter *trans* suppression are disclosed by Mette et al., The EMBO Journal, Vol. 18, No. 1, pp. 241-148, 1999 and by Mette et al., The EMBO Journal, Vol. 19, No. 19, pp. 5194-5201-148, 2000, both of which are incorporated herein by reference.

Suppression can also be achieved by insertion mutations created by transposable elements may also prevent gene function. For example, in many dicot plants, transformation with the T-DNA of *Agrobacterium* may be readily achieved and large numbers of transformants can be rapidly obtained. Also, some species have lines with active transposable elements that can efficiently be used for the generation of large numbers of insertion mutations, while some other species lack such options. Mutant plants produced by *Agrobacterium* or transposon mutagenesis and having altered expression of a polypeptide of interest can be identified using the polynucleotides of the present invention. For example, a large population of mutated plants may be screened with polynucleotides encoding the polypeptide of interest to detect mutated plants having an insertion in the gene encoding the polypeptide of interest.

Gene stacking

The present invention also contemplates that the trait-improving recombinant DNA provided herein can be used in combination with other recombinant DNA to create plants with a multiple desired traits. The combinations generated can include multiple copies of any one or more of the recombinant DNA constructs. These stacked combinations can be created by any method, including but not limited to cross breeding of transgenic plants, or multiple genetic transformation.

**Plant Cell Transformation Methods**

Numerous methods for transforming plant cells with recombinant DNA are known in the art and may be used in the present invention. Two commonly used methods for plant transformation are *Agrobacterium*-mediated transformation and microprojectile bombardment. Microprojectile bombardment methods are illustrated in U.S. Patents 5,015,580 (soybean); 5,550,318 (corn); 5,538,880 (corn); 5,914,451 (soybean); 6,160,208 (corn); 6,399,861 (corn) and 6,153,812 (wheat) and *Agrobacterium*-mediated transformation is described in U.S. Patents 5,159,135 (cotton); 5,824,877 (soybean); 5,591,616 (corn); and 6,384,301 (soybean), all of which are incorporated herein by reference. For *Agrobacterium tumefaciens* based plant transformation system, additional elements present on transformation constructs will include T-DNA left and right border sequences to facilitate incorporation of the recombinant polynucleotide into the plant genome.

In general it is useful to introduce recombinant DNA randomly, i.e. at a non-specific location, in the genome of a target plant line. In special cases it may be useful to target recombinant DNA insertion in order to achieve site-specific integration, for example to replace an existing gene in the genome, to use an existing promoter in the plant genome, or to insert a recombinant polynucleotide at a predetermined site known to be active for gene expression. Several site specific recombination systems exist which are known to function implants include cre-lox as disclosed in U.S. Patent 4,959,317 and FLP-FRT as disclosed in U.S. Patent 5,527,695, both incorporated herein by reference.

Transformation methods of this invention are preferably practiced in tissue culture on media and in a controlled environment. "Media" refers to the numerous nutrient mixtures that are used to grow cells *in vitro,* that is, outside of the intact living organism. Recipient cell targets include, but are not limited to, meristem cells, callus, immature embryos and gametic cells such as microspores, pollen, sperm and egg cells. It is contemplated that any cell from which a fertile plant may be regenerated is useful as a recipient cell. Callus may be initiated from tissue sources including, but not limited to, immature embryos, seedling apical meristems, microspores and the like. Cells capable of proliferating as callus are also recipient cells for genetic transformation. Practical transformation methods and materials for making transgenic plants of this invention, for example various media and recipient target cells, transformation of immature embryo cells and subsequent regeneration of fertile transgenic plants are disclosed in U.S. Patents 6,194,636 and 6,232,526, which are incorporated herein by reference.

The seeds of transgenic plants can be harvested from fertile transgenic plants and be used to grow progeny generations of transformed plants of this invention including hybrid plants line for selection of plants having an enhanced trait. In addition to direct transformation of a plant with a recombinant DNA, transgenic plants can be prepared by crossing a first plant having a recombinant DNA with a second plant lacking the DNA. For example, recombinant DNA can be introduced into first plant line that is amenable to transformation to produce a transgenic plant which can be crossed with a second plant line to introgress the recombinant DNA into the second plant line. A transgenic plant with recombinant DNA providing an enhanced trait, e.g. enhanced yield, can be crossed with transgenic plant line having other recombinant DNA that confers another trait, for example herbicide resistance or pest resistance, to produce progeny plants having recombinant DNA that confers both traits. Typically, in such breeding for combining traits the transgenic plant donating the additional trait is a male line and the transgenic plant carrying the base traits is the female line. The progeny of this cross will segregate such that some of the plants will carry the DNA for both parental traits and some will carry DNA for one parental trait; such plants can be identified by markers associated with parental recombinant DNA, e.g. marker identification by analysis for recombinant DNA or, in the case where a selectable marker is linked to the recombinant, by application of the selecting agent such as a herbicide for use with a herbicide tolerance marker, or by selection for the enhanced trait. Progeny plants carrying DNA for both parental traits can be crossed back into the female parent line multiple times, for example usually 6 to 8 generations, to produce a progeny plant with substantially the same genotype as one original transgenic parental line but for the recombinant DNA of the other transgenic parental line

In the practice of transformation DNA is typically introduced into only a small percentage of target plant cells in any one transformation experiment. Marker genes are used to provide an efficient system for identification of those cells that are stably transformed by receiving and integrating a transgenic DNA construct into their genomes. Preferred marker genes provide selective markers which confer resistance to a selective agent, such as an antibiotic or herbicide. Any of the herbicides to which plants of this invention may be resistant are useful agents for selective markers. Potentially transformed cells are exposed to the selective agent. In the population of surviving cells will be those cells where, generally, the resistance-confering gene is integrated and expressed at sufficient levels to permit cell survival. Cells may be tested further to confirm stable integration of the exogenous DNA. Commonly used selective marker genes include those conferring resistance to antibiotics such as kanamycin and paromomycin (*nptII*), hygromycin B (*aph IV*) and gentamycin (*aac3* and *aac*C4) or resistance to herbicides such as glufosinate (*bar* or *pat*) and glyphosate (*aroA* or EPSPS). Examples of such selectable are illustrated in U.S. Patents 5,550,318; 5,633,435; 5,780,708 and 6,118,047, all of which are incorporated herein by reference. Selectable markers which provide an ability to visually identify transformants can also be employed, for example, a gene expressing a colored or fluorescent protein such as a luciferase or green fluorescent protein (GFP) or a gene expressing a *beta*-glucuronidase or *uid*A gene (GUS) for which various chromogenic substrates are known.

Plant cells that survive exposure to the selective agent, or plant cells that have been scored positive in a screening assay, may be cultured in regeneration media and allowed to mature into plants. Developing plantlets regenerated from transformed plant cells can be transferred to plant growth mix, and hardened off, for example, in an environmentally controlled chamber at about 85% relative humidity, 600 ppm $CO_2$, and 25-250 microeinsteins $m^2$ $s^{-1}$ of light, prior to transfer to a greenhouse or growth chamber for maturation. Plants are regenerated from about 6 weeks to 10 months after a transformant is identified, depending on the initial tissue. Plants may be pollinated using conventional plant breeding methods known to those of skill in the art and seed produced, for example self-pollination is commonly used with transgenic corn. The regenerated transformed plant or its progeny seed or plants can be tested for expression of the recombinant DNA and selected for the presence of enhanced agronomic trait.

**Transgenic Plants and Seeds**

Transgenic plants derived from the plant cells of this invention are grown to generate transgenic plants having an enhanced trait as compared to a control plant and produce transgenic seed and haploid pollen of this invention. Such plants with enhanced traits are identified by selection of transformed plants or progeny seed for the enhanced trait. For efficiency a selection method is designed to evaluate multiple transgenic plants (events) comprising the recombinant DNA , for example multiple plants from 2 to 20 or more transgenic events. Transgenic plants grown from transgenic seed provided herein demonstrate improved agronomic traits that contribute to increased yield or other trait that provides increased plant value, including, for example, improved seed quality. Of particular interest are plants having enhanced water use efficiency, enhanced cold tolerance, increased yield, enhanced nitrogen use efficiency, enhanced seed protein and enhanced seed oil.

**Discovery of Trait-improving Recombinant DNA**

To identify recombinant DNA that imparts an enhanced trait to plants, *Arabidopsis* ce;;s were transformed with a candidate recombinant DNA construct and screened for an improved trait. A two-step screening process was employed which comprised two passes of trait characterization to ensure that the trait modification was dependent on expression of the recombinant DNA, but not due to the chromosomal location of the integration of the transgene. Twelve independent transgenic lines for each recombinant DNA construct were established and assayed for the transgene expression levels. Five transgenic lines with high transgene expression levels were used in the first pass screen to evaluate the transgene's function in T2 transgenic plants. Subsequently, three transgenic events, which had been shown to have one or more improved traits, were further evaluated in the second pass screen to confirm the transgene's ability to impart an improved trait. The following Table 3 summarizes the improved traits that have been confirmed as provided by a recombinant DNA construct.

In particular, Table3 reports "PEP SEQ ID" which is the amino acid sequence of the protein cognate to the DNA in the recombinant DNA construct corresponding to a protein sequence of a SEQ ID NO. in the Sequence Listing.

"annotation" refers to a description of the top hit protein obtained from an amino acid sequence query of each PEP SEQ ID NO to GenBank database of the National Center for Biotechnology Information (ncbi). More particularly, "gi" is the GenBank ID number for the top BLAST hit. The components of "annotation" are "e-value" which provides the expectation value for the BLAST hit; "% id" which refers to the percentage of identically matched amino acid residues along the length of the portion of the sequences which is aligned by BLAST between the sequence of interest provided herein and the hit sequence in GenBank; "GenBank ID" which provides a reference number for the top BLAST hit in GenBank; and " description" which refers to the description of that top BLAST hit.

"traits" identify by two letter codes the confirmed improvement in a transgenic plant provided by the recombinant DNA. The codes for improved traits are:

"CK" which indicates cold tolerance improvement identified under a cold shock tolerance screen;
"CS" which indicates cold tolerance improvement identified by a cold germination tolerance screen;
"DS" which indicates drought tolerance improvement identified by a soil drought stress tolerance screen;
"PEG" which indicates osmotic stress tolerance improvement identified by a PEG induced osmotic stress tolerance screen;
"HS" which indicates heat stress tolerance improvement identified by a heat stress tolerance screen;
"SS" which indicates high salinity stress tolerance improvement identified by a salt stress tolerance screen;
"LN" which indicates nitrogen use efficiency improvement identified by a limited nitrogen tolerance screen;
"LL" which indicates attenuated shade avoidance response identified by a shade tolerance screen under a low light condition;
"PP" which indicates improved growth and development at early stages identified by an early plant growth and development screen;
"SP" which indicates improved growth and development at late stages identified by a late plant growth and development screen provided herein.

Table 3

| PEP SEQ ID | annotation | | | | Traits | | | | | |
|---|---|---|---|---|---|---|---|---|---|---|
| | e-value | % id | GenBank ID | description | | | | | | |
| 205 | 5.00E-34 | 85 | gi|21281159| | gb|AAD48981.1| contains similarity to Solanum lycopersicum (tomato) wound induced protein | HS | | | | | |
| 206 | 1.00E-66 | 76 | gi|21553397| | gb|AAM62490.1|putative zinc finger protein | LN | | | | | |
| 207 | 1.00E-118 | 93 | gi|7573441| | ref|NP_191871.11| cyclin family protein | HS | | | | | |
| 208 | 0 | 93 | gi|22136838| | ref|NP_566299.1| GPI transamidase component Gpi16 subunit family protein | CK | CS | | | | |
| 209 | 1.00E-171 | 97 | gi|15028251| | ref|NP_566244.1| transmembrane protein, putative | CK | | | | | |
| 210 | 2.00E-57 | 98 | gi|37202020| | emb|CAB81279.1| putative protein | HS | PP | PEG | | | |
| 211 | 8.00E-37 | 100 | gi|26451972| | dbj|BAC43077.1|unknown protein | PP | SS | HS | | | |
| 212 | 0 | 86 | gi|42562765| | ref|NP_175971.3|transcription factor-related | CK | | | | | |
| 213 | 0 | 100 | gi|21280989| | gb|AAM44902.1|putative catalase | PP | SP | | | | |
| 214 | 0 | 89 | gi|22087278| | gb|AAC97995.1| Similar to gb|Z30094 basic transcripion factor 2, 44 kD subunit from Homo sapiens. | LN | PP | HS | | | |
| 215 | 0 | 77 | gi|12324313| | gb|AAD55662.1| Highly similar to non intermediate filament IFA binding protein | DS | | | | | |
| 216 | 0 | 100 | gi|21537362| | gb|AAM61703.1|protein kinase-like protein | CK | | | | | |
| 217 | 0 | 87 | gi|4678332| | emb|CAB41143.1|putative peptide transporter | CK | | | | | |
| 218 | 1.00E-144 | 86 | gi|14532890| | gb|AAK64127.1|unknown protein | PEG | | | | | |
| 219 | 1.00E-145 | 95 | gi|3242721| | gb|AAC23773.1|putative acetone-cyanohydrin lyase | DS | | | | | |
| 220 | 0 | 84 | gi|15293165| | gb|AAK93693.1|putative 3-methyladenine DNA glycosylase | CK | PEG | CS | HS | | PP |
| 221 | 1.00E-178 | 79 | gi|6321581| | ref|NP_011658.1|Btn2p [Saccharomyces cerevisiae] | PP | | | | | |
| 222 | 0 | 98 | gi|30387605| | ref|NP_178499.2| MATE efflux family protein | LN | | | | | |
| 223 | 1.00E-147 | 90 | gi|9758099| | ref|NP_198887.1| zinc finger (C2H2 type) family protein | CS | | | | | |
| 224 | 1.00E-139 | 95 | gi|23505833| | gb|AAN28776.1|At3g51780/O RF3 | CK | | | | | |

| PEP SEQ ID | annotation | | | | Traits | | | | | |
| --- | --- | --- | --- | --- | --- | --- | --- | --- | --- | --- |
| | e-value | % id | GenBank ID | description | | | | | | |
| 225 | 0 | 100 | gi\|6729028\| | gb\|AAF27024.1\|putative nodulin [Arabidopsis thaliana] ref\|NP_187169.1\| GDSL-motif lipase/hydrolase family protein | CS | | | | | |
| 226 | 0 | 100 | gi\|21436143\| | gb\|AAM51318.1\|unknown protein | CS | | | | | |
| 227 | 0 | 97 | gi\|31711910\| | gb\|AAM64944.1\| betaine aldehyde dehydrogenase, putative | PP | | | | | |
| 228 | 1.00E-128 | 80 | gi\|21554268\| | ref\|NP_171616.1\|33 kDa ribonucleoprotein, chloroplast, putative / RNA-binding protein cp33, putative | PEG | SS | | | | |
| 229 | 0 | 95 | gi\|6321563\| | emb\|CAA58159.1\| glutamic-dependent asparagine synthase | CK | CS | SS | | | |
| 230 | 1.00E-132 | 57 | gi\|25403213\| | pir\|\|A86468probable zinc finger protein | PP | PEG | | | | |
| 231 | 2.00E-54 | 52 | gi\|20127115\| | gb\|AAM10965.1\| putative bHLH transcription factor [Arabidopsis thaliana] | CK | | | | | |
| 232 | 1.00E-160 | 72 | gi\|18401370\| | ref\|NP_566566.1\|protein phosphatase 2C family protein | CK | HS | PEG | CS | | |
| 233 | 1.00E-33 | 45 | gi\|21593875\| | gb\|AAM65842.1\|putative RING-H2 zinc finger protein | PP | HS | | | | |
| 234 | 2.00E-34 | 51 | gi\|55419648\| | gb\|AAV51937.1\|AP2/EREBP transcription factor ERF-2 | CK | | | | | |
| 235 | 2.00E-73 | 59 | gi\|3955021\| | emb\|CAA09367.1\|HB2 homeodomain protein | CK | CS | | | | |
| 236 | 1.00E-51 | 58 | gi\|4689366\| | gb\|AAD27870.1\|BRH1 RING finger protein | HS | PP | PEG | | | |
| 237 | 1.00E-149 | 76 | gi\|52354283\| | gb\|AAM14913.1\| putative malonyl-CoA:Acyl carrier protein transacylase | HS | | | | | |
| 238 | 1.00E-154 | 85 | gi\|20260680\| | gb\|AAM13238.1\|putative NADPH-dependent mannose 6-phosphate reductase | PP | PEG | | | | |
| 239 | 2.00E-89 | 64 | gi\|21593394\| | ref\|NP_567300.1\| short-chain dehydrogenase/ reductase (SDR) family protein | HS | | | | | |
| 240 | 0 | 82 | gi\|42795470\| | gb\|AAS46245.1\|HMG-CoA synthase 2 | CK | HS | SS | | | |
| 241 | 1.00E-113 | 83 | gi\|20385588\| | gb\|AAM21344.1\|MADS-box protein 4 | SP | | | | | |
| 242 | 1.00E-135 | 89 | gi\|21280889\| | ref\|NP_196254.1\| ribosomal protein S8e family protein | SS | HS | | | | |
| 243 | 5.00E-28 | 43 | gi\|4567308\| | gb\|AAD23719.1\|putative RING zinc finger protein | PEG | | | | | |
| 244 | 3.00E-35 | 33 | gi\|25352200\| | pir\|\|T52379zinc finger protein ZPT3-3 | PEG | | | | | |

(continued)

| | annotation | | | | Traits | | | | | |
|---|---|---|---|---|---|---|---|---|---|---|
| PEP SEQ ID | e-value | % id | GenBank ID | description | | | | | | |
| 245 | 0 | 83 | gi|10800918| | emb|CAC12995.1|putative AUX1-like permease | CK | PEG | CS | | | |
| 246 | 3.00E-61 | 87 | gi|21554008| | gb|AAM63089.1|cold-regulated protein cor15b precursor | CS | HS | PP | | | |
| 247 | 0 | 100 | gi|25406415| | pir||D96781cytochrome P450, probable, 64213-66051 | PEG | | | | | |
| 248 | 0 | 92 | gi|3885330| | gb|AAC77858.1|putative cytochrome P450 | PP | | | | | |
| 249 | 1.00E-159 | 100 | gi|21593400| | gb|AAM65367.1|phi-1-like protein | LN | LL | | | | |
| 250 | 1.00E-89 | 76 | gi|45825151| | ref|NP_200258.2| zinc finger (B-box type) family protein | DS | | | | | |
| 251 | 1.00E-144 | 72 | gi|3643085| | gb|AAC36698.1|protein phosphatase-2C; PP2C | PP | CK | | | | |
| 252 | 2.00E-90 | 91 | gi|21537084| | gb|AAM61425.1|unknown | CK | PEG | | | | |
| 253 | 2.00E-51 | 100 | gi|28393947| | ref|NP_174092.1| glycine-rich protein | HS | PP | | | | |
| 254 | 0 | 91 | gi|23197704| | ref|NP_565909.1| radical SAM domain-containing protein | PEG | | | | | |
| 255 | 0 | 93 | gi|4902476| | emb|CAB43520.1|MAP kinase | CK | CS | | | | |
| 256 | 1.00E-100 | 72 | gi|34146806| | gb|AAC34217.1| putative alcohol dehydrogenase | CS | | | | | |
| 257 | 2.00E-87 | 82 | gi|3548814| | gb|AAC34486.1|E3 ubiquitin ligase SCF complex subunit SKP1/ASK1 (At19), | CK | LL | CS | | | |
| 258 | 1.00E-113 | 81 | gi|7939553| | dbj|BAA95756.1|expansin-like protein | PP | HS | | | | |
| 259 | 1.00E-163 | 52 | gi|13506810| | gb|AAK28345.1|receptor-like protein kinase 3 | CK | PP | | | | |
| 260 | 1.00E-163 | 68 | gi|12597803| | gb|AAG60115.1|hypothetical protein | PP | | | | | |
| 261 | 1.00E-161 | 100 | gi|20258881| | gb|AAM14112.1|putative ubiquinone/menaquinone biosynthesis methyltransferase | LL | | | | | |
| 262 | 1.00E-133 | 92 | gi|12597757| | ref|NP_176849.1| nodulin MtN3 family protein | SS | PEG | | | | |
| 263 | 0 | 95 | gi|6911875| | sp|P53780|METC_ARATH Cystathionine beta-lyase, chloroplast precursor (CBL) (Beta-cystathionase) (Cysteine lyase) | PP | SS | | | | |
| 264 | 1.00E-100 | 100 | gi|21389647| | gb|AAM48022.1|photoassimila te-responsive protein PAR-1b-like protein | SP | DS | | | | |
| 265 | 0 | 100 | gi|25402889| | ref|NP_173376.1| very-long-chain fatty acid condensing enzyme, putative | PEG | PP | | | | |

(continued)

| PEP SEQ ID | annotation | | | | Traits | | | | | |
| --- | --- | --- | --- | --- | --- | --- | --- | --- | --- | --- |
| | e-value | % id | GenBank ID | description | | | | | | |
| 266 | 0 | 88 | gi|6324999| | sp|P20438|CG12_YEAST G1/S-specific cyclin CLN2 gb|AAA65725.1| cyclin 2 | DS | | | | | |
| 267 | 0 | 100 | gi|3218550| | dbj|BAA28775.1|Cdk-activating kinase 1At | LL | | | | | |
| 268 | 1.00E-169 | 100 | gi|6321880| | ref|NP_011956.1|Nucleolar protein involved in the assembly of the large ribosomal subunit; contains a sigma(70)-like motif, which is thouqht to bind RNA | SP | | | | | |
| 269 | 0 | 89 | gi|21436315| | gb|AAM51327.1|putative histidyl-tRNA synthetase | CK | HS | CS | | | |
| 270 | 1.00E-150 | 100 | gi|14318575| | ref|NP_116708.1|20S proteasome beta-type subunit | PP | PEG | SS | | | |
| 271 | 0 | 99 | gi|6325396| | pir||S69027 ammonium transport protein MEP3 | LL | DS | | | | |
| 272 | 5.00E-76 | 72 | gi|7442240| | sp|O24543|AX2E_PHAAU Auxin-induced protein 22E (Indole-3-acetic acid induced protein ARG14) | DS | | | | | |
| 273 | 0 | 94 | gi|9759247| | dbj|BAB09771.1|serine/threoni ne protein kinase-like protein | LL | | | | | |
| 274 | 0 | 97 | gi|39647867| | emb|CAE26387.1|phosphogly cerate kinase | CK | DS | CS | | | |
| 275 | 0 | 89 | gi|9758468| | dbj|BAB08997.1|monosacchar ide transporter | PP | PEG | | | | |
| 276 | 1.00E-66 | 80 | gi|6319966| | ref|NP_010046.1|Phosphorela y intermediate protein, phosphorylated by the plasma membrane sensor Sln1p in response to osmotic stress and then in turn phosphorylates the response regulators Ssk1p in the cytosol and Skn7p in the nucleus | HS | | | | | |
| 277 | 2.00E-88 | 83 | gi|21536637| | ref|NP_565524.1| stress enhanced protein 2 (SEP2) | DS | LN | | | | |
| 278 | 0 | 90 | gi|6322724| | ref|NP_012797.1|Required for transcription of rDNA by RNA Polymerase I; DNA-independent RNA Polymerase I transcription factor | SS | | | | | |
| 279 | 0 | 94 | gi|6320705| | pir||S69555 myo-inositol transport protein ITR1 - yeast) | HS | SS | PP | | | |
| 280 | 5.00E-24 | 49 | gi|25453551| | pir||T52011ethylene responsive element binding factor 3 | LL | | | | | |
| 281 | 1.00E-150 | 100 | gi|4580468| | gb|AAD24392.1|putative cAMP-dependent protein kinase | PP | | | | | |
| 282 | 1.00E-169 | 91 | gi|21436057| | ref|NP_193037.1| oxidoreductase, zinc-binding dehydrogenase family protein | LL | SS | | | | |

(continued)

| PEP SEQ ID | annotation | | | | Traits | | | | | |
| --- | --- | --- | --- | --- | --- | --- | --- | --- | --- | --- |
| | e-value | % id | GenBank ID | description | | | | | | |
| 283 | 1.00E-165 | 83 | gi|21280925| | gb|AAM44967.1|putative cinnamyl alcohol dehydrogenase | HS | | | | | |
| 284 | 0 | 100 | gi|20334800| | ref|NP_568453.1| alcohol dehydrogenase, putative [Arabidopsis thaliana] | PEG | | | | | |
| 285 | 0 | 100 | gi|22136298| | gb|AAM91227.1|alcohol dehydrogenase | PP | | | | | |
| 286 | 1.00E-144 | 89 | gi|20259173| | sp|O04202|IF35_ARATH Eukaryotic translation initiation factor 3 subunit 5 (eIF-3 epsilon) (eIF3 p32 subunit) (eIF3f) | CS | SS | CK | | | |
| 287 | 6.00E-74 | 96 | gi|21593170| | ref|NP_196239.1| RNA-binding protein, putative | PP | | | | | |
| 288 | 1.00E-112 | 80 | gi|9759521| | dbj|BAB10987.1|nuclear cap-binding protein; CBP20 | PP | | | | | |
| 289 | 1.00E-177 | 95 | gi|12083276| | gb|AAG48797.1|putative delta 9 desaturase | CS | PEG | | | | |
| 290 | 1.00E-111 | 94 | gi|12083264| | gb|AAG48791.1|putative GTP-binding protein RAB11 D | LN | SS | PP | | | |
| 291 | 1.00E-134 | 91 | gi|23505935| | gb|AAP86673.1| 26S proteasome subunit RPN12 | HS | SS | | | | |
| 292 | 3.00E-53 | 86 | gi|26452894| | dbj|BAC43525.1|putative DNA-directed RNA polymerase 14 kDa subunit AtRPAC14 | CK | HS | PP | SS | PEG | CS |
| 293 | 5.00E-94 | 100 | gi|21554412| | gb|AAM63517.1|probable glutathione peroxidase At2g31570 | CK | CS | PEG | | PP | |
| 294 | 1.00E-116 | 92 | gi|6143884| | ref|NP_187617.1| immunophilin, putative / FKBP-type peptidyl-prolyl cis-trans isomerase, putative | SP | CK | | | | |
| 295 | 1.00E-114 | 100 | gi|6671929| | gb|AAF23189.1|putative GTP-binding protein (ATFP8) [Arabidopsis thaliana] | PP | | | | | |
| 296 | 1.00E-161 | 92 | gi|7670024| | ref|NP_566563.1| ubiquitin-conjugating enzyme, putative | HS | PP | SS | | | |
| 297 | 1.00E-132 | 95 | gi|21554045| | gb|AAM63126.1|20S proteasome subunit PAC1 | SS | | | | | |
| 298 | 1.00E-111 | 94 | gi|21593047| | gb|AAM64996.1|GTP-binding protein Rab11 | PP | | | | | |
| 299 | 0 | 93 | gi|23308437| | ref|NP_190336.1| malate dehydrogenase [NAD], chloroplast (MDH) | SS | HS | | | | |
| 300 | 3.00E-94 | 80 | gi|6562282| | emb|CAB62652.1|rac-like GTP binding protein Arac11 | PEG | | | | | |
| 301 | 0 | 100 | gi|21554607| | gb|AAM63631.1|ubiquitin activating enzyme-like protein | SS | | | | | |
| 302 | 5.00E-26 | 81 | gi|15217910| | ref|NP_173453.1|homeobox-leucine zipper protein-related | PP | | | | | |
| 303 | 1.00E-129 | 86 | gi|23505995| | ref|NP_177122.2| acid phosphatase, putative [Arabidopsis thaliana] | SP | PP | | | | |

(continued)

| PEP SEQ ID | annotation | | | | Traits | | | | | |
|---|---|---|---|---|---|---|---|---|---|---|
| | e-value | % id | GenBank ID | description | | | | | | |
| 304 | 1.00E-157 | 100 | gi\|28827628\| | gb\|AAO50658.1\|putative C-4 sterol methyloxidase | PP | | | | | |
| 305 | 9.00E-19 | 100 | gi\|21592539\| | ref\|NP_565794.1\| hydroxyproline-rich glycoprotein family protein [Arabidopsis thaliana] | CS | SS | CK | | | |
| 306 | 0 | 97 | gi\|15965196\| | ref\|NP_385549.1\|PROBABLE ENOLASE PROTEIN | SP | LL | | | | |
| 307 | 1.00E-153 | 100 | gi\|16080137\| | pir\|\|A69990 UTP-glucose-1-phosphate uridylyltransferase homolog ytdA | LL | | | | | |
| 308 | 1.00E-147 | 98 | gi\|26246388\| | ref\|NP_752427.1\|Pyrroline-5-carboxylate reductase | LN | | | | | |
| 309 | 1.00E-135 | 94 | gi\|23126946\| | ref\|ZP_00108826.1\|COG0345 : Pyrroline-5-carboxylate reductase | PP | | | | | |
| 310 | 0 | 100 | gi\|16263079\| | ref\|NP_435872.1\|probable alcohol | PEG | | | | | |
| 311 | 0 | 99 | gi\|15888903\| | pir\|\|H97551 probable aminotransferase aatc | LL | | | | | |
| 312 | 0 | 90 | gi\|16080158\| | ref\|NP_390984.1\|glycine betaine aldehyde dehydrogenase | CS | CK | | | | |
| 313 | 1.00E-141 | 94 | gi\|15614866\| | ref\|NP_243169.1\|UTP-glucose-1-phosphate uridylyltransferas | PP | | | | | |
| 314 | 0 | 99 | gi\|23111329\| | ref\|ZP_00097007.1\|COG0205 : 6-phosphofructokinase | CS | PP | CK | | | |
| 315 | 1.00E-150 | 87 | gi\|37526393\| | ref\|NP_929737.1\|UTP--glucose-1-phosphate uridylyltransferase (UDP-glucose pyrophosphorylase) (UDPGP) | PEG | SS | | | | |
| 316 | 0 | 99 | gi\|16128895\| | ref\|NP_415448.1\|aspartate aminotransferase | PP | | | | | |
| 317 | 0 | 97 | gi\|16080149\| | ref\|NP_390975.1\|glucose-1-phosphate adenylyltransferase | DS | | | | | |
| 318 | 0 | 96 | gi\|48732455\| | ref\|ZP_00266198.1\|COG1012 : NAD-dependent aldehyde dehydrogenases | HS | PP | | | | |
| 319 | 0 | 97 | gi\|16129263\| | ref\|NP_415818.1\|4-aminobutyrate aminotransferase | SS | PP | | | | |
| 320 | 0 | 99 | gi\|16128583\| | ref\|NP_415133.1\|putative PLP-dependent aminotransferase | PP | | | | | |
| 321 | 0 | 99 | gi\|30063716\| | ref\|NP_837887.1\|putative aminotransferase | CS | CK | | | | |
| 322 | 0 | 99 | gi\|16130084\| | ref\|NP_416651.1\|bifunctional: putative glutamate synthase (N-terminal); putative oxidoreductase (C-terminal) | SS | PP | | | | |
| 323 | 0 | 94 | gi\|16128664\| | ref\|NP_415214.1\|phosphogluc omutase | SP | PP | | | | |

(continued)

| PEP SEQ ID | annotation | | | | Traits | | | | | |
|---|---|---|---|---|---|---|---|---|---|---|
| | e-value | % id | GenBank ID | description | | | | | | |
| 324 | 0 | 96 | gi\|49176307\| | ref\|NP_417544.3\|probable ornithine aminotransferase [Escherichia coli K12] (EC 2.6.1.13) | CS | CK | | | | |
| 325 | 1.00E-175 | 80 | gi\|48729503\| | ref\|ZP_00263253.1\|COG0508 : Pyruvate/2-oxoglutarate dehydrogenase complex, dihydrolipoamide acyltransferase (E2) component, and related enzymes | CK | PP | | | | |
| 326 | 1.00E-173 | 80 | gi\|28869402\| | ref\|NP_792021.1\|2-oxoglutarate dehydrogenase, E2 component, dihydrolipoamide succinyltransferase | CK | PP | | | | |
| 327 | 1.00E-161 | 90 | gi\|37524574\| | ref\|NP_927918.1\|Transaldolas eB | PP | | | | | |
| 328 | 0 | 82 | gi\|37525385\| | ref\|NP_928729.1\|Dihydrolipoa mide succinyltransferase component of 2-oxoglutarate dehydrogenase complex (E2) | LN | PP | | | | |
| 329 | 3.00E-55 | 86 | gi\|16332334\| | ref\|NP_443062.1\|hypothetical protein slr0607 | CS | PP | HS | | | |
| 330 | 0 | 100 | gi\|15614388\| | ref\|NP_242691.1\|acetoin dehydrogenase E3 component | LL | | | | | |
| 331 | 0 | 99 | gi\|15615327\| | ref\|NP_243630.1\|dihydrolipoa mide dehydrogenase | LN | | | | | |
| 332 | 0 | 97 | gi\|16078525\| | ref\|NP_389344.1\|dihydrolipoa mide dehydrogenase E3 subunit of both pyruvate dehydrogenase and 2-oxoglutarate dehydrogenase complexes | HS | DS | | | | |
| 333 | 0 | 94 | gi\|15800431\| | ref\|NP_286443.1\|2-oxoglutarate dehydrogenase | CS | CK | | | | |
| 334 | 0 | 89 | gi\|22136798\| | gb\|AAM91743.1\|putative phosphate/phosphoenolpyruv ate translocator precursor protein | LN | | | | | |
| 335 | 0 | 100 | gi\|49176098\| | ref\|NP_415825.3\|putative polysaccharide hydrolase | HS | SS | | | | |
| 336 | 0 | 96 | gi\|15889444\| | ref\|NP_355125.1\|AGR_C_392 7p [Agrobacterium tumefaciens str. C58] ref\|NP_532838.1\| bacteriophytochrome protein | CK | HS | PP | PEG | CS | SS |
| 337 | 0 | 95 | gi\|15613173\| | ref\|NP_241476.1\|sulfite reductase (NADPH) | PP | CK | CS | PEG | | |
| 338 | 0 | 100 | gi\|30062749\| | ref\|NP_836920.1\|nitrate reductase 1, beta subunit | LN | | | | | |
| 339 | 0 | 100 | gi\|15804618\| | ref\|NP_290659.1\|glucosephos phate isomerase | PP | CS | | | | |
| 340 | 0 | 100 | gi\|23125493\| | ref\|ZP_00107424.1\|COG0243 : Anaerobic dehydrogenases, typically selenocysteine-containing | PP | PEG | HS | | | |

| PEP SEQ ID | annotation | | | | Traits | | | | | |
| --- | --- | --- | --- | --- | --- | --- | --- | --- | --- | --- |
| | e-value | % id | GenBank ID | description | | | | | | |
| 341 | 0 | 98 | gi|28868179| | ref|NP_790798.1|glucose-6-phosphate isomerase | CK | PP | SP | PEG | CS | |
| 342 | 0 | 96 | gi|6329427| | ref|NP_440155.1|isocitrate dehydrogenase (NADP+) | CK | CS | | | | |
| 343 | 0 | 85 | gi|37524705| | ref|NP_928049.1|sulfite reductase [NADPH] hemoprotein beta-component (SIR-HP) | DS | PP | PEG | | | |
| 344 | 1.00E-121 | 95 | gi|6728966| | gb|AAF26964.1|unknown protein | LN | | | | | |
| 345 | 0 | 96 | gi|6714417| | gb|AAF26105.1|unknown protein | LN | | | | | |
| 346 | 0 | 78 | gi|22136866| | ref|NP_177343.2|protease-associated zinc finger (C3HC4-type RING finger) family protein | CS | | | | | |
| 347 | 0 | 92 | gi|51971567| | ref|NP_850943.1| glutamine amidotransferase-related | CK | HS | CS | | | |
| 348 | 2.00E-65 | 88 | gi|26450572| | dbj|BAC42398.1|unknown protein [Arabidopsis thaliana] | CK | CS | | | | |
| 349 | 1.00E-115 | 100 | gi|6730712| | gb|AAF27107.1|Unknown protein | CK | CS | | | | |
| 350 | 0 | 100 | gi|20465757| | gb|AAM20367.1|putative cyclin protein | PP | | | | | |
| 351 | 8.00E-90 | 56 | gi|23297314| | ref|NP_849559.1|WRKY family transcription factor [Arabidopsis thaliana] | CK | CS | | | | |
| 352 | 1.00E-171 | 83 | gi|50940357| | ref|XP_479706.1|putative Shwachman-Bodian-Diamond syndrome protein | PP | HS | SS | | | |
| 353 | 2.00E-46 | 92 | gi|50929801| | ref|XP_474428.1|OSJNBa007 0M12.6 | CK | PP | | | | |
| 354 | 7.00E-54 | 92 | gi|5042333| | emb|CAB44664.1|BETL4 protein | PP | | | | | |
| 355 | 1.00E-56 | 34 | gi|42563228| | ref|NP_565108.2|zinc finger (CCCH-type) family protein | HS | SS | PEG | | | |
| 356 | 4.00E-61 | 72 | gi|51970440| | dbj|BAD43912.1|hypothetical protein | CK | PP | | | | |
| 357 | 7.00E-91 | 99 | gi|16331395| | ref|NP_442123.1|hypothetical protein slr0013 | SS | | | | | |
| 358 | 0 | 99 | gi|21229841| | ref|NP_635758.1|vanillate O-demethylase oxygenase subunit | CS | CK | | | | |
| 359 | 1.00E-142 | 100 | gi|16331855| | sp|Q55891|PCYA_SYNY3 Phycocyanobilin:ferredoxin oxidoreductase | CK | PP | CS | | | |
| 360 | 0 | 100 | gi|16331872| | ref|NP_442600.1|hypothetical protein slr0304 | SP | CK | | | | |

(continued)

| PEP SEQ ID | annotation | | | | Traits | | | | | |
| --- | --- | --- | --- | --- | --- | --- | --- | --- | --- | --- |
| | e-value | % id | GenBank ID | description | | | | | | |
| 361 | 3.00E-86 | 87 | gi\|21593344\| | gb\|AAM65293.1\|putative cold-regulated protein ref\|NP_178469.1\|late embryogenesis abundant domain-containing protein / LEA domain-containing protein | LN | | | | | |
| 362 | 8.00E-91 | 100 | gi\|16330328\| | sp\|P73690\|Y51L_SYNY3 Ycf51-like protein dbj\|BAA17736.1\| ORF_ID:sII1702~hypothetical protein | HS | | | | | |
| 363 | 5.00E-87 | 100 | gi\|15612647\| | ref\|NP_240950.1\|hypoxanthin e-guanine phosphoribosyltransferase | PEG | | SS | | | |
| 364 | 0 | 83 | gi\|6323679\| | sp\|P23748\|MPIP_YEAST M-phase inducer phosphatase (Mitosis initiation protein MIH1) (Mitotic inducer homolog) | CS | LL | PP | CK | HS | SS |
| 365 | 2.00E-33 | 90 | gi\|34898476\| | ref\|NP_910584.1\|EST AU082567(S21715) corresponds to a region of the predicted gene.~Similar to S.tuberosum ubiquinol cytochrome c reductase. (X79275) | PP | | | | | |
| 366 | 6.00E-54 | 81 | gi\|21592528\| | gb\|AAM64477.1\|ring-box protein-like | HS | SS | | | | |
| 367 | 0 | 76 | gi\|34910110\| | dbj\|BAB92553.1\| DNA cross-link repair 1B-like protein | CK | HS | | | | |
| 368 | 1.00E-156 | 100 | gi\|21436267\| | gb\|AAM51272.1\|putative nodulin-26 protein | LN | | | | | |
| 369 | 1.00E-100 | 81 | gi\|50900588\| | ref\|XP_462727.1\|putative phenylalkylamine binding protein sp\|Q9FTZ2\|EBP_ORYSA Probable 3-beta-hydroxysteroid-delta(8), delta(7)-isomerase (Cholestenol delta-isomerase) (Delta8-delta7 sterol isomerase) (D8-D7 sterol isomerase) dbj\|BAB92148.1\| putative C-8,7 sterol isomerase | LN | | | | | |
| 370 | 4.00E-39 | 46 | gi\|25361093\| | pir\|\|T00967hypothetical protein At2g26340 | HS | | | | | |
| 371 | 2.00E-78 | 89 | gi\|50906887\| | ref\|XP_464932.1\|cytochrome c bioqenesis protein-like | LL | PP | | | | |
| 372 | 1.00E-22 | 98 | gi\|50899510\| | ref\|XP_450543.1\|unknown protein | CK | LL | CS | PP | SS | |
| 373 | 5.00E-79 | 85 | gi\|50934647\| | ref\|XP_476851.1\|bifunctional phosphopantetheine adenylyl transferase dephospho CoA kinase-like protein | CK | CS | | | | |
| 374 | 8.00E-56 | 50 | gi\|38257027\| | dbj\|BAD01556.1\|ERF-like protein | PP | | | | | |
| 375 | 1.00E-64 | 55 | gi\|18423944\| | ref\|NP_568850.1\|basic helix-loop-helix (bHLH) family protein | PP | | | | | |
| 376 | 1.00E-39 | 42 | gi\|42567912\| | ref\|NP_568344.2\|myb family transcription factor | SP | PEG | | | | |

| PEP SEQ ID | annotation | | | | Traits | | | | | |
|---|---|---|---|---|---|---|---|---|---|---|
| | e-value | % id | GenBank ID | description | | | | | | |
| 377 | 6.00E-89 | 84 | qi\|37535020\| | re\|NP_921812.1\|putative HAM-1-like protein | LN | | | | | |
| 378 | 2.00E-49 | 59 | gi\|27804371\| | gb\|AA022987.1 \|MADS-box transcription factor CDM104 | LL | SS | | | | |
| 379 | 2.00E-68 | 60 | gi\|22137112\| | emb\|CAB72174.1\| responce reactor 4 [Arabidopsis thaliana] | CK | SS | | | | |
| 380 | 0 | 89 | gi\|50910245\| | ref\|XP_466611.1\|putative PLRR-4 polymorphic leucine-rich repeat protein | CS | CK | | | | |
| 381 | 2.00E-30 | 44 | gi\|17933450\| | gb\|AAK70215.1\|MADS-box protein | CS | CK | PEG | | | |
| 382 | 1.00E-93 | 54 | gi\|20502508\| | dbj\|BAB91414.1\|E2F-like repressor E2L3 | CK | PEG | CS | | | |
| 383 | 2.00E-38 | 58 | gi\|50909627\| | ref\|XP_466302.1\|unknown protein | PEG | | | | | |
| 384 | 3.00E-41 | 63 | gi\|50399946\| | gb\|AAT76334.1\|putative DNA-directed RNA polymerase II subunit | HS | | | | | |
| 385 | 4.00E-59 | 68 | gi\|37535924\| | ref\|NP_922264.1\|unknown protein | LN | | | | | |
| 386 | 8.00E-57 | 58 | gi\|50944571\| | ref\|XP_481813.1\|transfactor-like | HS | | | | | |
| 387 | 3.00E-73 | 42 | gi\|53792319\| | dbj\|BAD53026.1\|putative ring finger protein 1 | LN | | | | | |
| 388 | 9.00E-93 | 47 | gi\|25054862\| | ref\|NP_850517.1\| transcription factor, putative / zinc finger (C3HC4 type RING finger) family protein | LN | | | | | |
| 389 | 7.00E-91 | 66 | gi\|20269059\| | emb\|CAC84710.1\|aux/IAA protein | PEG | | | | | |
| 390 | 2.00E-72 | 45 | gi\|26450026\| | ref\|NP_172358.1\| myb family transcription factor (MYB60) | LL | LN | | | | |
| 391 | 1.00E-80 | 46 | gi\|50946213\| | ref\|XP_482634.1\|AP2/EREBP transcription factor-like protein | LN | | | | | |
| 392 | 8.00E-54 | 43 | gi\|29824137\| | ref\|NP_189337.1\| TCP family transcription factor, putative [Arabidopsis thaliana] | CK | CS | | | | |
| 393 | 0 | 99 | gi\|558543\| | emb\|CAA85320.1 \|C-termina\| zinc-finger | HS | PP | PEG | | | |
| 394 | 1.00E-124 | 62 | gi\|20259301\| | ref\|NP_566010.1\| SET domain-containing protein (ASHH3) | PP | SS | | | | |
| 395 | 4.00E-54 | 65 | gi\|21553740\| | gb\|AAM62833.1\|putative zinc finger protein | PP | PEG | CS | | | |
| 396 | 2.00E-54 | 40 | gi\|20465561\| | ref\|NP_974448.1\| zinc finger (C3HC4-type RING finger) family protein | LL | LN | | | | |
| 397 | 1.00E-112 | 65 | gi\|51557078\| | gb\|AAU06309.1\|MYB transcription factor | PP | LN | | | | |

| PEP SEQ ID | annotation | | | | Traits | | | | | |
|---|---|---|---|---|---|---|---|---|---|---|
| | e-value | % id | GenBank ID | description | | | | | | |
| 398 | 0 | 82 | gi\|15148926\| | gb\|AAK84890.1\|TGA-type basic leucine zipper protein TGA2.2 | LN | | | | | |
| 399 | 6.00E-78 | 47 | gi\|28558782\| | gb\|AA045753.1\|RING/C3HC4 /PHD zinc finger-like protein | LN | | | | | |
| 400 | 0 | 67 | gi\|42565068\| | ref\|NP_188743.3\|transducin family protein / WD-40 repeat family protein | HS | | | | | |
| 401 | 7.00E-29 | 46 | gi\|38638682\| | ref\|NP_177307.1\| AP2 domain-containing transcription factor, putative | CS | LL | LN | | | |
| 402 | 0 | 84 | gi\|50904461\| | ref\|XP_463719.1\|P0466H10.2 7 | PEG | | | | | |
| 403 | 0 | 88 | gi\|53796982\| | ref\|ZP_00357872.1\|COG0160 : 4-aminobutyrate aminotransferase and related aminotransferases | SP | CS | | | | |
| 404 | 0 | 94 | gi\|53796007\| | ref\|ZP_00357032.1\|COG0696 : Phosphoglyceromutase | PP | SS | PEG | HS | | |
| 405 | 0 | 93 | gi\|30697938\| | ref\|NP_201207.2\|expressed protein | DS | HS | | | | |
| 406 | 0 | 100 | gi\|13878095\| | gb\|AAK44125.1\|unknown protein | DS | CS | | | | |
| 407 | 0 | 97 | gi\|12324320\| | gb\|AAG52129.1\|hypothetical protein; 63994-65574 | LL | | | | | |
| 408 | 0 | 84 | gi\|22136086\| | gb\|AAM91121.1\|photorecepto r-interacting protein-like | CS | PP | | | | |

**Trait Improvement Screens**

DS- Improvement of drought tolerance identified by a soil drought stress tolerance screen: Drought or water deficit conditions impose mainly osmotic stress on plants. Plants are particularly vulnerable to drought during the flowering stage. The drought condition in the screening process disclosed in Example 1B started from the flowering time and was sustained to the end of harvesting. The present invention provides recombinant DNA that can improve the plant survival rate under such sustained drought condition. Exemplary recombinant DNA for conferring such drought tolerance are identified as such in Table 3. Such recombinant DNA may find particular use in generating transgenic plants that are tolerant to the drought condition imposed during flowering time and in other stages of the plant life cycle. As demonstrated from the model plant screen, in some embodiments of transgenic plants with trait-improving recombinant DNA grown under such sustained drought condition can also have increased total seed weight per plant in addition to the increased survival rate within a transgenic population, providing a higher yield potential as compared to control plants.

PEG-Improvement of drought tolerance identified by PEG induced osmotic stress tolerance screen: Various drought levels can be artificially induced by using various concentrations of polyethylene glycol (PEG) to produce different osmotic potentials (Pilon-Smits et al. (1995) Plant Physiol. 107:125-130). Several physiological characteristics have been reported as being reliable indications for selection of plants possessing drought tolerance. These characteristics include the rate of seed germination and seedling growth. The traits can be assayed relatively easily by measuring the growth rate of seedling in PEG solution. Thus, a PEG-induced osmotic stress tolerance screen is a useful surrogate for drought tolerance screen. As demonstrated from the model plant screen, embodiments of transgenic plants with trait-improving recombinant DNA identified in the PEG-induced osmotic stress tolerance screen can survive better drought conditions providing a higher yield potential as compared to control plants.

SS-Improvement of drought tolerance identified by high salinity stress tolerance screen:

Three different factors are responsible for salt damages: (1) osmotic effects, (2) disturbances in the mineralization process, (3) toxic effects caused by the salt ions, e.g. inactivation of enzymes.
While the first factor of salt stress results in the wilting of the plants that is similar to drought effect, the ionic aspect of salt stress is clearly distinct from drought. The present invention provides genes that help plants to maintain biomass, root growth, and/or plant development in high salinity conditions, which are identified as such in Table 3. Since osmotic effect is one of the major components of salt stress, which is common to the drought stress, trait-improving recombinant DNA identified in a high salinity stress tolerance screen can also provide transgenic crops with improved drought tolerance. As demonstrated from the model plant screen, embodiments of transgenic plants with trait-improving recombinant DNA identified in a high salinity stress tolerance screen can survive better drought conditions and/or high salinity conditions providing a higher yield potential as compared to control plants.

HS-Improvement of drought tolerance identified by heat stress tolerance screen: Heat and drought stress often occur simultaneously, limiting plant growth. Heat stress can cause the reduction in photosynthesis rate, inhibition of leaf growth and osmotic potential in plants. Thus, genes identified by the present invention as heat stress tolerance conferring genes may also impart improved drought tolerance to plants. As demonstrated from the model plant screen, embodiments of transgenic plants with trait-improving recombinant DNA identified in a heat stress tolerance screen can survive better heat stress conditions and/or drought conditions providing a higher yield potential as compared to control plants.

CK and CS-Improvement of tolerance to cold stress: Low temperature may immediately result in mechanical constraints, changes in activities of macromolecules, and reduced osmotic potential. In the present invention, two screening conditions, i.e. cold shock tolerance screen (CK) and cold germination tolerance screen (CS), were set up to look for transgenic plants that display visual growth advantage at lower temperature. In cold germination tolerance screen, the transgenic *Arabidopsis* plants were exposed to a constant temperature of 8°C from planting until day 28 post plating. The trait-improving recombinant DNA identified by such screen are particular useful for the production of transgenic plant that can germinate more robustly in a cold temperature as compared to the wild type plants. In cold shock tolerance screen, the transgenic plants were first grown under the normal growth temperature of 22°C until day 8 post plating, and subsequently were placed under 8°C until day 28 post plating. As demonstrated from the model plant screen, embodiments of transgenic plants with trait-improving recombinant DNA identified in a cold shock stress tolerance screen and/or a cold germination stress tolerance screen can survive better cold conditions providing a higher yield potential as compared to control plants.

Improvement of tolerance to multiple stresses: Different kinds of stresses often lead to identical or similar reaction in the plants. Genes that are activated or inactivated as a reaction to stress can either act directly in a way the genetic product reduces a specific stress, or they can act indirectly by activating other specific stress genes. By manipulating the activity of such regulatory genes, i.e. multiple stress tolerance genes, the plant can be enabled to react to different kinds of stresses. For examples, PEP SEQ ID NO: 229 and PEP SEQ ID NO: 372 can be used to improve both salt stress tolerance and cold stress tolerance in plants. Of particular interest, plants transformed with PEP SEQ ID NO: 364 can resist heat stress, salt stress and cold stress. In addition to these multiple stress tolerance genes, the stress tolerance conferring genes provided by the present invention may be used in combinations to generate transgenic plants that can resist multiple stress conditions.

PP-Improvement of early plant growth and development: It has been known in the art that to minimize the impact of disease on crop profitability, it is important to start the season with healthy vigorous plants. This means avoiding seed and seedling diseases, leading to increased nutrient uptake and increased yield potential. Traditionally early planting and applying fertilizer are the methods used for promoting early seedling vigor. In early development stage, plant embryos establish only the basic root-shoot axis, a cotyledon storage organ(s), and stem cell populations, called the root and shoot apical meristems, that continuously generate new organs throughout post-embryonic development. "Early growth and development " used herein encompasses the stages of seed imbibition through the early vegetative phase. The present invention provides genes that are useful to produce transgenic plants that have advantages in one or more processes including, but not limited to, germination, seedling vigor, root growth and root morphology under non-stressed conditions. The transgenic plants starting from a more robust seedling are less susceptible to the fungal and bacterial pathogens that attach germinating seeds and seedling. Furthermore, seedlings with advantage in root growth are more resistant to drought stress due to extensive and deeper root architecture. Therefore, it can be recognized by those skilled in the art that genes conferring the growth advantage in early stages to plants may also be used to generate transgenic plants that are more resistant to various stress conditions due to improved early plant development. The present invention provides such exemplary recombinant DNA that confer both the stress tolerance and growth advantages to plants, identified as such in Table 3, e.g. PEP SEQ ID NO: 372 which can improve the plant early growth and development, and impart salt and cold tolerance to plants. As demonstrated from the model plant screen, embodiments of transgenic plants with trait-improving recombinant DNA identified in the early plant development screen can grow better under non-stress conditions and/or stress conditions providing a higher yield potential as compared to control plants.

SP-Improvement of late plant growth and development: "Late growth and development" used herein encompasses the stages of leaf development, flower production, and seed maturity. In certain embodiments, transgenic plants produced using genes that confer growth advantages to plants provided by the present invention, identified as such in Table 3, exhibit at least one phenotypic characteristics including, but not limited to, increased rosette radius, increased rosette dry weight, seed dry weight, silique dry weight, and silique length. On one hand, the rosette radius and rosette dry weight are used as the indexes of photosynthesis capacity, and thereby plant source strength and yield potential of a plant. On the other hand, the seed dry weight, silique dry weight and silique length are used as the indexes for plant sink strength, which are considered as the direct determinants of yield. As demonstrated from the model plant screen, embodiments of transgenic plants with trait-improving recombinant DNA identified in the late development screen can grow better and/or have improved development during leaf development and seed maturation providing a higher yield potential as compared to control plants.

LL-Improvement of tolerance to shade stress identified in a low light screen: The effects of light on plant development are especially prominent at the seedling stage. Under normal light conditions with unobstructed direct light, a plant seeding develops according to a characteristic photomorphogenic pattern, in which plants have open and expanded cotyledons and short hypocotyls. Then the plant's energy is devoted to cotyledon and leaf development while longitudinal extension growth is minimized. Under low light condition where light quality and intensity are reduced by shading, obstruction or high population density, a seedling displays a shade-avoidance pattern, in which the seedling displays a reduced cotyledon expansion, and hypocotyls extension is greatly increased. As the result, a plant under low light condition increases significantly its stem length at the expanse of leaf, seed or fruit and storage organ development, thereby adversely affecting of yield. The present invention provides recombinant DNA that enable plants to have an attenuated shade avoidance response so that the source of plant can be contributed to reproductive growth efficiently, resulting higher yield as compared to the wild type plants. As demonstrated from the model plant screen, embodiments of transgenic plants with trait-improving recombinant DNA identified in a shade stress tolerance screen can have attenuated shade response under shade conditions providing a higher yield potential as compared to control plants. The transgenic plants generated by the present invention may be suitable for a higher density planting, thereby resulting increased yield per unit area.

LN-Improvement of tolerance to low nitrogen availability stress

Nitrogen is a key factor in plant growth and crop yield. The metabolism, growth and development of plants are profoundly affected by their nitrogen supply. Restricted nitrogen supply alters shoot to root ratio, root development, activity of enzymes of primary metabolism and the rate of senescence (death) of older leaves. All field crops have a fundamental dependence on inorganic nitrogenous fertilizer. Since fertilizer is rapidly depleted from most soil types, it must be supplied to growing crops two or three times during the growing season. Enhanced nitrogen use efficiency by plants should enable crops cultivated under low nitrogen availability stress condition resulted from low fertilizer input or poor soil quality.

According to the present invention, transgenic plants generated using the recombinant nucleotides, which confer enhanced nitrogen use efficiency, identified as such in Table 3, exhibit one or more desirable traits including, but not limited to, increased seedling weight, increased number of green leaves, increased number of rosette leaves, increased root length and advanced flower bud formation. One skilled in the art may recognize that the transgenic plants provided by the present invention with enhanced nitrogen use efficiency may also have altered amino acid or protein compositions, increased yield and/or better seed quality. The transgenic plants of the present invention may be productively cultivated under nitrogen nutrient deficient conditions, i.e. nitrogen-poor soils and low nitrogen fertilizer inputs, that would cause the growth of wild type plants to cease or to be so diminished as to make the wild type plants practically useless. The transgenic plants also may be advantageously used to achieve earlier maturing, faster growing, and/or higher yielding crops and/or produce more nutritious foods and animal feedstocks when cultivated using nitrogen non-limiting growth conditions.

Stacked Traits: The present invention also encompasses transgenic plants with stacked engineered traits, e.g. a crop having an improved phenotype resulting from expression of a trait-improving recombinant DNA, in combination with herbicide and/or pest resistance traits. For example, genes of the current invention can be stacked with other traits of agronomic interest, such as a trait providing herbicide resistance, for example a RoundUp Ready trait, or insect resistance, such as using a gene from *Bacillus thuringensis* to provide resistance against lepidopteran, coliopteran, homopteran, hemiopteran, and other insects. Herbicides for which resistance is useful in a plant include glyphosate herbicides, phosphinothricin herbicides, oxynil herbicides, imidazolinone herbicides, dinitroaniline herbicides, pyridine herbicides, sulfonylurea herbicides, bialaphos herbicides, sulfonamide herbicides and gluphosinate herbicides. To illustrate that the production of transgenic plants with herbicide resistance is a capability of those of ordinary skill in the art, reference is made to U.S. patent application publications 2003/0106096A1 and 2002/0112260A1 and U.S. Patents 5,034,322; 5,776,760, 6,107,549 and 6,376,754, all of which are incorporated herein by reference. To illustrate that the production of transgenic plants with pest resistance is a capability of those of ordinary skill in the art reference is made to U.S. Patents 5,250,515 and 5,880,275 which disclose plants expressing an endotoxin of *Bacillus thuringiensis* bacteria, to U.S. Patent 6,506,599 which discloses control of invertebrates which feed on transgenic plants which express dsRNA for suppressing a target gene in the invertebrate, to U.S. Patent 5,986,175 which discloses the control of viral pests by transgenic plants which express viral replicase, and to U.S. Patent Application Publication 2003/0150017 A1 which discloses control of pests by a transgenic plant which express a dsRNA targeted to suppressing a gene in the pest, all of which are incorporated herein by reference.

Once one recombinant DNA has been identified as conferring an improved trait of interest in transgenic *Arabidopsis* plants, several methods are available for using the sequence of that recombinant DNA and knowledge about the protein it encodes to identify homologs of that sequence from the same plant or different plant species or other organisms, e.g. bacteria and yeast. Thus, in one aspect, the invention provides methods for identifying a homologous gene with a DNA sequence homologous to any of SEQ ID NO: 1 through SEQ ID NO: 204, or a homologous protein with an amino acid sequence homologous to any of SEQ ID NO: 205 through SEQ ID NO: 408. In another aspect, the present invention provides the protein sequences of identified homologs for a sequence listed as SEQ ID NO: 205 through SEQ ID NO: 408. In yet another aspect, the present invention also includes linking or associating one or more desired traits, or gene function with a homolog sequence provided herein.

The trait-improving recombinant DNA and methods of using such trait-improving recombinant DNA for generating transgenic plants with improved traits provided by the present invention are not limited to any particular plant species. Indeed, the plants according to the present invention may be of any plant species, i.e., may be monocotyledonous or dicotyledonous. Preferably, they will be agricultural useful plants, i.e., plants cultivated by man for purposes of food production or technical, particularly industrial applications. Of particular interest in the present invention are corn and soybean plants. The recombinant DNA constructs optimized for soybean transformation and recombinant DNA constructs optimized for corn transformation are provided by the present invention. Other plants of interest in the present invention for production of transgenic plants having improved traits include, without limitation, cotton, canola, wheat, sunflower, sorghum, alfalfa, barley, millet, rice, tobacco, fruit and vegetable crops, and turfgrass.

In certain embodiments, the present invention contemplates to use an orthologous gene in generating the transgenic plants with similarly improved traits as the transgenic *Arabidopsis* counterpart. Improved physiological properties in transgenic plants of the present invention may be confirmed in responses to stress conditions, for example in assays using imposed stress conditions to detect improved responses to drought stress, nitrogen deficiency, cold growing conditions, or alternatively, under naturally present stress conditions, for example under field conditions. Biomass measures may be made on greenhouse or field grown plants and may include such measurements as plant height, stem diameter, root and shoot dry weights, and, for corn plants, ear length and diameter.

Trait data on morphological changes may be collected by visual observation during the process of plant regeneration as well as in regenerated plants transferred to soil. Such trait data includes characteristics such as normal plants, bushy plants, taller plants, thicker stalks, narrow leaves, striped leaves, knotted phenotype, chlorosis, albino, anthocyanin production, or altered tassels, ears or roots. Other enhanced traits may be identified by measurements taken under field conditions, such as days to pollen shed, days to silking, leaf extension rate, chlorophyll content, leaf temperature, stand, seedling vigor, internode length, plant height, leaf number, leaf area, tillering, brace roots, stay green, stalk lodging, root lodging, plant health, barreness/prolificacy, green snap, and pest resistance. In addition, trait characteristics of harvested grain may be confirmed, including number of kernels per row on the ear, number of rows of kernels on the ear, kernel abortion, kernel weight, kernel size, kernel density and physical grain quality.

To confirm hybrid yield in transgenic corn plants expressing genes of the present invention, it may be desirable to test hybrids over multiple years at multiple locations in a geographical location where maize is conventionally grown, e.g. in Iowa, Illinois or other locations in the midwestern United States, under "normal" field conditions as well as under stress conditions, e.g. under drought or population density stress.

Transgenic plants can be used to provide plant parts according to the invention for regeneration or tissue culture of cells or tissues containing the constructs described herein. Plant parts for these purposes can include leaves, stems, roots, flowers, tissues, epicotyl, meristems, hypocotyls, cotyledons, pollen, ovaries, cells and protoplasts, or any other portion of the plant which can be used to regenerate additional transgenic plants, cells, protoplasts or tissue culture. Seeds of transgenic plants are provided by this invention can be used to propagate more plants containing the trait-improving recombinant DNA constructs of this invention. These descendants are intended to be included in the scope of this invention if they contain a trait-improving recombinant DNA construct of this invention, whether or not these plants are selfed or crossed with different varieties of plants.

The various aspects of the invention are illustrated by means of the following examples which are in no way intended to limit the full breath and scope of claims.

**Example 1.**

This example illustrates the identification of recombinant DNA that confers improved trait(s) to plants

A large set of genes of interest were cloned from a genomic or cDNA library using primers specific to sequences upstream and downstream of the coding region. Transformation vectors were prepared to constitutively transcribe DNA in either sense orientation (for enhanced protein expression) or anti-sense orientation (for endogenous gene suppression) under the control of an enhanced Cauliflower Mosaic Virus 35S promoter. The transformation vectors also contain a *bar* gene as a selectable marker for resistance to glufosinate herbicide. The transformation of *Arabidopsis* plants was carried out using the vacuum infiltration method known in the art (Bethtold et al. Methods Mol. Biol. 82:259-66, 1998). Seeds harvested from the plants, named as T1 seeds, were subsequently grown in a glufosinate-containing selective medium to select for plants which were actually transformed and which produced T2 transgenic seed. The plants and seeds were screened for an enhanced trait or a surrogate for an enhanced trait.

**Soil Drought tolerance screen**

This screen identified genes for recombinant DNA that imparts enhanced water use efficiency as shown in *Arabidopsis* plants transformed with recombinant DNA that wilt less rapidly and/or produce higher seed yield when grown in soil under drought conditions

T2 seeds were sown in flats filled with Metro/Mix® 200 (The Scotts® Company, USA). Humidity domes were added to each flat and flats were assigned locations and placed in climate-controlled growth chambers. Plants were grown under a temperature regime of 22°C at day and 20°C at night, with a photoperiod of 16 hours and average light intensity of 170 $\mu$mol/m$^2$/s. After the first true leaves appeared, humidity domes were removed. The plants were sprayed with glufosinate herbicide and put back in the growth chamber for 3 additional days. Flats were watered for 1 hour the week following the herbicide treatment. Watering was continued every seven days until the flower bud primordia became apparent, at which time plants were watered for the last time.

To identify drought tolerant plants, plants were evaluated for wilting response and seed yield. Beginning ten days after the last watering, plants were examined daily until 4 plants/line had wilted. In the next six days, plants were monitored for wilting response. Five drought scores were assigned according to the visual inspection of the phenotypes: 1 for healthy, 2 for dark green, 3 for wilting, 4 severe wilting, and 5 for dead. A score of 3 or higher was considered as wilted.

At the end of this assay, seed yield measured as seed weight per plant under the drought condition was characterized for the transgenic plants and their controls and analyzed as a quantitative response according to example 1 M.

Two approaches were used for statistical analysis on the wilting response. First, the risk score was analyzed for wilting phenotype and treated as a qualitative response according to the example 1L. Alternatively, the survival analysis was carried out in which the proportions of wilted and non-wilted transgenic and control plants were compared over each of the six days under scoring and an overall log rank test was performed to compare the two survival curves using S-PLUS statistical software (S-PLUS 6, Guide to statistics, Insightful, Seattle, WA, USA).

Table 4 provides a list of recombinant DNA constructs that improve drought tolerance in transgenic plants.

Table 4

| Pep SEQ ID | Construct_ id | Orientation | Wilt Response Risk score | | | Seed Weight/plant | | | Survival Analysis of wilt response | | |
|---|---|---|---|---|---|---|---|---|---|---|---|
| | | | RS mean | p-value | c | delta | p-value | c | diff time to wilting | p-value | c |
| 215 | 19116 | ANTI-SENSE | 0 | 0.795 | S | -0.341 | 0.795 | / | 0.52 | 0.077 | T |
| 250 | 70677 | SENSE | 0.045 | 0.982 | S | -5.73 | 0.982 | / | 0.14 | 0.07 | T |
| 219 | 72712 | SENSE | 0.002 | 0.986 | S | -2.822 | 0.986 | / | 0.64 | 0.242 | / |
| 266 | 72714 | SENSE | 0.01 | 0 | S | 1.253 | 0 | S | 0.14 | 0.059 | T |
| 271 | 72721 | SENSE | 0.05 | 0.834 | T | -0.129 | 0.834 | / | 0 | 1 | / |
| 272 | 72959 | SENSE | 0.001 | 0.172 | S | 0.341 | 0.172 | / | 0.42 | 0.32 | / |
| 317 | 73534 | SENSE | 0.006 | 0.732 | S | -0.195 | 0.732 | / | -0.14 | 0.724 | / |
| 264 | 74244 | SENSE | 0.012 | 0.984 | S | -0.512 | 0.984 | / | 0.13 | 0.54 | / |
| 332 | 74453 | SENSE | 0.655 | 0.001 | / | 0.886 | 0.001 | S | -0.15 | 0.468 | / |

S: represents that the transgenic plants showed statistically significant trait improvement as compared to the reference (p<0.05, p value, of the delta of a quantitative response or of the risk score of a qualitative response, is the probability that the observed difference between the transgenic plants and the reference occur by chance)
T: represents that the transgenic plants showed a trend of trait improvement as compared to the reference with p<0.2
/: represents the transgenic plants didn't show any alteration or had unfavorable change in traits examined compared to the reference in the current dataset.

**Heat stress tolerance screen**

Under high temperatures, *Arabidopsis* seedlings become chlorotic and root growth is inhibited. This screen identified genes for recombinant DNA that imparts enhanced heat tolerance as shown in *Arabidopsis* plants transformed with the gene of interest that are more resistant to heat stress based on primarily their seedling weight and root growth under high temperature.

T2 seeds were plated on 1/2 X MS salts, 1/% phytagel, with 10 $\mu$g/ml BASTA (7 per plate with 2 control seeds; 9 seeds total per plate). Plates were placed at 4°C for 3 days to stratify seeds. Plates were then incubated at room temperature for 3 hours and then held vertically for 11 additional days at temperature of 34°C at day and 20°C at night. Photoperiod was 16 h. Average light intensity was ~140 $\mu$mol/m$^2$/s. After 14 days of growth, plants were scored for glufosinate resistance, root length, final growth stage, visual color, and seedling fresh weight. A photograph of the whole plate was taken on day 14.

The seedling weight and root length were analyzed as quantitative responses according to example 1M. The final grow stage at day 14 was scored as success if 50% of the plants had reached 3 rosette leaves and size of leaves are greater than 1mm (Boyes et al. (2001) The Plant Cell 13, 1499-1510). The growth stage data was analyzed as a qualitative response according to example 1L. Table 5 provides a list of recombinant DNA constructs that improve heat tolerance in transgenic plants.

Table 5

| Pep SEQ ID | Const ruct_ id | Orientation | seedling weight | | | Root Length | | | growth stage | | |
|---|---|---|---|---|---|---|---|---|---|---|---|
| | | | delta | p-value | c | delta | p-value | c | RS mean | p-value | c |
| 258 | 10903 | ANTI-SENSE | 1.26 | 0 | S | 0.131 | 0.053 | T | 0.86 | 0.051 | T |
| 205 | 12360 | SENSE | 1.305 | 0 | S | 0.142 | 0.052 | T | 0.307 | 0.043 | S |
| 405 | 12824 | SENSE | 1.309 | 0 | S | 0.25 | 0.002 | S | 0.63 | 0.063 | T |

(continued)

| Pep SEQ ID | Const ruct_ id | Orientation | seedling weight | | | Root Length | | | growth stage | | |
|---|---|---|---|---|---|---|---|---|---|---|---|
| | | | delta | p-value | c | delta | p-value | c | RS mean | p-value | c |
| 211 | 12927 | SENSE | 1.527 | 0 | S | 0.268 | 0.013 | S | 0.803 | 0.018 | S |
| 207 | 15210 | SENSE | 1.189 | 0 | S | 0.121 | 0.029 | S | 0.636 | 0.079 | T |
| 214 | 17309 | SENSE | 1.29 | 0 | S | 0.176 | 0.01 | S | 0.581 | 0.044 | S |
| 242 | 19801 | SENSE | 1.156 | 0 | S | 0.148 | 0.041 | S | 0.413 | 0.141 | T |
| 233 | 19845 | SENSE | 1.242 | 0 | S | 0.148 | 0.051 | T | 0.479 | 0.016 | S |
| 239 | 19850 | SENSE | 1.234 | 0 | S | 0.217 | 0.015 | S | 1.883 | 0.001 | S |
| 237 | 19981 | SENSE | 1.406 | 0 | S | 0.36 | 0 | S | 2.199 | 0 | S |
| 220 | 71546 | SENSE | 1.496 | 0 | S | 0.24 | 0.016 | S | 0.313 | 0.052 | T |
| 246 | 71556 | SENSE | 1.394 | 0 | S | 0.26 | 0.015 | S | 0.194 | 0.076 | T |
| 276 | 73029 | SENSE | 0.88 | 0.002 | S | 0.115 | 0.061 | T | 0.092 | 0.105 | T |
| 318 | 74125 | SENSE | 0.988 | 0 | S | 0.171 | 0.058 | T | 0.206 | 0.053 | T |
| 283 | 74329 | SENSE | 1.19 | 0 | S | 0.124 | 0.08 | T | 0.043 | 0.239 | / |
| 329 | 74402 | SENSE | 1.407 | 0 | S | 0.291 | 0.019 | S | 0.181 | 0.087 | T |
| 335 | 74503 | SENSE | 1.159 | 0 | S | 0.171 | 0.019 | S | 0.206 | 0.02 | S |
| 340 | 74553 | SENSE | 1.141 | 0 | S | 0.184 | 0.02 | S | 0.704 | 0.03 | S |
| 299 | 74669 | SENSE | 1.105 | 0 | S | 0.133 | 0.049 | S | 0.051 | 0.362 | / |
| 352 | 74903 | SENSE | 1.167 | 0 | S | 0.168 | 0.096 | T | 0.836 | 0.041 | S |
| 362 | 74980 | SENSE | 1.084 | 0 | S | 0.165 | 0.027 | S | 1.07 | 0.033 | S |
| 364 | 75337 | SENSE | 1.695 | 0 | S | 0.216 | 0.009 | S | 0.369 | 0.072 | T |
| 367 | 75352 | SENSE | 1.342 | 0 | S | 0.198 | 0.003 | S | 0.311 | 0.053 | T |
| 370 | 75358 | SENSE | 1.314 | 0 | S | 0.131 | 0.034 | S | 0.012 | 0.365 | / |
| 384 | 75409 | SENSE | 1.264 | 0 | S | 0.172 | 0.041 | S | 0.716 | 0.039 | S |
| 386 | 75550 | SENSE | 1.117 | 0 | S | 0.18 | 0.032 | S | 0.384 | 0.074 | T |
| 400 | 75571 | SENSE | 1.182 | 0 | S | 0.185 | 0.042 | S | 0.496 | 0.088 | T |
| 404 | 75909 | SENSE | 1.264 | 0 | S | 0.237 | 0.021 | S | -0.01 | 1 | / |

S: represents the transgenic plants showed statistically significant trait improvement as compared to the reference (p<0.05) T: represents the transgenic plants showed a trend of trait improvement as compared to the reference with p<0.2

/: represents the transgenic plants didn't show any alteration or had unfavorable change in traits examined compared to the reference in the current dataset

**Salt stress tolerance screen**

This screen identified genes for recombinant DNA that imparts enhanced salt tolerance, a surrogate for enhanced water use efficiency, as shown in *Arabidopsis* plants transformed with the gene of interest that are tolerant to high levels of salt based on their rate of development, root growth and chlorophyll accumulation under high salt conditions.

T2 seeds were plated on glufosinate selection plates containing 90 mM NaCl and grown under standard light and temperature conditions. All seedlings used in the experiment were grown at a temperature of 22°C at day and 20°C at night, a 16-hour photoperiod, an average light intensity of approximately 120 $\mu$mol/m$^2$. On day 11, plants were measured for primary root length. After 3 more days of growth (day 14), plants were scored for transgenic status, primary root length, growth stage, visual color, and the seedlings were pooled for fresh weight measurement. A photograph of the whole plate was also taken on day 14.

The seedling weight and root length were analyzed as quantitative responses according to example 1M. The final growth stage at day 14 was scored as success if 50% of the plants reached 3 rosette leaves and size of leaves are greater than 1mm (Boyes, D.C. et. al. (2001), The Plant Cell 13, 1499-1510). The growth stage data was analyzed as a qualitative response according to example 1L. Table 6 provides a list of recombinant DNA constructs that improve high salinity tolerance in transgenic plants

Table 6

| Pep SEQ ID | Construct id | Orientation | Seedling Weight at day14 | | | Root Length at day 11 | | | Root Length at day 14 | | | Growth Stage | | |
|---|---|---|---|---|---|---|---|---|---|---|---|---|---|---|
| | | | delta | p-value | c | delta | p-value | c | delta | p-value | c | RS mean | p-vallue | c |
| 228 | 19617 | SENSE | 0.662 | 0.023 | S | 0.211 | 0.138 | T | 0.019 | 0.845 | / | 0.467 | 0.06 | T |
| 229 | 19750 | SENSE | 0.794 | 0.001 | S | 0.324 | 0.035 | S | 0.192 | 0.001 | S | 3.409 | 0.001 | S |
| 240 | 19942 | SENSE | 0.496 | 0.022 | S | 0.123 | 0.294 | / | 0.103 | 0.208 | / | 1.302 | 0.103 | T |
| 270 | 72743 | SENSE | 0.522 | 0.043 | S | 0.018 | 0.856 | / | 0.194 | 0.004 | S | 0.651 | 0.055 | T |
| 278 | 72920 | SENSE | 0.377 | 0.033 | S | 0.251 | 0.001 | S | 0.133 | 0.004 | S | 0.191 | 0.233 | / |
| 315 | 73516 | SENSE | 0.41 | 0.005 | S | 0.079 | 0.292 | / | 0.034 | 0.372 | / | 1.252 | 0.053 | T |
| 263 | 74237 | SENSE | 0.711 | 0.002 | S | 0.18 | 0.135 | T | 0.187 | 0.066 | T | 2.74 | 0.01 | S |
| 282 | 74327 | SENSE | 0.554 | 0.006 | S | 0.169 | 0.056 | T | 0.162 | 0.005 | S | 1.469 | 0.005 | S |
| 291 | 74366 | SENSE | 0.623 | 0.008 | S | 0.125 | 0.228 | / | 0.108 | 0.248 | / | 0.695 | 0.062 | T |
| 335 | 74503 | SENSE | 0.666 | 0.002 | S | 0.258 | 0.027 | S | 0.103 | 0.221 | / | 2.431 | 0.01 | S |
| 336 | 74504 | SENSE | 0.976 | 0.001 | S | 0.356 | 0.013 | S | 0.261 | 0 | S | 3.178 | 0.001 | S |
| 296 | 74622 | SENSE | 1.092 | 0.003 | S | 0.261 | 0.005 | S | 0.217 | 0.013 | S | 1.457 | 0.065 | T |
| 297 | 74628 | SENSE | 0.423 | 0.057 | T | -0.062 | 0.725 | / | 0.226 | 0.02 | S | 0.087 | 0.31 | / |
| 301 | 74647 | SENSE | 0.161 | 0.442 | / | -0.083 | 0.569 | / | 0.208 | 0.001 | S | 0.573 | 0.043 | S |
| 352 | 74903 | SENSE | 0.446 | 0.009 | S | 0.001 | 0.99 | / | 0.116 | 0.034 | S | 1.234 | 0.05 | T |
| 357 | 74977 | SENSE | 0.496 | 0.019 | S | 0.2 | 0.012 | S | 0.198 | 0 | S | 0.764 | 0.043 | S |
| 363 | 74993 | SENSE | 0.379 | 0.017 | S | 0.242 | 0.075 | T | 0.108 | 0.07 | T | 0.163 | 0.319 | / |
| 366 | 75316 | SENSE | 0.475 | 0.078 | T | 0.287 | 0.002 | S | 0.178 | 0.011 | S | 4 | 0 | S |
| 364 | 75337 | SENSE | 0.934 | 0.004 | S | 0.217 | 0.124 | T | 0.314 | 0.002 | S | 1.831 | 0.013 | S |
| 378 | 75431 | SENSE | 0.429 | 0.083 | T | 0.076 | 0.397 | / | 0.096 | 0.271 | / | 0.522 | 0.121 | T |
| 379 | 75455 | SENSE | 0.286 | 0.281 | / | 0.314 | 0.001 | S | 0.221 | 0.006 | S | 0.396 | 0.095 | T |
| 372 | 75463 | SENSE | 0.601 | 0.011 | S | 0.141 | 0.221 | / | 0.163 | 0.049 | S | 1.252 | 0.058 | T |

(continued)

| Pep SEQ ID | Construct id | Orientation | Seedling Weight at day14 | | | Root Length at day 11 | | | Root Length at day 14 | | | Growth Stage | | |
|---|---|---|---|---|---|---|---|---|---|---|---|---|---|---|
| | | | delta | p-value | c | delta | p-value | c | delta | p-value | c | RS mean | p-vallue | c |
| 394 | 75543 | SENSE | 0.396 | 0.042 | S | 0.136 | 0.054 | T | 0.088 | 0.192 | T | 1.426 | 0.078 | T |

S: represents the transgenic plants showed statistically significant trait improvement as compared to the reference (p<0.05) T: represents the transgenic plants showed a trend of trait improvement as compared to the reference with p<0.2

/: represents the transgenic plants didn't show any alteration or had unfavorable change in traits examined compared to the reference in the current dataset

**Polyethylene Glycol (PEG) induced osmotic stress tolerance screen**

There are numerous factors, which can influence seed germination and subsequent seedling growth, one being the availability of water. Genes, which can directly affect the success rate of germination and early seedling growth, are potentially useful agronomic traits for improving the germination and growth of crop plants under drought stress. This screen identified genes for recombinant DNA that imparts enhance osmotic stress tolerance, a surrogate for enhanced water use efficiency, as shown in *Arabidopsis* seed when PEG was used to induce osmotic stress on germinating transgenic lines of seeds.

T2 seeds were plated on BASTA selection plates containing 3% PEG and grown under standard light and temperature conditions. Seeds were plated on each plate containing 3% PEG,1/2 X MS salts, 1% phytagel, and 10 $\mu$g/ml glufosinate. Plates were placed at 4°C for 3 days to stratify seeds. On day 11, plants were measured for primary root length. After 3 more days of growth, i.e. at day 14, plants were scored for transgenic status, primary root length, growth stage, visual color, and the seedlings were pooled for fresh weight measurement. A photograph of the whole plate was taken on day14.

Seedling weight and root length were analyzed as quantitative responses according to example 1 M. The final growth stage at day 14 was scored as success or failure based on whether the plants reached 3 rosette leaves and size of leaves are greater than 1mm. The growth stage data was analyzed as a qualitative response according to example 1L. Table 7 provides a list of recombinant DNA constructs that improve osmotic stress tolerance in transgenic plants.

Table 7

| Pep SEQ ID | Gene | Orientation | Seedling Weight at day14 | | | Root Length at day 11 | | | Root Length at day14 | | | Growth Stage | | |
|---|---|---|---|---|---|---|---|---|---|---|---|---|---|---|
| | | | delta | p-value | c | delta | p-value | c | delta | p-value | c | RS mean | p-value | c |
| 275 | 10180 | ANTI-SENSE | 0.322 | 0.024 | S | 0.154 | 0.112 | T | 0.077 | 0.233 | / | 2.679 | 0.014 | S |
| 210 | 12919 | SENSE | 0.523 | 0.001 | S | 0.15 | 0.104 | T | 0.134 | 0.211 | / | 1.067 | 0.084 | T |
| 218 | 16004 | ANTI-SENSE | 0.58 | 0.005 | S | 0.109 | 0.465 | / | 0.137 | 0.048 | S | 0.96 | 0.183 | T |
| 243 | 19705 | SENSE | 0.415 | 0.098 | T | 0.225 | 0.014 | S | 0.185 | 0.104 | T | 2.87 | 0.009 | S |
| 244 | 19737 | SENSE | 0.71 | 0.065 | T | 0.163 | 0.454 | / | 0.127 | 0.477 | / | 2.147 | 0.036 | S |
| 238 | 19757 | SENSE | 0.876 | 0.006 | S | 0.095 | 0.477 | / | 0.173 | 0.016 | S | 2.298 | 0.019 | S |
| 236 | 19902 | SENSE | 0.635 | 0.012 | S | 0.233 | 0.044 | S | 0.1 | 0.104 | T | 2.314 | 0.016 | S |
| 230 | 19964 | SENSE | 0.421 | 0.002 | S | 0.313 | 0.027 | S | 0.388 | 0.004 | S | 2.051 | 0.038 | S |
| 247 | 71330 | SENSE | 0.35 | 0.003 | S | -0.09 | 0.453 | / | 0.137 | 0.189 | T | 3.292 | 0.003 | S |
| 220 | 71546 | SENSE | 0.892 | 0.003 | S | 0.32 | 0.005 | S | 0.266 | 0.028 | S | 3.207 | 0.005 | S |
| 310 | 73480 | SENSE | 0.257 | 0.053 | T | 0.12 | 0.058 | T | 0.069 | 0.487 | / | 3.231 | 0.004 | S |
| 254 | 73651 | SENSE | 0.581 | 0.04 | S | 0.053 | 0.73 | / | 0.034 | 0.609 | / | 2.992 | 0.004 | S |
| 252 | 73685 | SENSE | 0.467 | 0.024 | S | 0.137 | 0.171 | T | 0.006 | 0.933 | / | 1.556 | 0.026 | S |
| 262 | 73769 | SENSE | 0.532 | 0.005 | S | 0.243 | 0.073 | T | 0.043 | 0.7 | / | 2.939 | 0.02 | S |
| 284 | 74340 | SENSE | 0.56 | 0.003 | S | 0.258 | 0.049 | S | 0.237 | 0.036 | S | 3.541 | 0 | S |
| 293 | 74374 | SENSE | 0.587 | 0.081 | T | 0.361 | 0.011 | S | 0.251 | 0.009 | S | 3.001 | 0.003 | S |
| 336 | 74504 | SENSE | 0.801 | 0.003 | S | 0.185 | 0.021 | S | 0.111 | 0.021 | S | 4 | 0 | S |
| 337 | 74528 | SENSE | 0.552 | 0.026 | S | 0.107 | 0.338 | / | 0.112 | 0.418 | / | 4 | 0 | S |
| 341 | 74554 | SENSE | 0.726 | 0.001 | S | 0.337 | 0.012 | S | 0.224 | 0.065 | T | 3.462 | 0.001 | S |
| 343 | 74590 | SENSE | 0.511 | 0.034 | S | 0.067 | 0.671 | / | 0.052 | 0.753 | / | 2.479 | 0.029 | S |
| 289 | 74608 | SENSE | 0.448 | 0.043 | S | 0.175 | 0.298 | / | 0.241 | 0.109 | T | 2.617 | 0.016 | S |
| 300 | 74670 | SENSE | 0.855 | 0 | S | 0.154 | 0.059 | T | 0.063 | 0.447 | / | 2.331 | 0.013 | S |
| 355 | 74951 | SENSE | 0.558 | 0.009 | S | 0.488 | 0.003 | S | 0.517 | 0.001 | S | 3.302 | 0.003 | S |
| 363 | 74993 | SENSE | 0.677 | 0 | S | 0.147 | 0 | S | 0.028 | 0.499 | / | 3.211 | 0.005 | S |

| Pep SEQ ID | Gene | Orientation | Seedling Weight at day14 | | | Root Length at day 11 | | | Root Length at day14 | | | Growth Stage | | |
|---|---|---|---|---|---|---|---|---|---|---|---|---|---|---|
| | | | delta | p-value | c | delta | p-value | c | delta | p-value | c | RS mean | p-value | c |
| 376 | 75418 | SENSE | 0.339 | 0.06 | T | 0.023 | 0.763 | / | 0.263 | 0.016 | S | 0.811 | 0.246 | / |
| 381 | 75456 | SENSE | 0.43 | 0.044 | S | 0.23 | 0.03 | S | 0.198 | 0.003 | S | 2.674 | 0.004 | S |
| 383 | 75492 | SENSE | 0.434 | 0.018 | S | 0.073 | 0.024 | S | 0.09 | 0.093 | T | 0.955 | 0.186 | T |
| 402 | 75536 | SENSE | 0.214 | 0.066 | T | 0.146 | 0.015 | S | 0.221 | 0.025 | S | -1.33 | 0.996 | / |
| 389 | 75564 | SENSE | 0.407 | 0.043 | S | 0.053 | 0.623 | / | 0.074 | 0.569 | / | 1.839 | 0.058 | T |
| 395 | 75567 | SENSE | 0.44 | 0.02 | S | 0.077 | 0.522 | / | 0.107 | 0.291 | / | 2.4 | 0.034 | S |
| 393 | 75590 | SENSE | 0.431 | 0.006 | S | 0.21 | 0.022 | S | 0.195 | 0.014 | S | 2.848 | 0.006 | S |

S: represents the transgenic plants showed statistically significant trait improvement as compared to the reference (p<0.05) T: represents the transgenic plants showed a trend of trait improvement compared to the reference with p<0.2

/: represents the transgenic plants didn't show any alteration or had unfavorable change in traits examined compared to the reference in the current dataset

**Cold shock tolerance screen**

This screen identified genes for recombinant DNA that imparts enhanced sold tolerance as shown in *Arabidopsis* plants transformed with the genes of interest that are more tolerant to cold stress subjected during day 8 to day 28 after seed planting. During these crucial early stages, seedling growth and leaf area increase were measured to assess tolerance when *Arabidopsis* seedlings were exposed to low temperatures. Using this screen, genetic alterations can be found that enable plants to germinate and grow better than wild type plants under sudden exposure to low temperatures.

Eleven seedlings from T2 seeds of each transgenic line plus one control line were plated together on a plate containing ½ X Gamborg Salts with 0.8 Phytagel™, 1% Phytagel, and 0.3% Sucrose. Plates were then oriented horizontally and stratified for three days at 4°C. At day three, plates were removed from stratification and exposed to standard conditions (16 hr photoperiod, 22°C at day and 20°C at night) until day 8. At day eight, plates were removed from standard conditions and exposed to cold shock conditions (24 hr photoperiod, 8°C at both day and night) until the final day of the assay, i.e. day 28. Rosette areas were measured at day 8 and day 28, which were analyzed as quantitative responses according to example 1M. Table 8 provides a list of recombinant nucleotides that improve cold shock stress tolerance in plants.

Table 8

| Pep SEQ ID | Construct id | Orientation | rosette area at day8 | | | rosette area at dav28 | | | difference in rosette area between day 28 and day 8 | | |
|---|---|---|---|---|---|---|---|---|---|---|---|
| | | | Delta | p-value | c | delta | p-value | c | delta | p-value | c |
| 209 | 13222 | SENSE | -0.562 | 0.978 | / | 0.396 | 0.074 | T | 0.581 | 0.058 | T |
| 216 | 14837 | SENSE | -0.08 | 0.661 | / | 1.007 | 0.002 | S | 1.154 | 0.002 | S |
| 212 | 14841 | SENSE | 0.662 | 0.023 | S | 1.026 | 0.001 | S | 1.088 | 0.004 | S |
| 231 | 19814 | SENSE | 0.38 | 0.008 | S | 0.328 | 0.027 | S | 0.55 | 0.01 | S |
| 234 | 71072 | SENSE | -0.054 | 0.58 | / | 0.736 | 0.005 | S | 0.662 | 0.027 | S |
| 224 | 71148 | SENSE | -0.43 | 0.899 | / | 0.189 | 0.14 | T | 0.643 | 0.02 | S |
| 217 | 71253 | SENSE | 0.318 | 0.004 | S | 0.442 | 0.019 | S | 0.54 | 0.009 | S |
| 251 | 71689 | SENSE | -0.566 | 0.907 | / | 0.62 | 0.01 | S | 0.879 | 0.043 | S |
| 312 | 73513 | SENSE | 0.48 | 0.009 | S | 0.61 | 0.022 | S | 0.439 | 0.074 | T |
| 314 | 73527 | SENSE | 0.499 | 0.034 | S | 0.9 | 0.001 | S | 1.087 | 0.001 | S |
| 321 | 74115 | SENSE | 0.254 | 0.17 | T | 0.896 | 0.002 | S | 1.029 | 0.002 | S |
| 324 | 74165 | SENSE | 0.142 | 0.28 | / | 0.293 | 0.115 | T | 0.61 | 0.018 | S |
| 286 | 74345 | SENSE | -0.109 | 0.746 | / | 0.819 | 0.004 | S | 1.148 | 0.009 | S |
| 333 | 74418 | SENSE | 0.421 | 0.002 | S | 1.012 | 0.003 | S | 0.565 | 0.038 | S |
| 337 | 74528 | SENSE | 0.395 | 0.004 | S | 0.544 | 0.018 | S | 0.725 | 0.009 | S |
| 294 | 74618 | SENSE | 0.274 | 0.003 | S | 0.781 | 0.011 | S | 0.941 | 0.033 | S |
| 305 | 74685 | SENSE | -0.238 | 0.923 | / | 0.758 | 0.001 | S | 1.076 | 0 | S |
| 360 | 74919 | SENSE | 0.221 | 0.214 | / | 0.358 | 0.053 | T | 0.388 | 0.108 | T |
| 356 | 74940 | SENSE | 0.28 | 0.143 | T | 0.903 | 0.001 | S | 0.952 | 0.002 | S |
| 358 | 74954 | SENSE | 0.197 | 0.176 | T | 0.602 | 0.013 | S | 0.712 | 0.024 | S |
| 364 | 75337 | SENSE | 0.669 | 0.002 | S | 0.695 | 0.002 | S | 0.603 | 0.014 | S |
| 381 | 75456 | SENSE | 0.413 | 0.034 | S | 0.895 | 0.002S | S | 0.998 | 0 | SS |

(continued)

| Pep SEQ ID | Construct id | Orientation | rosette area at day8 | | | rosette area at dav28 | | | difference in rosette area between day 28 and day 8 | | |
|---|---|---|---|---|---|---|---|---|---|---|---|
| | | | Delta | p-value | c | delta | p-value | c | delta | p-value | c |
| 380 | 75491 | SENSE | 0.324 | 0.047 | S | 0.914 | 0.001 | S | 1.11 | 0.001 | SS |

S: represents the transgenic plants showed statistically significant trait improvement as compared to the reference (p<0.05)
T: represents the transgenic plants showed a trend of trait improvement compared to the reference with p<0.2
/: represents the transgenic plants didn't show any alteration or had unfavorable change in traits examined compared to the reference in the current dataset.

**Cold germination tolerance screen**

This screen identified genes for recombinant DNA that imparts enhanced cold tolerance as shown in *Arabidopsis* plants transformed with the genes of interests are resistant to cold stress based on their rate of development, root growth and chlorophyll accumulation under low temperature conditions.

T2 seeds were plated and all seedlings used in the experiment were grown at 8°C. Seeds were first surface disinfested using chlorine gas and then seeded on assay plates containing an aqueous solution of 1/2 X Gamborg's B/5 Basal Salt Mixture (Sigma/Aldrich Corp., St. Louis, MO, USA G/5788), 1% Phytagel™ (Sigma-Aldrich, P-8169), and 10 ug/ml glufosinate with the final pH adjusted to 5.8 using KOH. Test plates were held vertically for 28 days at a constant temperature of 8°C, a photoperiod of 16 hr, and average light intensity of approximately 100 umol/m$^2$/s. At 28 days post plating, root length was measured, growth stage was observed, the visual color was assessed, and a whole plate photograph was taken.

The root length at day 28 was analyzed as a quantitative response according to example 1M. The growth stage at day 7 was analyzed as a qualitative response according to example 1L.Table 9 provides a list of recombinant DNA constructs that improve cold stress tolerance in transgenic plants.

Table 9

| Pep SEQ ID | Construct_id | Orientation | Root Length at day 28 | | | Growth Stage at day 28 | | |
|---|---|---|---|---|---|---|---|---|
| | | | delta | p-value | c | RS mean | p-value | c |
| 269 | 10814 | ANTI-SENSE | 0.218 | 0.009 | S | 3.148 | 0.007 | S |
| 208 | 17465 | SENSE | 0.269 | 0.027 | S | 3.246 | 0.004 | S |
| 223 | 17919 | SENSE | 0.366 | 0.002 | S | 4 | 0 | S |
| 346 | 18020 | SENSE | 0.112 | 0.105 | T | 3.016 | 0.014 | S |
| 406 | 18026 | SENSE | 0.407 | 0.002 | S | 2.111 | 0.039 | S |
| 226 | 18844 | SENSE | 0.464 | 0.012 | S | 2.927 | 0.005 | S |
| 225 | 19647 | SENSE | 0.479 | 0.002 | S | 3.129 | 0.008 | S |
| 232 | 19720 | SENSE | 0.295 | 0.003 | S | 4 | 0 | S |
| 229 | 19750 | SENSE | 0.224 | 0.006 | S | 3.161 | 0.006 | S |
| 245 | 19812 | SENSE | 0.261 | 0.002 | S | 3.022 | 0.014 | S |
| 235 | 19949 | SENSE | 0.226 | 0 | S | 3.093 | 0.01 | S |
| 220 | 71546 | SENSE | 0.3 | 0.001 | S | 3.181 | 0.006 | S |

(continued)

| Pep SEQ ID | Construct_id | Orientation | Root Length at day 28 | | | Growth Stage at day 28 | | |
|---|---|---|---|---|---|---|---|---|
| | | | delta | p-value | c | RS mean | p-value | c |
| 274 | 73061 | SENSE | 0.306 | 0.002 | S | 3.447 | 0.001 | S |
| 256 | 73271 | SENSE | 0.062 | 0.209 | / | 4 | 0 | S |
| 257 | 73282 | SENSE | 0.216 | 0.023 | S | 4 | 0 | S |
| 255 | 73342 | SENSE | 0.366 | 0 | S | 4 | 0 | S |
| 292 | 74383 | SENSE | 0.182 | 0.019 | S | 4 | 0 | S |
| 336 | 74504 | SENSE | 0.368 | 0.001 | S | 4 | 0 | S |
| 337 | 74528 | SENSE | 0.202 | 0 | S | 4 | 0 | S |
| 339 | 74541 | SENSE | 0.338 | 0.002 | S | 4 | 0 | S |
| 341 | 74554 | SENSE | 0.239 | 0.001 | S | 4 | 0 | S |
| 342 | 74578 | SENSE | 0.53 | 0 | S | 4 | 0 | S |
| 289 | 74608 | SENSE | 0.261 | 0.003 | S | 4 | 0 | S |
| 351 | 74880 | SENSE | 0.21 | 0.04 | S | 2.977 | 0.017 | S |
| 359 | 74907 | SENSE | 0.253 | 0.004 | S | 1.951 | 0.096 | T |
| 372 | 75463 | SENSE | 0.467 | 0.002 | S | 4 | 0 | S |
| 373 | 75475 | SENSE | 0.307 | 0.009 | S | 3.496 | 0 | S |
| 382 | 75480 | SENSE | 0.268 | 0.012 | S | 2.955 | 0.018 | S |
| 392 | 75554 | SENSE | 0.149 | 0.039 | S | 4 | 0 | S |
| 395 | 75567 | SENSE | 0.236 | 0.003 | S | 4 | 0 | S |
| 347 | 75850 | SENSE | 0.616 | 0 | S | 3.053 | 0.002 | S |
| 348 | 75861 | SENSE | 0.272 | 0.001 | S | 4 | 0 | S |
| 349 | 75875 | SENSE | 0.138 | 0.066 | T | 4 | 0 | S |
| 403 | 75991 | SENSE | 0.038 | 0.296 | / | 2.667 | 0.051 | T |

S: represents the transgenic plants showed statistically significant trait improvement as compared to the reference (p<0.05) T: represents the transgenic plants showed a trend of trait improvement as compared to the reference with p<0.2

/: represents the transgenic plants didn't show any alteration or had unfavorable change in traits examined compared to the reference in the current dataset

**Shade tolerance screen**

Plants undergo a characteristic morphological response in shade that includes the elongation of the petiole, a change in the leaf angle, and a reduction in chlorophyll content. While these changes may confer a competitive advantage to individuals, in a monoculture the shade avoidance response is thought to reduce the overall biomass of the population. Thus, genetic alterations that prevent the shade avoidance response may be associated with higher yields. Genes that favor growth under low light conditions may also promote yield, as inadequate light levels frequently limit yield. This screen identified genes for recombinant DNA that imparts enhanced shade tolerance in *Arabidopsis* plants that show an attenuated shade avoidance response and/or grow better than control plants under low light intensity. Of particular interest, we were looking for plants that didn't extend their petiole length, had an increase in seedling weight relative to the reference and had leaves that were more close to parallel with the plate surface.

T2 seeds were plated on glufosinate selection plates with ½ MS medium. Seeds were sown on 1/2 X MS salts, 1% Phytagel, 10 ug/ml BASTA. Plants were grown on vertical plates at a temperature of 22°C at day, 20°C at night and under low light (approximately 30 uE/m$^2$/s, far/red ratio (655/665/725/735) ~0.35 using PLAQ lights with GAM color filter #680). Twenty-three days after seedlings were sown, measurements were recorded including seedling status, number of rosette leaves, status of flower bud, petiole leaf angle, petiole length, and pooled fresh weights. A digital image of the whole plate was taken on the measurement day. Seedling weight and petiole length were analyzed as quantitative responses according to example 1M. The number of rosette leaves, flowering bud formation and leaf angel were analyzed as qualitative responses according to example 1L.

Table 10 provides a list of recombinant DNA constructs that improve shade tolerance in plants

Table 10

| Pep SEQ ID | Construct_ id | Orientation | Petiole length at day23 | | | seedling weight day23 | | | Leaf angle at day23 | | | Number of rosette leaves at day23 | | |
|---|---|---|---|---|---|---|---|---|---|---|---|---|---|---|
| | | | delta | p-value | c | delta | p-value | c | RS mean | p-value | c | RS mean | p-value | c |
| 407 | 11810 | ANTI-SENSE | -0.138 | 0.03 | S | 0.158 | 0.245 | / | 0.246 | 0.296 | / | -0.1 | 0.822 | / |
| 267 | 16014 | SENSE | -0.267 | 0.012 | S | -1.129 | 0.015 | / | 0.042 | 0.425 | / | 0.773 | 0.209 | / |
| 249 | 71571 | SENSE | -0.548 | 0.005 | S | -0.212 | 0.177 | / | 0.325 | 0.224 | / | 0.611 | 0.22 | / |
| 273 | 72984 | SENSE | -0.219 | 0.064 | T | -0.126 | 0.178 | / | 0.12 | 0.315 | / | 0.476 | 0.28 | / |
| 280 | 73044 | SENSE | -0.722 | 0.083 | T | -0.572 | 0.173 | / | -0.032 | 1 | / | -1.35 | 0.992 | / |
| 307 | 73476 | SENSE | -0.01 | 0.905 | / | 0.103 | 0.354 | / | 0.739 | 0.177 | T | 1.896 | 0.053 | T |
| 311 | 73482 | SENSE | -0.394 | 0.107 | T | -0.095 | 0.611 | / | 0.045 | 0.375 | / | 0.252 | 0.376 | / |
| 306 | 73487 | SENSE | 0.064 | 0.457 | / | -0.131 | 0.583 | / | 1.656 | 0.051 | T | 1.131 | 0.15 | T |
| 261 | 74306 | SENSE | -1.107 | 0.13 | T | -1.924 | 0.116 | / | 0.291 | 0.261 | / | -0.103 | 0.591 | / |
| 282 | 74327 | SENSE | 0.029 | 0.777 | / | 0.105 | 0.762 | / | 0.666 | 0.12 | T | 1.252 | 0.116 | T |
| 330 | 74476 | SENSE | -0.092 | 0.072 | T | -0.613 | 0.107 | / | 0.271 | 0.246 | / | -0.026 | 0.511 | / |

S: represents the transgenic plants showed statistically significant trait improvement as compared to the reference (p<0.05)
T: represents the transgenic plants showed a trend of trait improvement as compared to the reference with p<0.2
/: represents the transgenic plants didn't show any alteration or had unfavorable change in traits examined compared to the reference in the current dataset.

**Early plant growth and development screen**

This screen identified genes for recombinant DNA that imparts enhanced early plant growth and development, a surrogate for increased yield, as shown in *Arabidopsis* plants examined in a plate based phenotypic analysis platform for the rapid detection of phenotypes that are evident during the first two weeks of growth. In this screen, we were looking for genes that confer advantages in the processes of germination, seedling vigor, root growth and root morphology under non-stressed growth conditions to plants. The transgenic plants with advantages in seedling growth and development were determined by the seedling weight and root length at day14 after seed planting.

T2 seeds were plated on glufosinate selection plates and grown under standard conditions ($\sim$100 uE/m$^2$/s, 16 h photoperiod, 22°C at day, 20°C at night). Seeds were stratified for 3 days at 4°C. Seedlings were grown vertically (at a temperature of 22°C at day 20°C at night). Observations were taken on day 10 and day 14. Both seedling weight and root length at day 14 were analyzed as quantitative responses according to example 1M.

Table 11 provides a list recombinant DNA constructs that improve early plant growth and development.

Table 11

| Pep SEQ ID | Const ruct_i d | Orienta tion | Root Length at day10 | | | Root Length at day14 | | | Seedling Weight | | |
|---|---|---|---|---|---|---|---|---|---|---|---|
| | | | delta | p-value | c | delta | p-value | c | delta | p-value | c |
| 213 | 13478 | ANTI-SENSE | 0.242 | 0.062 | T | 0.159 | 0.075 | T | 0.603 | 0.013 | S |
| 227 | 19525 | SENSE | 0.249 | 0.183 | T | 0.245 | 0.001 | S | 0.385 | 0.062 | T |
| 221 | 70109 | SENSE | 0.282 | 0.001 | S | 0.23 | 0.001 | S | 0.564 | 0 | S |
| 248 | 71332 | SENSE | 0.071 | 0.548 | / | 0.093 | 0.17 | T | 0.419 | 0.009 | S |
| 220 | 71546 | SENSE | 0.307 | 0.057 | T | 0.231 | 0.013 | S | 0.561 | 0.006 | S |
| 246 | 71556 | SENSE | 0.408 | 0.008 | S | 0.291 | 0.011 | S | 0.328 | 0.263 | / |
| 408 | 72418 | SENSE | 0.144 | 0.063 | T | 0.17 | 0 | S | 0.46 | 0.006 | s |
| 253 | 72636 | SENSE | 0.486 | 0.026 | S | 0.339 | 0 | S | 0.705 | 0.006 | S |
| 279 | 72957 | SENSE | 0.228 | 0.053 | T | 0.158 | 0.089 | T | 0.371 | 0.08 | T |
| 309 | 73418 | SENSE | 0.242 | 0.082 | T | 0.148 | 0.089 | T | 0.212 | 0.085 | T |
| 316 | 73530 | SENSE | 0.239 | 0.023 | s | 0.17 | 0.003 | S | 0.442 | 0.001 | S |
| 313 | 73550 | SENSE | 0.181 | 0.046 | S | 0.113 | 0.016 | S | 0.304 | 0.045 | S |
| 259 | 73913 | SENSE | 0.243 | 0.069 | T | 0.091 | 0.26 | / | 0.452 | 0.015 | S |
| 325 | 74106 | SENSE | 0.25 | 0.003 | S | 0.192 | 0.015 | S | 0.34 | 0.052 | T |
| 318 | 74125 | SENSE | 0.104 | 0.549 | / | 0.139 | 0.048 | S | 0.524 | 0.002 | S |
| 320 | 74126 | SENSE | 0.149 | 0.082 | T | 0.065 | 0.284 | / | 0.354 | 0.127 | T |
| 322 | 74127 | SENSE | 0.381 | 0 | S | 0.212 | 0.006 | S | 0.63 | 0.002 | S |
| 323 | 74128 | SENSE | 0.213 | 0.091 | T | 0.12 | 0.161 | T | 0.541 | 0.002 | S |
| 326 | 74130 | SENSE | 0.14 | 0.002 | S | 0.144 | 0.001 | S | 0.003 | 0.955 | / |
| 327 | 74132 | SENSE | 0.134 | 0.275 | / | 0.144 | 0.049 | S | 0.336 | 0.025 | S |
| 328 | 74144 | SENSE | 0.137 | 0.217 | / | 0.134 | 0.065 | T | 0.492 | 0.012 | S |
| 319 | 74161 | SENSE | 0.205 | 0.02 | S | 0.131 | 0.018 | S | 0.459 | 0.035 | S |
| 263 | 74237 | SENSE | 0.253 | 0 | S | 0.189 | 0.002 | S | 0.408 | 0.013 | S |
| 265 | 74256 | SENSE | 0.28 | 0.04 | S | 0.202 | 0.012 | S | 0.461 | 0.034 | S |
| 260 | 74305 | SENSE | 0.184 | 0.063 | T | 0.131 | 0.026 | S | 0.257 | 0.121 | T |
| 281 | 74323 | SENSE | 0.13 | 0.08 | T | 0.067 | 0.054 | T | 0.426 | 0.005 | S |

(continued)

| Pep SEQ ID | Const ruct_i d | Orienta tion | Root Length at day10 | | | Root Length at day14 | | | Seedling Weight | | |
|---|---|---|---|---|---|---|---|---|---|---|---|
| | | | delta | p-value | c | delta | p-value | c | delta | p-value | c |
| 285 | 74341 | SENSE | 0.185 | 0.044 | S | 0.07 | 0.106 | T | 0.144 | 0.191 | T |
| 293 | 74374 | SENSE | 0.103 | 0.187 | T | 0.171 | 0.008 | S | 0.518 | 0.023 | S |
| 302 | 74385 | SENSE | 0.051 | 0.461 | / | 0.043 | 0.693 | / | 0.214 | 0.098 | T |
| 303 | 74386 | SENSE | 0.211 | 0.12 | T | 0.136 | 0.092 | T | 0.081 | 0.616 | / |
| 304 | 74387 | SENSE | 0.11 | 0.528 | / | 0.17 | 0.052 | T | 0.378 | 0.027 | S |
| 350 | 74548 | SENSE | 0.169 | 0.041 | S | 0.09 | 0.046 | S | 0.336 | 0.08 | T |
| 288 | 74604 | SENSE | 0.157 | 0.231 | / | 0.181 | 0.014 | S | 0.586 | 0.001 | S |
| 290 | 74609 | SENSE | 0.24 | 0.029 | S | 0.102 | 0.227 | / | 0.457 | 0.014 | S |
| 287 | 74616 | SENSE | 0.192 | 0.047 | S | 0.137 | 0.125 | T | 0.286 | 0.204 | / |
| 295 | 74619 | SENSE | 0.181 | 0.415 | / | 0.153 | 0.149 | T | 0.448 | 0.056 | T |
| 296 | 74622 | SENSE | 0.083 | 0.481 | / | 0.071 | 0.331 | / | 0.311 | 0.076 | T |
| 298 | 74631 | SENSE | 0.203 | 0.02 | S | 0.115 | 0.019 | S | 0.049 | 0.633 | / |
| 353 | 74915 | SENSE | 0.202 | 0.004 | S | 0.136 | 0.027 | S | 0.409 | 0.012 | S |
| 354 | 74927 | SENSE | 0.103 | 0.148 | T | 0.117 | 0.021 | S | 0.319 | 0.056 | T |
| 356 | 74940 | SENSE | 0.127 | 0.001 | S | 0.117 | 0.049 | S | 0.344 | 0.028 | S |
| 371 | 75312 | SENSE | 0.191 | 0.055 | T | 0.155 | 0.006 | S | 0.526 | 0.001 | S |
| 365 | 75339 | SENSE | 0.168 | 0.029 | S | 0.031 | 0.704 | / | 0.21 | 0.208 | / |
| 374 | 75440 | SENSE | 0.15 | 0.037 | S | 0.059 | 0.566 | / | 0.039 | 0.923 | / |
| 372 | 75463 | SENSE | 0.392 | 0.006 | S | 0.284 | 0.011 | S | 0.432 | 0.15 | T |
| 375 | 75488 | SENSE | 0.101 | 0.289 | / | 0.083 | 0.113 | T | 0.444 | 0.003 | S |

S: represents the transgenic plants showed statistically significant trait improvement as compared to the reference (p<0.05) T: represents the transgenic plants showed a trend of trait improvement as compared to the reference with p<0.2

/: represents the transgenic plants didn't show any alteration or had unfavorable change in traits examined compared to the reference in the current dataset

**Late plant growth and development screen**

This screen identified genes for recombinant DNA that imparts enhanced late plant growth and development, a surrogate for increased yield, as shown in *Arabidopsis* plants examined in a soil based phenotypic platform to identify genes that confer advantages in the processes of leaf development, flowering production and seed maturity to plants.

*Arabidopsis* plants were grown on a commercial potting mixture (Metro Mix 360, Scotts Co., Marysville, OH) consisting of 30-40% medium grade horticultural vermiculite, 35-55% sphagnum peat moss, 10-20% processed bark ash, 1-15% pine bark and a starter nutrient charge. Soil was supplemented with Osmocote time-release fertilizer at a rate of 30 mg/ft$^3$. T2 seeds were imbided in 1% agarose solution for 3 days at 4 °C and then sown at a density of ~5 per 2 ½" pot. Thirty-two pots were ordered in a 4 by 8 grid in standard greenhouse flat. Plants were grown in environmentally controlled rooms under a 16 h day length with an average light intensity of ~200 μmoles/m$^2$/s. Day and night temperature set points were 22 °C and 20 °C, respectively. Humidity was maintained at 65%. Plants were watered by sub-irrigation every two days on average until mid-flowering, at which point the plants were watered daily until flowering was complete.

Application of the herbicide glufosinate was performed to select T2 individuals containing the target transgene. A single application of glufosinate was applied when the first true leaves were visible. Each pot was thinned to leave a single glufosinate-resistant seedling ~3 days after the selection was applied.

The rosette radius was measured at day 25. The silique length was measured at day 40. The plant parts were harvested at day 49 for dry weight measurements if flowering production was stopped. Otherwise, the dry weights of rosette and silique were carried out at day 53. The seeds were harvested at day 58. All measurements were analyzed as quantitative responses according to example 1M.

Table 12 provides a list of recombinant DNA constructs that improve late plant growth and development.

Table 12

| Pep SEQ ID | Const ruct id | Rosette Dry Weight | | | Rosette Radius | | | Seed Dry Weight | | | Silique Dry Weight | | | Silique Length | | |
|---|---|---|---|---|---|---|---|---|---|---|---|---|---|---|---|---|
| | | delta | p-value | c | delta | p-value | c | Delta | p-value | c | delta | p-value | c | delta | p-value | c |
| 213 | 13478 | 0.054 | 0.365 | / | 0.217 | 0.013 | S | 0.052 | 0.347 | / | 0.293 | 0.084 | T | 0.079 | 0.021 | S |
| 241 | 19787 | 0.233 | 0.052 | T | 0.067 | 0.022 | S | 0.66 | 0.019 | S | -0.02 | 0.75 | / | 0.06 | 0.006 | S |
| 268 | 72751 | 0.287 | 0.012 | S | -0.141 | 0.714 | / | -0.991 | 0.973 | / | 0.094 | 0.099 | T | -0.024 | 0.675 | / |

S: represents the transgenic plants showed statistically significant trait improvement as compared to the reference (p<0.05) T: represents the transgenic plants showed a trend of trait improvement compared to the reference with p<0.2

/: represents the transgenic plants didn't show any alteration or had unfavorable change in traits examined compared to the reference in the current dataset

**Limited nitrogen tolerance screen**

Under low nitrogen conditions, *Arabidopsis* seedlings become chlorotic and have less biomass. This screen identified genes for recombinant DNA that imparts enhanced nitrogen use efficiency as shown in *Arabidopsis* plants transformed with the gene of interest that are altered in their ability to accumulate biomass and/or retain chlorophyll under low nitrogen condition.

T2 seeds were plated on glufosinate selection plates containing 0.5x N-Free Hoagland's T 0.1 mM $NH_4NO_3$ T 0.1% sucrose T 1% phytagel media and grown under standard light and temperature conditions. At 12 days of growth, plants were scored for seedling status (i.e. viable or non-viable) and root length. After 21 days of growth, plants were scored for BASTA resistance, visual color, seedling weight, number of green leaves, number of rosette leaves, root length and formation of flowering buds. A photograph of each plant was also taken at this time point.

The seedling weight and root length were analyzed as quantitative responses according to example 1M. The number green leaves, the number of rosette leaves and the flowerbud formation were analyzed as qualitative responses according to example 1L. The leaf color raw data were collected on each plant as the percentages of five color elements (Green, DarkGreen, LightGreen, RedPurple, YellowChlorotic) using a computer imaging system. A statistical logistic regression model was developed to predict an overall value based on five colors for each plant.

Table 13 provides a list of recombinant DNA constructs that improve low nitrogen availability tolerance in plants.

Table 13

| Pep SE Q ID | Constr uct id | Number of green leaves | | | leaf color | | | Root Length | | | Rosette Weight | | |
|---|---|---|---|---|---|---|---|---|---|---|---|---|---|
| | | RS mean | p-value | c | delta | p-value | c | RS mean | p-value | c | delta | p-value | c |
| 334 | 10150 | 1.496 | 0.007 | S | 0.974 | 0.007 | S | -0.285 | 0.022 | / | -0.024 | 0.572 | / |
| 222 | 10335 | 0.993 | 0.014 | S | 0.953 | 0.01 | S | -0.359 | 0 | / | -0.057 | 0.122 | / |
| 277 | 11145 | 0.368 | 0.41 | / | 1.059 | 0.004 | S | -0.141 | 0.211 | / | -0.086 | 0.143 | / |
| 361 | 11735 | 0.522 | 0.554 | / | H32 | 0.003 | S | -0.134 | 0.147 | / | 0.082 | 0.001 | S |
| 206 | 12030 | 0.57 | 0.321 | / | 0.944 | 0.027 | S | -0.28 | 0.005 | / | -0.031 | 0.545 | / |
| 368 | 12189 | 0.134 | 0.791 | / | 0.76 | 0.034 | S | -0.147 | 0.024 | / | -0.082 | 0.099 | / |
| 308 | 73465 | 0.591 | 0.195 | T | 0.586 | 0.061 | T | -0.03 | 0.631 | / | -0.04 | 0.249 | / |
| 328 | 74144 | 0.845 | 0.069 | T | 1.021 | 0.003 | S | 0.181 | 0.04 | S | 0.028 | 0.481 | / |
| 331 | 74417 | 1.051 | 0.021 | S | 1.4 | 0.001 | S | -0.155 | 0.019 | / | -0.071 | 0.16 | / |
| 338 | 74588 | 0.484 | 0.31 | / | 1.059 | 0.01 | S | -0.101 | 0.167 | / | -0.051 | 0.608 | / |
| 369 | 75321 | 0.607 | 0.041 | S | 0.317 | 0.423 | / | -0.095 | 0.238 | / | 0.056 | 0.021 | S |
| 377 | 75419 | -0.779 | 0.198 | / | 0.635 | 0.019 | S | -0.116 | 0.024 | / | 0.009 | 0.795 | / |
| 385 | 75424 | -0.354 | 0.537 | / | 1.489 | 0 | S | -0.245 | 0.003 | / | -0.062 | 0.165 | / |
| 391 | 75506 | 0.969 | 0 | S | 0.588 | 0.049 | S | 0.081 | 0.108 | T | -0.026 | 0.545 | / |
| 388 | 75528 | -0.026 | 0.932 | / | 0.229 | 0.594 | / | -0.042 | 0.534 | / | 0.111 | 0.058 | T |
| 396 | 75544 | 0.197 | 0.369 | / | 1.033 | 0.006 | S | -0.283 | 0.034 | / | -0.134 | 0.021 | / |
| 398 | 75546 | 0.577 | 0.001 | S | 1.108 | 0.002 | S | -0.321 | 0.006 | / | 0.01 | 0.765 | / |
| 390 | 75553 | -0.182 | 0.643 | / | 0.425 | 0.058 | T | -0.104 | 0.12 | 1 | -0.096 | 0.104 | / |
| 397 | 75556 | 0.766 | 0.01 | S | -0.544 | 0.179 | / | 0 | 1 | / | 0.271 | 0 | S |
| 399 | 75558 | 0.343 | 0.406 | / | 0.861 | 0.008 | S | -0.102 | 0.035 | / | -0.106 | 0.003 | / |
| 387 | 75575 | 0.087 | 0.83 | / | 1.147 | 0.001 | S | -0.117 | 0.093 | / | -0.03 | 0.325 | / |

(continued)

| Pep SE Q ID | Constr uct id | Number of green leaves | | | leaf color | | | Root Length | | | Rosette Weight | | |
|---|---|---|---|---|---|---|---|---|---|---|---|---|---|
| | | RS mean | p-value | c | delta | p-value | c | RS mean | p-value | c | delta | p-value | c |
| 401 | 75583 | -0.621 | 0.399 | / | -0.166 | 0.595 | / | -0.01 | 0.906 | / | 0.162 | 0.002 | S |
| 344 | 75834 | 0.425 | 0.038 | S | 0.589 | 0.03 | S | -0.067 | 0.343 | / | 0.028 | 0.429 | / |
| 345 | 75835 | 0.486 | 0.266 | / | 0.228 | 0.499 | / | -0.16 | 0.026 | / | 0.159 | 0 | S |

S: represents the transgenic plants showed statistically significant trait improvement as compared to the reference (p<0.05) T: represents the transgenic plants showed a trend of trait improvement compared than the reference with p<0.2

/: represents the transgenic plants didn't show any alteration or had unfavorable change in traits examined compared to the reference in the current dataset

**Statistic analysis for qualitative responses**

Table 14 provides a list of responses that were analyzed as qualitative responses

Table 14

| response | screen | categories (success vs. failure) |
|---|---|---|
| wilting response Risk | Soil drought tolerance screen | kon-wilted vs. wilted |
| Score | | |
| growth stage at day 14 | least stress tolerance screen | 50% of plants reach stage1.03 vs. not |
| growth stage at day 14 | salt stress tolerance screen | 50% of plants reach stage1.03 vs. not |
| growth stage at day 14 | PEG induced osmotic stress tolerance screen | 50% of plants reach stage1.03 vs. not |
| growth stage at day 7 | cold germination tolerance screen | 50% of plants reach stage 0.5 vs. not |
| number of rosette leaves at day 23 | Shade tolerance screen | 5 leaves appeared vs. not |
| flower bud formation at day23 | Shade tolerance screen | flower buds appear vs. not |
| leaf angle at day 23 | Shade tolerance screen | >60 degree vs. <60 degree |
| number of green leaves at day 21 | limited nitrogen tolerance screen | 6 or 7 leaves appeared vs. not |
| number of rosette leaves at day 21 | imited nitrogen tolerance screen | 6 or 7 leaves appeared vs. not |
| Flower bud formation at day 21 | limited nitrogen tolerance screen | flower buds appear vs. not |

Plants were grouped into transgenic and reference groups and were scored as success or failure according to Table 16. First, the risk (R) was calculated, which is the proportion of plants that were scored as of failure plants within the group. Then the relative risk (RR) was calculated as the ratio of R (transgenic) to R (reference). Risk score (RS) was calculated as $-\log_2 RR$. Subsequently the risk scores from multiple events for each transgene of interest were evaluated for statistical significance by t-test using S-PLUS statistical software (S-PLUS 6, Guide to statistics, Insightful, Seattle, WA, USA). RS with a value greater than 0 indicates that the transgenic plants perform better than the reference. RS with a value less than 0 indicates that the transgenic plants perform worse than the reference. The RS with a value equal to 0 indicates that the performance of the transgenic plants and the reference don't show any difference.

**Statistic analysis for quantitative responses**

Table 15 provides a list of responses that were analyzed as quantitative responses.

Table 15

| response | screen |
|---|---|
| seed yield | Soil drought stress tolerance screen |
| seedling weight at day 14 | heat stress tolerance screen |
| root length at day 14 | heat stress tolerance screen |
| seedling weight at day 14 | salt stress tolerance screen |
| root length at day 14 | salt stress tolerance screen |
| root length at day 11 | salt stress tolerance screen |
| seedling weight at day 14 | PEG induced osmotic stress tolerance screen |
| root length at day 11 | PEG induced osmotic stress tolerance screen |
| root length at day 14 | PEG induced osmotic stress tolerance screen |
| rosette area at day 8 | cold shock tolerance screen |
| rosette area at day28 | cold shock tolerance screen |
| difference in rosette area from day 8 to day 28 | cold shock tolerance screen |
| root length at day 28 | cold germination tolerance screen |
| seedling weight at day 23 | Shade tolerance screen |
| petiole length at day 23 | Shade tolerance screen |
| root length at day 14 | Early plant growth and development screen |
| Seedling weight at day14 | Early plant growth and development screen |
| Rosette dry weight at day 53 | Late plant growth and development screen |
| rosette radius at day 25 | Late plant growth and development screen |
| seed dry weight at day 58 | Late plant growth and development screen |
| silique dry weight at day 53 | Late plant growth and development screen |
| silique length at day 40 | Late plant growth and development screen |
| Seedling weight at day 21 | Limited nitrogen tolerance screen |
| Root length at day 21 | Limited nitrogen tolerance screen |

The measurements (M) of each plant were transformed by $\log_2$ calculation. The Delta was calculated as $\log_2 M$ (transgenic)- $\log_2 M$(reference). Subsequently the mean delta from multiple events of the transgene of interest was evaluated for statistical significance by t-test using S-PLUS statistical software (S-PLUS 6, Guide to statistics, Insightful, Seattle, WA, USA). The Delta with a value greater than 0 indicates that the transgenic plants perform better than the reference. The Delta with a value less than 0 indicates that the transgenic plants perform worse than the reference. The Delta with a value equal to 0 indicates that the performance of the transgenic plants and the reference don't show any difference.

**Example 2**

This example illustrates the identification of homologs of the cognate proteins of the genes identified as imparting an enhanced trait.

A BLAST searchable "All Protein Database" was constructed of known protein sequences using a proprietary sequence database and the National Center for Biotechnology Information (NCBI) non-redundant amino acid database (nr.aa). For each organism from which a DNA sequence provided herein was obtained, an "Organism Protein Database" was constructed of known protein sequences of the organism; the Organism Protein Database is a subset of the All Protein Database based on the NCBI taxonomy ID for the organism.

The All Protein Database was queried using amino acid sequence of cognate protein for gene DNA used in trait-improving recombinant DNA, i.e. sequences of SEQ ID NO: 205 through SEQ ID NO: 408 using "blastp" with E-value cutoff of 1e-8. Up to 1000 top hits were kept, and separated by organism names. For each organism other than that of the query sequence, a list was kept for hits from the query organism itself with a more significant E-value than the best hit of the organism. The list contains likely duplicated genes, and is referred to as the Core List. Another list was kept for all the hits from each organism, sorted by E-value, and referred to as the Hit List.

The Organism Protein Database was queried using amino acid sequences of SEQ ID NO: 205 through SEQ ID NO: 408 using "blastp" with E-value cutoff of 1e-4. Up to 1000 top hits were kept. A BLAST searchable database was constructed based on these hits, and is referred to as "SubDB". SubDB was queried with each sequence in the Hit List using "blastp" with E-value cutoff of 1e-8. The hit with the best E-value was compared with the Core List from the corresponding organism. The hit is deemed a likely ortholog if it belongs to the Core List, otherwise it is deemed not a likely ortholog and there is no further search of sequences in the Hit List for the same organism. Likely orthologs from a large number of distinct organisms were identified and are reported by amino acid sequences of SEQ ID NO: 409 to SEQ ID NO: 19247. These orthologs are reported in Tables 2 as homologs to the proteins cognate to genes used in trait-improving recombinant DNA.

## Table 2

```
SEQ ID NO:  homolog SEQ ID NOs
206: 9461 19072 14856  5315  7726 11678  8466  4567  9541  4039  2736   826
     4434 18755  7163 19021 16621 18594
207:11732  5088 14017 14658  8659 17828 14150 11838  7420   577 13978  3089
     9378  8177  5473  4151 15455  3299  3397 17568 12473  2946 17428  3126
    15408  3997 18806 10067  3246 19105
208:11715 12490 18003 15562  5946 18565  5550 13362  1866  4207  6966  6785
    16958 17740 12495  9774  3632  7059   937 10643 12198  6865 13806  1023
    12708 14552 16746 18201 16712  7735
209: 3105 15418  1368 15360  5654  7902  3038 16108 14416 16444 10078 17851
    12848 15183 12997 18090 18617  1312 16630  4080  4082  7689 19205 17262
    13305  5780 10544 14798 16363 16166 17737
210:17539 13894 15187 11374 17968  9384  7645 13991  5852
211:16101 13052  9940 10298  9899 15964 10104 18651  6853 12619  1551  4764
      684 13219 15864  9975  3261  4237  2710 18739 15477  7248   582 15025
212:11577 10930 12130 13904 13619 10462  9686 10796  9854 12277 14708  4822
      437  6953  7206  9936  2948  7987 13119  2409
213:11281  4744  9418  8607  3777  4299  2607  2319  7251  7279  9577 14778
    18625  6286  4491 14417  1831  7825  8703  3214 17914 10229 11951 17625
      970   966  7114 17802 17749 17813 17788 17783 17787 17765 17808 17806
    17764 17726 17746 17810 17834 17732 17745 17767 17728 17730 17744 17747
    17781  2457 15330 15328  7187  1588 17923  1156  5341 16738 11147 11237
    10534 13932  6444  2913 14896 13736 10170 11594 17581  1429 12223  7798
    16703 10946 10966 10944 10961 10934 11706 11679 11677 11704  3305  1863
    17474 15572  4795  6459   794  8522 15354 13706  8632  5186  8156  7182
     1057 12383 18370 18371 12218 14763  6085  9619  6084  3613 11617 15462
     4013 14623 13880  3030  1260 16762 11244  3082  9189 11858 14221 19035
     2439  4014  1840  8134 16791 14100  8244  8347 15753  6777 10859  2830
    16405  8205 13954 11632 18494 11891  2663  1366 18247 15522 10282 14126
    10940  8802 12556   997  3391  4295  7548  5823 17340  5932  2459 10562
    14935 17104 15135  2045  2256 14248 15748   638 11436 10293 14019 12831
     7142  6143  5949 14114 17359 10556 13121  2699 12459  3820 10865 15441
     5257  8413 14080 10255 12573   772  6606  3642  8804  3457  1348 16022
     7874  5815 15304   598  3258  3259  1401  4197  4837  5770  9990 11860
     5998 13653 19240 18528   798  5714  7362  5197 13400  6454  4954   587
    15889  1391  1208  2577  2564  4704  4774  5289 11843 15452  3750 18789
      950 13589 16248 18822 15416  9549  8091  2839  2840 16123  8406  8410
    15495  1886 15489 18634 15405  8526  8546  8530 11309 10415 12157 12164
    12160 12156 18974  9416 10400  9468  6180  6183 17850 11085  6361  1846
     9141  7414 12282  2648 18663 16836  2696 12925 19084  7529  7271  9600
    16269  8179 15909  8971 14368 11123 15003  7928 18412 13507  8354  2167
     8528  8545 13622 11823  5877  9383 18047 12323  7126 13778 15775  6818
    14349  1843  1841  5467 18619  2511 13117 12441 18799  4847  4844  4846
     1482  1029 13661  9950  7913 18555 13411 12291 15118 13667 11824 17679
    15097 13368  4611 15383  5294  5297  5295 10809  6024 13660 13664 18078
    18063 18075 18080 18064 18061  2901  2896 11461  9567  2623 15619 13681
    16483 19066 18430 14575 17264 14824 16907  3361  3539  9690  9562 13782
```

```
     14476 16298   672  4789 18071 16409 11810  9328 15142 16658 19113 15890
     11168 14282  7337  2737 18782  2977  6021  6936 15780 15779 14381  3119
      8277  5647  3188  5391  2197  8529 16229  4706 11648 18333 12351 10590
      4964 12869 12571 17461 15151  2108 12256 13689  6178 11145 10127 14620
       521 12494  9664 13352 18819 17275 16855  6094 16051  1861  1543 17278
      3184  3785  7977 10869 14020  1073  8390 13292  3685 12532  3942  3338
      7643  5224  7941 17062  4374 17281 15560 18682  6397  9485 11017 18260
     11733  7788  6676  7371 15931  2677  1855  7550 12942 16195  2434 13586
     14216  2671 18036  5925 11102  2387  3968 15553 12886 12211  6446 13317
     15962  2965  6866  2247  2576 18624 13803  8821 12537 18487  2386 15498
     10683  8191 15227 10351 14762 14780 14761  1568  1570  1823 11984 13721
      7268 10834 14940  2764  6670  1577  8050  4950 17318 17533   743  1273
     13143  2621  6532  6553 10775 11080 10240  6827 13829 14551 14376  8122
     11203 11186  9158  4943 11793  4606  4451  4941  4925  4421  4923  4447
      4439 13094 16831  5632 16842  7972  8319  4463 18291  1153  5593 14256
     15648 10411 15778  3836  8646 11057 10802  2547 10675 10705 12481 17144
     11933  9742   760   816  2393 15797 15801  4227 10125 15598 14171  4499
     16812 14907  3633  2579  6096 11005
214:17205 12417  5182 11592  1218 18584  9104  3562 17644 15108  2456 12621
      2781 16359  2519 11488 17691  5523  3592  4365 17685 18564  9255  3981
     17471 14932  6896 17261 14309  4464
215:11565 17980  9870  6731  7560  8022 11032  6899 11020 17043 17521 13209
      6078  3756  6314  4852  7764 17958  8539  3600  2654 15211  8089 10817
       882  2355 12320 10576   857
216: 4548  1814  1811 18872  5005  8334  4062 18447 18595
217:10206  9379 12609  7556 16810 18633  7572  6772  9785 18400  4394  8373
      3586 13142 13290 11170  2118  4015  4419  3154  4988 15178  2746 16499
      7153 17079 12443  9989  7666  3147 16089  8157 15468 13125 13112  1739
     16612 16627 16628  7970
218:14011  6366 12194 19166 14872  8536  9025 15878  5246  1466 10734 18375
     19051  3284 11851  1926 16644 15376 15389 19007 13011 16761 14930 17632
     17097 11908 10938 10056  5151  1836   696 16029 14064  3132 16092 16678
     14296  5107  9978 12711 18145  4922
219: 1266 12114  9582  1006 14040  2995  2574 14277  1487 12000 15871   545
     17801  6330 11820  4719  4718  7544  2089  6943  5642  3726 15195 15394
     15397  4250 16556  6724  9453 12301  8101 15949  7115 16130  3091 15504
      5479  9235 18681 12554  6544  9548 12244 14153 18006 13786  4349   944
     15755  5484 11621  9159  4302   731 19190 13815  4148  1580  2614
220:17643 10449 11152   786  6249 12066 11428 11925  8370  4891 10927  3165
      2073  1089  2824 12422 12197  9475 18563 14844  5667  5051  7973 17063
     16714 12884  8011 16641 16666  6510 19040  4239 14002 16784  2028 10196
     16806 16609 10112  4683  7040 10524 11614 15345  5811  2298  3507 15078
     14109 12786  4216 13136 18305  3837  5287 12384 17153  5425  7719 17446
      5024 14077  1542 14745  3608  6018  7127 18148 18190  1256  9632 13003
      8636  4022 12857 10814  3807 15717  2030 10386 15202
221: 9897
222:16503  6946 12512  8166  7603 13367 17779 17108  5677 12483  4102  3789
      8969  6176  4890  8723  5819 17099  6806 13035 12703 18886  4929  7208
     12867  8233 11471  2758 17866  8378 10583  7058  5503  7152 12439 15584
      8164  7820  9948  5042 10327 12796  9868 16562 10986 16295 19149  3946
      8716 15320 18991 18402  1483 13460 17671 10764  2962 17886 17864 10600
     10129  2817  2842  1695 16096 16759 15370 16173 13739  9388  8737  9863
     10045  2379 17056 18355  1404 10235 18253 14229 15388 10130  2085 12476
      9115  4120  3415  7852 14972 14948 10772 14572  8634  6691  8768 12084
      1558 16813 13074  5000 14208 12827  3545  7743 17653 16255  8780  6849
     13159  2512 17454  5691  2761  6441  8258 13861  2288 18638   679   927
     17856 10134 15581  6881 10442 11642
223: 5413  6647 13564 11787  2900 17371  5719 18538 15525 16814
224: 5509 13877 15894 17059 12524 14960 15820 11498  4900  3665 14166  6804
      1956 10375  3288 15483 14981  7447 12253  3522  5292  8172
225:19221 12606 17232  9256 19165 15129  4321  4677  9193  9192  9191  9195
     17128  4398 15386 16721  7270 15262  9606  9608  6404  3730  4073  8257
      5783  7717 17495 17574 18125 10532  2806  8453
226: 5355 10381 12506  9031
```

```
227:19210 13824 18756  2469 12148 19017  8260  8261 12127 18655  8112  7521
      7032 14012  3050 11148  4438  1566 16589  8148 15936 11007  7676 16727
     17306 14797 11542 14204  3088 11432  2312 18876 16653  8788  7477  1897
     11235  7590  5594 17421  1171  2099 18271  4351 19063 15226 14546 18240
     16886 18163 18160 14050 17165  8432  8098  7178 12519 15335 15336 15559
     18118 13139 11776  1732 18200  2705 15454 14111  7831  1225 17488 14609
     13273 12285 12720   771  1304 17239 18465 10463  6196  5321 10439  1315
     12739  2300 13232  3894  3310 11754 17956 16710  1595  9767 12365 12980
       312   878 19048 14611  1373 10205  3247 15298  7562 10090  3516 17816
     14670  5400  7355  9322  5109 12003 11372  9135 17202  8964 12126 11501
     15692 16891 14540  2285  1520  8928  3021 13685 15478 12561 13562 11325
      3904  3248  7591  8153  8174 11539 18693 12236 10326 16285 14984  7073
      7485 12635 18797 15967 18439 13075  4234  4864 18936  3640 11030  2385
      6954  1768  2406  1951 16202 10053 11969  6069 10741  4310  4346  7301
     15020  7030 13902  3676 15245  7967  2114  9755  9758 11041  1399   607
      3844  5187  1248 15419  5059 16475 12458 11038 14975  2305 10096 10098
     12979 16663  2458  3448 14447  9214  9217  3996  7238 12789  2038  6685
     19174  5335  4231 17456 11781  4994 14877 14271   742 13905 16267 11286
      4963 13433 13634 11742 10897 15826  1081 15615 12163  7022 11209  8104
     13217  1514  1513 11401 13849  9872  3392  7714 17437 13797  3209  5004
     16821 12740 13800 12560  5279  8730  9292  9265  9298  6765  9810  2380
     18328 16833  1946  9995  6746 13050  9269 12948  6484 17397 14869 14870
      6414 17620 14459  2373   434  3656   423  5401 16694 18030  5537  6578
     12369 12850   900 10894 10868  8960 16150 14909  2286 14529 12399 15856
      8705 16311  9271  8715  9294  2423  2855  8463  9779 12614 17865 12958
228:  3536  1432  1435  9457  3524  2486 15369 15566  8392 15107  8262  4848
     11182  1390 12883 12866 16691 12187  8176
229:11285  6998  4222  1194  3135   690  7978 12021 17770  4522 10591  3470
      5744 10394  9316 16338 10498  6985  6465 10932 16071  6165 14004  8007
      1454 18502  4382  3345  8449 13229   482 13158   471 13890 11377 13258
     11770 18675  2967  3041  9750 10249  9108 17334 18037 18728  7830  1674
      1812  3749 10193 14618  2134 13432  4763  4579  7829  2269  2651  7694
     12856 14831 16875  5171  3878 13759  8459 14779 18185  9776 11388 12319
      8800 15396 11006 11865   781  2655  9557  1652 18641 13834   883  1441
     17087 19062  1555 10780  5359 15260 15387  1477 18785 18732  8532  4811
      6656 14777 10943 17228  6869  6289   693 18549 12475 17800 10372 14986
      2345 15774 18087 18112 10403 10425 10356 10444  5049 17881  7747  7751
      2408 15831 15137  2702 11149 10004  6144  4336 14513 13952 14531  6916
     15501 12581  5620  6080 19010 12938  9860 18618 16724  4517  4518  8087
     15741  4127  1790 13173  7904 13171  1511 15876 11013 18888 11027 19169
      7975  3702  2402  7358 18232  9847  1293  3935 18026  5050 12496 11265
       445  8793 17777 18956  3689 15600  4183 12296  9947  6217  2318  9905
     10822 18897 10020  8608  4703 12426  2450 11693 13869  4378  5451 12152
     13395  3602 11015  6386 14969 14971 12677 12679 10108 11200  2362 13275
     13272 10488  1952  9959 13141  1597  9919 13656 18699 16615  9593   528
     11155  2311 12062  6200  7555 16257 13871 11381  8072 17475 17975  9464
     15555 14752 11676  9806  9636 18597 16145 10274  6007   803 11807  2508
      4932 18774 12096  4136  1786 13203 10939   670 18646 16184 18599  2581
     16610   716 14042  8562 14736 14186 13709  2192  8832 11194  4318  7133
     13421  7722 12759  8612  1003 16212  6268  7759 12304 18917 10512 14485
      1335   426 10569 15216   904  8558  5540  5521 18933  8714 16607  1525
      4021  8226 16343 17502  9713 14937  6678 15165  4655  5966  1262 18385
     15085  5600 19146 15327  3657   567 16109  7392  5220  6531  6901
230:  2130  5614 18926 11907 12420  4735  2310  5895 15061  9587  2282 11177
      7927  8439 14508  9449  4903  6372 13595  3196 15544   444  2920  6319
      5298
231:  1909 14373 14375 12706  8598 11874  6536  7394  9724 13690   867  9458
      4523  1703 13763  6958 14051 15346 19124  5833 13480 17342 13749   660
     13092  1043  3787  3666  4743  1789  7649  5971  2220 13980 15157  9574
      5328 11666 10119  5694 17374 18016 12563  2301  5762 11980 19089  1594
     16383 17962   574 10694 10755 11071  8789
232:  8297  5825  6027  9280 10798 11842 13809  8602  3051 16110 15280  5826
      4654  4160 13647 16427  8769 17058 13265 16636 17796  5282 11608 18218
      5544  4639  3751  1980  1599  1367  5678 16090  8359   982   980 15158
```

```
233:13431   7705   4748   2942
234: 6174   6173   5382   5585  13230    416  11225    654  17280  15733  10352   2087
     2106   7696   7472  13351   5940  16730  15960  14680   7112  17267  15783  12008
    15950  18731  18038   6273  19246    440  16299  18152   4005   5505   5331  15201
    15155  17171
235:13748  17177  17005  17507   2003   2731  14085  19141   3528   7773  12053
236:18719   6450   2954   2513  11280   3537  12705   8142   1722  15652   6054   2368
     8633  16862   2353   8396   6821    901   1338   8023   2885   5209   1400  10805
    14006  19175    920  11551   5722  15177
237: 9531  15827  11924  13062  15631  18390  15024  17683   9770   8469    447  16320
    10886   4868   5684  12984  10616  11070   6145  17628   9655  18501  12917  16620
      597  18585  13613  12010   9368  16625   7805  18988  17040   2689  18413  15186
     4542   7399   3101   7259   8108  17966   5070   4026   3314   8938  18997  16673
     3681  10328   5476   7286   2797   2795   6805   4951  10810   6689   3620   2650
    14993    439   5143  11726   8225  12969  13519  18776   3574   3419  10778  17155
    15100   9912    592    462  13348   3653  17555  18894  19069   3737   5311  15989
     6483   2095  11816   5552  11157  18998   5013   1198  11659  14169   1837   6978
    16566  19112    754   5846   9635   3808  13332   9836  10998  10558  14850   3711
    11087   7095   6789  14582   2405  17733   2395   2191  14821  17898  18539  11894
    19049  13683   4869   7423    810  16624  14264  11173   7205   2304  12791  18911
      540   7390  15746   4661  14474  12750  12431   1930  14703  16154   5100  14265
     7779   4485  11790  10166  19098  17981  18351  18316  13483   3429   5764  18602
    17718   5081  16590   2682   9244  15241   1591   6844  19002   1306  17939  12928
    13095   5145  11495   5383   9618   7331   6645   7908   8026  15413  16201  16348
     6937  18476  14667   4293   4973  13840   9324  17032  12167  10184   4904   3931
     4933   3240   1440  13853  10065  14734  11467  11799  16412  15244   3709   3663
     6202   3325  18397    919   1255  17985  17487   8231   9424  16669   6940  15923
    18306   7486   1129   7915  17212  18228   4913   9838  18846  17927  15550   4884
     5386   7487  11184   7359   6942    737   1844  16997   1729  13810   3821  12333
    11543  16481   7489  14980  10354
238: 5969  19041  17447  17839  13044   6002   5996   6451   6051   5841   5420   5975
     3313   5976   6030   4007   6044   6032   6035   7314   5390   5402   5406   5404
     5442   5483   5460   5447   5462   5466   5448   5403   5409   8111   6279   5010
     5457  13908  18485  10958  15764  15541   7610   6194   1287   2415    722   9613
    18552  15880   6449   6448  18427   1941  18981    813  17756  16493  16643   8243
    16018   8285   5367    681  17196   1880  13382  13353   6922   7721   6921  13781
      602  16375  16374   1074  17578  12505  15049  16420  10531   6595  14804   3356
      436   5388   6589  13334   1533   1682  11580  12397  16098  13659   6440   2483
    14084  12530   3910  19087  19100  14923  18518  12688  13533  14541  14664  14880
     6006   6025   6048   6947   6462   6467   6950   6471   6466   6470   5429   5982
    13335   5873  10054   2377   2376   2960   9973   5950   5427   5999   6005  17954
     5077   2687    812    698  11828   7347   3916  16904   8032   5443   5444   6845
     6861    774   6097   7864   6681  14657  14656  14322   2315  12334  11946   9215
    16352  16388   6184  19241   4820  10263   5192   4384  18473  18304   2125  12402
     3477  17630   8856  12634   8795   6223   7518   9472   2061   7998  14430  17592
    13210   4691  11026  13772   2757  14910  10539   1080  12022  17482  17713  12113
     7912  18577  13796  13788   6701   5397   7440  13096  15002  13893   6826   5886
     2947   5423   5973   5468
239: 7931  13758   8114   3373  11499  11529   1416   3738  13929  15590   1947  10552
      663  16209  16266   6195  18471   8560  14830  12408  10330   3753   5574  10312
     5387  10060   2091   2632  16023   6345  13028  16568  16816   1275  13202   4405
     1977   5058   9376  14258   9597  18150  15433   1990   3791   7832  12466  10094
     2631  13168  12715   4562  16993   2448  10742  17882  16751  11056   3456   1427
     1894  13370   1955   1637  15678
240:18268    843  14440  12547    863    849   1380    877    899   5838  17044  18986
     6087   1561   7134  12815  18752   9764  17742  13930  17791   4063   5217   1087
     2750  13402   6192  18436   8296  13072  16555   3399  18332  15403   1358  13201
     4618  12737  12313   2681   6906  12257   5347   9811   2778  18084  13271   3849
    15380   9079   5286  17837  12566  12567  12570  15930  18679   3212  16901   3880
     4979   7632  12905  11429  14344    636  16963  15406  12534  14968  17309   8138
    18401  18515  17014  17565   3768   9667   1496  15404   4486  16528  18777  14536
241: 7389   4712  18984   2834   7240   7425   7411   3882   2159   7263   7262   4838
    15917   7350   7269   7288   7239   1037   8013   6310   7326   7209   7237   7235
     7221   7299   7296   3899  15830  16083   4115   2148   8292  18656   7538   5827
```

```
     14078 13597  7517  7511  7494  7198 11579  7426  7444  3270 15500  8339
       627  1316 19032 11289  6299  1170  3567  3150  3151  8702  7648  7468
     13733 10019 10017 11534 10089  4520 16146 11919 11926 11887 15101 11886
     15102  3432 18952 18380 15422  8333  8284  8313  2075  6604 10245  3095
      7407 10445  2441 10489 10658  7473 15243  4952  7044  3124 16107  2221
      2241  7332  7339  7336  1359  1360  8314 17763  8310 12788  8318  7964
      8340  8343  8346  1962  8847  2058  2413  2416 16080 14542  7363  3127
      8003  7373  7372 15767 12500 16065  6387 14998 11845 11762 11109 15506
      7190  5075  5076  7367  7365 13195  7449  7467  6802  7491  7448  7535
      7520  7513  7533  7515  7290  7294  1975 13363   688 12002   692 12927
     12921  8317
242:  8884 11880  8404 14936 10220 13873 14731 19162  9931   728  7746  2765
      9313 11758 15528 15679  3801  6959
243:  6493  7546 16565  6399  3230 15571 10046 16614
244:  6067 18383 14924  7767   500 11341  8391 18054  9488 14419 15709  2123
      6463  8207 11093 16740  4794
245:  6356 10815  5961  3168 13304  7307  3538  9862 18320 10893 17933  9310
      2801 14818  6864
246:  1702 18677 18678 16723  3824
247:  9993  6350  2350  3660 13279  6773   833 13639 14922  1925 15263 13746
     10150 11999 10994  4619 12040  8110 12045 18224  3989 14287   443 18853
     16144 14510  8332 17924  4224  4989  9778 14496 18885
248:17218  8464 14878  5889  5871  5869  5920  5919  5144 16260  4470 11700
      2569  4480  4481  1067 19234 17408  9777 15352 13852 15549 13123 10984
      7295 13833  5464  7742  7738  7740 16169 11981  1726  5866  5864  5914
      5835  5839  5834  6468 11794  5893 10285  5917  5915  5861  4458  3378
      8006 12101 12540  6524 14197  1213 10988  7939 13889 17151 16020 16044
     13018  6266  9286 14709 12147 15902 18955  7996  3302  1807  3643 15994
       668 15993 11303 11315  5891  5890 11226 14904 10319 10318 10320 10301
     10300 10297 10322 18548 10284  9861 11008  4328  3779
249:  6780  5494  6854 15194  6088  2074 16016 10722 17010  9739  9706  9707
      5452  9678 17017  9722   493  3339  6621   496 10507 18235  8249 10055
     13499  2237 18808  6046  1677 10950 14628  6208 13735 16093
250:  8596 10295  6751 17589 14070  7098  7038  8951  4311 10311 10232
251:  8165 16137 11727 14848 10797  6870 15697 10219  2543 16471 18468   994
      4654 17908 17268  3959  9832  2808 15373  1237  3647 10686 15612 11675
      9784 16308 10978 15081 16090   982   980 15158
252:  5700 10459 14135 16707 10635 10022   652 10461  9410 10216  9602 16193
      6261 16715
253:15564 16365  3189  7229  6136 12380  9773  7880  7495  3648 11586 10617
     15395  5490  7523  7999  7532 16605  6957  8187 15574 18540 10138 10140
      1018  6766  2447  2433  3052  1186  5859  4179  1019  5303  1189 10139
     10143  4305  2399  3428  4306  6822  9455  3149  7484  2398  5380  4304
      9413  1187  6127  1241 18838   450  4874 11239  4893  6917  7500  9358
      9435  1085  4896  8624  6837  8081  4145  1822 10767  3535 15569 10732
     14952 18828 10079 18778  4121 16057 11153 17223  4347 17531 10638 13982
     16515 17774 10902  2175 17000  6764  6335  4406  6128  7811  1665 15653
       504 18289 11587  9507  6050 13490 13492  5006 15225  1047  4835 17918
     11808 17365 17363  6878 19092 18823  2719  9344 10875  5018 14749  5736
     13845   579 12944  2821  3032  5475  5456 16834 16986 14473  1845 18340
     15071   457  2837 15068  3413 14266 17618  3416  2841 16807 18622 18623
     17736  5553  5511 16913  1154 13787   551 16936 15134 16914   599  1103
     15032 14996 14489 14495   424   562   596 19235 16200 18098 18767 13087
     13081 13089  9698  4339 11800 11802 11652  5068  5052  5230  6547  7219
     12394  6405  7222 11028  7663 13663 13083  3825  6548  7001 13106  9236
      8416 10317  5712  5727 12386  9896  5016  7107  3856  2669  7129  3712
      2686 12346  9873  3816  5017  7608  6570 10983  7140  9789  7660 10935
      6850  4358  7421
254:  5008 12646 17688  8974  7014 13777 16381 18947  6884  5695  8070  6494
     18567  5248 10972  8883  2749 17493  5368  4695  2619 17562   565   866
      7934 18910  2163  8893  7103  8331  2771 17230 10464 12038  6156 11251
      6447  7626 12981 17857 18307 13138  7233 10185 14032  2442 10198  7579
     10288  5236 17638  5130 14500  5679  6723 17712 14696 18814  7293  3229
     10783  5784  1563 12227 12937  3202 13523 18544  2290  2092 18131 18458
```

```
        6187   2328   9027   9029   7272   4260  16038  14698
255:13844  13458  16344  13531   8991  10799  11067  14090   4077  19037   2887   8049
   11386  11116  14226   7690  12362   8745   8824   8279   7431   2378   6457    691
     689  15215  10186  18272  19197   7202   2174   1280  10910  15451   2634   5194
    3064  16764   9386  16140   9008   5381  11014   2599  19137   3083   6341   2620
    7439
256:18791   7383   5747  11955   5810  13684   7469   8124   3372   8114   3373   8123
    4217  17629   4204    591   6282   4519  14590  14131   9918  18109  18122  18107
   18123  10592   7481   7463   2612   6371   6375  10073  14990  11339  14608  11060
    7879    630   4828   3591   6406  15590  11021  17060    790   5787   2670   6720
   15939   2455   8857  13434  11738   3008   9102  17680   1196  10417  13536  14243
   17599   2902   9828  18056   5234  17355  18761   9371  11729   4637  10566  16772
   12408  14108   9500  10312  12735   5387  17976   5569   1191  15171  10790  17721
   17700  17675  17672  17720  17696  17677  17654  17719  17668  17704  17642  17702
   17698  17652   6324   6380  17768  15277  15321  15302  15275  15326  15324  15305
   15301  15283  15308  15236  15306  15329  15255  15271   7606   1292  14494   1603
    4524  11636  15988  18060  15311   2602   8083   5114   4690   3719   4672  17429
   17430   4233   8801   6584   7117  12873  15833    907   1575   8208   7089   6782
    2275  10256  15480  12342  11054  17184   9375   9393   6297   8043  18829   3281
   11962   9376  16594   6181   6879   6185  10213   7891   5849  12308  15458   2082
   13755  11977   5283  15433  15434   7662  18141  18135  18166  18146  18143  18313
    5892   1829   2536  10168  15018   3116   3117   4803  11056  11602  18167  18179
   18178  18198  18180  18177  18199   2128   4574   6875  10412   6321   3049    560
257:10236   1778   2762   8305  15457   1517  18065   3918   4604  14043   1834  15769
   10542  13088  16947   9842   2117   1780   1783   6887  10786   3092   3074   2425
     518   6885  18899   4918   8216   4901  16336   4899  16103   7720   3649   4873
    4866   4875    628   1283  15291  13148   3611   1217   9366  15616
258:17425  15853   8859   9683   7091   5216   2201   2205  11262   3346  13482  13109
   13107  13108   9794   8281  14641  13444   2715   1066   6137  12970   6142   6140
   17183   6163   6141  17181  17192   9831   5159  13051  14297  10948   7031    811
   16445   7171  15854  13126    710  17296  17319  17285  17286    873   9312  16119
   16120  10141   6168  17224   4466  14934  13246  14008  11772  10397  13068  13455
   17650  10953  14295   1735  10585  13288   8496   6041   7961  19038   6612   1629
    6017   6013  14627  11526  15297   4441   6040   6148   9420   7099   8711  19132
   11098   3943  10624   6800
259:12322   4714   7090   7343   3817   9397   4548   3010   2769   9109   3414  14347
   13708  13372   5943  11868  11465  10063   4752  17817  13361  13360  13357   5105
    5573   9024   6424  13872  13868   7092   4717   4716   7087   6575  17611  18568
    4570  15716  10227   8510   7634  10157
260: 6150  16463   4354   8132   5126  13216   2066   7809   7718   2988  12642  13615
   10029  17953  14902  16838  18868   8290   5596
261: 2464   6735  15726  18074   4533   7642   7488  10955  10687   7802   3503  13093
    8940   2616  12104   8987  18900   3879   8561   4110  16434   2625  16297  10309
    4244   1026   8472   8897  17846    740   3692  12517  16930  18079    655   5583
   14539   3696   8399  11724   9362   5698  10595  14766   5215   4170   9716  19244
    7813   5862    585   4135    648  19138  11445   2739  10042  18353   8100   2019
    2690  19052  12373  13945  16595   1140  12664   1560  16857  13624  12453  13808
    5173  13766   6628   9260  12260   8687  18121  17463   1120   7797  13969  17724
    5314  16826   2063   1088  14372   9553  17994   2673  16245   6149   8458  19093
    7324  10811   7710  17194  17284  12321  12033  10898  10152   9002  13471   5605
    8885   4972  13637   9833  12092   1700  19012   2081  13874  11373   2852  11365
   18154  11821   9406  17339   4707   1102   1166   1787  11844   5166  10551  12580
   19135   3752  15624   1063  14987   1535  17889   8739  13192   2052    610   1714
   10124  18536   8139  16889   5258   2981   8679   4369  16639  16037   7893
262: 2033   2767   3670   4459   4432  16170  10905  16867   8606  18942   7697   3565
   14407   8288
263: 1035   9303  17522   3596  16423  10242  10744  14550  18944  13113  10874   8143
    8831   7585   8109   1310   5138    850  11127   7958   8986   2453   2560   4205
    4004   4675   4673  10345  17998   1374    821   8649   8481   9682   7380   6431
    3360   3374   5754  11483  13329   1203   1340   1339   4399   3283   6668   6381
    6241   7284   7512   6230  11328  15128  12214  10951  11193   6497   2195  16892
   16809   4053  15715  15901  16798  11263   1719   3634   2158   5478  15076   1257
   13172  15036  14456  14458   3438  18769   2703  12701  18877  14893   6253  18425
    3403   3914   5746   4245   5745  17084  15381  17772  15379   9309  10296  12410
```

61

```
      5703 15992  3780 18405  2278  4687  7254  4645 15181  9150 10286 18596
      6188  9199 10890 10879 15662 18680 17535 16335 16334   564 15325  9790
     15285  2090 15282  6549  4833  1118  7329 18041 15620 16076  8589 10725
     17516 14852  6421  5690  5688  5119 12105 18454 13900  6564 14418 14619
      4297 10339 10360 10639 12762 12763 12741 14511 13719 10909 10773 16790
      9869  1727  6880 10960 11304  7110  5769 13197  7056  9945  5261 17830
     14774 14773 12199  3137  5595 14014  2941  2957 17451  9205  2466 12448
     15051  3366 18339  9670 13214  1239  3486 18561  7055  9712 10923 13525
      8438 13359  2418  9400 11234  2070 18866 15848  7243  7241  2726 10370
      7457 14298  1838 10174 11329 12913 10405 11972  3408 12813 15659  8843
      2880 13850  4880  4385 17717 17265 10460 11220 15829  9685 15344  2178
     11324 12998 18296 15580 12407 18636 16217  1071  4776  2384 16294   517
      1409 13377   442   855  3498  5899  7009  3369  9524  7955 17042  2463
      6860   797 13680  1818  8229  6189  1892  2482  5086 11387 12139 19047
     19046  2561 11674 16039 19016  6993  4118 16629 14564 16309  2735   832
      4286 11133  1920 16941  7793  7847  3846 12352  3027  2187  7317 11751
     16395 14634
264:  7571 12286 11656  6312  7806 13041  4308
265: 13716 16282  3622 16128  2432  5091  7256  5094  7466  7464  5092  1341
      1344 16034 16318  1345  5493 10131 13605 14704  4059 10144 19195  9113
266: 16179  6376
267: 10695 10080 15222  3336 16070 17906 15461 16778 11541  8321 14840 16441
     14461  3078 16553 14929 14615 18225  1799  3781  9074  1362  8894  9856
     13182 12587 11210 16207 19053  9477  3450  8475 11378 10077 18659  5908
     11662 10239  3546  9196 11788 16729 12687 17436 12472 14335 14313  3350
      5657 13623 11806 15691 18889  5828 12962  7549 17152 14791  7328  7327
     15844 10068 11042 19158 16711 15570 16355  8068 10996
268:  2886  9957 17982 10633  7244  6714  1461  2113  6871   836 13502  6798
     15168 18396  3234  2200  9380  5880  9621  8447  9787 12043 15448  9694
     13218  4705 13753 14029  2998
269: 10688  4111 17553 10437 15096  5485  9680 10385 10653 11747 16874 14594
     16211 14686 17690  8241  4055  7427  7803  1987  4186  4214 15651  1363
      8864 11780 13023  7633 12224  6490 13751  8037  3589 11702  5195  5909
      2222  9907 14842 17686  6624  7566  3583 12297 12724 14468 10207 18644
     10608  1622  4841 16104  1605 16551 18689 12343  9207  2436  3822 16818
     10642 18674  2352  4626  1675 17661  3134   617  2708   961  3106 10629
     14827 11171  6053 12518  7784  5323  2580  9759  1709  2179 18964 14835
      6852 13452 11718 10486 13090 18057  3263  8170  8744 16373  1654 15378
     14170  9209  2584  3407   889  1439 10830  1585  4816 16025 12545 13281
     13346  2590  1758  9650 17819 18091 16148  3389 17615 14976 10238 13875
      2611 14065  5696 12279  6074  6729  2745 19185  5681 16920 13388 14163
      8813  3618 14834 15087  3721  1485  9949 10283  7215  3309 13415  5439
      4469  5933 17289 11789  6384  5731 10264  7605  8842  1099 14475  3909
     13435 15200  7527 11012 18453 15198  4161  4104 18753 12971 17172  9631
     12235  8497 13245 18019 16873  3661  4577  6121  6122 17843 14241  6559
     15677 16369 18284  6292  1387 19123  4430 18460  6119 14240  4809  5037
      3176 14246   822 19212  3604  8652 11848  9111 17377 14244 10482  5532
     14726
270:  8430 11246   888 19061 13527  4203  9211 18507 12612  6037  9813 12222
      4042 17351  7357  2554 15576  2530 17209  1254   553  6291  4213  8660
      8211 13160 12189  4353  2593 12910 18282 14806 17244 10015  2741  7736
      6107 16064   536   683
271:  4877 12594 12592 12593  1168 17709 14622  9708  3469 12557  7679 13757
      6458 11104 10895  7774  6856  6232 12922  3157  8983 11307 15719 12415
     10416  6486  7002  1957 16783  5624 16422 11181  7291  1626  1648  5699
      2204   719  6203  5597  6211 12836 12810 17491 17492 18863  8852 18169
     17979  9998 10497 12247 18972 16293 11825  9133  5644  7508  4196 18115
     16165 15640 12480  1900  2109  1803  1697  5219 12900 13417 18843 17971
      2072  9088 12825 17178 18422 11750 10836 11653 13491 11689 11447  7592
     19119  3201  3872 19058  6475   860  5497 10226  9099 13710 10051  6443
      8235  3185 11456   806   429  8818 10483 17327 13496 11075  4592   412
     11434 10429 11433  7422  7409  7408 15322  7907 13817 17105  7191 17711
     10243 15851 13418 13147  2556  3866  2791  8273  3086  3070  3072  5538
     11777  6320 16611  6100 14771 16623  3786  8242 18082 19027 13724 15983
```

```
      1891  2867  7775 11619 13324  6616  6618  1125 18094 17722 15364  6683
      2157 12555 13024  3783 14194 14316  5306  6778 15785  1282 18433  3128
     14481  1991  8973  5580  5591 11725  2566 10376 13740  6263  1971   808
      3748 17701 17699  6514 18566  6515  2929  3160  3162  8065 13297 13642
     14227 10467  9564  4460 17984 13310 14903 13295   458  5622  3047  4732
     12237  4733 18448 14026 12245 17550 13325 16872  6622  9208 11356 10084
      4865 11454  4461  5057  1856  7815  6736 15122 17560 15649  4241 14979
      8033  2588  2812  2571  4071   964  2367   963 12429 10641  3853  6277
       489  5174 11089 13091 10627 15133 18593  3984  9394 10678 10661  9365
     14707 10023 10677  9319 15093  6963 13933 15000  2932 18188 14891 18189
     15816 13816  5516   671  5424 15377 10753   661 15431 12258  3839 10438
      5562  6313 12903  3897  7885 12208  9401  9143  6513 14927 16596 13242
      5450 11198 18338 18696 17858 17860 18786  5393  7594 12681  3631  2024
      2005  4693  1526  2397  8766 11511  1179  1723 15556  5205 10095  4713
272:  2476  4605  3590 17269 12552 12550  7501 14751 15392  5592 11796 16372
      4149 18640  6540 15725 18637 18044  9775 14074 17762  9431 16817  2976
      2041 10920 10918 10919  6246  6245  3529  4129 11740  9275 11166  2997
      7120 16741 14429  4680 14300   930 18404 10446 13276 14192 17400
273:  7563  7558 12847 18712 16770 17497   544 10669 10670 17546  5362 18031
     10396  6596 11136  3476  3497   622
274: 13296  2414  7482   715  5029 17139  8523 12280  5587  9163 13837  7094
      3044  4280  2153  1112  4738  4010  5324 10529 10800 18319  7203  6138
      5128 10413 17368  9596  3799  5190  8551  2711  8694 12467  5167  4009
      3862  9114  6874 16580 10234  6060 13741  8220  7478 11407 13856 11295
     15070 13298  4415  1606 18172  6499 13961 14716  3655 10066  2800  7266
     18464 16192 12428 17016  1810  7183  9659 15622  4254 18781   824  3874
      5645 16717 15897  2604 11084  9641  9704 10541  3208 13670  1672  7074
      5735  2600  5765  6912  5346 13728 13703 12240 12238  9145 15565 11169
      1405  9756 15771  5179  5706 15772 12901  6709 14021  4168 18206 11626
     14139  2779   886 11568  1437  9403 16000 16756 12529 13059 15915 12623
      3252  1720 12209 16495 19227  9883 18208  5924 11326 13150  3307  8365
      6995 12798  4949 14471 13560  8423 12166  9082  5041  6058 19128  7756
     17440 14799  2510 13503 10992 12818  6613 11175 12853   793  2299  8291
     12461  7179 18106 14045  2984 11966   741  2565  2532 11685  3928 10924
      8919  1372  7471  6106 13429 13236 18258  6786  6823  2446  3040  3042
     14676 10735   724   727  9601 15533  1649 10137  4857  4842  5365   644
      6812 10912  3603 12030 14912  8616  9170  2322  3245  3250  3260 12840
      5264 16328  8655  6283 13350 10698  8590 10697 11885 11195  9476 14668
     15473 11567 12864  9709 14945 12215  4742 14584 16884 16447  1996 10199
      2734  6807 13557  8666 15469 17395 14955 14176  5518 18711  2827 13154
      2898  5322  5319  4739  8890 17300  4495  5063 10549 13137  4195  1854
      2527 19139  9946 15252 12776  5280 10043   935 17588  4373  9663 17287
      8521  6169 11290 10749 17136 17121  7881 14438  4888  5759 16329  2927
     15532  1013 18288 17804 13542  8044 16866  1327 12341  4863 16757  9526
     15323  6503 10634 17375 10795  1116 14645  6760   893 10543  5316 17304
      5733 17142 14098  8524 12290 10516  7883   909  7984   778 14722 16684
     12239  3006 15438 17394 17665 11558  8342 18264 14928 14478 17344 13381
      7085 13720 18912 17156  2264 12779  1586  5275 10989  5818 11896 11897
      5619 10840  5543  1650 10582 11709 16690 14269 14810  4431  4788  4790
      7944 16870  7387  6769  7960 12491 18360  5026  6658 16937 12538  4612
     10580 18193  3979  8422  3818  8505  8507  8504 17778  1759  8816 14646
     11797  4240  6897 15192  6066 11963 13198 18204 18963  2573 14426  7752
     15906 16059  1220 10047
275:  6567  7596 11364 11453 12046  5398 13331 16182  8916  1973  5797  2518
       891 18583 13323   733 12250  8328  5812  9967  3368 10248  3335  3895
     11052   890 13631 12434  2940 12421  5281  9331 17926 19188  6236 17460
     16804  5671  5831 16672 15497 17552 11625 14132 11383  9972 12803 11064
      4584  6770 13599 15368 17312  1216 10021 16522  2292  1932  4386  8493
      8247  6504 15981 15979  5650  5649  5636 17849  6811 18134   763  6640
     16829 15593  7070  4330  3192 14604  8972 14445 17869  2903  6742  8271
      8817  6090  5296 15391 13676 16526  2983  6105  3619 16242  6828  2683
     19079 14386  8407 13407 14384 14377 14357  3028 15348  4817 14803 10025
     13175 13161 14681  6147 13177 17575 17576 14449 17873  4915  3262 16036
     17573 10706  4912 13077 18586  9900 18940   711  3290 16031  6781 18658
```

```
     18668 18670 18671 18666  9878  3171  3207   880  3843  7249  3754  4372
      5498 10341 13017  6682  8822  8212  6976  1984  7674  1906 14209 10975
      9849 16316  6726  3249 11988 11991  5817  4766 10578 10662   430 14671
     17412 15883  5437 11773
276:  6415 11627 16734  2790 12959  6986 10082 18111  1354 12820  3964
277: 18650  5609 10555 10768 10537 17527  6952 15558 10041 18763  7499
278: 15266 15482 12044 12553  4614 11192  2672 11183  9823 14522 16221  3544
      6281  5112  1760 18159  8238  8228  5482  8190  1965
279:  6567 11364   749 11453 12046 10052 17706 18365  8385  5398  1544 16182
     12144 16111  3341  9565  9725 15117 15657   533  1137  2518  1887  1885
     10842 13590  3994  2630 16766 15463 13036  9551   936 11557 18445 17217
      9331  4033  4622  9786 11206 10471  4352  4386  1932  8493  8247  6504
     15979 15981  9941  8839 15593  2313   916   918  2006  7070  4330 18529
     10148 12565 10731  4682 10251 16953 15509  1699  7405 16425  9783 10979
      5799 15687 18301  1950  7096   910  1135 16009  3792  3289 14462 14467
     14464 17453 13343 13384  6719 13341 13729  2729  3534  2831 16392 12337
     12350  6742  7072  7075  8817  6944  7570  3680 13593 12535 18281 19020
     11696  4598  8914 14214  4159  4105 15044 16776  5558 16175  4191 15013
     12168 16055 10803  2983 16242  6105  8407  9666 12514  4363  6781  8770
      9878  3171  5652 17141  6726  3249 11991 11988  5817  4766 10662 17219
       430 14671  4886 16660  5437 11773  5761  5410 18705 13903
280:  6173  6174  2365 17259  4411  4410  5588 11225 15721 14985  7696 11506
     13351  5940 10368  6787  1959 12498  2454 16341 15783 18676 11293  4855
     14524 17078 10180  5505 15201 15155 17171
281: 17893  7011  6207 11190 17567 12492 19233 19215  4300  7003 18070 16733
      1554  9581   726 13534  2346 14727 18724 10857 13387  9212  9168  8573
      7609 18442  6632
282: 18444  2704 17326 11744  5133 10947  2939  7795  9151 17727 19011 18210
      7640 17186 13914 16210  7796  9182 16289 11073 10329 18021   486  3056
     19189  2823  2718 19238 18251 17572  9916  7688 13457 19033  5682  5161
      4926  8080  7018  6841 11443 11095  9499 17065  7800  7601 19208 15218
      5336 11076  4603  5978  5434  4035 12125 16835  3501  5152  1055 16745
      4798  4689  6204 11348 14326 16719  1911 13223 11698 14123  2728 17489
     15170  7007  6500 18519 18845  2992 11872 15371 12170  5290  1076 11637
     15888  1378 18303  6977  8150 12792  5180  4001 10701 11105 11240  6832
     13140  8860  9478  1883 13213  8435 17266 17026 13464  9672 18431  3659
      8631 16129 14941 14947 14446 11292  1493 13419 17608  7564  6391 18516
     10217 13189  2412 15180  5510 18242 18310 13799 18244  2324 14518   605
      8344  5123  8185  1105 10401   467   762 13128  6945  8993  9326 16082
     11795  5213  5623 10420  2991  2993  2096   960 12805 15251 15269 12807
     12808 15270  7006 12219  3037  4391 12138 16426 16377  9381 17166
283:  1002   570 11876  2649 17648  4380 18089 12995 10728 17836   473   498
       497   494   479   478   546   532   531   456  1015  5353  5378 15904
     12957  2173 17947 10011  2928  8192  4334  8436 10105 16670  8736  5369
     14463   773 19068 10713 10613 14516 10631 12652 10059 18613 15333  6327
      1206  4265  5431  4488 13563 11252 14434  2444  4287 11202  4967 15204
      6609  6389 17214  9049 11603 12082  4333  5394  4878 14480 15253 13831
     15728 16916 16176  5417 13947   492 14257 12736  5030   809 15363 18256
      6671 14781 15290  4190  9653  3334  3142 11578  7479 14674  7636  7429
      9011  6705 16995 13053  8444 18496  3690  3684 12996  8128 16753 11423
     14784 17995  8105  2055  1197 18721 13278 14156 17631 16351 11812   921
      6316  6511  9688  8236  2236  1666  8183 14222 10270 10871 13354 10281
     14643  9877  8303 10404  3508 14918 11058 10754 12023 19160 14926 12436
      7311  1532 16945 16640  2936 12181   940 15494  1817 14016  6809 14081
      5214  2516 10962 12360 14278 18013  9804 13658  9805 16113  1205  8511
      3525  3575  7878  7199  7600  1982 16131 16501  2291 11620  3695 16984
     14292 18773  7186  6323 10513  8845  5757 18101  8446 16588  5206 18358
     17179 16606 15686 12142 10606 12316  7617  1093  8519  8670 17382  3800
      2626 13178 17695  1423  1940 18278  6601  6598  6593  6300  9545  9495
      9511 16384  2953 18423 13134  1744 13300  7493 10787  7818 18632 14702
      4730   543   542 15813 15825 15773 15794  7790  7792   449   474   476
      1020 12896  1634 10792 12939 15517 17601 14591  2504  2344  5239 13365
     10738  5272  3907 12787  1422  1424  1426  1425 19071 14066 16496 11686
      5098  8250  5868  9903 15732  4474  5641 13540 13553  4126 13784  2325
```

```
       7406  2261  8915 18702  3635  9611  8644  7577 12242 12269 12246 12271
      11172  5545 15259 17564  4679  8798 17419  4927 18557 12287 12288  5930
       5928  5923  5935  4130 13063  2794  1707  4094  1716  3417  2027 11025
       7823  7826 17538  6576  6577  6574  5948  7932  7947  2012 12569  6675
        828 12106 11826  3708  2515  5501  5922  5901  5897  9808  3022 11269
      11272 11268 13793 17409 14651  1584  7567 17116  9026  7438  5942 11535
        435   507   506   508   510   554  8989  8959  8956  3505 18140  9977
       8791 16310 17659  5330  7514  6280 14200 16736 11690   454   452   549
        513   525   529   526  1017  1016 14261 17867 18881
  284: 9361 16893  9841  9508  9489  9510  8548  2151  9737  8904  9699  9733
       8878  9765  8911  9771  8935  4737 11143 11141  9599 17965 17946 17964
      17930 17871 17945 17942 17925  2429  2430 17944 17921  2431 17872 11759
      11211 11749 11746 11767 11233 11249 11099 12527  7839 13474  6634 12615
      19181 11446  8245 13314  7012  9594 13153 10676  2100 18285 18354  1169
      18730  7863  8452  9676  8923 14136 13408  4155 15234  6994 16883 12775
      11442 11441 11444 11418 11402 11403 11421 11420 11424 11425 18440 14044
      10044  1907 16100  2578 17862 18275 14037  7727   902 14299 11996 11521
      11993 11519 11525 11522 11500 11520 11502 11990 11487 11503 11497 11496
      11462 11466 11459 11468 11463  1731  6352  9028 10971 14876 17905  1007
       3912 18214  2424  5743 14161  3251  9701  4116 13262 16196 16194 14886
      15430  3744 15496   538 12595 12454   572 14919 10603  6393  2474 18937
       1350 12992  4791 14059 10593  6231  8906  1357 17622 13397 12455  6425
      13336  4445   601  2279  9629  4564  4514  4515  4530  9668  9673  3298
      18324 14202 14217  7836  7995 14054 14052 10447 13260  9743  9740  3296
       6847  7835 18362 18346 18368 18348 18361 18344 10389  5830  9745  9744
       9734  9738  5898  5896  5900  5959  5956  5951  5954  5902  5904  5931
       5934  5906  5905 14576 11634  3067 17147 17162 12627 18342 18367 10707
       6343  6340 11120  7435  7434  3295  2856 18352 14219 14223 18326 14205
      14206 16199 16161 16312 16181 16174 16205 16159 15704 15701 16135  6905
       6403  6367  6979  6939  6931  6908  7028  7027  7051  6935  6930  6982
       7088  7050  6996  6910  7084  7081  3301 18321 14207 10407 14201 16085
      11852 11841 11830 18849  4290  8356 15621 13592  9691 10911  8256 17163
      17159 17160 14938  4909 19243 12355 11757  1229  1231  9627  8814 17187
       9822  9486 19151 12596 19152 13551 13607 19142 19154 12590 19153 12628
      13627 13610 13570 13567 13569 13632 13644 13645 13629 13651 13649 12617
      19196 11827 11786 11801 11798 11782 11769 13633 11124  4276 15799 15730
      15776 15759  9072  8275 14415  3276 15817  9692  8865  8908  9741  4544
       9671  8863  4594  4597  4541  9647  4560  8820  9077  9633 13601 18798
      18794 10819 11333 11311 11342 11337 11336 11334 13814 15375  6867  6502
      12768 13425  8239  1903 13934 13960 13956 13936 17174 15112  3232  3233
       2359  5824 10492  5266 14180  3722  5850 10430 10432 10427 10408 10410
      10414  5844 10426 10468 10452 10453 14379 10525 10547 10553 10570 10577
       6810  6794  6790  6796  6813  6833  6815  6831 17566  6190 10237 10088
      17358  9958 18903 18833 18520  4587  4590  4536 13045 15942  2775 15428
       7650 17148 17157  9287  4268  4253 18883 13477 12390 12613 19171 14402
      19173 19177 12629 12631 19156  1669  9637  4258 12862  9649  8837 13571
       9843  9513 13603 13584 13583 13588 13587  9818  9516  9480  9481  9795
       9793  9484  4065  4038  3526  3520  3492  3518  3504  3472  3523  3459
       4043  4036  4066  3558  3557  4020  3556  4019  3540  3542  2968  4034
       4060  4032  4947 18852 18851 11998 12013 12035 11997 12057 12055 12014
      12039 12019 12034 12017 12011 12061 12037 12060 12041 18856  4270 10450
       6432  6429  6422 16725 16742 16701 16705 16743 16722 16706 12395 12378
      12376 12326 12381 12345 12382 12361 16700 16681 16685 16682 16680 16136
      16698 16133 16561 16598 16586 16564 16584 16578 16540 16538 12357  9768
       8934  9702  9730  8881  4272  4257 18859 18873 18871  4259  4273 18858
      11353 11352 11350 18834 18836 18875 18902 18901  9735  8903  8876  8902
       8877  3588  1869  7078 16399 11481 11480 11485 11484 13012
  285:11893  9361 16893  9508  9510  9841  9489  8548  2151  9737  8904  9699
       9733  8878  9765  8911  9771  8935 17925 17965 17964  2430 17945 17942
      17871 17930  2431 17946  2429 17872 17944 17921 11759 11211 11767 11749
      11746 11233 11249 11099 12527  7839 13474  6634 19181 12615 11446 16547
       8245 13314  7012  9594 13153 10676  1169 18285 18354 18730  7863  8452
       9676  8923 14136 13408 13475  4155 15234  6994 16883 12775 11442 11441
      11444 11421 11420 11402 11418 11403 11424 11425  1305 18440 14044 10044
```

```
        1907 16100  2578 17862 18275 14037  7727   902 14299 11525 11522 11496
       11993 11500 11503 11521 11519 11502 11996 11487 11520 11990 11497 11462
       11468 11459 11466 11463  1731  6352  9028 10971 14876 17905  1007  3912
       18214  2424  5743  1204  3251 14161  9701  4116 13262  4502 16194 16196
       14886 15430  3744 15496   538 12595 12454   572 14919 10603  6393 15683
        2474 14250 18937  1350 12992  4791 14059 10593  6231  8906  1357 17622
       13397 12455  6425 13336  4445   601  2279  9629  4564  4514  4515  4530
        9673  9668 18324  3298 14202  7836 14217  7995 14054 14052 10447 13260
        9743  9740  3296  6847  7835 18362 18346 18348 18368 18361 18344 10389
        5830  9745  9744  9734  9738  5898  5896  5900  5956  5959  5951  5954
        5902  5904  5934  5931  5906  5905 14576  3067 17147 12627 18342 18367
       10707  6343  6340 18321  3301 14207 10407 14201 16085 11852 11830 11841
        8356 13592  9691 18195 10069  9922 11349 11618 15487 10182  3804  4909
       19243 12355 11757 10954  9627  8814 17187  9822  9486 19151 19152 12596
       13607 13551 19142 19154 19153 12628 12590 13627 13610 13632 13570 13567
       13629 13569 13644 13645 13651 12617 19196  4986 11389  4987 11398 11827
       11786 11782 11801 11798 11769 13633 11124  4276 15799 15759 15776 15730
        9072  8275 14673 15817  9692  8865  9741  8908  9671  4597  4544  4594
        8863  4541  9647  4560  8820  9077  9633 13601 18798 18794 13814 18690
        6439 14133 12768 13425  9224  4509  2132 10382  1903 13934 13956 13960
       13936 17174 15112  4944  1133  2359  5824 10492  5266 14180  3722  5850
       10430 10432 10427 10408 10414 10410  5844 10426 10468 10453 10452 14379
       10525 10547 10570 10553 10577  6810  6794  6790  6796  6813  6833  6815
        6831  6748  6749 17566  6190 10237 10088 17358  9958 18520  4590  4587
        4536 13045 15942  2775 15428  7650 17148 17157  9287  4268  4253 18883
       13477 12390 19171 12613 19177 19173 14402 12629 12631 19156  1669  9637
        4258 12862  9649  8837 13587  9513 13603 13584  9843 13588 13571 13583
        9516  9818  9480  9793  9481  9795  9484  4065  4038  3526  3520  3492
        3472  3518  3504  3523  3459  4043  4036  4066  4019  4020  3557  3556
        3540  3542  3558  4034  4060  4032  4947 12061 11997 12035 12013 11998
       12055 12057 12019 12039 12034 12017 12014 12060 12011 12041 12037 18856
        4270 10450  6432  6429  6422 16725 12326 12395 12376 12378 16743 16742
       16722 16706 16705 16701 12381 12345 12382 12361 16681 16685 16682 16700
       16136 16698 16680 16133 16598 16586 16564 16561 16578 16584 16540 16538
       12357  9768  8934  9702  9730  8881  4272  4257  4273 11353 11352 11350
       18875  9735  8903  8876  8902  8877  1869  3588 16399 11480 11481 11485
       11484 13012
  286:  2777  5039 16750  1471  3285  2150 10618  8803 11961  2889  6846  3739
        1995  3694 17476 12331 12602  7992 10443 10584  2551 12607 18523 16013
        7530  4332 17943  9036  8140 14652  9799  6082  2538  5800  6597 12897
       15296
  287:  9566  5643  6213 14305  9062 12764  1510  7131 14437 14832  5009  2243
       12243  6637  4770  3936 13672 11255 11221  5921  7391  1432  1435  9457
       15566  8392  5977 14724 12745  2394 16446 15400 12866 12883  3287  7541
        6534  7866 15513 14892
  288: 15365 18498 13541  7184  7109  1034  7848  1309  1974  5407  4483  3120
       15279  6672 19131 14159  8869  2364 10622  4552 13626 10133 10574   461
       17086  6452  4595  3599  3294 11158 18667
  289: 16368 12503  3630  9835 13104 15863 12124 11301 13962  8408 17039 12299
        8002  7757  7854  2421 17941  9614  9944   947  7876  3401  2851 18366
        5872 12894  5554  6523  3967 10214 17229 11034  6907 17354 15997 17279
       15293 11343 12782  3434  8664 10980 11284  7151  5262  5760 15952 13577
        8200  5778 14238  2958  3987  6408  4512  4532  4535  4513 13926  8393
        2633  2640   913 15415 11965 16677  6715 13715  4773 19199  8085  1519
       13439 15432 14319 13712  8738 11125 16975  6895  8732 12851   477 14392
       15611  4147  9651  6491  6488  3806 13714 17754 17750   460 12140  3732
        1981  2760 18374   632  8844  2235 13441  8846  6661  6659   865 14395
       14433 14958  8575  7966
  290: 11915 12531 10674  5108 10673 10671  5110 10656 15342  7321 11367 11854
        5888 14613  5002  7871 18610 18248  5952 11256 11273  1008  9456  9482
        1098 17829 10769  8759 15842  9296 11737 16394 15796 11222 12888 17948
       15606 15608 13316  8168 13652 16659 15073 13630 10782 17520 17523  7375
       12579  4555 16775 18273  8782  8833  8828  5913
  291:  9661 14871 18815 14431 12470  9259  5881 17640  2549  5199  3645 16654
```

```
       14864 17074  1797 10867  4767 18221  9718   751 13915 18164  9703  6358
         739 12122 15132 14974  8785  2372  6430 14127  1550 18042  7607  1574
        8968 14165 14000 12131 19081 16106  2645 17386  6251 15629 11509 17303
       11118 16393  9726 11174  4106 10619  5469  2895  7622 19078 15694  5576
       11721 12733 17199 15873
  292:15315 12929 13897 13194  6285 13409 13879  5211   428  4840   623  9603
        9165  2034 13804
  293: 1857 13794  6221 18932 10387 11856  4252  4256  4255  3425 12892 11901
        8638  8630   978  5301  5141 15472  1119 11213 13891  2603  8637 12207
       16699 14669 12027 15111 13878  2754 11266  9938 10663  7917 17855  2691
        8215   717   483  2996  8158 13042  3905 19024  3005  9180  3573 10211
       16275 10831 13060  6460   704  1245 17402  1160  1753  4132 11888 18985
       16323 17693  7097 11035 15312  1997 18441  5708  7627 16437  8154  1791
       19031  8274  3741  8747  6445 12846 11110 18117  7167 10833 15412 17468
       10824 16088 12354 15985  6684 17751  4202 14270   885 19125  8213 14055
        1676 14412  4092  4079  2784  4093  2239 12447 18014 14252 18350  2529
        3662  1065 15060 10364 16853 15185  2643 18058  3747  1498  1443  1881
        8360 12988 11224 12576   631  3784  7653 10644  8103  2707  7143   952
       11597  6171 18896 19187  2242  9521  9760 15027  6679 15641 11298  6919
        1036  2296 18614  2154 11537 13573  3099 10710  5656 11644 15999   973
       13920 19091 18890  2891 18318  3629  1252   879  5997  1961  7982  7936
  294: 7105  3084  1994 18783  2497  7816 18184  3440 14124 18751  1967 15867
       14672  7886 17222  1877 17983 17378  9387  3723  9501 16163 14128  6267
        8384  5802  8872  3745 18948 14939  2793 16550  7047  2103   612  1522
       15922 13547 13079 15943  5970  5968  4359 17714 10659 16490
  295:11915 12531 17198  7368  2744 18649  3181 14613  8489  5002  7871 13313
       18248 12632 11291  6927  1098 17829 10769 11161   488 14181 16980 14805
        3254  2964  9225  9296 14276 11739 15796  7315 11804 15606 15608 13316
        5763 12456 13652 16659 10739 12059 10782 17523  7375 10346  8720 16775
        2653 18273  8782  8833  5913
  296:  862  5137 16001 12941   770 15554 18605  8082  3087  8425  2773  6680
        6816  5218 18416  5855  1839 14492 16795  3595  1910 13985 10118 12109
        7502 14989  5062  3113 10472  7870  1136  3790  8761  8515 14301 13855
        5798  7675 15995 13529  1460 10887 18807 12633  7553
  297:  438 13973 13308 11956 16007  7173  7175 12391  2026  8512 16573 16216
       11162  7158  2209  9879 16890 10061  9391  8377 11208 13866 15446 10737
       16951 18861  3386 14949 16049  2268   422  2498 12158 11196   999 11422
        6909   971 17103 12945 16236 14279  1815  5256 15390   801  4660  4185
        5533  3055 15173 13105  8055  2461  5801  2810 15573 17102 17242 11720
       15557  6135 14995  2480 13243  5433
  298:11915 12531 15342  7321 11367  5888 14613  8489  5002  7871 18610 18248
        5952 11253  1008 15996  1098 17829 10769 17874  4262  6648  9296 16112
        2214 16394 17950  5329 18025 16063 17293 15606 15608  6546  4664  4292
       10730 18652 12515 11405 18158  3549 13330  3902  6434 11902  9414 17888
        2240 17635 14946 12828  8168 13652 16659 15073 13630 10782 17520 17523
        7375 12579  4555 16775  8782  8833  8828  5913
  299:  414  1917  1934  1936  1364 14900  8480 12103 17664  4215  5090 10064
       10748  4581  3710  7147 15265 14787  5241  3993 17960 12064 10702 17494
       13847 14233  7728 11629  9459 11576 12484  6071  3597 18443  1876 10502
        6159  6154  6160 17140  7783 10647 17310 17311 17393 17332 17314 17372
       17366 17388 17349 17364 17345 17317 17369 16206 17390 16927 16906 16839
       16226 17418 16925 16825 16190 16228 16948 13935 13163  2803 17357 18002
         410 14420 10711   853   839  6111  2440 15718  4166 12588  1480  8764
       11330  3359 17935  6269 13179 14129  3075  2639  2657 15001  6623  6619
        6649  6642  5607  7804  6646  9579 15233 12379 18050 17729 14558 12543
       12546 14954   409  5200 10554  7507  3337  7801  2712 11045  6157 11716
       12255 12259 11719  1915 13946  1212  1210  1236  1240  1242  1771  1769
        1800  1777  1796  1794  1802  1269  1805  1244  1773  1272  1247  1775
        1267  1264  1261  1214  7754 12783  1502 14776 17545 17503 17499  6363
       14199 17682 16929 17526 14196 17540 17684 12050  2170  2176  2149  2147
       17258  2145 12029 17252 12048  1642 17294 17292 17282  2873 17336 12024
       13998 11552  3881  3451 17233 14483 14506  3978 14486 14482 13999  3939
        3887 14507 14487  3940 11528 14013 13996 13997 18688 18722  6334  5664
        8553  2931  4402  4401 15288  6179 17089  7845 17088 17055 17067  6182
```

```
        7247 17064 17465 17481 17469 17467 13921 13899 12068 12069 12063 12065
        9522 13942 13924 14829  5375 12544 19216  5610  7450  4163  9575  7246
        9628  9059  4165  6857  6838  8944  7667  9634  7668  9652  7101 12424
       15341  5621  5608 11722 12248 11687 11714 12217 11695 11691 11697 12220
       11694 12249 12212  9731 12216 11723 12210  5724 15847
   300:17992 14813  6116  9580  2888  7234  8357  9926 14330 15460  6476 11921
        8487 19217 12340 14973 18168  6988 14092 13614  9589  1042 12559 12558
       10145 13807  3131 12159  7765 17049   491  5373 16006 13937 16869 16844
       13943 13939 13917 18662  1028 18302  8760  8210  7918   968  6155 18132
        4210  4212  4208   465 12388  1139  2520  7618 19214  6643 11734 11547
       11050  7539 10093  8107 10903 12707 10224  4971 14365 16659 18553  9554
        5668  1601 16122  1462 10321 11228  9914 18521 17523 16603 16599 16600
       14253 10841 12283 13811 12709  5758  3941  3385  9514  9509  8569 18996
        9826 15445 15986  7285 18975  3467  9261 16796 16794 15896 15895
   301:10900  8457 13754  4644  5428  3481  1939 10756  4267 18389 14849  6797
        2161 10604 16911 13860  9866  9857 14350  3615  1201  1403  1653 19242
       15740  4248 18572  5142 13938  7102 11968  3015 10490  2894 18449 14314
       18543 17977 17076   425 10771  5506
   302: 6516  3244
   303: 9157  6322 11978 11130  7319  6594 12072 12604  1630 15835 15625  2667
       11873 10353  7250 12387 18220  5040 16397   744   682 13620 15592  3376
        7637  5327 15172  9136  3454  8680  3406  2563  8386 15628   583 17951
       16220 16241 15828  2354  1711 18844 14933 14606  8084  2951  2023 12230
        1131  1022  7453  6888  3757  3490  3410   965 14448 10758  3736 13099
        5418  3869 16033  5419  9116  5463  9118 14280
   304:13016  4501 12800 15199 17376 16048  7771  6495  2349 13559 15944 16686
       11650  4749  7034 12604  1630 12213  1041  8874 12774 13164 15543 16462
        2893  9060  8653  3035  6124  2097 11863  3682  3447 14809  7584 15021
        3973 13598 10693  4630
   305: 3217  3571
   306: 9517 16283 17490  7415   537  6886  2849 12335 14505 13819  6814  6115
       17752  9736 18191  8424  2155 19176 17694  8682 15351  5379 11881 11938
       16662 17579 11271 16360  7659 16032  9586  7670   675  6708 13311 11396
        4758 17299  5191  7346  8937 16585  5606   502  8374  3761 16767  3107
       12145 13731  8754 11287  1587 17321   939 11556 13038  2216  5067 17391
        5842  5661  9584 18461 17532 15765 12358 17023 16827   854 15019  3746
       18406  3158  4638 16647  9956  5285 10517  6010  8267  3102  8875 17859
        3533  5163 14049 12819  8830 10422  1545 11903 18692  1408  5782 10419
        8743  2507 14022 15821  4156 18202 14875  3437  2493  5683 14057  2774
        2307 11546  2862 18609 15789 16720 12528  4710  7020 14477  8362 15861
       12266  3664  5927 10107   911 10257  9367 12658 15086  2329 12591 10770
       17651 14336   501  3598  8283  9930  7604 10652 19129  5069 14901 15868
       14717  1446 11406 12649  3069  5305 17167  2725  1528   841  7652 12267
       19206  6331   417 12854 18813  2969  4834 15665 14583  9988  3933 12960
        8189  8063 18363  9630  3955  8707 13020 13437  2029  7702 16563  9892
        9894  9909  5345  4993 17674 14807 17748   923 13039 12302 12305  4593
        6333  4582  4585  4504  4505  4507  4525  4528  4580  8892 16476  3071
       15617 13247 16828 13307 12568 16763 13025  4662  9544  9547  2848  8455
        1875 17554 14725  6339 12637  3861 16635  9893 16631  9377  4729 18714
        5265  7136 17773 19222 15795  6317 17731 13215 15661  6496 15845  3683
       18173  8985  3238 16239  8182   847  8939 13251 17330 15347  1090  6654
       16830 14533  1434 10162  1307  1608 12047  9254  4623  6733  9845  3976
       11791  6023 10860  8387  8388  3436  1289  4200 11245 14746 18697 10596
        9834  4867 18801  4048  4500  4051  4125  4041  6284 16362  6481  2203
        3253 17931 15047 13987  1184 17227 13858  6911  1625  7441 15207  4112
        1349 17655  3809 17405 15646 11605 16230 15136 12284 11241 11022  9598
       16189 16789  8351 11101  3405 17442  6972 13910   898 13694 11524 12489
        6077 12564 18758 16224 10614 12449  9505  4294 14642 15832  1908 11003
        9272 12730 18514  6417 17206  1004   475 12462 19223 18691  3383  6603
        6725  3889  2521 17328   842 17029  3484 17880  2854 17158 11122 15188
       19211  2820  7370  5183  5883 12698  4454  7017 10197  5646  9715  1157
       12651  7968  7684  1763  7207  2196 13537 12099  2202  2253  3365  8144
       13004 16497 15811  6265  2124  8582  7976  7231 18294  6438 17932  6485
       13284 18660 16797  5384  6617 15439 18738 18562 13641  3118 11948  3760
```

```
        5015 11023  3770 18364  1660 19130  1072  4534 12826 18768  2417 17234
        2882  8417  9871  5837  9911  9132 11165  3873 13863 17480  9515  4131
        6588 18170 16222  4230  5085  9852  8230  8227 18403  9997  2267 16180
       18524 17253  7957  4709 14254  6788  4558 15464 10901 12863 16622  6999
        4559 19065  4408 16389 18716   942 17799  2335  4291 14534 15459  7048
        9917  8849  9747  6004  9915  4583 16306  3700 14454  3960 12902  9166
        9769  2495  9891  9890  3163  4044  9913  8502
   307:16966  1919 12583 14001 11131  6894  6752 19213  6877  6738  5028 11478
       12673 16234 15971 15875 12694 12533 13333 11870 12183 12169 12146 12093
        6307 12143 12171 12089 12074 12123 12073 12137 12118 12117 12051 12665
        3465 18929  8042 12488 12878 13761 17647  6876  6840  6272  6835 11243
       10315 19201  9766  4261 15639 16402 19042  7713  7791 16291  6973   721
        1355  7033 11065  9853 14782 18575 14554  3292  6118  8034 13958 13312
        1115 12766  8654 12755 10949 16650 16061 13056  2685 18534  4047 11361
        8443 17255  5349  1847  9017   446  3511 12923  4315  3637 10479 18867
        2225  9308 11987 12463  3000  5517 17776 14688 13231 15599  7869 11941
       17438 16655 15176  6229 11914 18483  8880 14025 14630 18182 17877 15768
        4778 13925  8338  5136 14825  4905  9898  7630  1639 11601 17037  2945
       10431 17797  9885  7574 13621 10863  9055 11151 11755  7946  9333  6329
        4591  4091 13981  5065 16803 14822  6964  3463 10454  2177  9360 11197
       13234 15420 10808 13424 10292  8329  2865 13338 13339 16185 10202  8955
       15242  8322  8223 16632 15869  4551 10418  2231  3358 17915  9080 15089
        6338  2733 11560 12364 18802 12721  4025 10092 15814  3303  6611  7498
       13931  1868  8572  9825 14345 10581 10007 18356  8308  6981 10752  8173
        2904  2938 12670 15524  5073 14565 14327  4609 17256 18506 11581  9098
        6914  3347 13049 18254  8152  8151  1224 10181  2869 14649 12087  9901
        7952  1465 11039  2613  4283  3706  5396 10832  3362  5953  8848 17193
        7462  8895 14087 12914 18645  2396  9188 10926 11609  6271  7379  3493
       14614  3502 19167 13184 11475   419 18267  8970  9234  6938 16418 13285
       18292 18269 18212 10727 18238 12508 12510   709   315 17725  8434 13254
         861 17182 12639 13959  6309  2860  8674 12332  8623  8648  8678 12309
        8647  8629  8697 18930 18017 15456  9239  3499  1491  3279  7455 13389
       18685 13086  1576  8896  4057  3140  1215  8021  8040 11229 16223  4381
        3805  7364   313 11613 14318  7069 18729  4708 12085
   308:17723 13349  5426 10342   646  8286 11681 15655  7162  2974 11778 13876
       18386 10114  3206  2802  8806  8486 14598  6551  3387 18415  4506 19086
         720 13013  8851  6410 18004  1501  6469 11711  8962 16976  6226  1146
        8776  5079 13857 14566 18277  2698  3433 15273  9154  8961  1782 12468
        2069  5555  4356 15859 12450 17580 17519 12015  4117 12311 12833   903
       17841  3317  8289 13436  3400  4303  5816 15343 14218  3390  2829 15319
        5048  4640 11046  9300 18120  5536  5686 10941  8685 12753 15582 12001
       15643  5455 16989  2119 14187 11932 18938  1334 15886   938  4813  3811
       10654 15534 18497 14811  1552  2661  5080 15338  6315  5225 13561 15213
        1490 14525 15884 12359  6669   955 18171  5358 14624  2813  2542 12020
        1749  1631 12016  6303  6949  7842 16125 18957 16094  7410  2120 16642
       12078 11363 10559 12794 19168 13662 15228 13544  2606 18630 18727  5929
        1497   976 11871  3924  5803  1809  7834  2156 15294  9809  5613 12582
         819 10715 15196 13261 16533 11473 12028 18392  2674  4882 18532  3509
       14873 10440  4142  8651  6824   745  9875 16149 17563 17373 18734 18230
        8224 13813  8272  3398  4907 13315  4016  1330 18116  6568 17201 11312
       15066 14302  4800 18992  4086  4549   309  6162  5244  5565  1899 11667
       14224 16251  7386  6416 14759 14889  3100 10072  9146
   309:17723 13349  5426   646  8286 11681 15655  7162  2974 11778 13876 18386
       10114  1104  3206  2802  8806  8486 14598  6551  3387 18415  4506 19086
         720 13013  8851  6410 18004  1501  6469 11711  8962 16976  6226  1146
        9010  8776  5079 13857 14566 18277  2698  3433 15273  9154  8961  1782
       12468  2069  5555  4356 15859 12450 17580 17519 12015  4117 12311 12833
         903 17841  3317  8289 13436  3400  4303  5816 15343 14218  3390  2829
       15319  5048  4640 11046  9300 18120  5536  5686 10941 12753 15582 12001
       15643  5455  2119 14187 11932 18938 15886  1334   938  4813  3811 15534
       10654 18497 14811  1552  2661  5080 15338  5225 13561  1490 15213 14525
       15884 12359  6669   955 18171  5358 14624  2813  2542 12020  1749  1631
       12016  6303  7842 18957 16125 16094  7410  2120 16642 12078 11363 10559
       12794 19168 13662 15228 13544  2606 18630 18727  5929  1497   976 11871
```

```
        3924    5803    1809    7834    2156   15294    9809    5613   12582     819   10715   15196
       13261   16533   12028   18392    2674    4882   18532    3509   14873    4142   10440    8651
        6824    9875   16149   17563   17373   18734    8224   18230   13813    8272    3398    4907
        4016    1330   13315   18116    6568   15447   11312   15066   14302    4800   18992    4086
        5244    5565    1899   11667   14224   16251     308    7386    6416   14759   14889    3100
       10072    9146
310:   10103   18209    3920    6083   11927   11920   17472    4786   17648   10273    6306    9064
       11347    7551    6834    6260    7334   11100    5786   17161   16591   12957   15792   17989
       13513   14343   10276    5369    3379    1500    8808   10294    1398   10594     823    5710
       18613    7856   14705   13453    6480   18733   15333    3235    4488    6348    1223    5579
         469    6158    2323   15204   12704    7135    2864    5709   13549   16473   12094   11847
       15189    7404   18021   16376   15728   17758   17759   10289    5408    2799   11658    9568
       17803    1830    1347   15363   15824    3963   16520   17045    2659    4190    7035    7889
        1332   11043   18965   13554    4100    4076    1488   17195    7119    7429   14837   14242
        8145    8222    8282    6690    6705   10469   14943    3690    3684    6191   16970   14567
        1302   14760   10891   10888   14125   13193   13805    9451   12868   17957   11774    2050
       15443    2709    1596   17631   11812    4600    3958   17798    2617   17937    6512    3483
         753   10957    6419   16465    9968    9604   17150   19182    9662    7557    3962    5101
       13495    9576   17734    4975    3891    6104    8565   17439   12438     852   18005   14679
        2768    3555    7354    6809    2693    1475   19143    2516    5214   12911   11562    3868
        5630    3865    3819    5007    6948   14549    2971    7275     459   19164    5974   12430
        5655     967   14881   17890   17331   14820    7963   10759   18574   16972   18773     837
        2068    2133    8845   19013   19054   12054     659   17185     640    3444   14093    6479
       14245   10973    7988    6296    3530    8812     464   16581    3767   15891   15332    1402
       13224     796    5729    2002   14571    6300    9442   18423   12656    9032   10645   15161
       11805   15637   12696    5239    8059    7922   17929    6756   13692    3094    1298   11589
        8861    3514    3093   18298    1301    4779   11321    3090   13713   11607   19071    1621
       18085    5907   15732   13127   11970    1178   14188    9969   12336   10649   10172    6305
        4760    2694    3243   15374    8000   18588   13076   17379    6898   10609    9061    3321
        5901    9808    3022   19029    2112    2759    9640   16516    2738   15881    6161    3225
       17681   11663   14225   11836   18207    6210   17844   11690   11113    7824   10588
311:   16284    3606   17484    3724   12088   11103    5785    8336    4133    5435   12206   18097
        7672    3473   10039   15998   16601   11983   18950   16664   16546    6636    7838   10509
       16277   12119   13264   14523    7959    1624    6101    6558    6991   17341    3713   18780
        6732   14843   10839   11368   11376    7124    2198   11419   10982    5660   10500    4144
       13648   18842   16054    4831    5477   19155    1618    5333    2295   10956   16508    1271
         635    5739    7739   16017   14157    6758    9889   14914   12966    7210    3982   17122
       10837   12989    6556    2697   15231   12324    7578   11435    3445   18211   15350   15035
        2143   11631   16505    5245    1415    1572    6829   15515   15116     827    1386    4407
        7692   12842   18133    4853    9605    5531    3036    4948   16400   12572   17847    3054
       13870   12907    7292   10290   17401    2526   15417   16488   10985    3934   18892   19082
        7937   16671    8368   18511     818   13398   14650   10501    6270   14970   12668   13951
        7076    9820   11096   14699   17676   16290   17907   15362    5385   15054    3855    5338
       10212   17090    9087    2224   12811   12933    4815    8067    9429   13643   15267     687
        6819   12318    2260     485    6093    9503    3108    7687   10087   17708    3704   11310
       17316   18100    2782    3166    4676    7310   10111   10921    2460   16998   13700    1896
        3890    3893   11460    1365    4697    5147    8506   17646   12460   10187   13390    2371
       13266   12499   15209    1819    8062    1842   16386   16407   12516   17315    4818    7619
       12585    6346   16345    9939   15062   17660   18237   18639   16965   18174   11756    7709
       12790   15507    5527    5421    5857   13673   19101     922   13675    2047   15119   17607
       15523    5604    2583    2462   11728    3180    3264   15426   15516    9555   10457    2383
       10519   14033
312:   19210   19017    2469   12148    8868    9122    8261    8260   12127   18655   10968    4278
        7032   14012    3050    4438    1566   16589    8148   15936   18723   14802   11007    7676
       11542   14797    3088   18432   15023    5711    4424   12601   17203   15601   15229   15790
       10278   11432    7413   18876   16653   11235    6737    1684    6956    9399    1897    9520
        8790    7451    8788    7477   17421    4351   19063   15226   16886   18163   18160    7887
       17356    9131    9840   10435   14050   17165    8098   15335   15336   18118   19218    2079
        1732    2705    7831   14111   14609   17488   13273    1225   14741   12285   12720   17703
        3155    1304   17239     771   11531    6196    6407    6374    3679    4189   11047   18086
        4317   16026    1757   18381   13635   10439   11504   15742   17243   17247    5416    8996
        6566    6509   14737    1315    5853    7602    1598   13609   13378    6003    6112   13287
       12739    3813   12645    6983   15316    2300    3894    3310    6507   18804    5203   18754
       11533    4836   17956   11754    9830   16710   12891   13671    9767   12365   14611   19048
```

```
 1373 17645 10205  7562  3247 15298 10090  3516 14670 14578  5400  7355
 7287  6657 12597  3186 12801  9322  2963 12003 11372  9135  8891 18576
 8964 12126 15692 16891  1520 15478  3021 13685 12561 11325 13562  3904
 3248  7591  8174 11539 16285 14984 17248 15849  1431 17593 14701  7485
 6569 18797 13075 11030  4234  1951  2406  6694  8640 11969  4529 17591
 1573  2001 14631 16366 15065  3460 15627  3676  7030 15020  1278  7967
 2114  9755  9758 11041  1399   607  3844  5187  1248 15419 16475  5059
12458 11038 14975 10096 12979 16663  2458  3448 14447 17143  8069  8074
 9214  9217  3996 12789  7238 19174   227 12457 16704  5335  6029 17456
 4994 11781 14877  4963 13433 13634 11742  1081 15826 15615 12163  7022
11209 13217  8104 13738 13750 13742  1514  7714  3392  9872 11401 13849
13797 16821  3209  5004  9810  2380 18328 10246 13309 16833 16143  1946
 6746 13050 17027 12948  6484 17397 14869 14870 13608  7545 14441  6414
11135 15223 15074 15007 17620 14459   434   423  5401  2373 18030 16694
 5537  6578 12369 12850   900  8960 10894 10868 14909  2286 14529 16150
12399 15856 18280 17002   516 15539  8705  4792 16311 11664  8715  2423
 2855  8463 17865  9779 12958
313:16966   643  4364  1919 12583 14001 11131 11866  6894  6752 19213  6877
 6738  5028 11478  5031 12673  6883 16234 15875 15971 12694 13333 11870
12533 12089 12074 12093 12073 12051 12146 12143 12183 12123 12665 12117
 6307 12137 12118 12171 12169 15777  2586  9343  7157 16204  4467  5936
 5848  2944  3465 18929  8042 12488 10936 12878  5308 13761 10847 17647
 6876  6835  6272  6840 11243 19201 10315  9766 15639 16402  1251 19042
 8345  7713 17138  7791 14235  9974 16291  6973   721 14514  1355  7033
11065  8783 18451 18470  9853 14782  8294 14554  3292  6118  8034  8326
 7882 13312  1115 12766  8654 11936 15710 14341 12674 12755 14585 15809
10949 16787 10621 16244 15680 15675 16650 13056 16061  2685   925 18534
 4047 16841 11361 15666  8443 17255  9128  5349  1847  3511 12923  4315
10473 13515  9149  9152 10479 18867  2225  9308  5129 11987  3000  5517
17117 17091 17739 13231 15599 15256  7869  7260  1005 16655 15176 11941
17438 16917  6229 11914 18483  8880 14025 14630  1176 14629 18182  5047
17877 19064  2724 15768  3835  4778 13925 15822  8338  5136 14825  4905
 9898  7630  1639 11601 17037 11036 15239  2945 10431  7289 17797  9885
 7574  4849 13621 10863  9055 11628 11151 12401 11755 16894  8403  7946
 9333  6329  4591  4589  4091 13981 15919 11572 11657 18700  4138  5065
  470 17414 16358 16803 11665  6964 14822 10703   307   844 19122  3975
 5260  3463 18429 10454  2177  9360 11197 14283 13234 15420  8184  9953
17197 10808 13188 13424  7005 10292  8329  2865  2866  2403 13339 13338
 1290 16185 11883 10202 15242  8955  8322 11191 11212 18103 17940 17254
 8223  6379 16632 17934 15869  2503  3636  4551 10418  2231  2872  3358
17915 15089  6338  2733 11560 12364  5805 18802  9140  8445  2502  3312
 7828 12721  4696  8008   872  4025 10092 15882 15814 12371  3303  6611
13977  7498 13931  1868  8572 14345  9825 10581 10007 12149 18356  8308
 7036  6981 10752 14493  8173  8136  2938 12670  2904  6932  5073 14565
 2037 14327  4609 17256  2721 18506 16138  6028 16225 16203 16156 16198
 9121 18322 16246 16178 16172  9129 16219  9117 16243 16158 11581  3347
 6914 13049 18254  8151  8152  1224 10181  2869  9901  1465 11039  6971

 3706  2613  4283  5396 10832  3362  5953  8848 17193  7462 18645  2396
 9188 10926  6271  7379 19167 14614  3493  3502 13184 11475   419  2246
 1541   420 10820  1865  1864 19193  5121 11566  3364 15356 16339 18267
 8970  9234  2382  6938 16418 13285 18212 18292 18269 10727 18238 18293
 6362 18215 18239 12508 12510   709  1152   315 17725 17697  8434 13254
  861 13959 17182 12639 16406 12542 12541  6309  2860  3570  8648 12309
 8647 12332  8623  8674  8629  8678 18930 18017 15456  3499  1491  3279
 7455 13389 13086 18685  1576  8896  4057  3140  1215  8021 11229 16223
 4381  3805  7364  3228 15465 11613  9609 14318  7069 18729  4708 12085
314:17506  3672 14034 18715 14894 10880  1849 16469   512 10254 10470  5726
14472 10171 14406  3718 16079  9837  9339  5104 17771  5508  5020   834
17380 15334  5096  4999  9288  8295  5472 13752  1964 16799  2401 13069
13691 13422  7593  2540  2717  5882 11638 13697 17811  5581  4456  1381
15933  2472  7620 16823  4482  2452  7400 16262 17807 18192 13790 18757
18673 10586  4772 12650 18934  3174  5720  2083  2874  4669  9314  9839
 9762 10310 13888  5627  9147  8405  3728 11400  6409 15786  2828 15900
```

```
  3340  3474  1459   892 13842 16047 11176 15514 13638   969 12485  7833
  7242 10503 11553  4417 11411  1986  1221  1227  3527 18941 12683 12977
 12129 15069  5885  1534 16021 15292 17543  4538  2792  5539  5519 17904
   876  3164 18558  1300 10402 13522 14027 15033 18919 18259  4068  2850
 17343 19026  4479 10493  5549  1874 18434   645  9644 14808  5548  6344
 15331 13114  3446  2644 17210 13825  5662 15153 16313  7693 12780 16574
  4686 11440 14816  4338 16346  1636 19236 17410 10101  7456   991 12872
  3699  4028  6992  9962  4496  3842 16910 12042  2255  7046 17485 15064
   817  8460  4635 12802 15113  4751 14557  1668  6768  8195 14547  2541
  6164 13760  1788 12403 15927 17057  1507  4337  7148  3237 19060 16254
 13277  9223 15237  9177 15837  2341 10611 11649  2838 18156 12666 16249
  3138  6863 17584  2129  6563 15963 14009  3652  9433  2262  2534 13801
  3231 14588  7872 11469 13974 17240 11323 14501 16931 12006   441 18417
  2918 12097 12837 10016 18066 15815  3716 13070 14839 10827 10253 14272
 13944  1870 15148  9697 10347 12080 19157  9054 18345  4503 12292  4395
 16938   463  7974 14120 16183  3471  4782 14865 15284 15286  6396  8031
  8017 18816  9981  9980 11274 17812 14966 10272  3420  3624 16040  3020
  1155  1159 13046  8442
315:16966   643  4364  1375  1919 11131 11866 11486  6894  3621  6752  5028
 11478 12673  6883 16234 15971 15875 12694 13333 12533 11870 12169 12183
 12171  6307 12665 12143 12137 12123 12118 12117 12146 12074 12089 12051
 12093 12073 15777  2586  9343 18929  8042 12488 10936 12878  5308 13761
 17647  6876  6835  6272  6840 11243 19201 10315 15639 16402 19042  7713
  7791  9974 16291  6973 15084  3115   721 14514  7033  1355 11065  1512
 18451 18470  9853 14782  8294 18575  3292  8034  6118  8654 11936 15710
 14341 12755 14585 10949 16787 15680 15675 16244 10621 16340 16061   925
 18534  4047 11361 17255 12923  4315 10473  9152 13515  9149  9357 10479
 18867  9308  2225 11987 12463  5517 17117 17091 17739 15599 13231  7869
  7260  1005 11941 17438 16655 15176 16917 19150  8880 14025 14630 14629
 18182 17877  2724 15768  3835  4778 13925  8338 10373 14825  9898  7630
  1639 11601 17037 10431  7574 10863  9055 11151 11755  8403  9333  6329
  4591 13981 15919 11657 11572 17414   470 16803  6964 14822 10703  3975
 19122   307   844  5260  3463  2177  9360 11197 14283 13234  9953  8184
 10808 13424 10292  8329  2865  2866 13338 13339 16185 10202 15242  8955
  6473 11191 11212 18103  2638  8223  6379 16632 15869 10418  2231  9080
  6295 15089  6338  2733 11560 12364  5805 18802  8445  2502  3312  7828
  1747 12721  4696  8008   872  4025 10092 15814  3303  6611 13977  1096
 13931  1868 14345  8572  9825  5794 10581 10007 18356  8308  7036  6981
 10752 14493  8173  8136  2938 12670  2904  6932  5073 14565 14327  4609
 17256 18506 16219 16178  6028 16243 16138 18322 16158 16225 16172 16203
 16156  9121 16198 16246  9129 11581  6914 13049  8151  8152  1224 10181
  2869  9901  1465 11039  4283  2613  3706  5396 10832  3362  7462 18645
  9188 10926  6271  7379 19167 14614  3493 13184  3502 11475   419 17288
 16086  4069 10820  1865  1864   420  3364 19193 18267  8970  9234 16418
 18292 18212 10727 18269 18238 18293 18239  6362 18215 12508   709   915
  1152 17725 13254   861 13959 12639 17182 12541 12542  2860  6309  8648
  8697  8623 12332 12309 15456  3499  1491  3279  7455  8896  4057 16223
  4381  3805  3228 15465   313 11613 14318  7069 18729  4708 12085
316:18488 18007  3371 18022  6240  5577  1141  4858 15264 13008  8036  7857
 16648 14599 16141 17352 11264  2922  7855 12100 16569  4670 11835  7156
  2615 16602 15671 16213  1068 10487 18989  7745 13625  8276 11591 15968
 18537 13558  1106  2391 17916 11931  7844 18020 10668  5894  2814 16160
  4671  4553 13144  7837  8429  9643  9229  9330 12172  9648 15361 12367
 18376  8325  3269 16474  6652  2059 10267  2067 10188  3795  3794   974
  5364  7376  8980 15893 16484  2807  2826 15414  9081 10784  3221 11000
 16877   358 18878 11643 14101 17431 14104 11922 14106 14118 14103 11917
 14117 11877  9156  2076  7599 18286  4992  7700  9492 17313 12079 14348
  4084  9494  7706  1297 17046  3961  5911 12363  8462  8465  1521 13528
 11661 18323 10495 18481  1858  9034  9035 18850  7483  6754 17200  7821
 11939 13965  4088 11316  6817 17715 11701   788  7905 18810  5360 14586
  1033 14950 16858  8889 15056 15910 11640  9983 15575 11391 17626 17605
 17606 17623 16231 16151 16155 16095 13014 16069 12870  3257  6401 17505
 17504 17525 17524  1679  2836 11399  7377   432 16819 10332  2411 10331
  2410 12098 12865  3223  1027 13770 13767 16781 16777 16779 16801 17741
```

```
       16774    4860  15510  12777  12778  14071  14067  14053  14069  14091  14072  14075
        8376   11001   9987   7853   5184   7393   7395   3219   3220  17517   3203  17996
        3205   17513  10899   3204  18474  18023   9302   1882  17692   3594   3098   1993
        6587   16786  17678   7822  18795  14425   2040  14424  14895  13347  18509  13344
       18011    3992   4426  15577  11803  18607  12719   3019   2751   1413   1611  15567
        5584    4242  18484  18027  18467  18028  17973  17972  17970  17969   7432   4113
        4887    4360  12444   1657   8005  17457  10146    563  18067   7398   4018  13922
        5489     530   1353   5529   6153   8586   6257   7997  19219   8567  12135   5520
       18325   18513  18472  11430  15382  18181   7353   7349   7351   7348   7218   7214
        7201    7224   7220   7200  17333   2490  10136  15058  19237  15045  15907  16396
       16398   15912  15057  15636   2471  16403  15079  15075  18489    363   8942
317:   10700    2789   1693  18488  18007  10307   6070  15804  12667  12669   9807  11134
       16489   16485  15579   4935   7730   2883  18491    357  12474  11486  15568  10324
        1141    4114   4023   1324   1516   8779   8066   8071   8383   8036   7857  16769
       18034    5299   8547   7169   7318   7344   8910   8912   8092  19202   8015   8690
       14341   12620  15737  16340  15484  15467  14520  14452  14455  16969  14400   1724
       12630    9906   2004    524  14484   4721   3025   7855  12963  18219  16511  16512
        3275   16587   4756  17235   1571   2857  15727  10976  10974  12274  12273  13779
       13783   10612  18384   9855   5254  10252  13600  18187  16897   7678  11829   2404
        9908    5704  14398  15012   5140   2309   6667  10169   6704  16922  15850   6152
       11114    5014   8102   9465   6114   2638  18541   4403  15642  11464   2905  11622
        8995    1743   1762   1746  19009   1346   7223   2208   2193  17096   7104  12577
        5887    6803  11121  18419  14992   7575  10188   3795   3794    974   7376   8980
        2807    2826   7369   3221  16877  11643  14101  17431  14104  11922  14106  14118
       14103   11917  14117  16324  16321   8905  10383  15152   2076   7599  12174  12185
       12182   18286  13955  17910  14653   7417  12493  18627  16619  10333   1521  17614
       17616   17619  11661  18323  10495  18481   1001  18455   7303   7306   7304  16744
        3625    7860   4775  17411  18535   1033  15056  15910   7850  11458  17423  17422
       10530   10527  10535  15563   3257   1704   1706  14317   6401  17505  17504  17525
       17524   15208  11409  11410   2505   6022  14351  16028   7381   7377    432  17322
       15540    6527   5564   5567  15538  12098  12865   3223   4128  11048   1117   6455
       13770   13767  16099    845  18437  15972  15973  16097   3580  14537  10850  10852
       13827   15803  12523   8855  16421  10106  11083  11745   4860  12777  12778  14071
       14067   14053  14069  14091  14072  14075   8376  11001   9987   7853   5184   7393
        7395    3219   3220  17517   3203  17996   3205  17513  10899   3204  18474  18023
        8058   16661   9537   7732  11831  15167   7258   5125  10598  17302  18438   7525
       16517   11964  13291  11188  16233   7480  12520   6579  13347  18509  13344  18011
        7526   11573   6489  13802  11785  11585   3344   9693  17863   1377   9412   1040
       18484   18027  18467  18028  17973  17972  17970  17969   6464   3216  10159  11232
       10456     998  11571   4985   4969   4968   4962   4942  14048   4370   3286    503
       12392   17021   5556   3215   6951  10681   7213   3129   5208   6043   9884    620
        6698    7398  13922   6759   6761   6763   4298   7130   7141   1286  18325   1553
        1564    8604  15411  10151  17595  15945    327  17353   1384   7353   7349   7351
        7348    7218   7214   7201   7224   7220   7200   2490  12445  12412  12411   8097
        7923    4277  11898  11906  11911  11909  11934   6086  17673   4895  15058  19237
       15045   15907  16396  16398  15912  15057  15636   3112  16403  15079  15075  10699
       13848   18489   6779    418  17149
318:   12599     499  18756   7177   8260  11959  18655  10968   8112   4278   7032   3823
       14255    3548   1566  16589  16232   8773   8775   2276   2312   7316   8907  11235
       12972   10692  17421  14546  18163  18160   7004   8135   7178  15335  15336  15470
        4765   14111   1225   5814   4193  13232   8948   1200  11059  17361  12365  13450
        2535   17627  10090   3516   6657   7287  11489   9322   5109   3778   9014  12003
        3354   15913  17202  14866   9711   2285   8928  14738  10494  14577  11588  18862
       19117     954   8153  13468   9213   1431   7485   4537   4429  11904  12478  12758
       12095   14845   1768  12437  16579  15770   2675  13701   7914   9070  10222  14563
        2114    9758   9755  11041   5187   1248   2305  10098  10096  11267  19200  14447
        9214    9217   3996  12789   7238   2038   6685  19174   5335   4231  18968  10872
        8252   15615  12163  11209   5713   2380   6538  13050   3892   6414  11753  17620
        5399   16694  18030   5537  16265  17092   6578  12369  12850    900  10894   8960
       10868   11664   8715   2423   8463   9779  12614
319:    1589   13174  17604  12728   8019   1121   5422    814   2032   3576    403  12973
        5779     586   7625  13532  14465   8035   5342  14260  12366  13545  11833  15130
       16303    3048  15435   2835  12465   8722   4550  15303  15548  15934   3788  10866
       10597    2843   6036  13084  15810   8431  10743   5277   5274   9812   9270   2756
```

```
      1410 18832  3278 11379 13579 11278  4074  7651  8619 13040  8311  3715
     16215 15077 14079 15855 12165  4158 14545  5156 12317 14284  1515  7176
      4843 14556 12712 12300  9895  9617 18976  7164 12344  3521  1713  9817
      8246 14610  4404 18916 18913  3122 11641  5193  5344 15313  4566  4856
      5071  5093  4448  8287 13657  6526  4058  4427  6492  3925 10279 13235
      2054  8492  5680  7588 11513 15092   977  6674 12978 14138 14142 19136
      8941 12026  6528 11069 13846 18499 15946 14691  5796 15067 10116  4991
      9572  9076  3395 18068  9445 17137  8421 13493 10070 12598 12451  5291
     12469  9327 12751 12747  1725 12393 11867  6201  2489  7875 15355 19025
     10704  2049 10788 15175  2284 16846  8929  5947 13843 15877 12575  2501
      3898  8155 13467  7071  7873 13993 15899 13380  6767 18512 11884 11979
      3330 15689 16649  4862  8196  6687  2400 11639  2206  6934 11974  3561
     17225 17146 16272  5307  8299  6795  5491  4199 12086 13575  6650  3908
     12452  9496 12843  7943  4725 12680 12281  8051   358 12760  4561  2302
      8823  6716  7236 10428 18993 18407  1484 18587  1395  9297  4090 12363
      8462  8465  1521 11345 10914  8048  3177  2098  1828  5023 13427  5602
      5276 15578 11178  2819   615  9528 14414  9527 11254  3937  5441  1651
      8198 12353 10853  8300 10913 10928  6110  5189  4050 12823 14443 11949
     16408 11950 13618 13617  8525  8047 14589 17449  9626 10655  7897 16072
     10344  1825 16933  5370  1337 16438  5326  2492 15690  4379  2818 13301
     17586  9487   949  3316 11112  8259 10981 15481 11590  1960 10987 10959
       327  6605 17324  1442 14285 16347  3109  8163  9798  2999  6258 19224
     11771 10012  5150 13606   363   331 14915 19232  2868 18598  8400  8016
      9887 12616
320:15632  2952 18051  1872  3610  1143  8771  9483 12004 11992 10480  8336
      4133 12206  1141  7895 10039 16601 11983 10259  6521 16664  6287  8036
     14786 12091 12228 18962 14523  7959  1698  6558  1392 17879  6216  6732
      4889 17426  4101 11419  8871 10630  5660 18687  5945  6925  5477 13616
     15059 18276 14625  7855 10956  9543 14068 14195  3384 17085 14647 10436
     19111  2582  9763  1531  5352 11010  3327 14602 18949  5178 14833  3564
     10837  7935  6556  2697 15231 10250  1721 19094 16923 14173  4694 16114
     14956 11138 10083 15037 18032  1014  9937  3923 19096  7692  5154 12842
     14677  3036 12572 17847  5502 13294  7292 10290 10985  1326  5578  5496
     16671 15401 16633 14650 13687  7867  4516  3489  2481  9181  1640 17370
      1859  9696 16524  9721 11096 15425 14800 15362 15449  2265  2374  2181
     12009 14700 10716  9087  2723 12811  3421 18898  8500 11713 17270 18029
     11943  9460 17570 15160  6926  4444 10794  6710  6707  4243 14028  6791
      4228 19004  3795 10188  3794   974  7376  8980 12318  2807  2826 15644
      2680  6093   358 11643  4153   838 14101 17431 11922 14104 14106 14118
     14103 11917 14117 11877  9156 17708  2076  7599 18286 16788 18725   702
      6728  1024  3627 12363  8462  8465  1521 11661 18323 10495  3704 15618
      1858 17316 18100  2782  4676  3166  7310 10111  1033  7850 13576 15542
     16151 16155 16095  3257  6401 17505 17504 17525 17524  1896  1207  7377
       432 16819 10332  2411 10331  2410 12865  3223  4128 11048  1117 13770
     13767 16781 16777 16779 16801 17741 16774 17646  4860 12777 12778 14071
     14067 14053 14069 14091 14072 14075  8376 11001  9987  7853  5184  7393
      7395  3219  3220 17517  3203 17996  3205 17513 10899  3204 12460  1407
     10187 13347  6722 10917 13636 16218  5693 11859  5188  8765 11074  7398
     13922 12516 17315  6346 16345  9939 15062 18325 18698 17660 16965   327
      7298  5459 18530 17826 12176 15633  7353  7349  7351  7348  7218  7214
      7201  7224  7220  7200  2490  1737  3491 13673 13675 15119 13602 15058
     19237  3264 15516   363 10316 16637  2383 10379 10952 10563
321: 4787  1070  1143 14654 12004  9483 11994 11992 10480  8077  7492 18097
      1141  7895 10039 16601 11983 18391  9162 16546  6287  8036 16050 12091
     15109  5055 14523  7959  1698  6558  4665  9086 14143  3713  1389 17879
      6216  6732  4288 10839 16448 11376 17426  7384 15099 11717 18687  3375
      6925  2064 13616 18615 18276  1079  7855 10956 16326 14023 14195 18092
      2595  1419  5878 14647  8095  9681  9889  1418  7628  1691 11010  3327
      1291 14602  5178 18949  8748 10837  9976 15008 12989  6556  6602  4099
     15231 12324 11078 12716 18545 15123 19094  1721 11505 16923 16087 11631
      4694  4728 14956 14961 17360 12636 15037 18032  1014 19096  9937  3923
      5154 12842 10269 14580 17215  4853  9605  4948 15110 12572 17847  5502
     13294 16560  7292 14293 10985  1326  5496  5578 13565 16671 15401  9097
     16633 17335   818 13398 13687  4516  7867  9181 17370  1640  2481 16524
```

```
 9820 11096 18560 16290  4367  2374  2265  2181  3855  5338 16697  9087
 3421  4284 13612  9460 15160 17570  6926  4444 13949  6326 10794 10325
 4243  6710  6707 14028  6791  4228 19004 10188  3795  3794   974  7376
 8980  2807  7729 15644  2680  1890  6093   358 11643 10156 14101 17431
14104 11922 14106 14118 14103 11917 14117 11877  9156 17708  2076  7599
18286 17450 10636 10883 19245  2706  6728 16273  1024  3627  4931 10230
13967 10244  5157  3863 17448 17445 13484 12298   523 16683 14982 14290
11257 16905 18550 17602 17600 16815   656 17707  2933  4668  7938 12926
11953 18469 12363  8462  8465  8395  1521 11661 18323  3704 15618  1858
17316 17901  1025  4676  7310 13111  3166  9748 15261  7953 14931 10111
17383  6243 14337 11009   802  1489  9751  5535 19225  4741  8379  9986
 5615  9570  7631 18249  1579 18874 13594 14957  2833 13097 11855 17444
15990   800  2048  5863  1033  7850 13576 15542 16151 16155 16095  3257
 6401 17505 17504 17525 17524 17093 15754 18311  6989 11460 14526  7397
11457  1896 12536 12551  1207  1365 18241  7377   432 16819 10332  2411
10331  2410 12865  3223  4128 11048  1117 13770 13767 16781 16777 16779
16801 17741 16774 17646  4860 12777 12778 14071 14067 14053 14069 14091
14072 14075  8376 11001  9987  7853  5184  7393  7395  3219  3220 17517
 3203 17996  3205 17513 10899  3204  4087  5453   600 10187 12890  1263
 2658 12511  2978 16319 11645  9321 14259 12460 12499  5106  3023 13950
 1407  1842  3668 11242 10666  8675  8209  5693  9304  5717  1495  8302
13979  6722 12398  8064 10221 16218  9573  7398 13922  3342 12516 11763
 2356 17878 17315 16011  9939 15062 18325 18698 17660 14814 16965  2892
17826 12176 18530 15633  7353  7349  7351  7348  7218  7214  7201  7224
 7220  7200  2490  5527  1737  3491 13673 13675 15523  3264 15516 16637
10316  8850  2383  9979 10952
322:13867 13356 18488 18007  3183 12200 18931  2950  6222  3371 18022   360
 5198 14399  1141 15264 17705  8488 14597 11044  8036  7857  4692  3130
12522 17069 10657  3462 13989  1149  6120 11450  8772 10736 17216  9050
 6700  9882 18213 18369 14503  9964 15053 16115  7855 18183  9714 15235
18772 14715  3139  7921  9020 14812   701  2770  3381  7111  7814  9167
13764  3884 17541  3901  1277 12193 12982  4219  6713  2609  3190 11470
 8428 19080  2937  2587 15925 15924  2921  2742   590  8886  9243 17583
11582  6545 15518  6109 10855  5127 14268  9185  2748 10188  3795  3794
  974  7376  8980 10081 14401 16645  2807  2826 15414  3221 11000 16877
  358 11643 13747 14101 17431 14104 11922 14106 14118 14103 11917 14117
11877  9156  2076  7599 18286 17450 10636 10883 19245  2706 17448 17445
13484 12363  8462  8465  1521 11661 18323 10495 18481  5492  1858  3922
17901  1025 15261  1033 15056 15910 16151 16155 16095 13014 16069  3257
 6401 17505 17504 17525 17524  1530  2022  7377   432 16819 10332  2411
10331  2410 12098 12865  3223  4128 11048  1117 13770 13767 16781 16777
16779 16801 17741 16774  4860 15510 12777 12778 14071 14067 14053 14069
14091 14072 14075  8376 11001  9987  7853  5184  7393  7395  3219  3220
17517  3203 17996  3205 17513 10899  3204 18474 18023   600 13347 18509
13344 18011  8209  8302 18008 10221 15948 17612  8147 11081 18398 16052
 2853 15154 15669  4108   413 16888  8441  3479  1048 17549  9749 17291
14470  2008 13574  5988  3547 13320 12107  4540  6031 16301  6076 18372
10579  4109 11366 19231 11108  8482  5412 12150 14436 18176  1610   674
 6891 13730  8642  6095  3638   993 17479 11156  6014  4301  1954 10818
 7039 15055 12418 10003  1924 13798 11179 15926 13552  9569 18113  9432
15846 14535  3145 12007 16132 12934  1538 16847 15583  6741  5534 14502
 4641  7188 15672  4220 11219 12375  7172  3983  2230  6072  5854 17508
17766 18490 15885  4617  4368 16850 13940  3991  1557 11615  2624 18508
18457 10864 18750  6697  1128 12487 18977  5445 11199 10746 16932  5601
16270   559  5072 16012  1609 13543 18010  5876 18551 11710 16973  8611
17125 17786   896 15150  6585  6520 13129  4965  3423 18175  2832 10945
18855 16525  7611  1748  5964 14612 11300 17123  1873 14105  1285 15156
11382  8729 12956 17420  6607  6377  5035  9405 13286  9963  8621  9241
13064  2473 14203 19067 17080 10484 16852  9591  6583  5237  3885 18484
18027 18467 18028 17973 17972 17970 17969  5302 16042  6762  8202 17109
 9781  3057 12195 18035 14637  2924  5223   595  5651  5083 15409 17949
13812  7398 13922 18325 17657 18513 18472 17113 17362  7353  7349  7351
 7348  7218  7214  7201  7224  7220  7200  2490  6747  4859 15058 19237
15045 15907 16396 16398 15912 15057 15636 16403 15079 15075 18489 13426
```

```
      5879 12748
323: 8509  1134 18488 18007  1916  3371 18022  8888 12349 10147 14312 15530
     13340 18024  1141 12261 15264  9171 10433 11548  8036  7857 15149 17899
      2020  9112  4452  4484  2223 11002  7352  6808  7161 17556  6400  7983
      2949  1905  8251  4178  4037  2160  7855  3729 10163 14692  2870  4621
     15928  5084 11180  7057 11633  9253 16002 13530  3418 11611 17115 17307
     17434 13885  9657 11119 15654 12427  3412  4802  5132  6456  1469  3913
      3614 13992 17416 16667 11029 13494  5616  2334  2102 11128 18331 15535
     16171  9966  6234 18709  6008 11068 15752 16073 16342  8681  8695  6474
      6357  9359  3239 11051 16468 10672 11216 17792  3226 13446  4327 14062
      7614  2101 12204 18837  1898  5001 15038  7000  4563  3830 12987 10357
      8622 16416 18809 13442 10188  3795  3794   974  7376  8980  2807  2826
     15414 11189  3221 11000 16877   358 11643 14101 17431 14104 11922 14106
     14118 14103 11917 14117  9717 11877  9156  2076  7599 18286  2975 17450
     10636 10883 19245  2706 17448 17445 13484 13267 12765 11397  7419 12363
      8462  8465  1521 11661 18323 10495 18481  1858 17901  1025 15261 14337
     11009   802  1489  9751  5535 19225  4741  8379  9986  5615  9570 18249
      1579 18874 14957  2833 13097 11855 17444  2048  5863 16811 12374 13302
      5440 14857 13516 10812  9419  6771 11768 12141 18295 11218  3543  6242
     11404 18052  2875  1963  2636  7150  5500 15645  3024 19039   805 11538
     17169  2943 12915  4471 17129  2420  9530  8029 15921 16041 11094 10747
     13795  2144 17511 12731  6924 15147 18864  7598  2259  7950 18104  7123
     13183  9498   651  5099  8353 13883  4389  2662 11940 19159 16364  4653
     14754 12986  3439  9888  4998  8161 19192 17068 10691   934   606   769
      8468 18297  1397  3167 10721 18909 18127   820  8767 11231 10209 18039
      4769  4271   914  5454 17761  1436 16749 12983  7990  3461 11482  2488
     19116  2605 15929  1132 11154  5499 16543 12081  4575 10781 15011 17427
     14182   608  1033 15056 15910  7850 13576 15542 16151 16155 15597 16095
     13014 16069  3257 14796  6401 17505 17504 17525 17524  1010  2022  7377
       432 16819 10332  2411 10331  2410 12098 12865  3223  4128 11048  1117

     13770 13767 16781 16777 16779 16801 17741 16774  5158  4860 15510 12777
     12778 14071 14067 14053 14069 14091 14072 14075  8376 11001  9987  7853
      5184  7393  7395  3219  3220 17517  3203 17996  3205 17513 10899  3204
     18474 18023   600  7911 16191 13238 15975  4924  9729 14251  6091  4326
      6820 13828  4812  9933 17784 16424  4768 15749  7309 10904  2071  4194
     15043  2025 10355 13347 18509 13344 18011  8209  8302 10221 17990  6825
     13135 15948  8147 11081 18398 17612 16052  2853 15154 15669  4108   413
     16888  8441  3479  1048 17549  9749 17291 14470  2008 13574  5988  3547
     13320 12107  4540  6031 16301  6076 18372 10579  4109 11366 19231 11108
      8482  5412 12150 14436 18176  1610   674  6891 13730  8642  6095  3638
       993 17479 11156  6014  4301  1954 10818  7039 15055 12418 10003  1924
     13798 11179 15926 13552  9569 18113  9432 15846 14535  3145 12007 16132
     12934  1538 16847 15583  6741  5534 14502  4641  7188 15672  4220 11219
     12375  7172  3983  2230  6072  5854 17508 17766 18490 15885  4617  4368
     16850 13940  3991  1557 11615  2624 18508 18457 10864 18750  6697  1128
     12487 18977  5445 11199 10746 16932  5601 16270   559  5072 16012  1609
     13543 18010  5876 18551 11710 16973  8611 17786   896 15150  6585  6520
     13129  4965 18175  2832 17125 10945  3423 18855 16525  1748  5964  7611
     14612 11300 17123 14105  1285 15156 11382 12956 17420  6607  6377  1873
      5035  9405 13286  8729  9963  8621  9241 13064  2473 14203 19067  4331
     17080 10484 16852  4017  9591  3885  6583  5237 18484 18027 18467 18028
     17973 17972 17970 17969  5302 16042  6762  8202 17109  9781  3057 12195
     18035 14637  2924  5223   595  5651  5083 15409 17949 19109 11395 17509
     10365 16956  9754  9720 13131  5389  6525 18579 18245 18559 15802  6580
     13199 10789 12935 13695 14236 14994  5044  6962 15953 11413  2116 18736
      3803 13906  7398 13922 15230  9222  2858 19186 18325 18513 18472 17119
       327 12058 14220 15762  7353  7349  7351  7348  7218  7214  7201  7224
      7220  7200 18951   550   569   548   555 18970   552   571   576 18973
       566   556   568 18967 18971 18927 18954 18953 17025 17030 16532 17028
     17031 17024  6349  2490 13768  8873  3506  4724  6862  3554  4984 10907
     15058 19237 15045 15907 16396 16398 15912 15057 15636 16403 15079 15075
     13211 18489   363 12855 14320  9450  6933
324: 9727 17604  8019 17534  5342 14260 12366 11833 15130 15303 15548   578
```

EP 2 489 726 A2

```
        2843 10597  6036  3271 10743 13040  8311 16215 12165  5156 14545 12317
        1515  9617 18976 12344 13678  9850  9817 10925 16912  3080 15313  4566
        6526  5635 10279  9228   815  2054  8508 11583  3845  5204  7588 15092
       12713  3464   977 15385  9792 12978 14138 12026  3293  1369 12116  9076
       18068 12469  9327 12751  6201 19025  7875 12432  2049 10788 16846 18665
        7873 11979  3330 15689 17238  4586  8514  2400 17974  2546 17225 15437
        8299  4199  6795  5491 12086 12452 12843  7943 12052  6716  7236 10428
       12363  8462 11345  8048  2098  5602 13427  1651  7682  8300 10853 12353
        8198 10913 10928  5189 11954 11949 11950 16408  4232  4289 14167  2492
       15690 10153 15481 11590  3698 13133 10987 10959   328   327 14285  8163
        9798  6258   594 11771 17226   331  6717 19232  2907 18598  2868  8016
       15159
325:   14605  6517 16865 17621  6498 10877  4478   535  7655  7490 11610 12325
       10826 12155   329   359   362   360   357  9904 12647 12682 11322 13239
        8952  4187  1141  4906  6775  3519  6177 17529 16576 10915 19075  8253
        9925  8036  7079 14890 10540  2772  1228  9307  5251  8710  2343  9153
        7253  6428  3776  8966 16276  8950  5611  8203 14908  4343  6064  5226
        6370  1046  3282  3553 10371  7861 13057 10076  5103 18747 15674  3566
       16824  4745  7855  4568 10122  3173 14748  7024  1998 18270 18414  2000
        9723 16513  7623  3841 11201 10882 14758  7077  3123 15800  2884  8411
        9932 14183  7748 11559 13821 13207  5113  4397  5376  8746 17170 18657
        9642 15684 14665   326 15221 11049   941 18982  8593  6099 15254  1202
       16046 12502 17716  9851 15240 14039  1414 18848  2451  4881  9525  5374
       14767 14281  6641 17952 12133 15614  7506 15039 17536  2009 13100  8427
        6859  9928 19163  4498  2340  1509   433 13478 14041 14568  4457 18018
       17111  1472  9268  9056 10303  5984 19184 18805 15184  6590   747   858
        1988 17838 16755 13510 14046  9788  7458  8799  9811  4319 14371  3222
       14453 13005  1232 13882  5228  9624  2044  6117 10188  3795  3794   974
       15693 18573  7376  8980  2807  2826 11958 18157 10192  3850   358  8304
       11643 16949  2912 14101 17431 14104 11922 14106 14118 14103 11917 14117
       11877  9156  1742  2076  7599 18286  2961  2816  8027 11540   897 17297
       14617 12032 14635  2786 12115  1581 19172  8841  2722  3304  3031 18527
        3971  3488 14435  3300   653 17450 10636 10883 19245 12363  8462  8465
        1521 11661 18323 10495  1858  5752 18642 14905 11072 16126 15626  1970
       11741  1549  2732 16716 11426  1770  6081  9927 11814   614  9502  8796
        1798  7054  1033  7850 13576 15542 16151 16155 16095  3257  6401 17505
       17504 17525 17524 18409 15499  8232 16518  7377   432 13504 13500 16819
       10332  2411 10331  2410 12865  5789  3223  4128 11048  1117 13770 13767
       16781 16777 16779 16801 17741 16774  4860 12777 12778 14071 14067 14053
       14069 14091 14072 14075  8376 11001  9987  7853  5184  7393  7395  3219
        3220 17517  3203 17996  3205 17513 10899  3204 11670 14581 14168 11682
       12108  4806 15638  6252 19127  1701  5131  8012  3793 15088 12482 12339
         600  6047 14198 11699  7787 19095 10717 12562  3733  3009 12416  9506
        8209  6561  3146  8302   859  8778 10221  3688 17462 14789 17884 15014
        6858  7396 16527 17887  4569  4361  9985  2465  3152  9337 18012 12479
        1428  3121 12526  7010  6034  6248  5302 11518  3532  7398 13922 16467
        9227 13725  3045 14411 11840  6227  2107  5626  3017   649  5687 19170
       18325 17413 13970 18612  8010  7401 19015   328  7461 14921  4492 15663
        7353  7349  7351  7348  7218  7214  7201  7224  7220  7200  2490 10210
        1914 11630 12067 10526  7360 17483   990  2676  9138  1772 10340   333
        4344  2478  7841   363  1767  4651 11660 15488  7901  7724
326:   14605  6517 16865 16885 17621  6498 10877  4478   535  7655  7490 11610
       12325 10826 12155   362   359   357   360   329  9904 12647 12682 11322
       13239  8952  4187  2763  1274  1141  4906  6775  3519  6177  2747 18261
       11684 17529 16576 10915 19075  8253  9925  8036  7079  4625  7770 14890
       10540  2772 18979  1228  9307  5251  8710  2343  9153  7253  6428  3776
        8966 16276 18393 17006 14683  8950  5611  8203 14908  4343  6064  5226
        6370  1046  3282  3553 10371  7861 13057 10076  5103 18747 15674  3566
       16824 18741 17597  2126  4745  7855  4568 10122  3173 14748  7024  1998
       18270 15272 18414  2000  9723 16513  7623  3841 11201 10882 14758  7077
        3123   325 15800  2884  8411  9932 14183  7748 11559 13821 13207  5113
        4397  5376  8746 17170 18657  1764   875 11550  9642 15684 14665 11049
         941 17189 18982 16732  8593  6099 15254  1202 16046 12502 17716  3827
        5116  4146  9851 15240 14039  1414 17928 17894 18848  2451   522  4881
```

77

EP 2 489 726 A2

```
 9525  5374 14767 14281  6641 17952 12133 15614  7506 15039 17536  2009
13100  8427  6859  9928 19163  4498  2340  1509  2780  1486   433 13478
14041 14568  4457 18018 17111  1472  9268  9056 10303  5984 19184 18805
15184 17669  6590  5317  5461   747   858  1988 17838 16755 13510 14046
 9788  7458  8799  8811  4319 14371  3222 14453 13005  1232 13882  5228
 9624  6117  2044 10188  3795  3794   974 15693 18573  7376  8980  2807
 2826 11958 11654 15238  3291 12252 18157 10192  3850  9625  8304  8603
11643 16949  2912 14101 17431 14104 11922 14106 14118 14103 11917 14117
11877  9156  1742  2076  7599 18286  2961  8027  2816 12032 11540   897
17297 14617 14635  2786 12115  1581 19172  8841  2722  3304 18527  3031
17821 17385 10997 14435  3300   653 17450 10636 10883 19245  3971  3488
 2706 17448 17445 13484 10247 12363  8462  8465  1521 11661 18323  1858
 5752 18642 14905 11072 16126 15626  1970 11741 16716  1770 11426  1549
 9927 11814  9502   614  6081  2732  1798  8796  7054 13263  9753 13032
17901  1025 15261 14337 11009   802  1489  9751  5535 19225  4741  8379
 9986  5615  9570 18249  1579 18874 14957  2833 13097 11855 17444  2048
 5863 16811 12374  1033  7850 13576 15542 16151 16155 16095  3257  6401
17505 17504 17525 17524 18409 15499  8232 16518  5559  9033  6961  3195
15502  7377   432 13504 13500 16819 10332  2411 10331  2410 12865  5789
 3223  4128 11048  1117 13770 13767 16781 16777 16779 16801 17741 16774
 4860 12777 12778 14071 14067 14053 14069 14091 14072 14075  8376 11001
 9987  7853  5184  7393  7395  3219  3220 17517  3203 17996  3205 17513
10899  3204 11670 14581 14168 12108 11682  4806  6252 15638 19127  5131
 1701  3793  8012 15088 12482 12339   600  1430 18717 10571 13132  2647
 8686  9426  6047 11699 14198  7787 10717 19095 12562  3733  3009 12416
 9506  6561  8209  3146  8302   859  8778 10221  3688 17462 14789 17884
15014  6858  4361  7396 16527  4569 17887  2465  9985 18012  9337 12479
 3121  1428 12526  7010  7398 13922 16467  9227 13725  3045 14411 11840
 6227  2107  5626  3017   649  5687 19170 18325 17413 13970 18612  8010
 7401 19015   328  7461 14921  4492 15663  7353  7349  7351  7348  7218
 7214  7201  7224  7220  7200  2490 10210  1914 11630 10526 12067  7360
17483   990  2676  9138  1772 18635 10340   333  4344  2478  1751  7841
 1767  4651 11660 15488  7901
327:15072 18488 18007  9610  3775  9607  3371 18022  9772  3587  3646  3626
 3623  3605   360  5669  1833 18114 14999  9687  1141  4861  8045 15264
11935 19083  8036  7857 16570  5268  5235  7145  5870  6620 10640 18589
10476 17211  3947  5034 18317  4157 18510  7855  3915 11082 10349 17126
 1913 14073 10520  6696 14675 12497 10231  5640  3617  8159  1342 12396
14967 15398  3877 18205  1444  8485 12841  4970  1296 14659  6073   317
  332 12025   887 15872 14897 13021  3998 17919 17868 16067  1616  1878
 6009 11314   547  2796 13566 13927  5860  2980 15914  9417  4366 17477

 5293  4666  6056   767  5618 16102 12310 15083  2136 11779 13414  3319
 9174  9169  7113 18279  9434   869 12657 14439 12954 14521   780  8186
 9746  7582  7597 16451 10188  3795  3794   974  7376  8980 13650  2807
 2826 15414 12722  3221 11000 16877 11643  5414 15634 14101 17431 14104
11922 14106 14118 14103 11917 14117 11877  9156  2076 15756  7599 18286
 4123 10977  5267  4264  4246  4226  4247  4221  4211  4209  3742 17450
10636 10883 19245  2706 17448 17445 13484 12363  8462  8465  1521 11661
18323 10495 18481  1858 19106 17961  3487  5697 16728  1033 15056 15910
 7850 13576 15542 16151 16155 16095 13014 16069  3257  6401 17505 17504
17525 17524  1966  2022  7377   432  8618 16819 10332  2411 10331  2410
12098 12865  3223  4128 11048  1117 13770 13767 16781 16777 16779 16801
17741 16774  4860 15510 12777 12778 14071 14067 14053 14069 14091 14072
14075  8376 11001  9987  7853  5184  7393  7395  3219  3220 17517  3203
17996  3205 17513 10899  3204 18474 18023  5912 12513   600  7430  7911
16191 13238 15975  4924  9729  6091  4326  6820 13828  4812  9933 17784
16424  4768 15749  7309 10904  2071  4194 15043  2025 10355  5253 13347
18509 13344 18011  3930 15274  2484  6139 18484 18027 18467 18028 17973
17972 17970 17969 13244  9306 12654  7398  9689 13922  6420 15605 14007
13995  2438  2437 19194 13145  3988 15004 11945  4150 10313 10359 18325
18513 16544 14378 18243 18234 18231 18233 18229 18472  4879 15706  2293
15916  7353  7349  7351  7348  7218  7214  7201  7224  7220  7200  2490
 6757  4810 15090   319   316   322   320   323 15058 19237 15045 15907
```

78

```
    16396 16398 15912 15057 15636  2327 16403 15079 15075 10665 18489   363
      331  2956 15490 14175
328:14605  6517 16865 16885  9665  3001 17621  6498 10877  1351   708  4478
      535  7655  7490 11610 12325 12155   359   329  9904 12647 12682 11322
    13239  8952  7452  4187  6693  2763  1274  1141   629 17824  7900  4906
     6775  6177  3519  2747 18261 11684 17529 16576 10915  2317 19075  2548
     8253  4198  9925  8036  7079  4625  7770 14890 14154 10540  2772 18979
     8825  1228  9307  8673  5251  8710  2343  9153  6428  1481 17991  3776
     8966 12834 16276 18393 17006 14683  8950 11563  4180  5611   519  8203
    14908  4343  6872  6064  5226  6370  1046  3282  3553 16693   848 10371
    15841  7612  7861 13057  5103 18747 17001 15674  3566 16824 18741 17597
     2126  2014  4745  7855  4568 10122 10969  3173  2544 14748  7024  1998
    18270 15272 14644 18414  2000  9723  5269 16513  7623  3841 11201 10505
    10882 14758  2552  5991  7077  8626  3123  5046   325  8727   777 13918
    15800  2884  3466  8448 17617  8411  8918  4089 12752  9932 14183  7748
    11559 13821 13207  7858 15656 13994  5113  6395   825  4397 12175  5376
     8746 17170 18657   875  1764 11550  9642 15684 14665   326 15221  2042
    11049 13548  6784   941 18982 16732 17189   332  8593  6099  1740 15546
    18142 15254  1202  1281 15791 16046 15474 13180 11077 12502 17716  3827
     5116  4146  9851 15240   987 14039 12727  1414 17894 17928 12662 18848
      522  2451  4881 13061  9525 10424  5374   427 14767 10179 14281  6641
    17952 12133 15614 17071  7506  3584 15039 17536  2009 13100  8427 16760
     6859  9928 19163  7166  4498 14733  2340  3068  1816  1509  2780  1486
      433 16785 13478 18959 17854 14041 16004 14568 15205  4457  2876 18018
    14884  1600 17111 10757  1472   766  9268  9056 10303  5984 19184 18805
    18151 15184 17669 18943  6590  5461  5317  6134   747   858  1988 17838
    10028 16755 13423  5944 13510  9955 14046  9788  7458  8799  8811  4319
    14371  6600  1850 17710  3222  4455  5432 14453 13005  1232 18987  5228
    13882  9624  2044  6117 10338 10188  3795  3794   974 15693 18573  7376
     8980  2807  2826 11958 11654 15238  3291 12252 18157 10192  3850  9625
      358  8304  8603 11643 16949  2912  1565 15603 13762  2805  2809  8467
     2804 10050 17132 14101 17431 14104 11922 14106 14118 14103 11917 14117
    11877  9156  1742  2076  7599 18286  2961  2360  8027  2816 11540   897
    17297 14617 12032  3304  3031 18527 14635  2786 12115  1581 19172  8841
     2722 17450 10636 10883 19245  2706  3971  3488 17821 17385 17448 17445
     3300 13484 10997 14435   653 10247 12363  8462  8465  1521 11661 18323
    10495  1858 18642  5752 14905 11072 16126 15626  1970   614  9927  9502
     6081  8796  1798  1549 11741 11426 16716  1770 11814  2732 13032  7054
    17901  1025 13263  9753 15261 14337 11009   802  1489  9751  5535 19225
     4741  8379  9986  5615  9570 18249  1579 18874 14957  2833 13097 11855
    17444 13520  2048  5863 16811 12374 13302  5440 14857 13516 10812  9419
     6771 11768 12141  3543  6242 11404 10175 18052  1963  2636 17662  7150
     2875  5500 15645  3024 19039   805 11538  2943 12915 17169  4471 17129
     2420  9530  8029 15921 16041 11094 18222 10747 13795  2144 17511 12731
     6924 15147 18864  7123  7598  2259  7950 18104 13183  9498   651  5099
     8353 13883  1033  7850 13576 15542 16151 16155 16095  3257  6401 17505
    17504 17525 17524 18409 15499  8232  6961 16518 15502  5559  9033  3195
     7377   432 13504 13500  2411 16819 10332 10331  2410 12865  5789  3223
     4128 11048  1117 13770 13767 16781 16777 16779 16801 17741 16774  4860
    12777 12778 14071 14067 14053 14069 14091 14072 14075  8376 11001  9987
     7853  5184  7393  7395  3219  3220 17517  3203 17996  3205 17513 10899
     3204 11670 14581 14168 11682 12108  6252 15638 15088  1701  4806 19127
     3793  5131  1430  8012   600 10571  2647 12339 12482 13132 18717  5066
     9426  8686  7911 13347  6047 14198 11699  7787 19095  3733 10717  3009
     9506 12562 12416  8209  6561  8778  8302  3146 10221   859  3688 15948
     8147 14789 17462 17884 15014  6858 18012  4361  4569 16527  7396 17887
     2465  9985  3152 13120  5302  3121  7010  1428  6034  6248 16042  6762
    11518  8202 17109  9781  3532  3057 12195 18035 14637  2924 12479  5223
     2218  9337   595 12526 17290  5651  2890  5083 15409   581  2226 17949
     6501 15384 19109  7049 11159 11395  7613 14638 12405  1009 15823 12586
     5832   468 13131  5389  6525  7398 13922 16467 13725  3045 14411 11840
     6227  2107  5626  3017  5027   649  5687  5560 19170 18325 17413 13970
    18612  8010  7401 19015  7461  6703   453 14921  4492 15663  7353  7349
     7351  7348  7218  7214  7201  7224  7220  7200  2490 10210  7497  5053
```

```
         1914 11630 10526  7360 12067 17483   990  2676  9138  1772 18635 17757
        10340  5732   333 15080   321   324 15058 19237  4344  2478  1751  7841
         1767  4651 11660 15488  7901  7724
    329:17587 18488 18007  3371 18022 12974  8171 13047  1141 15264  8036  7857
        17407  5542  7855   325   326   332  3829 10188  3795  3794   974  7376
         8980  2807  2826 15414  3221 11000 16877 11643 14101 17431 14104 11922
        14106 14118 14103 11917 14117 11877  9156 15521 15519 15512  2076  7599
        18286 17450 10636 10883 19245  2706 17448 17445 13484 12363  8462  8465
         1521 11661 18323 10495 18481  1858 17901  1025 15261 14337 11009   802
         1489  9751  5535 19225  4741  8379  9986  5615  9570 18249  1579 18874
        14957  2833 13097 11855 17444  2048  5863 16811 12374 13302  5440 14857
        13516 10812  9419  6771 11768 12141  3543  6242 11404 18052  1963  2636
         7150  2875  5500 15645  3024 19039   805 11538  2943 12915 17169  4471
        17129  2420  9530  8029 15921 16041 11094 10747 12731 13795  2144 17511
         6924 15147 18864  7598  2259  7950 18104  7123 13183  9498   651  5099
         8353 13883  4389  2662 11940 19159 16364  4653 14754 12986  3439  9888
         4998  8161 19192 17068 10691   934   606   769  8468 18297  1397  3167
        10721 18909 18127   820  8767 11231 10209 18039  4769  4271   914  5454
        17761  1436 16749 12983  7990  3461 11482  2488  2605 15929 19116  1132
        11154  5499 16543 12081  4575 10781 15011 17427 14182 11494  4324   686
         8119 16545 10123  1033 15056 15910  7850 13576 15542  6776 16151 16155
        16095 13014 16069  3257  6401 17505 17504 17525 17524  2022  7377   432
        16819 10332  2411 10331  2410 12098 12865  3223  4128 11048  1117 13770
        13767 16781 16777 16779 16801 17741 16774  4860 15510 15508 12777 12778
        14071 14067 14053 14069 14091 14072 14075  8376 11001  9987  7853  5184
         7393  7395  3219  3220 17517  3203 17996  3205 17513 10899  3204 18474
        18023   600  7911 16191 13238 15975  4924  9729  6091  4326  6820 13828
         4812  9933 17784 16424  4768 15749  7309 10904  2071  4194 15043  2025
        10355  5253 18478  2062 13347 18509 13344 18011  8209  8302 10221 15948
         8147 11081 18398 17612 16052  2853 15154 15669  4108   413 16888  8441
         3479  1048 17549  9749 17291 14470  2008 13574  5988  3547 13320 12107
         4540  6031 16301  6076 18372 10579  4109 11366 19231 11108  8482  5412
        12150 14436 18176  1610   674  6891 13730  8642  6095  3638   993 17479
        11156  6014  4301  1954 10818  7039 15055 12418 10003  1924 13798 11179
        15926 13552  9569 18113  9432 15846 14535  3145 12007  1128 16132 12934
         1538 16847 15583  6741  5534 14502  4641  7188 15672  4220 11219 12375
         7172  3983  2230  6072  5854 17508 17766 18490 16932 15885  4617  4368
        16850 13940  3991  1557 11615  2624 18508 18457 10864 18750  6697 12487
        18977  5445 11199 10746  5601 16270   559  5072 16012  1609 13543 18010
         5876 18551 11710 16973  8611 17786   896 15150  6585  6520 13129  4965
        18175  2832 10945  3423 17125 18855 16525  1748  5964 11300  7611 14612
        17123 14105  1285 15156 11382 12956 17420  6607  6377  1873  5035  9405
        13286  8729  9963  8621  9241 13064  2473 14203 19067 17080 10484 16852
         9591  6583  5237  3885 11942 14024  5941  2321 17997 12578  4450 13443
        18484 18027 18467 18028 17973 17972 17970 17969  5302 16042  6762  8202
        17109  9781  3057 12195 18035 14637  2924  5223   595  5651  5083 15409
        17949 19109 11395 13131  5389  6525 18579 18245 18559 15802  6580 13199
        10789 12935 13695 14236  5044  6962 15953 11413  2116 18736  3803 13906
         7398 13922 18325 18513 18472  6413  7353  7349  7351  7348  7218  7214
         7201  7224  7220  7200  2490 15058 19237 15045 15907 16396 16398 15912
        15057 15636 16403 15079 15075 10664 18489   363
    330:14603   515 18387 18343  2331  4920 18684  3585  1086  2105 10280  4477
          534 16554   580  5939 12908 11544  9876  8805  3452  7516   362   357
          360  5160  7225  7197 13693 11971  8978  7475  8946  4188  6695  4175
        17891 18486  5737  5750 15281  8580  1376  1379 17822  7898 10455 14342
         7382  7888  7445   738  5438 17922  9221 17902  7986  4172 12385 14898
        15214 12314  3725 15232  8116 18161  2487  2559 19074  4201  8255  9921
        14137  7777  5356 14888 14152  6278  2470  2755 18980   718 16531  1230
         3927  8672  9283  3995  6079  2339   851 11164  7252  9267  3883  1479
        17993  1590  2294  3774  5271  6390  8965 11604 10999 10176 17396 18394
        17008 14682 13895 15309   484 17399  8933  5612  9198  6892  6990  9137
         8204  8167 19059 18580 17301  6873  4340 11451  6065  9085 11944 11248
        18110 10733  7794  5227  7083  8278 15410 16695  3552  7053  2668 15839
        10369 14061  7862 13065 12871  3847 16010 12618  5102  5723  5348  9583
```

```
    9532 15763 13830 17548 15673  5777 18062 18742 15685 17435   421   835
    1904  3265  9700  2180  2753  4596 12734 10970 18093 18831  2545 10816
    7026 12691  6318  2499  1999  2567 19001  1394  6365  9719 12315  8324
   11811  5666 16514 18475 15956 10933  3273  5918  3838   706 10504  2553
   10861  5990  9018  8809  8627  8584 13596  2608 10275 12278  8725   779
   17013  4078  3280 18893 18315 14793 18945  4573  3468  2115 18906  8412
    2897  8921  7583  9677 14390 13256 14184  7750 11561  7723  7768 13823
    8577 13165  7859 18546  7116  5043  4449  2137  6398  7799  7196 12151
   10862 18748  5377  3616 17168 11492 15246 18824 12600   895  1785 14661
   15224 16802   985   326 14632 13546 16571  9996 16735   332 17191 18450
   18771  1741 18129  6098 15547 15586 15257 15805  1279 16479 15476 11079
   13181 16731 12486 18648 16167  4143 15009 15010 17134 12729  1417 10964
   14712  5504  5721  3199  2622 13058  2979 10423  8307  3641  4854  3541
   10183   735 16996   732 17406  9929  4647 12132 17070 14450  8676 17537
   14527 14925 11247  5570 16918 18300  6997 13078  7933 12177 10965  7654
   19120 14122  8609 10040  1506  2233  3802 17276 16782 19107 18939  3104
   10995  1912 16003  8348  8420 13916 14569 15206 15711  8756  7204 18526
    6423 11090 14885  1583 14210 10760 17110 10117   768  1474  2743  3191
    3911  8963  9058 10304 19183  5960 15372 18803  8355 18130   750 16208
    2485  1433  1895  2007 18492 17590  5481  5304  6130  3953  5967   748
     856  9398  1989 18983 10032  1308 16754 13420 17236  1270 10884 13511
    9952  4531 14047  9791   584  6920  6129  6133 14369 17710  6599 16186
   14499 14532 18914  3224  5430  4453  6290  4141  4547 14451 13001  5357
    5676 10109 18990  1226 13884  6373 11384 16391 15289  9623 10336  2046
   10225  5637 18760  2679 15287 15299 15005 10538  2274 15858  2366  4715
   18226  7226 10000 14899 12251 11655  2326 12860 12845 12858 15650  4472
   18162  7699 13374  1938  6890  2517   358 13718  9245  3740  8301 10036
   10037 11598 13156  6062 11813  4731  9348  6264 18880  2358  3478  2537
   16692  8028 12689 15658 12574  1734 17135 12440  3578 17624 18905  3691
   11371 14828  6677 14887 14573  6627 16604  3255  4171 10829  5926 18882
    5653  2514  7948 13401 11986  7884 18359  3148 17208  7280 18155 13200
    5115 19203 15440 13521 13501 12684  5790   561 14678  4152  7008  7589
   17500  5692  3103 16843  8691 14121  4961 15699 18887  9148 13225 11743
    4031 10545 13773  9282  6505  2815 11385  4383  5212 11549  3097  9160
     603  3512 11976 13010  1548 10287  8901  2266 12643  8381 14274 11452
   16500 16464 16442 16552 16470 13726  7065  7063 14056 10680  7067  7043
   17663 14404 11839 11995  2011  3141 12589 18452  3852  6660  2217  4465
    4565  3018  1851 13717 14662 18960 14978 15034 15793 17415 10448 11989
    6298 11606 12404  7683  2094 10265 13451  9523  9057  6506 14626 14596
   17585  4757  2467  2131  1958 16227 12425  8409   455  7459  3609  8867
    4350  2093 19030  2060 13896  8024 15664  1766 15442  6244  7496  4892
   10377 11975  3002  3671  4437  9335 19108 17895 16401 10085 16164  1056
    4981  1848 11707 15836  5124  1138 18857  3644 10102 10006  9263 10291
   12852  3475  6442 16263  9780  5177 11318  1765 19178 18695 18669  9242
    4650  8073  7725 10485 17246
331:14603   515 18387 18343 10567  2331  4920 18684  3585  2105 10280   534
    4477 16554   580  5939 12908 11544  9876  8805  3452  7516   360   357
     362   359   329  5160  7225  7197 13693 11971  8978  7475  8946  4188
    6695  4175 17891 18486 15281  8580  1376  1379 17822  7898 14342 10455
    7888  7382  7445   738  5438 17922  9221 17902  7986  4172 12385 14898
   11160 15214 12314  3725 15232  8116 18161  2487 19074  2559  8255  4201
    9552  9921 14137  7777  5356 14888 14152  6278  2470  2755 18980   718
   16531  1230  3927  9283  8672  3995  6079  2339   851 11164  7252  9267
    3883  1479 17993  2294  3774  5271  6390  8965 10999 11604 10176 17008
   14682 17396 18394 13895 15309 17399   484  5612  8933  9198  6892  6990
    9137  8204  8167 19059 18580 17301  4340  6873 11451  6065  9085 11944
   11248 18110 10733  5227  7794  7083  8278 15410  3552 16695  7053  2668
   15839 10369 14061 13065  7862 12871  3847 16010 12618  5102  5723  5348
    9583  9532 15763 17548 13830 15673  5777 18062 18742 15685 17435   835
     421  9700  1904  3265  2180  2753  4596 12734 10970 18093 18831  2545
    7026 10816  2499 12691  6318  1999  2567  1394  6365 19001  9719  8324
   11811 12315  5666 16514 18475 10933 15956  3273  3838   706 10504  5990
   10861  2553  9018  8809  8627  8584 13596  2608 10275 12278  8725   779
   17013  4078  3280  8926 18315 18893 14793 18945  4573  3468  2115 18906
```

```
  2897  8412  8921  7583  9677 14390 13256 14184 11561  7750  7723 13823
  8577 13165  7859 18546  7116  5043  4449  2137  6398  7799  7196 12151
 18748 10862  5377  3616  7581 17168 11492   895 15695 15224 16802   985
 14632   326 13546  9996 16571 18999 18450 18771 18129  6098  1741 15547
 15586 15805 15257 16479  1279 15476 11079 13181 16731 12486 18648 16167
  5118 15009 15010 17134 12729 10964  1417 14712  5721  5504  3199  2622
 13058  2979 10423  8307  4854  3641  3541 10183 16996   735   732 17406
  9929  4647 12132 17070 14450  8676 17537 14527 14925 11247  5570 16918
 18300  6997 13078  7933 12177 10965  7654 19120 14122  8609 10040  1506
  2233  3802 17276 16782 19107 18939  3104 10995  1912 16003  8420  8348
 13916 14569 15206 16935 15711  8756  7204 18526  6423 11090  1583 14885
 14210 17110 10760 10117  1474  2743   768  3191  3911  8963  9058 10304
 19183  5960 15372 18803  8355   750 18130 16208  2485  1433  2007 18492
  1895 17590  5481  5304  6130  3953   748   856  9398  1989 18983 10032
  1308 16754 13420 17236  1270 10884 12757 13511  9952  4531 14047  9791
   584  6920  6129  6133 14369 17710  6599 16186 14532 14499 18914  2449
  3224  5430  4453  4547  4141  6290 13001 14451  5357  5676 10109 13884
  1226 18990  6373 15289 16391 11384  2046  9623 10336 10225  5637  2679
 18760 15287 15299 15005 10538  2274  2366 15858  4715 18226  7226 10000
 14899 12251 11655  2326 12860 15650 12845  4472 12858 18162  7699 13374
  1938  6890  2517   358 13718  9245  3740  8301 10036 10037 11598 13156
  6062 11813  4731  9348  6264 18880  2358 16692  8028  3478  2537 12689
 15658 12440 17135 12574  1734  3578 17624 18905  3691 11371 14828  6677
 14887 14573  6627 16604  3255  4171  5653 18882  5926 10829 13669  2514
  7948 13401 11986  7884 18359  3148 17208  7280 18155 13200  5115 19203
 15440 13521 13501 12684  5790   561  4152  7008  7589 17500  3103  5692
 16843  8691  4961 14121  9148  4031 15699 11743 13225 18887 10545 13773
  9282  6505 11549 11385  4383  5212  2815   730  3097  9160   603 11976
 13010  1548  3512  8381  4054 10287  8901  2266 11452 16442 16464 16470
 16500 16552 13726  7063  7067  7043 10680  7065 14056 17663 14404 11839
 11995  2011  3141 12589 18452  4465  3852  6660  2217  3018  1851 13717
 14662 18960 14978 15034 15793 17415 10448 12404 11606  7683  2094 13451
  9523 10265  9057 14626  6506 14596 17585  4757  2467  2131  1958 16227
 12425  8409   327   328   455  7459  3609  8867 19030  4350  2060  2093
 13896  8024 15664  1766 15442  6244  7496  4892 10377 11975  3002  3671
  4437  9335 19108 17895 16401 10085 16164  1056  4981  1848 11707   324
   322   316   319 15836   333   323   321   320  5124  8234 18857  1138
  3644 10102 10006  9263   619 12852 10291  3475  5177  6442 16263  9780
 11318  1765 19178 18669  9242  4650  8073  7725 17246 10485
332:14603   515 18343 18387 10567  2331  4920 18684  3585  2105 10280  4477
   580 18136  8805  3452  7516   359   360  5160  7225  7197 13693 11971
  7475  8978  8946  6695  4188  4175 17891 18486  5737  5750 15281  8580
  1376  1379 17822  7898 14342  7445   738  5438 17922  9221 17902  7986
  4172 12385 15214 12314 15232  8116 18161  2487  2559 19074  4201  8255
  9921  7777  5356 14152 14888  6278 18980  2755  2470   718 16531  1230
  3927  8672  9283  3995  6079   851 11164  7252  9267  3883 17993  3774
  5271  6390  8965 10999 11604 10176 18394 17396 13895  8933  9137  8204
  8167 18580 19059 17301  6873  4340 11451  6065  9085 11944 11248 18110
 10733  5227  8278 15410 16695  3552 15839 10369 14061 13065  3847 16010
  5102  5723  9532 15763 13830 15673  5777 18742 17435   421   835  9700
  1904  3265  2180  2753  4596 10970 18093  2545 10816  7026 12691  2499
 19001  6365  1394  9719  8324 11811 16514 18475 10933 15956  3273  3838
   706 10504  2553 10861  8809  8627  8584 13596  2608   779  8725 12278
 17013  4078  3280 18893 18315 14793 18945  3468  8921  8412 14390 13256
 14184  7750 11561  7723 13823  8577 13165  7859 18546  7116  5043  2137
  6398  7196 12151 18748 10862  5377  3616 17168 15246 18824 11492 12600
  1785 15224   985 16802 13546  9996 18450 18771  1741 15547  6098 15257
 15476 13181 18648 12486 16167 15009 15010 17134 12729  1417 10964 14712
  5721  3199  2622 13058  2979 10423  8307  3641  4854  3541 10183 16996
 17406  4647 12132 17070 14450  8676 17537 14527 14925 11247  5570  6997
 16918 18300 13078  7933 10965 12177  7654 14122  8609 10040  1506 16782
 19107 18939 10995  3104  1912 16003  8420 15206 14569 16935 15711  8756
  7204 18526  6423 11090 14885  1583 14210 10760 17110 10117   768  2743
  1474  3191  3911  8963  9058 10304  5960 18803   750  2485  1433  1895
```

```
      2007 18492 17590  5304   856   748  9398  1989 18983 10032  1308 13420
     16754 17236  1270 12757  9952 13511 14047  9791   584  6599 17710 14369
     16186 14532 14499 18914  2449  3224  4453 14451  5357 18990  1226  9623
     10336 15287 15299 15005 10538  2274 15858  4715 18226  7226 11655  2326
     14899 10000 12251 15650 12860 12858 12845 18162 13374  7699  1938  6890
      2517 13718   358  9245  8301 10036 10037 11598 13156  6062 11813  6264
     18880  2358  3478  2537 16692  8028 15658 12689 12574  1734 17135 12440
     17450  3578 17624 18905  3691 11371 14828 14573 14887  6677  6627  3255
     16604  4171 16840  5926 10829 18882  5653 13669  2514  7948 13401 11986
      7884 18359  3148 17208  7280 18155 13200  5115 19203 15440 13521 12684
      5790   561 14678  3956  4152  7589  7008 17500  5692  3103 16843  4961
     14121  8691  9148  4031 15699 13225 18887 11743 10545 13773  9282  6505
     11549  4383  2815 11385  5212   730  3097  9160   603 13010  1548 11976
      3512 10287  8901  2266  8381  4054  6522  4061 14274 11452 16464 16442
     16470 16500 16552 13726  7065  7063 10680  7043  7067 14056 17663 14404
     11839 11995 12589 18452  3852  6660  2217  4565  1851  3018 13717 14662
     18960 14978 15034 15793 10448 17415 11989  6298 11606 12404  7683  2094
     13451  9523 10265  6506  2467  2131  1958 16227 12425  8409  7459  8867
      4350  2093  2060 13896 19030 15664  1766 15442  7496  4892 10377 11975
      3002  3671  4437  9335 17895 16401 10085 16164  1056  4981  1848 11707
      7139  5124 15836   322 18857  1138  3644 10102 10006  9263   619 10291
     12852  3475 16263  6442  9780  5177 11318  1765 19178 18669 18695  4650
      7725 17246 10485
333:17621  6498 10877  4478   535  7655  7490 11610 12155   359   329   357
      9904 12647 12682 11322 13239  8952  4187  2763  1274  1141  4906  6775
      6177  3519 17529 16576 10915 19075  8253  9925  8036  7079  4625  7770
     14890 10540  2772  9307  5251  8710  2343  9153  6428  1481  3776  8966
     16276 18393 17006 14683  8950 14908  4343  6064  5226  6370  3282  3553
     10371  7861 13057 18747 15674  3566 16824 18741 17597  4745  7855  4568
     10122  3173 14748  7024  1998 18270 15272 18414  2000  9723  7623  3841
     11201 10882 14758  7077  3123   325  8727   777 15800  2884  8411 14183
      7748 11559 13821 13207  5113  4397  5376  8746 18657  9642 15684 14665
       326 15221 11049   941 18982  8593  6099 15254  1202 16046 11077 12502
     17716  9851 15240 14039 18848   522  2451  4881  9525  5374 14767 14281
      6641 17952 12133 15614  7506 15039 17536  2009 13100  8427  6859  9928
     19163  4498  2340  1509   433 13478 14041 14568  4457 18018 17111  1472
      9268  9056 10303  5984 19184 18805 15184  6590  5461   747   858  1988
     17838 16755 13510 14046  7458  8799  8811  4319 14371  3222 14453 13005
      1232  5228  9624  2044  6117 10188  3795  3794   974 15693 18573  7376
      8980  2807  2826 11958 18157 10192  3850  9625  8603 11643 16949  2912
     14101 17431 14104 11922 14106 14118 14103 11917 14117 11877  9156  1742
      2076  7599 18286  2961  2816  8027 11540   897 17297 12032 14617 14635
      2786 12115  1581 19172  8841  3304  2722 18527  3031 17450 10636 10883
     19245  3971  3488  2706 12363  8462  8465  1521 11661 18323 10495  1858
     18642  5752 14905 11072 16126 15626  1970 11741  6081 16716 11426   614
      8796  9927  9502  1770  1798 11814  1549  2732  1033  7850 13576 15542
     16151 16155 16095  3257  6401 17505 17504 17525 17524 18409  8232 15499
      6961  7377   432 13504 13500 16819 10332  2411 10331  2410 12865  5789
      3223  4128 11048  1117 13770 13767 16781 16777 16779 16801 17741 16774
      4860 12777 12778 14071 14067 14053 14069 14091 14072 14075  8376 11001
      9987  7853  5184  7393  7395  3219  3220 17517  3203 17996  3205 17513
     10899  3204 11670 14581 14168 12108 11682 15638  6252 19127  4806  1701
      3793  5131 15088 13347  6047 11699 14198  7787 19095  3733  3009 10717
     12562  9506  8209 12416  6561  8778  8302  3146 10221   859  3688 15948
      8147 17462 14789 17884 15014  6858 17887  2465  7396  9985  4361  4569
     16527 18012  3152  5302 13120  6034 16042  6762 12479  3121  8202 17109
      9781  1428  6248  3057 12195 11518  7010  9337 18035 14637  2924  7398
     13922 16467 13725  3045 14411 11840  6227  2107  5626  3017   649  5687
     19170 18325 17413 13970 18612  8010  3601  7401 19015   328  7461 14921
      4492 15663  7353  7349  7351  7348  7218  7214  7201  7224  7220  7200
      2490 10210  1914 11630  7360 10526 12067 17483   990  2676  9138  1772
     18635 10340 15058 19237  4344  2478  1751  7841  1767  4651 11660 15488
      7901  7724
334:13396 17207  7300 12940 10409  6167  6302 16961  3782 14913 12949  6274
```

```
         1445    8025    4162    5021   10714    4634   11369    6304   16188   14951    7942    7940
        14879    4636    9184    9183     840    3277   17325    3714   15114   17528   17582    5185
         4423   19198   14370   11230   15609   10851    2333   11889    4722    8931   15588    1827
          680   11357   16987    3034    7121   14512   16943    2908   16250   18410     495   12608
         3954    2539    4400   14817    8967    8298
    335:17955   13227   14444    9858    3875    7843    5082    5361    8900   16139   16124    4656
        16646   13371    6550    5840    6552    8352   13524   11651    9350    7366   15095    9106
         2189   17114   15179   13572   17818   12991     884    8197   17100    7580   17298    7561
        18531   14944   12090   12849    8713    2165     481   10266    5405    7903   10155   10154
         5547    6554    5176   11960   18654    8367   18775    3308    3306   15052    6482
    336:12812    6537    6555   17107   18839   15750    6539    8474   19239   12797   15782    4902
        15115    9000    9001    9065    9067    9105    9103   17825    6565    6562    6557    9004
         9003    9012   16968    2568    9474    9470    1122    1147    1755    8315    8306    8312
        14711   14713   14695    7673    7677   14772   14775    2435   11752   13152    6435    4085
         4083    6533    6535    8614    8615    8617    8620    8518    8537    8542    8543    8570
        12746    8576    8592    8594    8601    8610    1645    1673    8635    8639    8641    8643
         8661    8663    8665    9257    9258    9264    9266    9262    9299    9295    9317    9318
         9320    9323    9338    9342    9341    9345    9364    9346    9373    9374    9389    9392
         9390    9396    9404    9408    9407    9411    9409    9423    9427    9425    9441    9428
         9443    9444    9447    9467    9448   10005   10001   10008   10014   10024   10013   10027
        10031   10034    8599    8597    8477    8478    8490    8479    8513    1692    1754    1710
         1752    1045   17015   15357   15358    9042    9041   12442   15367    9100    9101    9120
         9119    9123    9124   16510    1050     984    2213    2212    1659    1656    8981    8979
         9005    9007    7760    7737    7734    9044    9043    9069    9071   12814    2254    2258
         2272    2273    8160    4433    6702    6721    3343    6193   16536   19006   12307    3854
         3851   12771    1662    1661    2139    2138    2171    8566    8568    8667    8669   10026
         2162    2146    2210    2190    2142    2141    4959    1144    1148   17053   13859    1049
         1053    1052    1124   17011   16959    1059    1058    1054   13862    1142    1127    1061
         1062    1075    1077    1064   16992   16915   16964   16878   16876   16859   17012   16899
        16921   16946    1182    1095    1094   16856    1097   16854   16851    1126   13864   16939
        16942    1100   16940   13841   16960   14325   16944   16994   17052   16879    1108    1145
        16991   16880   17018   16967   13839   17048   16926   17022    1177    1114    1113   16895
         1111    1175   17047    1110    4898    5589    5599    4983   16542   13892   16864   16988
        17051   14321   14323   19043    2338    1536    2164    9068    1664   16509    1044     983
         6146    4976    3764    4974    3763   15421    9190   11574    8757   10135   14728   11967
         3187    4958    8755   11575    1172    1276   17066   16924   16962    1083    1092    1078
         1082    1181   16990   16903   16896    4982    4894    1694    1728    1750    1784    1635
        12761    8471    5674    4720    4702   16162   16593   16429   16592   13465   16651   12975
        14562   14228   13445    7037   12725    9992   13383    9994   13865   14324   16882    1539
         1540    1165    1164    1537    1185    1193    1188    1209    1190    1211    1192   16687
        16688   16689    3125    9504    1328   18686    1945   14841   14262   13283    9073    9075
        13355   17912   17911   12471    4663   13744    6639    2970    5233    6727    8683   18083
        16805   16999    1109   16617   10334    1733    4064   16482   12338    2688    8323    7698
        11227    1776    1602    1619    1620    1690    1689    1736    1781    4784    1615    1667
         1774    1779    1756    1738    1623    1641    1671    1717   12773   13030   15977   15991
        12233    5806     912   18647    5201    1678   15537   15536   16043   11137   17959    4029
        10751    9422    7965    3773   17175   15696    9656   14239    1456    8237    3965    2720
         4396   15798    4098   13654   16332    1457    8240    5097    7868    8335    6224   17118
         7782    6255    6237    7776    6233   14548   15040   15041    8516    8517   12031   12049
        12781   12784    6582    6560   14328    4917   14355   14359   14367   14396   19230   10194
        10191   10451    9437    1647    9436    9439    9440    9846    7906    2642    9463   13611
         8571   10086     662   19008    6288    4736   14530     846    9216   12880    8162   14794
        14790   14788   15359   13027   14387   11668    8337    8341   12881   12882   13026   13391
        12955   12785   12804   12806   13191   13220   10610   10605   10607   10623   10626   10625
        10533   18925   14732   14714   14729   14730   17061   14750   12898    7639    7641    7624
        13186   13185    4801   13976    8330    7761   16558    9139    9089    9090   12899    9046
         9045   13385   12932    9040    9038    9013    9015    9096    8218   13006   13009   11928
        11916   11918   11923   11335   13190   13187    7691   13406   13394   13392   12879   12877
         3727   18357   13517    3085   12328   12329    6664    6665    6666   12660    4761   11671
        14600    8169    6360    3351   10190    5867    3349   16478   16472   16477    7680   16504
        13155    2297   14382   14391    4797   15682    2785   12327   13048   18495   14115   17082
        12990     917   15503    5572    9144    5334    4799    5675   14308   14394   14366   14338
        14859   14310   14385   14860   14858   14332   14356    1688    1687    9560   11126    8014
         2169    2166    1696    8495    8491   12793    2227    2215    9469    8538    8540    9289
```

```
        9290  9291 15908 10650 13029 13031 13033 16432  6354  6353  5957  9874
       17852 11476  2846  5526  5528 19207 16548 12951  2135 12953  5530 12952
       14720  3496  5582  2342 15247 15911 17347 15712 13002  1680  2185  2184
        7322  4953  4759  4762  1130  3826  3812  3815 12767  8645  8473  8461
       12795  9126  9127  9094  9009  1683  1685 12861 13043  9093  9091  9078
       12904 12906  1448 11362  1449  1450  9325 12931 14358  2915  8053 14498
       11392  4282  7781 16258  9550  9590  4521  4977 18683 15969 16454  1011
        1246   996 11260   946   943   932 18672 14854  4980 15980 15976 15978
       15974 15874 15982 16521 12799  8419 11340 10885 16256 10835 10856 10806
       13518  3443 14861 16748 16758  8949 15743 13836  7261 17637  2188  2186
       14388 14397 15744  6427  6412  6411 14333 12961 16261 13674 14819  9048
        9047 12816 14747 12835 12809 12832 12817  1806 18749  4362  1325  9864
        1801 10091  8794  8786  8117  8113  8115  8060  8057  8093  5874  8838
        8829  8836  8858  8826  8810  8827  8815  9471  8054  5768  8056  5843
        8075  5829  5788  8149  5808  5807  8076  8078  8120  8118  8125  8130
        8127  8977  8976  9529  9538  9536  9559  9539  9540 18257 16900  7763
       11331 11313  7658  7661 14795 11338 14815 14307 13912  1804 15957  3848
        3831  3834 12744  4181  4740 14753 14230  4182  4711  8668  8758  8693
        8692  8726  8724  8728  8701  8706  8704  8689  8721  8709  8708  8774
        8781  8777  8784  8752  8731 14363 10892  4387 12605 17432  6543 10268
        8178  7656  7644 14769 14755 14693 14689  1638  1670  1712  1715  1730
        2238  2234  9063 14862  1681  1663  2251  2250 14311 14331  4911  4914
        4930  4934 14334  4936 14353 14354  4939 14361 14389  4978  1646  1617
        1604  1708  3870  3871 12743  8574 16919  9369  9370  2211  2252  8498
        8499  4897  4908  4919  4937  4955  4957  4960 16507 16486 16480 16491
        1032  1030   981   979   962   959   956  2232  2229 14329 14155 14306
       14340 14339 14393 14851 14853 14855  4821  7851 11477
  337:  4095 15046 10521 11731  1628  9595  7433   633  7621 18399  1383  7646
        5781 12525 14082  6175  5446 17124  2637 14579 18542  8588   804  8650
        5284 18904  5658  8106  6336  4263 11332 12874  6478 13913  9066 13379
       17596 18592 13115  2010  2419  4845 14997 18740   618  3431  3970 10518
       14706 16668  9305 10374 18493  5038 10849 14836  2271   992  9179 16837
        8717 10496 18477  7920 10916  3559 10271 17999  4030 18694  6969  4377
        9285  2666 10774 17320 13447 10398  3876  2057 18631 17938   558 17050
       15751 10685 10348  7899 13923  8201   604  6712  2475 19003  2910 16091
        2140 10720  3323  9194 11223 19145 10602   657 17840 11593 11595 18216
       16978 13665 12968 16708 15761  4008  9556 11066  3402  5250 17466  6487
       16702 11097 11261 15834 16616  4700  4701  9490  6730  3210  1853 14663
       14660 10844 13655  4649  6868 16252  4885  1523 15210 12821  1183 15471
        2051  5972  4643  3628  1352  5987  6581 18812 14639 16535  9679 11416
       14991  7257  4274  9585  6918  2388 17101  4046 16235  2330 12769  4218
         881  5507 11957  3735 14383 16187  2043  5111 10878 17577 19147   343
       17755 12154  7436 13269 13270  4312  7297  2111  3014  9674  4608 10651
       11599 13358
  338:10161  1021  6688  9452 16287 16288 15688 15702 15143 16317 15707 15713
       15714 15729  6744 14587 10601 14607  3011 12699 13448  9210  7228  7062
        7064  7066  2700  8792 17658 19226 18581  7704 14010 16417  1222  7789
         611 11394  8456 13854 13461  4750  2550 14364 19133 16371 11853   908
        3758  1219  7812  7093  7068 14739   666  6001 17547  7416 14427 12134
       15094 16314  2594 15063 12622  1314 15738 15705 15731 15734 15736 14785
         634 15145  2811  4375 16005 15760  6740 16286  9982  1607  5288  3077
        3076  3079  3060 17569  5586  1163  1294  1299  7042  7060  1318  1320
        1323  7531 10165
  339:13280  7465 13170 13488  8094  7023 15591 15585 15589 13440 10740 14846
       15781 14380  7542  4997  6793  6530 15127 14107 14099 12692 12638  6743
        4176  8397  8398 12234 12202 12231 12184  8401 12178 12201 12188 12226
        8402  1852 18620 17417  1931 18095 18096 18081 18076 17392 17346  1795
        1793 17515 17496 18138 17518 18108  8418  3043 18459  8099  9311 16771
        2036 14555  8712  1468  4416  4414  4413  4436  4435  4371  4357  4355
        4376  3798  3797  3315 11764  5938  3236 15423 18556  1084  9329  9332
        9334  9352  9349  9353  9355  9354  1556 12179  7144  4314  7509 15131
       13118 16078   945 13430 18194  4956  6929  1412  4783 12306  9385 12690
       12672  4342 13055 13054  7021  4081  7703  3033 17154  9984 15399  7217
        9277 13222 13034   341 11004 15248  1463  2015 17433 12644 15941 10807
```

```
      3266  8742  1972  3510  9558  4266   626  7388 14560  7227 13555 17967
      4546  8700 17272 10335 18466  1060  3720 12985  9961 12732 18869  8954
      2752 13820 10361 15865  2500 10465 18069 18784 16053   775  2727 16534
     13326 15757 12838 11061  3693 13785 10142 11449 11354  9684  5074  9660
     12700 15166 15169 13456 13485 13469 13473 13487 13505 13506 12663 12661
      5471 17875 16908 13205 17054  9363   989   988  4140  6615  6572  6635
      6591  6608  6592 11985  6631  6662  6633  6573  6638  3200 10745  1012
     15212 14003  8819 17558  3396 13373 19044  7278  8887 11236  5875 11288
     16665  5242  4870 10506   725 15807 18290 11584 11815 10709  5665  8913
      4443  4446  4940 18124 18126  6102 10718 13110  5792 18314  9497  3370
      3367 11346  7951 13887  1123  7016  8221 13167  1644  9351  6673 16460
     18895 16626  1321 13321  3707  6276  7282 17173  5804 19036 12603 15028
     11016 18046  8269  8121  2935 14015 17073 16077  9816  3949  6418 11142
     17987  1761 15163 15613 15594 15124 15595 15162 15146 15144 15126 15138
     15121 15140 15164 15139  3828  3810  3832  3814  3796 16726 10777 13774
     13776 10726 11111 10708 10724 10729 11982 13771 11437  8696 18408  9083
     11185  5772  5773  5793  5795  7325  5718  5716  5775  7308  7313 11294
     13971 13968 13919 13986 13966 13898 13983 13941 13948  5742  5738  7755
      7753  5740  5809  5753  5776  5755 17404  5756  5771  5263 13375 13376
      8038 14491 18265  7335 17735  1470  6663  1478  1476  1455  1451  1935
      1473  8041  1937  1458  1933  1452  5249  1447 11296 11297 11282 11259
     11277 11299 11279 11417 11344 11415 11258  2990 14315 10158  2987 18033
     18144 18603 18600 18147  1821  1893  2985  2986  2989  6848 17794  8531
      8557  8554  8535  8527  8550  8751  9638  9616  9639  9615  9620  9052
      9612  8994  8992  8920  8924  3380 10785  6038 10491  4075 16618  7274
      6061  5955  7685 17176 11024 17188  5903  8096  8061 16575 16559 16506
     16597 10546 17145  7669  5958  8947  8927  8930  8925  9016  9019  8957
      8984  8988  8990  9023  6337  5820  5822  9051  7330  5064  9053 18015
     11669  5821 14528 14509  5813 15700  2445 11351  6436 14764  8797 13073
     17133
340:13196 16439  6745  4610 11899 10523 19022  5232 11306  2056  2570 18196
       609  5025  5019  7865 15314  2678 12655  9546  9454 13253 18958  1923
      4335  9039 11412  8309  7587 15840  8854  5981  9219 16268 15747  4154
      6311 13498  4599  5366  3114  9197 17805  3156 14735  1918 13226 12950
      7323  7733  8437  9356 12112 17458 16494 13907 17075  9200  9250  9276
      9252  9278  9279  9281  6045  5162  7615  7595  8879  7374  9867  6042
      7749  8020  3743  8718 16238 16530 15604 18077 14517 14553  1871  1069
      2878  2845 12697  9970 12964 12965  3572 17241  4279 18703   616 17038
     17036 17007 17009 17034 17035 17003 17004  2716  1388 14685 12624  3453
      2426  4819 12501  3673 10048  1499  2916  4850  5117  7647 14030  9934
     15766 13237   539 11479 18921  7137 17775  5670 16492 18336 18337  1862
      6718 11040 13556  3974  6965  5054  3081  6968  7212 13037 16679  6364
      2629  2077  3766  6851  9951  7731  9942  5707 15735 10648 11283  2381
      2363 18601 10074  3268  2684  4642  9218   664  2494  2601  8219 17656
     10990  7785  3331  5146  4493  4620  2917 16768 15720  8671 15843  9645
      5541  6103 11053 14038 15349  2088   795  3297  1000  1559 12161 15353
     13769  2270 10599 11748 18825 18373  3182 10477 13228  5351  5273 17329
     10825 12254  8870  1199  4883 10343 12967  4238  1562 17883 11472 17019
     16370 10801 18263 14621 12659 15703   593 11508 12110 12723 12111  4392
     11861  4169 14911 16024 18137 17510 10528 18935 14916 17827 18611 15191
     12909 18197  1658  2086 16157 11091 12303 13953 12295 13403 14690   527
      3357  2859 11106 17963  1655 16934 15042 16307  2152  3172  3999  2695
      4223  1968 14466 15787  5255 12229 11570 11600 15937  7082  5673  6369
      8380  5325  3322  3328  5672  6368 13585 11692 17220  3531  3073  1889
      8613 17603 13282  6059 14374  6092 15505  3382 10113  9943  9960 13705
     10510 12419  8371  1356   697  2104   868 15905  6254 10906 12136  9705
      6214   589  2558  7015  7333  7345  7340 15278 15258 15276  7342  4805
     10215 18817 18643 12946  1331  1333 11882 13568  2641  9802 10173 17561
     11973  6328 16066 13399 15310 10568  6842  4296  9829 12919 13707  6529
      6625  1039  5395  4067  4526  9438 10350 15249 15857 13015  9800 17845
      3551 16820  7979  1633  9827  7991  9824 11672  9865  9204  9284  9220
      9249  8579  9175  9246  8549  9173  9248  8563  9232  9203  8578  8559
      9172  9178  8585  8581  8552  8556  9176  8587 13212 10149 13765  9201
      9226 14964  1718  7993 12641 18059  9231  3494 14770  9563  7665 10399
```

```
         4688  6342  7025 16979 16279 16302 16981 16413 16433 16414 16330 16333
        16331 16971 16296 16350 16954 16356 16974 16353 16300 16431 16304 16292
        16357 16955 16952 16325 16385 16435 16411 16410 16280 16430 16957 16354
        16387 16985 16415 16327 16455 16457 16390 16380 16950 16436 16382 16274
        16452 16456 16278 16453  5858  2370 14410 18969  3274  1393  8975   573
         7715 15030  1249 14601 17639  7403  1504  8265 10515  8316 15104  1901
        14063 11766 12976 11736 12162  1902  7470 15551  8361 10367 14060  8052
         9801  9803  7474  1051  2959  4990  7193 12916 17670 12887 10565 16240
         8866 11564 12294 12293 17789 14742 13578  3256
341:13280  7465 13170 13488  8094  7023 15591 15585 15589 13440 10740 14846
        15781  7542  4997  6793  6530 15127 14107 14099 12692 12638  6743  8397
         8398 12234 12202 12231 12184  8401 12178  1852 18620 17417  1931 18095
        18096 18081 18076 17392 17346  1795  1793 17515 17496 18138 17518 18108
         8418  3043 18459  8099  9311 16771  2036 14555  8712  1468  4413  4414
         4416  4436  4435  4357  4355  4371  4376  3798  3797  3315 11764  5938
         3236 15423 18556  1084  9329  9332  9334  9352  9349  9353  9355  9354
         1556 12179  7144  4314  7509 15131 13118 16078   945 13430 18194  4956
         6929  4783 12306  9385 12690 12672  4342 13055 13054  7021  4081  7703
         3033 17154  9984 15399  7217  9277 13222 13034 15248  1463  2015 17433
        12644 15941 10807  3266  8742  1972  3510  9558  4266   626  7388 14560
         7227 13555 17967  4546 11878  8700 17272 18466  1060  3720 12985  9961
        18869 12732  8954  2752 13820 10361 15865  2500 10465 18069 18784 16053
          775  2727 16534 13326 15757 12838  3693 13785 10142 11449 11354  9684
         5074  9660 12700 15166 15169 13456 13485 13469 13473 13487 13506 13505
        12661 12663  5471 17875 13205 16908 17054  9363   989   988  4140  6633
         6608  6592  6591  6631  6573  6635 11985  6615  6572  6662  6638  3200
         1012 10745 15212 14003  8819  3396 13373 17558 19044  7278  8887 16665
         5875 11288 11236  5242  4870   725 10506 11584 15807 18290 10709  5665
         8913  4443  4446 18124 18126  6102 10718 13110  5792 18314  9497  3370
         3367 15852 11346  7951 13887  1123  7016  8221  1644 13167  9351 16460
         6673 18895  5487  3178 16626  1321  3707  6276  7282 13321  5804 17173
        12603 18046  8269  2935 14015  8121 17073 16077  9816  3949  6418 11142
         6209  1761 15146 15163 15613 15594 15162 15595 15124 15121 15144 15140
        15164 15138 15126 15139  3828  3832  3814  3810  3796 16726 10777 13774
        13776 10726 11111 10708 10724 10729 11982 13771 11437  8696 18408  9083
        11185  5773  5772  5793  5795  7325  5718  5716  5775  7308  7313 11294
        13971 13968 13986 13898 13919 13966 13983 13948 13941  7755  7753  5738
         5742  5740  5809  5753  5776  5755 17404  5756  5771  5263 13376 13375
         8038 14491 18265  7335 17735  1451  1470  8041  1933  1478  1455  1935
         1473  1458  6663  1476  1937  1452  5249  1447 11296 11297 11299 11259
        11282 11277 11279 11417 11344 11415 11258  2990 14315 10158  2987 18033
        18603 18144 18147 18600  1821  1893  2985  2986  2989  6848 17794  8531
         8557  8554  8535  8527  8550  8751  9616  9638  9615  9620  9639  9052
         9612  8994  8992  8924  8920  3380  7669 10785 17145  5958 16618 16506
        11024 10491  4075  7685 16559  8096  6038  5955 17188  7274  6061 17176
        16597  8061  5903 16575 10546  8947  8927  8925  8930  9019  9016  8984
         8957  8988  8990  9023  6337  5820  5822  9051  7330  5064  9053 18015
        11669  5821 14509 14528  5813 15700   339  2445 11351  6436 14764  8797
        13073 17133
342: 4275 14267 13342  6967 16887  3675  9675  6057  3840 17900 16902 18661
        10421 17260  3986 15808  7849  8997 16487 15022  4823 12754 10395  8534
        13581  2017  5522  7701  2701  9728 17790 12191  4072  6250  7712  7019
        12120  7086  4916 18255  8520 18744 13466 18395  2589  9658 17251 12180
         4996  5774 10305  4826  3919 11530  6902  2861  4554  7041  2825 18879
         7681  3326  2822  8206  1820  3218 15526 18435 14428 14561 13249 10813
        11879  4832 15268  2787  6653 12610 13972  3977  7443  4056 17876 12742
        17551  9095   830 12330 18456  5153 17559 14538  3441  4281  6388   929
        12203 12005 16713 14490  3329 17610 15818 10223  1832 17892 16739 19247
        19209  8382  6262  3193  5986  8544 10873  2844 11163  3318  5155 18764
        11864  6332 19115 19114 11647  8254  9757 17455 18569 13668 16305 19055
        18604 14213 19057 18621 18578 14211 18907 13293 13257 13319 13318 13255
        13233 13274 13240  1288 14112   705  1322  1313 14151 14149 14096   669
         1265   703   700   639 13682 14145 14134   647  1259 14146  1243 14113
         1268 14130 13704  1317   707 14178 14148 14116   736   757   756   677
```

```
      14119 13679 14172 13688 14097   667   782 14088   783   678 14174   734
      14089   729   642   791   758   759  1233   761   787   784  1238 13686
       5633  3144 14179  6378 18102 19076 19103 19099 19102 19090 19088 18743
       9920 13711 13723  1195 19220  1467 17833 10277  5278  7877  6039  5512
       6020  6019  6016  6015  5994  5995  6012  5993  5515  5032 11146 19134
       2994 17095  8583  3311 11705 15026 10508  1167 12675 10097  1101  1091
       5963  8953 10466
343:   4095 15046 10521  1628  9595  7433  7621 18399  1383  5781 12525 14082
       6175  5446 17124  2637 14579  8588   804  8650  5284 18904  8106  6336
       4263 11332 12874  6478 13913  9066 13379 17596 18592 13115  2010  2419
       4845 14997 18740   618  3431  3970 10518 14706 16668  9305 10374 18493
       5038 10849 14836  2271   992  9179 16837  8717 10496 18477  7920 10916
       3559 10271 17999  4030 18694  6969  4377  9285  2666 11862 10774 13447
      10398  3876  2057 18631 17938 17050 10348   604  6712  2475 19003  2910
      16091  2140 10720  3323  9194 14883 11223 19145 10602   657 17840 11593
      11595 18216 16978 13665 12968 15761  3402  4008 16708 17466  9556 11066
       6487  5250 16702 11261 15834 16616  4700  4701  9490 17897  6730  3210
       1853 14666 10844 14663 13655 14660  4649  6868  4648  6855 16252  4885
       1523 15210 12821  1183 15471  2051  5972  4643  3628  1352  5987 14639
      16535  9679 14991 11416  7257  2330  4274  9585  6918  2388 17101  4046
      16235 12769  4685   881  5507 11957  3735 14383 16187  2043  5111 10878
      17577 19147 17755 12154  7436 13269 13270  4312  7297  2111  3014  9674
       4608 10651 11599   337  2316 13358
344:   5300  3500  4027  7686  9421  4052  8763 14058  5561  2575 18884 15708
       4543  1686 13252 13166  4602 14826 18707  5525  8193  7080 17793   986
       9233 14403
345:   5980  9971  4070  2303  9479  8998  7155 11775   933
346:  18418 12504 14095  1860  5339  1942 18388 11380   752 11688 14723  7312
      18500  6219 12070  1382 15676 13413 19034 10573 16153 13734 18099 17389
       1808  8009 14640 13476 14408  8018  7100  3096  2598 13386   625 11250
      11018  3111 10260 18653  2016   637 10620 13082  2021 11275  8483 13851
      15961 10302 12726 18421  6212  3159  2443  3658
347:  11270 17077  4946  4945 15527 18554 15862 15870 15860 13080 12083 14086
      18704 15951 14593  5514  8137 18330 17072  5748  4003  7232  9301  8840
      14744  3550  6166 17459 15531  4830 12695 14036  7418  7827 14592  3352
      16349  7118 15429  5566 10536  2972 15722  3016  1928  1508  3985 14595
      13481  9084 14959 13221 10228 16121 15492 12993  8591 15602  2592 11832
       1949  9134  9588  2207 16909  5169  5148 11680 17594 10679  5728  6123
       4235 11214 15511 12368 11088 18835 11905 10115 15436 15660  7540  3170
      11393 10848 15970 12714  2257 16752  3677  3857  6325  5243 14768 15918
      18582 14405 10218 11890  9037 16519  6235 12549 18827  2287 12400 15098
       7528
348:  13176  4345  6836 18766  3513 16800 19180  2524 12509  5751  7573  4388
349:  11912  5568  6508  3945 11062 10942  8564  5617 16983  5480 19110  2468
       3581 10481  7817 10475  5989  7810  3957  4139  6900  9519  8199  1162
      17815 16982 13169 11144 14543   490  5095  4658  5436  2277 16315  7320
       8369  7909  7971 14917  4572  5603  5011
351:   1944  3388 11320 11302 11319 11317 11305  7165  1174 11523 18349 13775
       7657  7283  3563 13470  3951 18978  3197  3932  3046  3062  3065  3063
       5791  3938 11517 12121 11055  4011 16549  3495  2533  3650 15029 17486
        451 17381  4511 14953  9732 15182  2627 10876 18227   511 19073  8217
      12521 15724  4425 15491  7189  8088 17498 17501 18186
352:  13975  6206 17120 13306 11947  1705 16008  3435 19023  7536  6974  2031
      13640  1494 18480 11512  3888  7159 18706  6893 11448   714  4753 10937
       6126  6218  8698 13988 10384 16502 11673 19019 10167  7265  4613  9695
      14636 17295 14469 15141  2783
353:   8835 11783  9669 12756  2337 13479  9821  8090 13818
354:  18918 14457
355:  17083 10967  5149 19161 12075 11238 18924  4468 11390 10560 17452 17271
      17530  2078
356:  16848 17598 13404  2320  4652  6293 12539  3765  8280 13130   520  3952
       9848 15670
357:   3703 18488 18007  3371 18022 12829  4322 10690  1141 15264  8036  7857
        924   975  7855  7926   325   326  4938 11635 10188  3795  3794   974
```

```
 7376  8980  2807  2826 15414  3221 11000 16877   358 11643 14101 17431
14104 11922 14106 14118 14103 11917 14117 11877  9156 15521 15519 15512
 2076  7599 18286 17450 10636 10883 19245  2706 17448 17445 13484 12363
 8462  8465  1521 11661 18323 10495 18481  1858 17901  1025 15261 14337
11009   802  1489  9751  5535 19225  4741  8379  9986  5615  9570 18249
 1579 18874 14957  2833 13097 11855 17444  2048  5863 16811 12374 13302
 5440 14857 13516 10812  9419  6771 11768 12141  3543  6242 11404 18052
 1963  2636  7150  2875  5500 15645  3024 19039   805 11538  2943 12915
17169  4471 15921 17129  2420  9530  8029 16041 11094 10747 13795  2144
17511 12731  6924 15147 18864  7598  2259  7950 18104  7123 13183  9498
  651  5099  8353 13883  4389  2662 11940 19159 16364  4653 14754 12986
 3439  9888  4998  8161 19192 17068 10691   934   606   769  8468 18297
 1397  3167 10721 18909 18127   820  8767 11231 10209 18039  4769  4271
  914  5454 17761  1436 16749 12983  7990  3461 11482  2488  2605 15929
19116  1132 11154  5499 16543 12081  4575 10781 15011 17427 14182 11494
 4324   686 11427  8119 16545  1033 15056 15910  7850 13576 15542 16151
16155 16095 13014 16069  3257  6401 17505 17504 17525 17524  2022  7377
  432 16819 10332  2411 10331  2410 12098 12865  3223  4128 11048  1117
13770 13767 16781 16777 16779 16801 17741 16774  4860 15510 12777 12778
14071 14067 14053 14069 14091 14072 14075  8376 11001  9987  7853  5184
 7393  7395  3219  3220 17517  3203 17996  3205 17513 10899  3204 18474
18023   600  7911 16191 13238 15975  4924  9729  6091  4326  6820 13828
 4812  9933 17784 16424  4768 15749  7309 10904  2071  4194 15043  2025
10355  5253 18478 13347 18509 13344 18011  8209  8302 10221 15948  8147
11081 18398 17612 16052  2853 15154 15669  4108   413 16888  8441  3479
 1048 17549  9749 17291 14470  2008 13574  5988  3547 13320 12107  4540
 6031 16301  6076 18372 10579  4109 11366 19231 11108  8482  5412 12150
14436 18176  1610   674  6891 13730  8642  6095  3638   993 17479 11156
 6014  4301  1954 10818  7039 15055 12418 10003  1924 13798 11179 15926
13552  9569 18113  9432 15846 14535  3145 12007 16132 12934  1538 16847
15583  6741  5534 14502  4641  7188 15672  4220 11219 12375  7172  3983
 2230  6072  5854 17508 17766 18490 15885  4617  4368 16850 13940  3991
 1557 11615  2624 18508 18457 10864 18750  6697  1128 12487 18977  5445
11199 10746 16932  5601 16270   559  5072 16012  1609 13543 18010  5876
18551 11710 16973  8611 17786   896 15150  6585  6520 13129  4965 18175
 2832 10945  3423 17125 18855 16525  1748  5964  7611 14612 11300 17123
14105  1285 15156 11382 12956 17420  6607  6377  1873  5035  9405 13286
 8729  9963  8621  9241 13064  2473 14203 19067 17080 10484 16852  9591
 6583  5237  3885 11942 14024  5941  6783  2321 18484 18027 18467 18028
17973 17972 17970 17969  5302 16042  6762  8202 17109  9781  3057 12195
18035 14637  2924  5223   595  5651  5083 15409 17949 19109 11395 13131
 5389  6525 18579 18245 18559 15802  6580 13199 10789 12935 13695 14236
 5044  6962 15953 11413  2116 18736  3803 13906  7398 13922 18325 18513
18472   328 16450  7353  7349  7351  7348  7218  7214  7201  7224  7220
 7200  2490  5078   333 15058 19237 15045 15907 16396 16398 15912 15057
15636 16403 15079 15075 11327 18489
358:  7510  2877   514  3654  3669 15610 18488 18007  3371 18022  8268  8266
14102   360   359   329   362   357 11217  7969  9372  8541  7890 18428
11507 18426  1141 15264  8036  7857 19000   613  8263 13146 19018 19126
18606  6915  6347 13472 10628  8494 13268  2909  7855 11569 13550  6426
 2973   325  7807 14215  3026 12947 15493   326 15366 11129  6026  3133
 5061  2597 18920  2219 18525  4049  7576 18045  2110  5513 16459 19228
14697 16792 16718  3560 18726  4631  9142  4307  4348  6586  4615  4629
 4995  4616   699  3687  5856 10188  3795  3794   974  7376  8980  9347
 9315  2807  2826 13328 18424 10646  5320 13791 17988 15414  3221 11000
16877 11643 14101 17431 14104 11922 14106 14118 14103 11917 14117 11877
 9156  2076  7599 18286 17450 10636 10883 19245  2706 17448 17445 13484
18745 15681  5992  2522  2523 15520 19014  6220 12363  8462  8465  1521
11661 18323 10495 18481  1858 17901  1025 15261 14337 11009   802  1489
 9751  5535 19225  4741  8379  9986  5615  9570 18249  1579 18874 14957
 2833 13097 11855 17444  2048  5863 12374 16811 13302  5440 14857 13516
10812  9419  6771 11768  5500 12141  3543  6242 11404 18052  1963  2636
 7150  2875 15645  3024 19039 12915   805 11538  2943 17169  4471  9797
17129  2420  8029  9530 15921  2555  7989  1311  5122 16041 11094 10747
```

89

```
13795   2144  17511  12731   6924  14303  15147  18864   7598   2259   7950  18104
 7123  13183   9498    651   5099   8353  13883   4389   2662  11940  19159  16364
14754  18608   4653  12986  17068   3439   9888   4998   8161  19192  10691    934
  606    769   8468  18297   1397   3167  10721  18909  18127    820  11516   4271
 8767  11231  10209  18039   4769    914   2605   5454  17761   1436  12263  16749
12435  12983  19116   7990   3461  11482   2488  14294   2357  15929   1132  11154
 5499  16543  12081   4575  10781  15011  17427  14182  11494   4324    686   1033
15056  15910   7850  13576  15542  16151  16155  16095  11455  13014  16069   3257
 4390   6401  17505  17504  17525  17524   6799   3353   6792   2022   3424   3404
 3409    792   7377    432  16819  10332   2411  10331   2410  18533  12098  12865
 3223   4128  11048   1117  13770  13767  16781  16777  16779  16801  17741  16774
 4860  15510  12777  12778  14071  14067  14053  14069  14091  14072  14075   8376
11001   9987   7853   5184   7393   7395   3219   3220  17517   3203  17996   3205
17513  10899   3204  18474  18023    600  17986  18923    575   4812   7911  16191
13238  15975   4924   9729   6091   4326   6820  13828   9933  17784  16424   4768
14684   9518  15749   7309  13646  10904   2071   6198   4194  15043   2025  10355
 5253  13347  18509  13344  18011   8209   8302  10221   1303  14421  17237  10579
15948  16301   1954   8147  11081   2008  13320  18398  17612   7188  16052   3547
12007   3983   2853  15154  15669   4108    413  16888   8441   3479   1048  17549
 9749  17291  14470  13574   5988  12107   4540   6031   6076  18372   4109  11366
19231  11108   8482   5412  12150  14436  18176   1610    674   6891  13730   8642
 6095   3638    993  17479  11156   6014   4301  10818   7039  15055  12418  10003
 1924  13798  11179  15926  13552   9569  18113   9432  15846  14535   3145  16132
12934   1538  16847  15583   6741   5534  14502  15885   4641  15672   4220  11219
12375   7172   2230   6072   5854  17508  17766  18490   4617    559   4368  16850
13940   3991   1557  11615   2624  18508  18457  10864  18750   6697   1128  12487
18977   5445  11199  10746  16932   5601  16270   5072  16012   1609  13543  18010
 6520   5876  18551  11710  16973   8611  17786    896  15150   6585  13129   4965
18175   2832  10945   3423  17125  18855  16525   1748   5964   7611   6607  14612
11300  17123  14105   1285  15156  11382  12956  17420   6377   1873   5035   9405
13286   8729   9963   8621   9241  13064   2473  14203  19067  17080  10484  16852
 9591   6583   5237   3885  18484  18027  18467  18028  17973  17972  17970  17969
 5302  16042   6762   8202  17109   9781   3057  12195  18035  14637   2924   5223
 5651    595   5083  15409  17949  19109  11395   8126   3348  12648   1969  13131
 5389   6525  18579  18245  18559  15802   6580  13199  10789  12935  13695  14236
 5044   6962  15953  11413   2116  18736  11857   1258   1235   3803   1582    755
13906   7398  13922   1826  18325  18513  18472    328    327  18793   6125  18821
 1158   6132   1546  12173   4539  12936  18946  18922   7353   7349   7351   7348
 7218   7214   7201   7224   7220   7200   2490    320    322    333    324    323
  321    319    316  15058  19237  15045  15907  16396  16398  15912  15057  15636
16403  15079  15075   8656  18489    331    363    330   4045
359:18246  18488  18007   3371  18022  16861   3411  13792   5847   5845   1141  15264
 8036   7857   5865   9815   5372  16808   5252   7273   7855  13022  13463  13462
  326    332   3697   4490   4040   4024  10188   3795   3794    974   7376   8980
 2807   2826   6239  15414   1879   3221  11000  16877  11643  14101  17431  14104
11922  14106  14118  14103  11917  14117  11877   9156  15521  15519  15512   2076
 7599  18286  17450  10636  10883  19245   2706  17448  17445  13484  12363   8462
 8465   1521  11661  18323  10495  18481   1858  17901   1025  15261  14337  11009
  802   1489   9751   5535  19225   4741   8379   9986   5615   9570  18249   1579
18874  14957   2833  13097  11855  17444   2048   5863  16811  12374  13302   5440
14857  13516  10812   9419   6771  11768  12141   3543   6242  11404  18052   1963
 2636   7150   2875   5500  15645   3024  19039    805  11538   2943  12915  17169
 4471  17129   2420   9530   8029  15921  16041  11094  10747  13795   2144  17511
12731   6924  15147  18864   7598   2259   7950  18104   7123  13183   9498    651
 5099   8353  13883   4389   2662  11940  19159  16364   4653  14754  12986   3439
 9888   4998   8161  19192  17068  10691    934    606    769   8468  18297   1397
 3167  10721  18909  18127    820   8767  11231  10209  18039   4769   4271    914
 5454  17761   1436  16749  12983   7990   3461  11482   2488   2605  15929  19116
 1132  11154   5499  16543  12081   4575  10781  15011  17427  14182  11494   4324
  686   8119  16545   1033  15056  15910   7850  13576  15542  16151  16155  16095
13014  16069   3257   6401  17505  17504  17525  17524   2022   7377    432  16819
10332   2411  10331   2410  12098  12865   3223   4128  11048   1117  13770  13767
16781  16777  16779  16801  17741  16774   4860  15510  12777  12778  14071  14067
14053  14069  14091  14072  14075   8376  11001   9987   7853   5184   7393   7395
```

```
       3219  3220 17517  3203 17996  3205 17513 10899  3204 18474 18023   600
       7911 16191 13238 15975  4924  9729  6091  4326  6820 13828  4812  9933
      17784 16424  4768 15749  7309 10904  2071  4194 15043  2025 10355  5253
      18478 13347 18509 13344 18011  8209  8302 10221 16888 15948  1610  8147
      11081 18398 17612 16052  2853 15154 15669  4108   413  8441  3479  1048
      17549  9749 17291 14470  2008 13574  5988  3547 13320 12107  4540  6031
      16301  6076 18372 10579  4109 11366 19231 11108  8482  5412 12150 14436
      18176   674  6891 13730  8642  6095  3638   993 17479 11156  6014  4301
       1954 10818  7039 15055 12418 10003  1924 13798 11179 15926 13552  9569
      18113  9432 15846 14535  3145 12007 16132 12934  1538 16847 15583  6741
       5534 14502  4641  7188 15672  4220 11219 12375  7172  3983  2230  6072
       5854 17508 17766 18490 15885  4617  4368 16850 13940  3991  1557 11615
       2624 18508 18457 10864 18750  6697  1128 12487 18977  5445 11199 10746
      16932  5601 16270   559  5072 16012  1609 13543 18010  5876 18551 11710
      16973  8611 17786   896 15150  6585  6520 13129  4965 18175  2832 10945
       3423 17125 18855 16525  1748  5964  7611 14612 11300 17123 14105  1285
      15156 11382 12956 17420  6607  6377  1873  5035  9405 13286  8729  9963
       8621  9241 13064  2473 14203 19067 17080 10484 16852  9591  6583  5237
       3885 11942 14024  5941 18484 18027 18467 18028 17973 17972 17970 17969
       5302 16042  6762  8202 17109  9781  3057 12195 18035 14637  2924  5223
        595  5651  5083 15409 17949 19109 11395 13131  5389  6525 18579 18245
      18559 15802  6580 13199 10789 12935 13695 14236  5044  6962 15953 11413
       2116 18736  3803 13906  7398 13922 18325 18513 18472 17557   328 15806
      15935  7353  7349  7351  7348  7218  7214  7201  7224  7220  7200  2490
       4329  7547   333   320   323 15058 19237 15045 15907 16396 16398 15912
      15057 15636 16403 15079 15075 10637 12507 18489   363
360:18341 18488 18007 16264  3371 18022  7638 13822  1141 15264  9022  8036
       7857  2766  5222  7855 10380  9924   326   695 18994 17274  1593 18800
      10188  3795  3794   974  7376  8980  2807  2826 15414  3221 11000 16877
        358 11643 14101 17431 14104 11922 14106 14118 14103 11917 14117 11877
       9156  2076  7599 18286 17450 10636 10883 19245  2706 17448 17445 13484
      12363  8462  8465  1521 11661 18323 10495 18481  1858 17901  1025 15261
      14337 11009   802  1489  9751  5535 19225  4741  8379  9986  5615  9570
      18249  1579 18874 14957  2833 13097 11855 17444  2048  5863 16811 12374
      13302  5440 14857 13516 10812  9419  6771 11768 12141  3543  6242 11404
      18052  1963  2636  7150  2875  5500 15645  3024 19039   805 11538  2943
      12915 17169  4471 17129  2420  9530  8029 15921 16041 11094 10747 13795
       2144 17511 12731  6924 15147 18864  7598  2259  7950 18104  7123 13183
       9498   651  5099  8353 13883  4389  2662 11940 19159 16364  4653 14754
      12986  3439  9888  4998  8161 19192 17068 10691   934   606   769  8468
      18297  1397  3167 10721 18909 18127   820  8767 11231 10209 18039  4769
       4271   914  5454 17761  1436 16749 12983  7990  3461 11482  2488 19116
       2605 15929  1132 11154  5499 16543 12081  4575 10781 15011 17427 14182
      11494  4324   686  1033 15056 15910  7850 13576 15542 16151 16155 16095
      13014 16069  3257  6401 17505 17504 17525 17524  2022  7377   432 16819
      10332  2411 10331  2410 12098 12865  3223  4128 11048  1117 13770 13767
      16781 16777 16779 16801 17741 16774  4860 15510 12777 12778 14071 14067
      14053 14069 14091 14072 14075  8376 11001  9987  7853  5184  7393  7395
       3219  3220 17517  3203 17996  3205 17513 10899  3204 18474 18023   600
       7911 16191 13238 15975  4924  9729  6091  4812  4326  6820 13828  9933
       4768 17784 16424 15749  7309 10904  2071  4194 15043  2025 10355  5253
      13347 18509 13344 18011  8209  8302 10221  1048 15948  8147 11081 18398
      17612 11108  1538 16052  2853 15154 15669  4108   413 16888  8441  3479
      17549  9749 17291 14470  2008 13574  5988  3547 13320 12107  4540  6031
      16301  6076 18372 10579  4109 11366 19231  8482  5412 12150 14436 18176
       1610   674  6891 13730  8642  6095  3638   993 17479 11156  6014  4301
       1954 10818  7039 15055 12418 10003  1924 13798 11179 15926 13552  9569
      18113  9432 15846 14535  3145 12007 16132 12934 16847 15583  6741  5534
      14502  4641  7188 15672  4220 11219 12375  7172  3983  2230  6072   559
       5854 17508 17766 18490 15885  4617  4368 16850 13940  3991  1557 11615
       2624 18508 18457 10864 18750  6697  1128 12487 18977  5445 11199 10746
      16932  5601 16270  3423  5072 16012  1609 13543 18010  5876 18551 11710
      16973  8611 17786   896 15150  6585  6520 13129  4965 18175  2832 16525
      10945 17125 18855  1748  5964  7611 14612 11300 17123 14105  1285 15156
```

```
        11382 12956 10484 17420  6607  6377  1873  5035  9405 13286  8729  9963
         8621  9241  2473 13064 14203 19067 17080 16852  9591  6583  5237  3885
        18484 18027 18467 18028 17973 17972 17970 17969  5302 16042  6762  8202
        17109  9781  3057 12195 18035 14637  2924  5223   595  5651  5083 15409
        17949 19109 11395 13131  5389  6525 18579 18245 18559 15802  6580 13199
        10789 12935 13695 14236  5044  6962 15953 11413  2116 18736  3803 13906
         7398 13922 18325 18513 18472 16404   327  5056  7353  7349  7351  7348
         7218  7214  7201  7224  7200  7220  2490  2336   322   319   316   324
        15058 19237 15907 15045 16396 16398 15912 15057 15636 16403 15079 15075
         8658 18489   331
   361:  1702  3903 18678 18677 16723  3824  7338 18840  5965 12718 13789
   362: 16281 18488 18007 18022  3371  7061  7892  1141 15264  8036  7857 19191
         7170  3442  7855 11765   326  2525 12433  5350 10188  3795  3794   974
         7376  8980  2807  2826 15414  3221 11000 16877   358 11643 14101 17431
        14104 11922 14106 14118 14103 11917 14117 11877  9156 15521 15519 15512
         2076  7599 18286 17450 10636 10883 19245  2706 17448 17445 13484 12363
         8462  8465  1521 11661 18323 10495 18481  1858 17901  1025 15261 14337
        11009   802  1489  9751  5535 19225  4741  8379  9986  5615  9570 18249
         1579 18874 14957  2833 13097 11855 17444  2048  5863 16811 12374 13302
         5440 14857 13516 10812  9419  6771 11768 12141  3543  6242 11404 18052
         1963  2636  5500  7150  2875 15645  3024 19039   805 11538  2943 12915
        17169  4471 17129  2420  9530  8029 15921 16041 11094 10747 13795  2144
        17511 12731  6924 15147 18864  7598  2259  7950 18104  7123 13183  9498
          651  4653  5099 11940  8353 13883  4389  2662  8468 19159 16364 14754
        12986  3439  9888  4998  8161 19192 17068 10691   934   606   769 18297
         1397  3167 10721 18909 18127   820  8767 11231 10209 18039  4769  4271
          914  5454 17761  1436 16749 12983  7990  3461 11482  2488  2605 15929
        19116  1132 11154  5499 16543 12081  4575 10781 15011 17427 14182 11494
         4324   686  8119 16545  1033 15056 15910  7850 13576 15542 16151 16155
        16095 13014 16069  3257  6401 17505 17504 17525 17524  2022  7377   432
        16819 10332  2411 10331  2410 12098 12865  3223  4128 11048  1117 13770
        13767 16781 16777 16779 16801 17741 16774  4860 15510 12777 12778 14071
        14067 14053 14069 14091 14072 14075  8376 11001  9987  7853  5184  7393
         7395  3219  3220 17517  3203 17996  3205 17513 10899  3204 18474 18023
          600  7911 16191 15975  4924 13238  9729  6091  4326  6820 13828  4812
         9933 17784 16424  4768 15749  7309 10904  2071  4194 15043  2025 10355
         5253 18478 13347 18509 13344 18011  8209  8302 10221 17291 15948 17479
         2853 15154 15669  4108 17549  9749 14470  4540 18372 11366 19231  5412
        12150 14436 18176 13730  8642  3638   993 11156  6014  4301  1954 10818
         7039 10003  1924 16132 13798 15583  9569 13320  9432 15846 16301 14535
         8482   674  7188 12934  6741  1610  5534  7172  3983  2230  6072 15672
         5854 17508 18490 15055  4617 16847   413  3479  1048 13574  5988  6031
        10579  4109  6095  1557 11615  5445 18508 16052 16888 13552 11081 12107
        13940 12007  8147 12487 18977 17766  8441  2008  3547  6891 18398  5601
        17125 17612 11179 15926 18113  3145 16012  1609 18010 14502  4220  6076
        11108 12418 16973  8611 17786 18551 11710  1128  4641  1538 16850 15885
         4965 15150  6585 18750  6697 10746 11219 12375 13129   559  6520  4368
         2832  3991 13543  3423  2624 18457 10864 11199 16932 16270  5072 17123
         5876 14612   896 15156 16852 18175 10945 18855 16525  1748  5964 11300
         8621  7611  9963  9241  6607  5035 14105  1285 11382 13286 12956 17420
         6377  1873 17080 19067  9405  8729 13064  2473 14203  6583 10484 11942
         9591  5237  2321  3769  3885  7954 17997 14024  5941  9954 18484 18027
        18467 18028 17973 17972 17970 17969 15630  5302  6762 16042 17109  8202
         9781  3057 12195 14637  2924 18035  5223 15409   595  5651  5083 17949
        19109 11395  6580 10789 13695 15802  5389 18245 18559  7398 13922 18325
        18513 18472   328   327  7707  7353  7349  7351  7348  7218  7214  7201
         7224  7220  7200  2490  3607 15058 19237 15045 15907 16396 16398 15912
        15057 15636 16403 15079 15075  9155 18489   330
   363: 18274 18488 18007 11117 10258  9446  3371 18022   329   360   359  7341
        17634 11532  3678 17861  1141 11850  6626  1745 15264 16539  2664  8699
         5767 11011  8036  7857  1343  6839  8733 11515 14442 16737  6301   658
         5340  6383  3012  4309  4229 16075  2351  8932 12221  5354  6706  3213
         3896 17512 15668 15120  4825 18765  7855  4601 10991  2531 10828 17127
         1411  5701 15745 17917  4012 11414  8414  1948   325  8882  3926  7772
```

```
      18792 16498 13428  7045  7919 16773  3833  8372  5231  6402  8899  4814
      12738 16127   326 12276 13535 12190 17785 10684  3455 14687  9274 12077
      15788 10337  9859 10204  9466 15723  7916 18347  6113 15339 18049 14162
      14158  7554  2228  5734  2183  1503  9273 13486  4699 18053   874  7758
       7168  2911 16068 10075  5165  6610  8188  7505  5836 10110  3449 15698
      17609  5486 10188  3795  3794   974  7376  8980  2807  2826 15414  3221
      11000 16877   358 11643 16928 14101 17431 14104 11922 14106 14118 14103
      11917 14117 11877  9156 15521 15519 15512  2076  7599 18286 16747 17450
      10636 10883 19245 18626  2706  5629 17448 17445 13484 12611 12363  8462
       8465  1521 11661 18323 10495 18481  1858  7476 18811 19045 17901  1025
      15261 14337 11009   802  1489  9751  5535 19225  4741  8379  9986  5615
       9570 18249  1579 18874 14957  2833 13097 11855 17444  2048  5863 16811
      12374 13302  5440 14857 13516 10812  9419  6771 11768 12141  3543  6242
      11404 18052  1963  2636  7150  2875  5500 15645  3024 19039   805 11538
       2943 12915 17169  4471 17129  2420  9530  8029 15921 16041 11094 10747
      13795  2144 17511 12731  1983  6924 15147 18864  7598  2259  7950 18104
       7123 13183  9498   651  5099  8353 13883  4389  2662 11940 19159 16364
       4653 14754 12986  3439  9888  4998  8161 19192 17068 10691   934   606
        769  8468 18297  1397  3167 10721 18909 18127 10323   820  8767 11231
      10209 18039  4769  4271   914  5454 17761  1436 16749 12983 10781  7990
       3461 11482  2488  2605 15929 19116  1132 11154  5499 16543 12081  4575
      15011 17427 14182 11494  4324   686  8119 16545  1033 15056 15910  7850
      13576 15542 16151 16155 16095 13014 16069  3257  6401 17505 17504 17525
      17524  2022  7377   432 16819 10332  2411 10331  2410 12098 12865  3223
       4128 11048  1117 13770 13767 16777 16779 16801 17741 16774  4860
      15510 12777 12778 14071 14067 14053 14069 14091 14072 14075  8376 11001
       9987  7853  5184  7393  7395 17909  3219  3220 17517  3203 17996  3205
      17513 10899  3204 18474 18023 18329   600  6542  7911 16191 13238 15975
       4924  9729  6091  4326  6820 13828  4812  9933 17784 16424  4768 15749
       7309 10904  2071  4194 15043  2025 10355  5253 18478 13347 18509 13344
      18011  4578  8209  8302 10221 15948  8147 11081 18398 17612 16052  2853
      15154 15669  4108   413 16888  8441  3479  1048 17549  9749 17291 14470
       2008 13574  5988  3547 13320 12107  4540  6031 16301  6076 18372 10579
       4109 11366 19231 11108  8482  5412 12150 14436 18176  1610   674  6891
      13730  8642  6095  3638   993 17479 11156  6014  4301  1954 10818  7039
      15055 12418 10003  1924 13798 11179 15926 13552  9569 18113  9432 15846
      14535  3145 12007 16132 12934  1538 16847 15583  6741  5534 14502  4641
       7188 15672  4220 11219 12375  7172  3983  2230  6072  5854 17508 17766
      18490 15885  4617  4368 16850 13940  3991  1557 11615  2624 18508 18457
      10864 18750  6697  1128 12487 18977  5445 11199 10746 16932  5601 16270
        559  5072 16012  1609 13543 18010  5876 18551 11710 16973  8611 17786
        896 15150  6585  6520 13129  4965 18175  2832 10945  3423 17125 18855
      16525  1748  5964  7611 14612 11300 17123 14105  1285 15156 11382 12956
      17420  6607  6377  1873  5035  9405 13286  8729  9963  8621  9241 13064
       2473 14203 19067 17080 10484 16852  9591  6583  5237  3885 11942 14024
       5941  2321 18484 18027 18467 18028 17973 17972 17970 17969 11616  5302
      16042  6762  8202 17109  9781  3057 12195 18035 14637  2924  5223   595
       5651  5083 15409 17949 19109 11395 13131  5389  6525 18579 18245 18559
      15802  6580 13199 10789 12935 13695 14236  5044  6962 15953 11413  2116
      18736  3803 13250 13906  7398 13922 11708  9202 18325 18513  6519 18472
        327   328  7929  7353  7349  7351  7348  7218  7214  7201  7224  7220
       7200 17337  7741  2490  6801  3458 13727 18735   316   321   333   322
        324   320   319   323  6750 15058 19237 15045 15907 16396 16398 15912
      15057 15636 16403 15079 15075 18489 13580  7766
  364:  8684 16027 13149 10126  8454 15932 11869 13702 13698  7664  8628  3039
        3267
  365: 17649  8270  3136 10804  6247  8834 17636 15016  8936  2194 13157  4323
        8741 11308   472 19056
  366:  5012  2906  8001 12356  6089 18788  4785  6186  6359  6711  3320 11092
  367:  7896 15587  5766 14289 18000 13369  8046 16461  9247 13124 10208 15984
        7503 11474  5470   765 11822
  368: 18223  5598 10881  8595 15561 10514  9251 17180 10854  6055 12225  2422
        8657 17250  3377 13990  4420  9030 19028  2248  7708 10035 12889 11370
      19121 11952  3762  3651  7635  9473 17896 19118  6913 15466  3944  6131
```

```
      3755  1161 13259  1151  5087 11439 18915  3175  5089 11438 16657  9710
     10306 19085  1527 15920 12676 13514 14962 10018 13289  7013  8470  7160
     10908   685   650 11493  4723  4184 18299   829 15453 12859 17424  9533
      2244  2847 14721 11910 12477  7174 15879  5488 12702  9161 14497 12241
      9336  7245  9646 15955 10132 17257  3161 18165 13393 18479 13438  1524
     14655 11554  4462  7586  7930 14718  5571 17190  9164 19070 13526  2863
     10779  7543  3731 10062  4442  2923 18088  2881  8943  3734  3058  4633
      3900  3667  5575  4124  5363   995  2692  5332  5916  7378  1953 15812
      2389  2390  2245  7361 10033 10128 18718 13416 17384 11063 18720   509
      3241  6075  2361   541 15106 15987  2306  2730   906  2263 12377 11712
       621  6011  6433 10564 10589  5120 18420 12406 14906  5170 16214   694
     13162  6259 18759  5659  7744  4422 17687   665  9006  8004 15938 15486
     12018  6903  5465  5202  3639 10189  4000 17403  4727 18570 18571 18591
     13103  7276  8327  8749 14147 13677  8146 10195  4684 11527 15892 11819
     15958  3517 15190 16361 11510
369:  2084 15197  8662  8366  2930  6655 12876  8740 17571 13116
370:  1613 19204 11892  6151 11834 16271  7629
371:  3593  6170  6205 17780 18378  1421  3053 15450  9886 15739 18891  6294
     10793 15529 13666 11730 18928  3004  6197 13102 16177 13928 14868 12232
      6033  7442 14783 15125  5741   864 13206  5210 12920 18629 12671 16466
      2407  5884  6651 14574
372:15623 14801   953 10002  6238 10071 13756 11624 16541 14110  3858  4119
      4412 10719
373:  4316 13909 16449 17769 15006  4781 10038 14140 13489 16035  4824  9107
      6225 16014  8175 17809  3179 14740 16152  8688 16780 13019 17782 17245
374:  6174  6173 17263  5382 13230   416 13835 10352 12036  2106  2087 12205
     12076 13351  5940  7910 11809 16730 15960 14680  8484  3972  4409  4808
     18796 15783 12008  3864   440 18038 19246 18731 16299  4005  5505  5331
     12153 14304 12102 17171
375:15552  2308 10821  4674 10575  4747 17033  5313   505  8735  1438  5247
      4174  4173 10712 12685   676 14249 18327  4576  3363 13911  9752  7128
      6923  5003   557
376:12640 10761 17514 13509 16058  1234  7711  5310
377:15091  4251  7569 13412  9186 14005 11205  6753  5318 13512  5181  3332
      4510  5730 19140 18482  2628 16322  6889 12710 13826 16117  1492  6630
     14504  6975  3194 11115  1922 18860 18335  3969 18746  3485 12262  4545
      3701 15340 14247  4624  9240  7559 17795  2280 18217  2121 14191 14648
     17689 18547  7154  2925 17348 10160 18995 18701 16015  2369 13208 13699
      5625  2618  8394 17231  2934  1031  4588   931  5715 10557 13832  6308
       799  3921  1420  3674  7819 11623   713 16019  7524 10823  6351 10511
     18737  6199  7305   958 10010 12423  8451 14867 14792  1835 18830  9571
     17441 12749 16458  2477  3430 14035 18847 18001  3771  1319  3705 18504
      5474  4726  5270   785 18382  4164  8734   957  4755
378:  7385  7389  4712  7216  7240  7425  7411 16142  3882  7263  7262  4838
      7264  7267  7288  7269  7239  8013  6310  7299  7296 15830  4115 14488
      4236  2148 12770  5634  8293 18656  9110  9130  5827 14078 13597  7511
      7494  7198 11579  7426  7786  7194  7444  3270 15500  8339  1316 19032
      6299  1170  8702 13733 10030 10478 10019 10089  4520  3432 18377 18380
      8333  8284  8313  2075  6604 16849  5563 10245  3095 10445 11875  6960
     10489  4952  7044  8314 17763  8310 12788  8318  7964  8340  8343  8346
      1962  8847  2058 16080  3127 15015 17613 17633  2348  8003  7373  7372
     15767 12500 16065 11929  8086  6387 14998  6461 11845 11762 11109  7190
     17213  5075  5076  7367  7365 13195  7449  7491  7448  7535 11930  7290
      7294  1927  1929  1975  1976 12927  8317
379:   789  2899  9238  3272  5648  3579 15295  6275 14031 16259  5495 10963
      5221  2572 18153 15647  4771  8787 11837  4871  6699 10308 17936 16583
     14963  7534 12772  9430 13248  6068 13071
380:11132 17164 10120 13780 18790  3980  6453 16881  5663  9535 19104  1529
     18961  2182  9230  8958 10766  3066 14515 16060  4872 16197  5238 18266
381:  8677  2479  7211  7425  4341  4838  7350  7264  8013  7326  7209  7235
     15830  5983  5979  5985 18656  7511  7198 15500  8339   627  6299 16863
      8702 15217 15219 15220 12265  2585 12272 12270 12268  8753  3061 11536
     11534  4520  3432 18952 18377 18380  8333  8284  8313  2075  4839  6604
      5557  3612  4952  7044  3124 13964 13963  7964  8343  8346 16080  7195
```

```
         8003  7373 16065  8853 11929  7949  7962  6461  3480 11762 11845 17213
        13195  7449 12348  7448  7446 11930  6644 17544  1979  1976   688 12002
          692 12927 12924 12921  8317
   382: 6843  6987 13364 15545 14423  4921  4285  2528  2557 18040  8350  9340
   383:15954  6049 18779 18043  2249 15965
   384: 7925 14352 11545 10615 16765 15479  8141 14076 15758  5411 12128 15947
         3153  8503 16045 11187  3686  9965  1284  5392 16793 15607  5705  3059
         7149
   385:12678 11761 19097 15082 10667  4225 11019  8039  8375 17367  9578 18072
   386:10572 14694  5590  4876  3577 12056 17885 18628 17398  7894  7122   673
         4475
   387:13459  5045 12999  2199 13901 17853 19179 15940  9881 12409  5036 14275
        18503  8364  2168  8079  4928  2375  2053 17350 16572 10390  3007  7945
         7762   641
   388:16871  9761 11846 15784 18262 18446 17041  2127  8909 17081  8129 10993
         5337  2955 10550  1632  4698 18308 13085 15485 13327 16105
   389: 2476  3590  2914 12550 12552  7501 14570 15819 11167  4325 19077  6830
         4556 10406 12930  5172  5060 12389  3569 16656  5168  9775 14074 17762
         9431  2976 16817 11358 11360  2041 11375 10922 11355 11359 14965 10920
        10918  3529  4796 11740  2289 15427  7180  9275  4129  7616 13151 18966
        10689 14083  6228  2080 13696  5628 10009   411 10499 15596  8605 17387
   390:13101  8214 18411  6355 17130 17131 17112 17106 11037  5175  5207 16634
        16638 18236 18250 13007 12625 10843 10358  4002   905  7460 17283 10362
        10388 10845 13303  2314 12875 17641  7106 16084  4137  9491  4494 14185
        14189 14160 13722 10262  9561 10261  2652 16419 14234  3867  3394  4122
        16608 17323 18139 16832 11596 16537 14193 14190 14231 14212 17832  7504
        18708  7840 16675 16237  7108 13984  2646 18664 14232  8358 12918  4473
         4497   431 13345 11107 14988 10858  8415 10522 18463  2013 10896 13582
         1453  8762 16443 14346 11683  2065 10233 10846 10363 10391 10393 14422
         9935 17273 17249  8363 10121 14942
   391: 7780  7778  5749 16030  8501 14291 14288  6980  8600  7808 14360 14094
         2039  3393 16652  5033  5164 14882 16247  4249 12196 17478  5851   448
        19005
   392: 9293 16557   723  6692  9622  2591 13628  6928  1592
   393: 5309 16676  1992  5415 17277  4097  6941 16134  3966 10723 12275 12413
         9592
   394:10929  8533 15300  1943  2871  3355  8440  1643  4780 15402 10696  4627
         2660  3426  1295  1614   466 18055 18870  5343  6172 13204  2509 14838
        16696 14719 12186  9237  5229 14362 18820 11703 10441  9402  3427  6904
         6394 15444 13539   480  4107  3143 11612 16378  9125  5638  5458  4628
        12830 16118 11849  7565 14432
   395:17920  6614 14177  8433 18119 14743  1547  2798  4829 13098  1792  7185
         5546 12686 17738 14286 17308 15203  7356 15424  8898 10366
   396: 1612 16074 15966  6215  9512  6739   712  4754  9542   926 15250 17094
         3886  1518 14141  3582 17743  2596 16577  7981  8476 11139  9991  7519
        16523  4476  3159  2443
   397: 6955 17338  4807 13366 13886  3990 12822  4206 11204  4966 12012 18854
        18590 18505  5937  6734  2714  7052  1150  4851  7568 10870  5135  3950
         2713   870 17666  8862   972  4428  1180 15407
   398:10762 10763 17831 18710  4571  2491 18826  7281  7980 12839  7132  3515
        11431 13508 15105 11150 12370 14765  7924  8625 10099  5689 10100  7454
        10434  8719  3324  3759  1396  7985  1985
   399: 6493 10378 18309  8131  4746  2656  3568 15048  5685  9021 16845  8807
        18908  2035 18616 19229  9844 10776 11514 15103  1336  4804 17204 14756
        12584 18287 12312  5524 13410  6108 12653 16440 10889  4657  4508 11491
         4103
   400:14710 10682 11646 10632  9092  7695  1867 15667  2172 14920 10057  4313
        15838  6000 16168 13497 12464  7428  3227 15903 11817  6518  7230 15193
        14479 15307  9534  8999 10201
   401: 4269   415  5196  1888 17473  5940 17913  2427  4192   894  2283  3972
         4409  4808 15898 14757 16253 13957
   402: 8349  2740  7671 12717  5139 12414  4134  7424 16056 14823
   403: 1589 17604 12728  8019  2032  1121  5422  4393   814  3576  1329 18252
        17534  7522  6755  8555 13532 14465  5342 14260 12366 13545 11833 15130
```

95

```
       16303   3048 15435 12548 12465  9395  4550 15303 15548 16379 15934  3788
       11207 10866 16567  9796 10597  2843  9814  6036 13084 15810 18073 12289
        2610  8431  8133 10743  5277  5274  9812  9270 16062  1410 18832  3278
       11379  7651  8619  4074 13579 11278  7552  3482  1921  3110 13040  3715
         588 16215 14079 15855 15077 12165  4158 14545  7138 12317 14284 16674
        7176  4557  4843  4827 14556 15959 12712 12693 12300  9895  9617 18976
        3521 15031  1253  9850  1713   948 17470  9817  8246 14610 14409  4404
       18149 18916  3122  6477 11641  5344  5193 15313  4566  4856  6382  6385
        8287 13657  6526 12895 13000 17835  5551  4058  4427  6492  3925 15887
        6882 13235  2054 11583  8492  7588 15092   977  3464  6674 11033 10561
       12912 12978 14142 19136   951 13846  6528 11069 16860 10474 14237 10178
        9206  9999 12994  1371  1369 18499 15946 14691  5796 15067 13591 14544
       10116  9572  9076  3395 18068  9445 17137 10070 12598 12451  5291  8421
       13493 12446 16898  3422 12469  9327 12751  1725 12393 11867 16709  6201
        2489 19025 15355 12432 10704 10788 15175 13881 15050 11031  2284 16846
        8929 13843 15877 12575  2501  3898  8155  8181 13467  7071 13380 18512
        6767 11884  3330 15689 16649  4862  2400 11639  2206  6774  7277 10750
       13838  5371  9923  6934 11974 17225  3561 17146 15437  5307  6795  8299
        5491  4199 12086 13575  6650 12452 12843  7943  4725  8051 12281 12760
        4561  2302  8823  7236 18407 18993  1395 18587 10428  1484  9297  4090
        6052   746  5639  5240 11345  8048 13454  3013  3772  3177  2098  1828
        5023 13427 15578 11178  5602  5276 14414  2819   615  9528  3937  5441
        9527 18009 11254 11735  1651 10853  8300 12353 10928  8198 10913 11954
       11949 16408 11950  8525  8047 17449 14589 10655 14519  9626 17814  7897
        3029  4632 16072  1825 10344 16933  7125 16977   487  5370  1337 10164
        7994 14863  4232  4289 11276  8180 14167 10838 18522 10765 13322  2506
       18787   776 16529  6541  4678  5631 12943 17667  6970 19144  3198  7846
        2492 15690 17586 13301  4379  2818 10177  3242  3316   949  9487  8259
       11112  6256 10981 15481 11590 14273  5962  3698 13133  1978 10200  2281
       15318 15337 15317  1960 10987 10959 10458  6605  1442 14285 16347  8163
        9798  2999  6258   594 19224 11771  3948   319 18283 17226 10012  7302
       16116 14915  6717 19232  2868 18598  8016  9887 12616
404:  4607  2496  4440 11086  9902 13066 10299 13067 10058  7029 11937 11215
        8917 18762  8922  6629  3169  4418  7255 17978  6392  4006 16582  4177
       13299 18105 19050  1884  8982 18462 13743 10049 17020  1107 14164 17443
        3211   807  9415  7769  2665  7716  9819  4793  8194  4487  9462  1406
        8450 11140  4167 18203  3917 15866  2776  1567  9782 15174 12626  4681
       12824 14018  4910  4659  2562  2122  2788  9880  2428   871  8389  7192
       16613  2982  3929 11900  8750  5259 18517 14460  1370 18379  7537 11555
        4777 12893 14616 15017  5702 12071  2926  2018  2392 18128 17823 17870
       13449 12192  1385  8320 11784  8264 14559  5910 11792  8248  1361 17542
        1038 17221  1250 12347 10203 15635  7181  7956 13405  1627 10660  3003
        6686 18312 14977  2919 13737  1505  4646  9493 11913  9654 14413 16822
       11895 12844  7146 17760 12372 14263 18841 10587 16428 18770 12264 16337
       14633 11408 17098  2635  7437 18818 16147 17848 15393  9910  6063  5725
        6571  5134 17464 10392  6472 17903
405:  3906  4734 13745  7402 14874   764
406:  5355  6984  4667 17305 11490 16367  8426 10381  1464 11818   624  7412
        3333  9382 15475 11760 13337
407:12506 19148 10381  2347  9031  1569
408:  1173  2332  4489
```

## Example 3

This example illustrates the construction of a consensus amino acid sequence of homologous proteins of homologous genes that impart an enhanced trait.

ClustalW program was selected for multiple sequence alignments of the amino acid sequence of SEQ ID NO: 406 and 17 homologs. Three major factors affecting the sequence alignments dramatically are (1) protein weight matrices; (2) gap open penalty; (3) gap extension penalty. Protein weight matrices available for ClustalW program include Blosum, Pam and Gonnet series. Those parameters with gap open penalty and gap extension penalty were extensively tested.

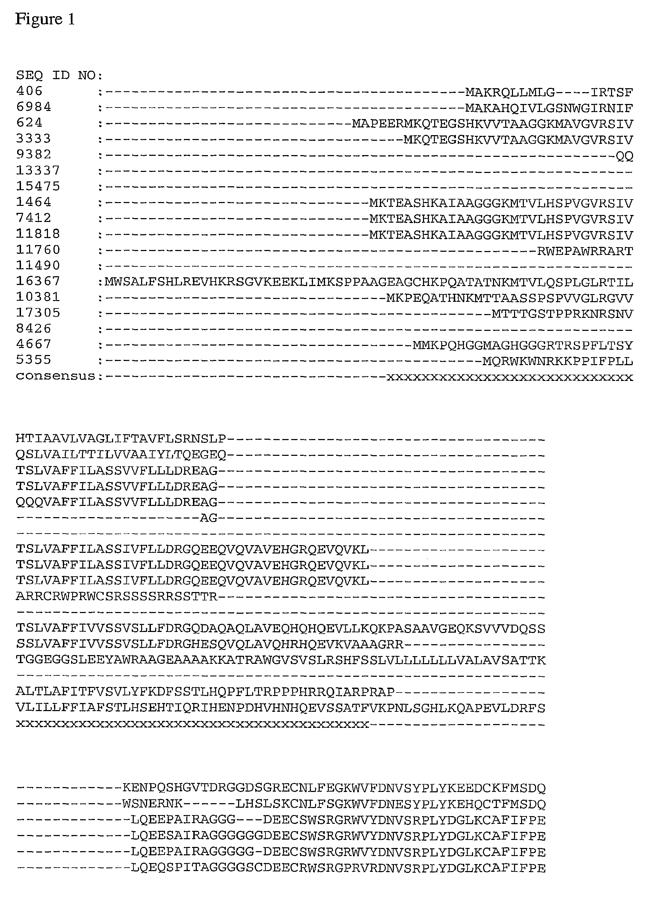

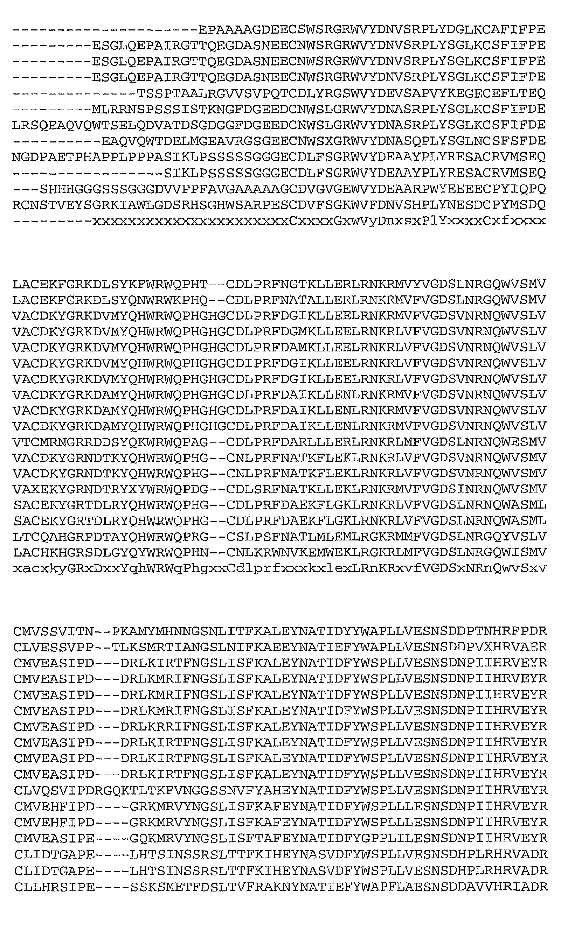

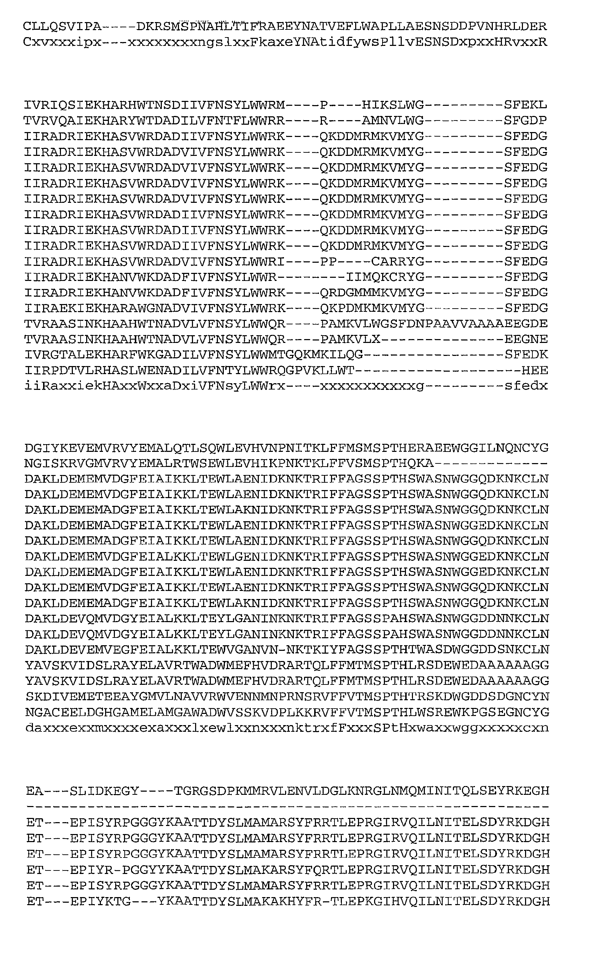

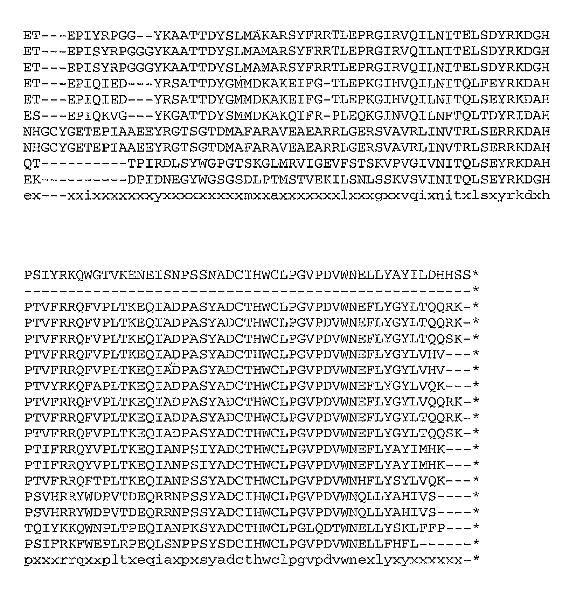

On the basis of the test results, Blosum weight matrix, gap open penalty of 10 and gap extension penalty of 1 were chosen for multiple sequence alignment. Shown in Figure 1 are the sequences of SEQ ID NO: 406, its homologs and the consensus sequence, as set forth in SEQ ID NO: 19248. The symbols for consensus sequence are (1) uppercase letters for 100% identity in all positions of multiple sequence alignment output; (2) lowercase letters for >=70% identity; symbol; (3) "X" indicated <70% identity; (4) dashes "-" meaning that gaps were in >=70% sequences.

The consensus amino acid sequence can be used to identify DNA corresponding to the full scope of this invention that is useful in providing transgenic plants, for example corn and soybean plants with enhanced agronomic traits, for example improved nitrogen use efficiency, improved yield, improved water use efficiency and/or improved growth under cold stress, due to the expression in the plants of DNA encoding a protein with amino acid sequence identical to the consensus amino acid sequence.

## Example 4

This example illustrates the identification of amino acid domain by Pfam analysis.

The amino acid sequence of the expressed proteins that were shown to be associated with an enhanced trait were analyzed for Pfam protein family against the current Pfam collection of multiple sequence alignments and hidden Markov models using the HMMER software in the appended computer listing. The Pfam protein families for the proteins of SEQ ID NO: 205 through 408 are shown in Table 16. The Hidden Markov model databases for the identified patent families are also in the appended computer listing allowing identification of other homologous proteins and their cognate encoding DNA to enable the full breadth of the invention for a person of ordinary skill in the art. Certain proteins are identified by a single Pfam domain and others by multiple Pfam domains. For instance, the protein with amino acids of SEQ ID NO: 214 is characterized by two Pfam domains, i.e. "C1_4" and "Ssl1". See also the protein with amino acids of SEQ ID NO:222 which is characterized by two copies of the Pfam domain "MatE". In Table 16 "score" is the gathering score for the Hidden Markov Model of the domain which exceeds the gathering cutoff reported in Table 17.

Table 16

| PEP SEQ ID NO | Pfam domain name | begin | stop | score | E-value |
|---|---|---|---|---|---|
| 206 | zf-A20 | 10 | 34 | 35 | 2.40E-07 |
| 206 | zf-AN1 | 104 | 144 | 64.5 | 3.10E-16 |
| 207 | Cyclin N | 33 | 168 | 12.1 | 0.00023 |
| 208 | Gpi16 | 9 | 603 | 1272 | 0 |
| 212 | BSD | 100 | 167 | 44.7 | 2.80E-10 |
| 212 | BSD | 179 | 244 | 67.5 | 3.90E-17 |
| 213 | Catalase | 18 | 401 | 977.7 | 3.80E-291 |
| 214 | Ssl1 | 22 | 277 | 646.3 | 2.20E-191 |
| 214 | C1 4 | 361 | 409 | 101.1 | 2.90E-27 |
| 215 | Bromodomain | 119 | 208 | 105.8 | 1.10E-28 |
| 216 | Pkinase | 75 | 343 | -9.1 | 1.60E-07 |
| 217 | PTR2 | 133 | 544 | 197.2 | 3.50E-56 |
| 218 | FTCD N | 5 | 195 | 377.6 | 1.70E-110 |
| 219 | Abhydrolase 1 | 50 | 272 | 30.4 | 3.00E-06 |
| 220 | HhH-GPD | 172 | 317 | 88.2 | 2.30E-23 |
| 222 | MatE | 40 | 200 | 121.9 | 1.60E-33 |
| 222 | MatE | 261 | 433 | 96 | 1.00E-25 |
| 224 | ubiquitin | 48 | 120 | 24.7 | 0.00029 |
| 224 | BAG | 138 | 219 | 94.2 | 3.50E-25 |
| 225 | Lipase GDSL | 37 | 365 | 370.9 | 1.80E-108 |
| 226 | DUF231 | 280 | 449 | 234.8 | 1.60E-67 |

(continued)

| PEP SEQ ID NO | Pfam domain name | begin | stop | score | E-value |
|---|---|---|---|---|---|
| 227 | Aldedh | 16 | 485 | 842 | 2.80E-250 |
| 228 | RRM 1 | 110 | 181 | 79.9 | 7.10E-21 |
| 228 | RRM 1 | 214 | 284 | 87.8 | 2.90E-23 |
| 229 | GATase 2 | 2 | 162 | 10.5 | 7.60E-12 |
| 229 | Asn synthase | 211 | 478 | 335.9 | 6.10E-98 |
| 230 | zf-C2H2 | 273 | 295 | 30.6 | 5.00E-06 |
| 231 | HLH | 62 | 113 | 39 | 1.50E-08 |
| 232 | PP2C | 39 | 323 | 106.9 | 5.20E-29 |
| 233 | zf-C3HC4 | 96 | 137 | 33.4 | 7.10E-07 |
| 234 | AP2 | 83 | 146 | 144.5 | 2.60E-40 |
| 235 | Homeobox | 84 | 145 | 72.6 | 1.10E-18 |
| 236 | zf-C3HC4 | 87 | 129 | 31.8 | 2.10E-06 |
| 237 | Acyl transf 1 | 52 | 354 | -13.2 | 6.40E-11 |
| 238 | Aldo ket red | 5 | 292 | 465.6 | 5.50E-137 |
| 239 | adh short | 13 | 179 | 55.1 | 2.00E-13 |
| 240 | HMG CoA synt | 5 | 453 | 992.9 | 1.00E-295 |
| 241 | SRF-TF | 9 | 59 | 121.8 | 1.80E-33 |
| 241 | K-box | 75 | 175 | 148.8 | 1.20E-41 |
| 242 | Ribosomal S8e | 1 | 237 | 327.1 | 2.70E-95 |
| 243 | zf-C3HC4 | 103 | 144 | 37.8 | 3.30E-08 |
| 244 | zf-C2H2 | 4 | 26 | 22.3 | 0.0015 |
| 244 | zf-C2H2 | 204 | 226 | 22.4 | 0.0014 |
| 244 | zf-C2H2 | 236 | 258 | 20.6 | 0.005 |
| 245 | Aa trans | 45 | 439 | 358 | 1.30E-104 |
| 246 | LEA 4 | 71 | 141 | 15.6 | 0.05 |
| 247 | p450 | 66 | 499 | 209.4 | 7.60E-60 |
| 248 | p450 | 40 | 503 | 289.8 | 4.70E-84 |
| 249 | Phi 1 | 25 | 278 | 515 | 7.40E-152 |
| 250 | zf-B box | 3 | 47 | 56.2 | 9.50E-14 |
| 251 | PP2C | 84 | 348 | 267.9 | 1.80E-77 |
| 254 | Radical SAM | 171 | 337 | 77.3 | 4.40E-20 |
| 255 | Pkinase | 25 | 283 | 174.5 | 2.30E-49 |
| 256 | adh short | 6 | 182 | 42.9 | 9.80E-10 |
| 257 | Skp1_POZ | 4 | 64 | 105.1 | 1.80E-28 |
| 257 | Skp1 | 112 | 190 | 173 | 6.70E-49 |
| 258 | DPBB_1 | 73 | 151 | 142.6 | 9.30E-40 |
| 258 | Pollen_allerg_1 | 162 | 239 | 163.9 | 3.70E-46 |
| 259 | LRRNT_2 | 18 | 58 | 40.7 | 4.40E-09 |

(continued)

| PEP SEQ ID NO | Pfam domain name | begin | stop | score | E-value |
|---|---|---|---|---|---|
| 259 | LRR_1 | 86 | 108 | 12.3 | 1.6 |
| 259 | LRR_1 | 109 | 133 | 15.7 | 0.15 |
| 259 | LRR_1 | 134 | 157 | 17.4 | 0.047 |
| 259 | LRR_1 | 158 | 177 | 8.3 | 11 |
| 259 | LRR_1 | 179 | 202 | 12.5 | 1.3 |
| 259 | Pkinase | 334 | 601 | 3.8 | 2.90E-08 |
| 260 | Methyltransf 11 | 135 | 225 | 25.7 | 0.00015 |
| 261 | Ubie_methyltran | 34 | 287 | 368.5 | 9.10E-108 |
| 261 | Methyltransf_11 | 88 | 205 | 70.4 | 5.00E-18 |
| 261 | Methyltransf 12 | 88 | 203 | 38.6 | 1.90E-08 |
| 262 | MtN3_ slv | 11 | 100 | 69.4 | 9.90E-18 |
| 262 | MtN3_ slv | 135 | 221 | 122.5 | 1.10E-33 |
| 263 | Cys_ Met_Meta_PP | 88 | 460 | 768.4 | 3.90E-228 |
| 263 | Beta_elim_lyase | 129 | 376 | -107.7 | 0.0016 |
| 264 | PAR1 | 1 | 181 | 469.5 | 3.60E-138 |
| 265 | Chal_sti_synt_C | 350 | 493 | 13.4 | 0.00012 |
| 266 | Cyclin_N | 43 | 195 | 113.1 | 7.30E-31 |
| 267 | Pkinase | 21 | 418 | 193.4 | 5.00E-55 |
| 268 | Brix | 96 | 271 | 216.4 | 5.60E-62 |
| 269 | tRNA-synt_2b | 81 | 250 | 166.9 | 4.70E-47 |
| 269 | HGTP_ anticodon | 402 | 486 | 17.1 | 0.00088 |
| 270 | Proteasome | 38 | 233 | 81.4 | 2.50E-21 |
| 271 | Ammonium_transp | 19 | 423 | 597.5 | 1.10E-176 |
| 272 | AUX_IAA | 8 | 204 | 332.3 | 7.20E-97 |
| 273 | Lectin_legB | 25 | 215 | -14.8 | 1.60E-09 |
| 273 | Lectin_legA | 236 | 279 | 31 | 3.70E-06 |
| 273 | Pkinase | 355 | 624 | 179.3 | 8.50E-51 |
| 273 | Pkinase_Tyr | 355 | 624 | 116.1 | 9.20E-32 |
| 274 | PGK | 2 | 395 | 675.6 | 3.30E-200 |
| 275 | Sugar_tr | 27 | 491 | 416.7 | 2.90E-122 |
| 275 | MFS_1 | 31 | 467 | 75.7 | 1.30E-19 |
| 276 | Hpt | 30 | 112 | 54.9 | 2.30E-13 |
| 278 | RRN3 | 37 | 620 | 1128.7 | 0 |
| 279 | Sugar_tr | 89 | 546 | 595 | 6.00E-176 |
| 279 | MFS_1 | 93 | 505 | 100.6 | 4.10E-27 |
| 280 | AP2 | 25 | 88 | 133.6 | 4.70E-37 |
| 281 | Pkinase | 4 | 205 | 28 | 1.20E-09 |
| 282 | ADH_N | 33 | 119 | 53.8 | 5.30E-13 |

(continued)

| PEP SEQ ID NO | Pfam domain name | begin | stop | score | E-value |
|---|---|---|---|---|---|
| 282 | ADH_zinc_N | 155 | 318 | 43.1 | 8.50E-10 |
| 283 | ADH_N | 34 | 149 | 129.2 | 1.00E-35 |
| 283 | ADH_zinc_N | 180 | 315 | 119.1 | 1.10E-32 |
| 284 | ADH_N | 40 | 165 | 112.8 | 8.90E-31 |
| 284 | ADH_zinc_N | 196 | 338 | 125.2 | 1.60E-34 |
| 285 | ADH_N | 43 | 173 | 105.4 | 1.50E-28 |
| 285 | ADH_zinc_N | 204 | 348 | 112.5 | 1.10E-30 |
| 286 | Mov34 | 23 | 133 | 136.1 | 8.40E-38 |
| 287 | RRM 1 | 36 | 107 | 101.5 | 2.20E-27 |
| 288 | RRM 1 | 36 | 107 | 73 | 8.20E-19 |
| 289 | FA desaturase | 54 | 269 | 135 | 1.80E-37 |
| 290 | Ras | 15 | 176 | 336.7 | 3.50E-98 |
| 291 | SAC3_GANP | 24 | 209 | 128.9 | 1.20E-35 |
| 292 | RNA pol_L | 6 | 83 | 75.5 | 1.50E-19 |
| 293 | GSHPx | 8 | 117 | 234.4 | 2.20E-67 |
| 294 | FKBP C | 115 | 214 | 127.7 | 3.00E-35 |
| 295 | Ras | 10 | 171 | 339 | 7.10E-99 |
| 296 | UQ con | 15 | 148 | 81.4 | 2.50E-21 |
| 297 | Proteasome | 28 | 215 | 246.5 | 4.90E-71 |
| 298 | Ras | 14 | 175 | 317.4 | 2.20E-92 |
| 299 | Ldh_1_N | 83 | 226 | 247.5 | 2.40E-71 |
| 299 | Ldh_1_C | 228 | 394 | 199.8 | 5.50E-57 |
| 300 | Ras | 8 | 209 | 221.5 | 1.60E-63 |
| 301 | ThiF | 30 | 167 | -12.5 | 3.50E-05 |
| 303 | Sterol desat | 10 | 215 | 128.3 | 1.90E-35 |
| 304 | Sterol desat | 12 | 227 | 195.1 | 1.50E-55 |
| 306 | Enolase_N | 3 | 133 | 227.6 | 2.40E-65 |
| 306 | Enolase_C | 138 | 424 | 567.5 | 1.10E-167 |
| 307 | NTP_transferase | 4 | 267 | 89.5 | 9.20E-24 |
| 308 | NAD_binding_2 | 16 | 185 | -39.1 | 1.10E-05 |
| 308 | NAD_Gly3P_dh_N | 17 | 154 | -24.6 | 0.00015 |
| 308 | F420_oxidored | 18 | 265 | 341.2 | 1.60E-99 |
| 309 | F420_oxidored | 4 | 255 | 278.2 | 1.40E-80 |
| 310 | ADH_N | 27 | 145 | 115.5 | 1.40E-31 |
| 310 | ADH_zinc_N | 175 | 318 | 138.1 | 2.10E-38 |
| 311 | Aminotran_1_2 | 44 | 399 | 178.8 | 1.20E-50 |
| 312 | Aldedh | 11 | 476 | 903.9 | 6.50E-269 |
| 313 | NTP_transferase | 5 | 269 | 52.4 | 1.40E-13 |

(continued)

| PEP SEQ ID NO | Pfam domain name | begin | stop | score | E-value |
|---|---|---|---|---|---|
| 314 | PFK | 6 | 281 | 515.1 | 7.10E-152 |
| 315 | NTP_transferase | 10 | 288 | 20.8 | 2.90E-11 |
| 316 | Aminotran_1_2 | 114 | 480 | 492.5 | 4.40E-145 |
| 317 | NTP_transferase | 94 | 349 | 346.3 | 4.40E-101 |
| 318 | Aldedh | 13 | 475 | 734.7 | 5.60E-218 |
| 319 | Aminotran_3 | 113 | 448 | 471.8 | 7.60E-139 |
| 320 | Cys_Met_Meta_PP | 117 | 395 | -276.6 | 0.0042 |
| 320 | Aminotran_1_2 | 118 | 471 | 184.5 | 2.30E-52 |
| 321 | Aminotran_1_2 | 121 | 483 | 125.6 | 1.20E-34 |
| 322 | DAO | 214 | 489 | -32.3 | 0.0011 |
| 322 | Pyr_redox_2 | 214 | 474 | 69.5 | 9.70E-18 |
| 322 | Pyr_redox | 343 | 433 | 55.7 | 1.40E-13 |
| 323 | PGM_PMM_I | 127 | 272 | 140.6 | 3.80E-39 |
| 323 | PGM_PMM_II | 297 | 407 | 69.6 | 8.70E-18 |
| 323 | PGM_PMM_III | 408 | 528 | 105.7 | 1.20E-28 |
| 323 | PGM_PMM_IV | 543 | 632 | 55.6 | 1.50E-13 |
| 324 | Aminotran_3 | 192 | 522 | 634.1 | 1.00E-187 |
| 325 | Biotin_lipoyl | 91 | 164 | 82.6 | 1.10E-21 |
| 325 | E3_binding | 198 | 234 | 74.5 | 3.00E-19 |
| 325 | 2-oxoacid_dh | 262 | 493 | 486.5 | 2.70E-143 |
| 326 | Biotin_lipoyl | 91 | 164 | 79.5 | 9.50E-21 |
| 326 | E3_binding | 198 | 234 | 76.7 | 6.40E-20 |
| 326 | 2-oxoacid_dh | 261 | 492 | 483.1 | 2.90E-142 |
| 327 | Transaldolase | 101 | 401 | 580 | 2.00E-171 |
| 328 | Biotin_lipoyl | 92 | 165 | 88.9 | 1.40E-23 |
| 328 | E3_binding | 201 | 237 | 69 | 1.40E-17 |
| 328 | 2-oxoacid_dh | 261 | 491 | 478.4 | 7.90E-141 |
| 329 | cNMP binding | 103 | 191 | 73.7 | 5.30E-19 |
| 330 | HI0933_like | 90 | 415 | -239.1 | 0.0006 |
| 330 | FAD_binding 2 | 91 | 401 | -113.1 | 0.0016 |
| 330 | GIDA | 91 | 420 | -208 | 0.00018 |
| 330 | DAO | 91 | 353 | -32.7 | 0.0012 |
| 330 | Pyr_redox 2 | 91 | 399 | 237.8 | 2.10E-68 |
| 330 | Pyr_redox | 261 | 354 | 100.9 | 3.30E-27 |
| 330 | Pyr_redox dim | 428 | 537 | 164.9 | 1.90E-46 |
| 331 | GIDA | 94 | 435 | -211.8 | 0.00032 |
| 331 | Pyr_redox 2 | 94 | 413 | 217.1 | 3.40E-62 |
| 331 | Pyr_redox | 271 | 368 | 107.5 | 3.60E-29 |

(continued)

| PEP SEQ ID NO | Pfam domain name | begin | stop | score | E-value |
|---|---|---|---|---|---|
| 331 | Pyr_redox dim | 443 | 552 | 193.7 | 3.90E-55 |
| 332 | FAD_binding 2 | 99 | 410 | -122.5 | 0.0042 |
| 332 | Pyr_redox 2 | 99 | 409 | 275 | 1.30E-79 |
| 332 | Pyr_redox | 266 | 362 | 114.7 | 2.30E-31 |
| 332 | Pyr_redox dim | 437 | 546 | 202.7 | 7.40E-58 |
| 333 | Biotin_lipoyl | 92 | 165 | 81.4 | 2.40E-21 |
| 333 | E3_binding | 202 | 238 | 68.7 | 1.60E-17 |
| 333 | 2-oxoacid_dh | 261 | 491 | 485.1 | 7.30E-143 |
| 334 | DUF6 | 117 | 242 | 57.9 | 3.00E-14 |
| 334 | DUF250 | 251 | 397 | 196.5 | 5.70E-56 |
| 335 | Alpha-amylase | 61 | 489 | -62.8 | 4.70E-06 |
| 336 | GAF | 141 | 304 | 86.6 | 6.80E-23 |
| 336 | Phytochrome | 315 | 501 | 33 | 8.10E-07 |
| 336 | HWE_HK | 520 | 600 | 115.8 | 1.10E-31 |
| 336 | Response_reg | 732 | 848 | 36.6 | 7.80E-08 |
| 337 | NIR_SIR_ferr | 69 | 137 | 83 | 8.50E-22 |
| 337 | NIR_SIR | 169 | 332 | 206.4 | 5.80E-59 |
| 337 | NIR_SIR_ferr | 348 | 419 | 75.4 | 1.50E-19 |
| 338 | Fer4 | 9 | 32 | 9.4 | 0.011 |
| 338 | Fer4 | 177 | 202 | 9.1 | 0.012 |
| 339 | PGI | 52 | 541 | 1099 | 0 |
| 340 | Molybdop_Fe4S4 | 2 | 68 | 80.6 | 4.30E-21 |
| 340 | Molybdopterin | 70 | 501 | 390.7 | 1.90E-114 |
| 340 | Molydop_binding | 627 | 738 | 174.9 | 1.80E-49 |
| 341 | PGI | 55 | 545 | 751.6 | 4.50E-223 |
| 342 | Iso_dh | 28 | 469 | 379.1 | 6.10E-111 |
| 343 | NIR_SIR_ferr | 78 | 147 | 71.9 | 1.80E-18 |
| 343 | NIR_SIR | 179 | 338 | 199.5 | 6.80E-57 |
| 343 | NIR_SIR_ferr | 354 | 425 | 67.3 | 4.50E-17 |
| 344 | DUF783 | 27 | 236 | 348.6 | 9.00E-102 |
| 346 | PA | 43 | 144 | 89.4 | 9.50E-24 |
| 346 | zf-C3HC4 | 232 | 273 | 34.7 | 3.00E-07 |
| 347 | Peptidase_C26 | 13 | 248 | 158.9 | 1.10E-44 |
| 348 | SURF5 | 17 | 143 | 252 | 1.10E-72 |
| 349 | DUF962 | 7 | 171 | 329.8 | 4.20E-96 |
| 350 | Cyclin_N | 191 | 318 | 198.5 | 1.40E-56 |
| 350 | Cyclin_C | 320 | 447 | 173.7 | 4.00E-49 |
| 351 | WRKY | 237 | 297 | 137.6 | 2.90E-38 |

(continued)

| PEP SEQ ID NO | Pfam domain name | begin | stop | score | E-value |
|---|---|---|---|---|---|
| 352 | SBDS | 6 | 244 | 261.6 | 1.40E-75 |
| 353 | DUF167 | 38 | 112 | 82.3 | 1.40E-21 |
| 355 | zf-CCCH | 2 | 25 | 30.5 | 5.40E-06 |
| 356 | DIM1 | 5 | 139 | 78.9 | 1.40E-20 |
| 358 | Rieske | 100 | 198 | 68.5 | 1.90E-17 |
| 359 | Fe bilin red | 112 | 333 | 416.6 | 3.20E-122 |
| 360 | Radical SAM | 123 | 282 | 47.3 | 4.70E-11 |
| 361 | LEA 4 | 107 | 180 | 55.3 | 1.80E-13 |
| 363 | Pribosyltran | 92 | 236 | 146.3 | 7.30E-41 |
| 364 | Rhodanese | 254 | 367 | 102 | 1.60E-27 |
| 367 | DRMBL | 248 | 368 | 129 | 1.20E-35 |
| 368 | MIP | 30 | 251 | 276.9 | 3.60E-80 |
| 369 | EBP | 23 | 219 | 362.2 | 7.50E-106 |
| 371 | CcmH | 1 | 139 | 16.6 | 6.30E-09 |
| 373 | CTP transf 2 | 30 | 170 | 48.4 | 2.20E-11 |
| 374 | AP2 | 46 | 109 | 145.7 | 1.10E-40 |
| 375 | HLH | 48 | 97 | 52.3 | 1.40E-12 |
| 376 | Myb DNA-binding | 59 | 104 | 58.1 | 2.50E-14 |
| 377 | Ham1p_like | 12 | 188 | 153.2 | 6.10E-43 |
| 378 | SRF-TF | 9 | 59 | 112.2 | 1.40E-30 |
| 378 | K-box | 74 | 174 | 76.9 | 5.80E-20 |
| 379 | Response reg | 10 | 152 | 75.9 | 1.20E-19 |
| 380 | Nuc sug_transp | 105 | 341 | 17.6 | 1.50E-14 |
| 381 | SRF-TF | 9 | 59 | 105.7 | 1.20E-28 |
| 381 | K-box | 70 | 167 | 6.3 | 0.00011 |
| 382 | E2F TDP | 16 | 81 | 111.2 | 2.60E-30 |
| 382 | E2F TDP | 151 | 231 | 128 | 2.30E-35 |
| 384 | RNA pol_Rpb6 | 61 | 114 | 86.8 | 5.90E-23 |
| 386 | Myb DNA-binding | 45 | 96 | 41.3 | 2.90E-09 |
| 387 | zf-C3HC4 | 83 | 122 | 41.3 | 2.90E-09 |
| 388 | zf-C3HC4 | 42 | 86 | 35.1 | 2.20E-07 |
| 389 | AUX_IAA | 11 | 234 | 381.5 | 1.10E-111 |
| 390 | Myb_DNA-binding | 14 | 61 | 49.1 | 1.30E-11 |
| 390 | Myb_DNA-binding | 67 | 112 | 38.4 | 2.10E-08 |
| 391 | AP2 | 49 | 121 | 137.7 | 2.80E-38 |
| 391 | AP2 | 151 | 215 | 101.8 | 1.80E-27 |
| 392 | TCP | 56 | 241 | 161.1 | 2.60E-45 |
| 393 | zf-C3HC4 | 383 | 424 | 43.1 | 8.30E-10 |

(continued)

| PEP SEQ ID NO | Pfam domain name | begin | stop | score | E-value |
|---|---|---|---|---|---|
| 394 | SET | 109 | 238 | 182.9 | 7.10E-52 |
| 395 | zf-C2H2 | 36 | 59 | 18 | 0.031 |
| 396 | zf-C3HC4 | 206 | 246 | 34.5 | 3.40E-07 |
| 397 | Myb_DNA-binding | 26 | 75 | 34.3 | 3.70E-07 |
| 397 | Myb_DNA-binding | 134 | 181 | 53.7 | 5.30E-13 |
| 398 | BZIP_1 | 171 | 234 | 26.9 | 6.50E-05 |
| 398 | BZIP_2 | 171 | 221 | 25.3 | 0.00019 |
| 399 | zf-C3HC4 | 111 | 152 | 37.5 | 4.10E-08 |
| 400 | WD40 | 15 | 51 | 16.6 | 0.079 |
| 400 | WD40 | 59 | 95 | 23.3 | 0.00075 |
| 400 | WD40 | 210 | 246 | 16.4 | 0.093 |
| 400 | WD40 | 329 | 366 | 21.2 | 0.0033 |
| 401 | AP2 | 15 | 80 | 89.1 | 1.20E-23 |
| 402 | Arm | 35 | 75 | 34.2 | 4.00E-07 |
| 402 | Arm | 297 | 337 | 21.5 | 0.0027 |
| 403 | Aminotran_3 | 42 | 384 | 494.7 | 9.80E-146 |
| 404 | iPGM_N | 3 | 383 | 716.6 | 1.60E-212 |
| 404 | Metalloenzyme | 393 | 512 | 147.2 | 3.80E-41 |
| 405 | DUF231 | 239 | 408 | 227.7 | 2.30E-65 |
| 406 | DUF231 | 231 | 404 | 334.6 | 1.50E-97 |
| 407 | DUF231 | 237 | 405 | 336.4 | 4.40E-98 |
| 408 | NPH3 | 204 | 452 | 364.6 | 1.40E-106 I |

Table 17

| Pfam domain name | accession number | gathering cutoff | domain description |
|---|---|---|---|
| 2-oxoacid dh | PF00198.12 | -112 | 2-oxoacid dehydrogenases acyltransferase (catalytic domain) |
| ADH N | PF08240.1 | -14.5 | Alcohol dehydrogenase GroES-like domain |
| ADH zinc N | PF00107.15 | 23.8 | Zinc-binding dehydrogenase |
| AP2 | PF00847.9 | 0 | AP2 domain |
| AUX_IAA | PF02309.6 | -83 | AUX/IAA family |
| Aa_trans | PF01490.7 | -128.4 | Transmembrane amino acid transporter protein |
| Abhydrolase_1 | PF00561.9 | 5.5 | alpha/beta hydrolase fold |
| Acyl_transf_1 | PF00698.10 | -120 | Acyl transferase domain |
| Aldedh | PF00171.11 | -295 | Aldehyde dehydrogenase family |
| Aldo_ket_red | PF00248.10 | -97 | Aldo/keto reductase family |

(continued)

| Pfam domain name | accession number | gathering cutoff | domain description |
|---|---|---|---|
| Alpha-amylase | PF00128.11 | -93 | Alpha amylase, catalytic domain |
| Aminotran_1_2 | PF00155.9 | -57.5 | Aminotransferase class I and II |
| Aminotran_3 | PF00202.10 | -207.6 | Aminotransferase class-III |
| Ammonium_transp | PF00909.10 | -144 | Ammonium Transporter Family |
| Arm | PF00514.11 | 40.1 | Armadillo/beta-catenin-like repeat |
| Asn_synthase | PF00733.10 | -52.8 | Asparagine synthase |
| BAG | PF02179.5 | 25 | BAG domain |
| BSD | PF03909.6 | 25 | BSD domain |
| Beta_elim_lyase | PF01212.10 | -114.4 | Beta-eliminating lyase |
| Biotin_lipoyl | PF00364.11 | -2.3 | Biotin-requiring enzyme |
| Brix | PF04427.7 | 11.4 | Brix domain |
| Bromodomain | PF00439.13 | 8.9 | Bromodomain |
| C1_4 | PF07975.1 | 25 | TFIIH C1-like domain |
| CTP_ transf_ 2 | PF01467.15 | -11.8 | Cytidylyltransferase |
| Catalase | PF00199.8 | -229 | Catalase |
| CcmH | PF03918.4 | -30.8 | Cytochrome C biogenesis protein |
| Chal_sti_synt_C | PF02797.5 | -6.1 | Chalcone and stilbene synthases, C-terminal domain |
| Cyclin_C | PF02984.7 | -13 | Cyclin, C-terminal domain |
| Cyclin_N | PF00134.12 | -14.7 | Cyclin, N-terminal domain |
| Cys_Met Meta PP | PF01053.9 | -278.4 | Cys/Met metabolism PLP-dependent enzyme |
| DAO | PF01266.11 | -36.5 | FAD dependent oxidoreductase |
| DIM1 | PF02966.6 | 25 | Mitosis protein DIM1 |
| DPBB 1 | PF03330.7 | 30 | Rare lipoprotein A (RlpA)-like double-psi beta-barrel |
| DRMBL | PF07522.3 | 25 | DNA repair metallo-beta-lactamase |
| DUF167 | PF02594.6 | 25 | Uncharacterized ACR, YggU family COG1872 |
| DUF231 | PF03005.5 | -58 | Arabidopsis proteins of unknown function |
| DUF250 | PF03151.6 | 125 | Domain of unknown function, DUF250 |
| DUF6 | PF00892.9 | 30 | Integral membrane protein DUF6 |
| DUF783 | PF05615.2 | 25 | Protein of unknown function (DUF783) |
| DUF962 | PF06127.1 | 25 | Protein of unknown function (DUF962) |
| E2F_TDP | PF02319.9 | 17 | E2F/DP family winged-helix DNA-binding domain |
| E3_binding | PF02817.6 | 10 | e3 bindinq domain |

(continued)

| Pfam domain name | accession number | gathering cutoff | domain description |
|---|---|---|---|
| EBP | PF05241.1 | 25 | Emopamil binding protein |
| Enolase_C | PF00113.11 | -34 | Enolase, C-terminal TIM barrel domain |
| Enolase_ N | PF03952.5 | -4 | Enolase, N-terminal domain |
| F420_oxidored | PF03807.5 | -34.5 | NADP oxidoreductase coenzyme F420-dependent |
| FAD_binding_2 FA_desaturase | PF00890.13 | -124.8 | FAD binding domain |
|  | PF00487.13 | -46 | Fatty acid desaturase |
| FKBP_ C | PF00254.16 | -7.6 | FKBP-type peptidyl-prolyl cis-trans isomerase |
| FTCD_N | PF07837.2 | -67.7 | Formiminotransferase domain, N-terminal subdomain |
| Fe_bilin_red | PF05996.2 | 25 | Ferredoxin-dependent bilin reductase |
| Fer4 | PF00037.14 | 8 | 4Fe-4S binding domain |
| GAF | PF01590.14 | 23 | GAF domain |
| GATase_2 | PF00310.10 | -106.2 | Glutamine amidotransferases class-II |
| GIDA | PF01134.11 | -226.7 | Glucose inhibited division protein A |
| GSHPx | PF00255.9 | -16 | Glutathione peroxidase |
| Gpi16 | PF04113.3 | -207.8 | Gpi16 subunit, GPI transamidase component |
| HGTP_anticodon | PF03129.9 | -2 | Anticodon bindinq domain |
| HI0933_like | PF03486.4 | -255.8 | HI0933-like protein |
| HLH | PF00010.15 | 8.2 | Helix-loop-helix DNA-bindinG domain |
| HMG_CoA_synt | PF01154.7 | -230 | Hydroxymethylqlutaryl-coenzyme A synthase |
| HWE_HK | PF07536.4 | 25 | HWE histidine kinase |
| Ham1p_like | PF01725.6 | -46 | Ham1 family |
| HhH-GPD | PF00730.13 | 13.5 | HhH-GPD superfamily base excision DNA repair protein |
| Homeobox | PF00046.17 | -4.1 | Homeobox domain |
| Hpt | PF01627.11 | 25 | Hpt domain |
| Iso_dh | PF00180.9 | -97 | Isocitrate/isopropylmalate dehydrogenase |
| K-box | PF01486.7 | 0 | K-box region |
| LEA_4 | PF02987.6 | 25 | Late embryogenesis abundant protein |
| LRRNT_2 | PF08263.1 | 18.6 | Leucine rich repeat N-terminal domain |
| LRR_1 | PF00560.20 | 19 | Leucine Rich Repeat |

(continued)

| Pfam domain name | accession number | gathering cutoff | domain description |
|---|---|---|---|
| Ldh_1_C | PF02866.6 | -13 | lactate/malate dehydrogenase, alpha/beta C-terminal domain |
| Ldh_1_N | PF00056.11 | -31.3 | lactate/malate dehydrogenase, NAD binding domain |
| Lectin_legA | PF00138.7 | 19 | Legume lectins alpha domain |
| Lectin_legB | PF00139.9 | -77 | Legume lectins beta domain |
| Lipase_GDSL | PF00657.11 | 10.9 | GDSL-like Lipase/Acylhydrolase |
| MFS_1 | PF07690.4 | 23.5 | Major Facilitator Superfamily |
| MIP | PF00230.8 | -62 | Major intrinsic protein |
| MatE | PF01554.8 | 59.6 | MatE |
| Metalloenzyme | PF01676.7 | -14.4 | Metalloenzyme superfamily |
| Methyltransf_11 | PF08241.1 | 17.1 | Methyltransferase domain |
| Methyltransf_12 | PF08242.1 | 21.4 | Methyltransferase domain |
| Molybdop_Fe4S4 | PF04879.5 | 13.6 | Molybdopterin oxidoreductase Fe4S4 domain |
| Molybdopterin | PF00384.11 | -50 | Molybdopterin oxidoreductase |
| Molydop_binding | PF01568.10 | 1.1 | Molydopterin dinucleotide binding domain |
| Mov34 | PF01398.10 | -4 | Mov34/MPN/PAD-1 family |
| MtN3_slv | PF03083.5 | -0.8 | MtN3/saliva family |
| Myb_DNA-binding | PF00249.18 | 19.1 | Myb-like DNA-binding domain |
| NAD_Gly3P_dh N | PF01210.12 | -44 | NAD-dependent glycerol-3-phosphate dehydrogenase N-terminus |
| NAD_binding 2 | PF03446.4 | -63.5 | NAD binding domain of 6-phosphogluconate dehydrogenase |
| NIR_SIR | PF01077.10 | -25 | Nitrite and sulphite reductase 4Fe-4S domain |
| NIR_SIR_ferr | PF03460.5 | 20 | Nitrite/Sulfite reductase ferredoxin-like half domain |
| NPH3 | PF03000.4 | 25 | NPH3 family |
| NTP_transferase | PF00483.12 | -90.5 | Nucleotidyl transferase |
| Nuc_sug_transp | PF04142.5 | -92.4 | Nucleotide-sugar transporter |
| PA | PF02225.10 | 13 | PA domain |
| PAR1 | PF06521.1 | 25 | PAR1 protein |
| PFK | PF00365.9 | -132 | Phosphofructokinase |
| PGI | PF00342.8 | -168.9 | Phosphoglucose isomerase |
| PGK | PF00162.8 | -95.1 | Phosphoglycerate kinase |
| PGM_PMM_I | PF02878.5 | -37.5 | Phosphoglucomutase/ phosphomannomutase, alpha/beta/ alpha domain I |

(continued)

| Pfam domain name | accession number | gathering cutoff | domain description |
|---|---|---|---|
| PGM_PMM_II | PF02879.5 | -20 | Phosphoglucomutase/ phosphomannomutase, alpha/beta/ alpha domain II |
| PGM_PMM_III | PF02880.5 | -11 | Phosphoglucomutase/ phosphomannomutase, alpha/beta/ alpha domain III |
| PGM_ PMM_ IV | PF00408.9 | -6 | Phosphoglucomutase/ phosphomannomutase, C-terminal domain |
| PP2C | PF00481.10 | -44 | Protein phosphatase 2C |
| PTR2 | PF00854.11 | -50 | POT family |
| Peptidase_C26 | PF07722.2 | 25 | Peptidase C26 |
| Phi_1 | PF04674.2 | 25 | Phosphate-induced protein 1 conserved region |
| Phytochrome | PF00360.9 | 11 | Phytochrome region |
| Pkinase | PF00069.14 | -70.8 | Protein kinase domain |
| Pkinase_Tyr | PF07714.4 | 65 | Protein tyrosine kinase |
| Pollen_allerg_1 | PF01357.10 | 17.2 | Pollen allergen |
| Pribosyltran | PF00156.14 | 2 | Phosphoribosyl transferase domain |
| Proteasome | PF00227.14 | -36.7 | Proteasome A-type and B-type |
| Pyr_redox | PF00070.16 | 5 | Pyridine nucleotide-disulphide oxidoreductase |
| Pyr_redox_2 | PF07992.2 | -20 | Pyridine nucleotide-disulphide oxidoreductase |
| Pyr_redox_dim | PF02852.11 | -13 | Pyridine nucleotide-disulphide oxidoreductase, dimerisation domain |
| RNA_pol_L | PF01193.11 | 16.9 | RNA polymerase Rpb3/Rpb11 dimerisation domain |
| RNA_ pol_Rpb6 | PF01192.12 | 25 | RNA polymerase Rpb6 |
| RRM_1 | PF00076.10 | 15.2 | RNA recognition motif. (a.k.a. RRM, RBD, or RNP domain) |
| RRN3 | PF05327.1 | 25 | RNA polymerase I specific transcription initiation factor RRN3 |
| Radical_SAM | PF04055.8 | 8.4 | Radical SAM superfamily |
| Ras | PF00071.11 | 18 | Ras family |
| Response_reg | PF00072.11 | -14.4 | Response regulator receiver domain |
| Rhodanese | PF00581.9 | 25 | Rhodanese-like domain |
| Ribosomal_S8e | PF01201.11 | 25 | Ribosomal protein S8e |
| Rieske | PF00355.15 | -7 | Rieske [2Fe-2S] domain |
| SAC3_GANP | PF03399.5 | -15.2 | SAC3/GANP/Nin1/mts3/elF-3 p25 family |

(continued)

| Pfam domain name | accession number | gathering cutoff | domain description |
|---|---|---|---|
| SBDS | PF01172.7 | -78 | Shwachman-Bodian-Diamond syndrome (SBDS) proteins |
| SET | PF00856.16 | 15.8 | SET domain |
| SRF-TF | PF00319.8 | 11 | SRF-type transcription factor (DNA-binding and dimerisation domain) |
| SURF5 | PF06179.2 | 25 | Surfeit locus protein 5 |
| Skp1 | PF01466.8 | -2 | Skp1 family, dimerisation domain |
| Skp1_POZ | PF03931.4 | 14.9 | Skp1 family, tetramerisation domain |
| Ssl1 | PF04056.4 | -151.8 | Ssl1-like |
| Sterol_ desat | PF01598.7 | -13 | Sterol desaturase |
| Sugar_tr | PF00083.12 | -85 | Sugar (and other) transporter |
| TCP | PF03634.3 | -38 | TCP family transcription factor |
| ThiF | PF00899.10 | -38.4 | ThiF family |
| Transaldolase | PF00923.8 | -49 | Transaldolase |
| UQ_con | PF00179.15 | -30 | Ubiquitin-conjugating enzyme |
| Ubie methyltran | PF01209.8 | -117 | ubiE/COQ5 methyltransferase family |
| WD40 | PF00400.19 | 21.4 | WD domain, G-beta repeat |
| WRKY | PF03106.5 | 25 | WRKY DNA -binding domain |
| adh short | PF00106.13 | -46.6 | short chain dehydrogenase |
| bZIP_1 | PF00170.10 | 16.5 | bZIP transcription factor |
| bZIP_2 | PF07716.4 | 15 | Basic region leucine zipper |
| cNMP_binding | PF00027.17 | 20.6 | Cyclic nucleotide-binding domain |
| iPGM N | PF06415.3 | -263.4 | BPG-independent PGAM N-terminus (iPGM_N) |
| p450 | PF00067.11 | -105 | Cytochrome P450 |
| tRNA-synt 2b | PF00587.14 | -40.5 | tRNA synthetase class II core domain (G, H, P, S and T) |
| Ubiquitin | PF00240.12 | 19.4 | Ubiquitin family |
| zf-A20 | PF01754.6 | 25 | A20-like zinc finger |
| zf-AN1 | PF01428.6 | 15 | AN1-like Zinc finger |
| zf-B box | PF00643.13 | 11.1 | B-box zinc finger |
| zf-C2H2 | PF00096.14 | 19 | Zinc finger, C2H2 type |
| zf-C3HC4 | PF00097.12 | 16.9 | Zinc finger, C3HC4 type (RING finger) |
| zf-CCCH | PF00642.14 | 10.7 | Zinc finger C-x8-C-x5-C-x3-H type (and similar) |

**Example 5**

This example illustrates the construction of plasmids for transferring recombinant DNA into plant cells which can be regenerated into transgenic crop plants of this invention. Primers for PCR amplification of protein coding nucleotides of recombinant DNA are designed at or near the start and stop codons of the coding sequence, in order to eliminate most of the 5' and 3' untranslated regions. DNA of interest, i.e. each DNA identified in Table 1 and the DNA for the identified homologous genes, are cloned and amplified by PCR prior to insertion into the insertion site the base vector.

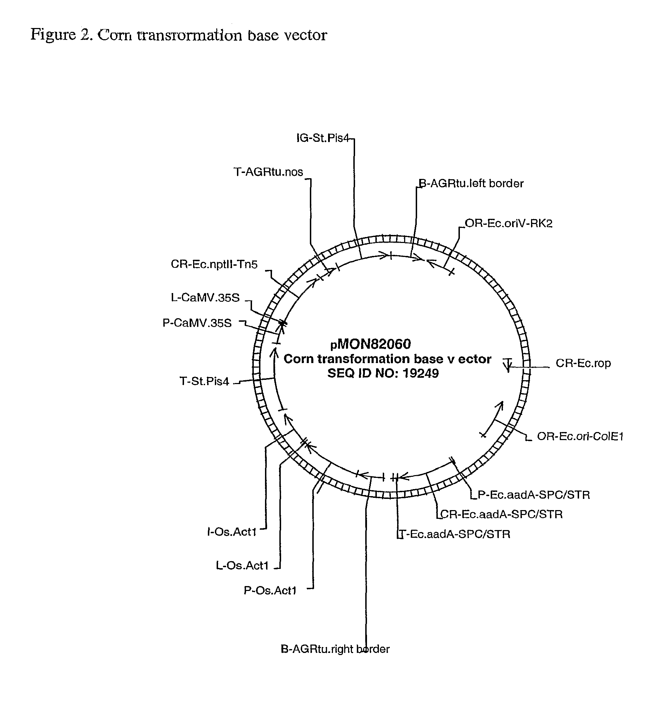

Elements of an exemplary common expression vector, pMON82060 are illustrated in Table 18. The exemplary base vector which is especially useful for corn transformation is illustrated in Figure 2 and assembled using technology known in the art. The DNA of interest are inserted in a expression vector at the insertion site between the intron1 of rice act 1 gene and the termination sequence of PinII gene.

Table 18. pMON82060

| function | name | annotation | Coordinates of SEQ ID NO: 19249 |
|---|---|---|---|
| Agro transformation | B-AGRtu.right border | Agro right border sequence, essential for transfer of T-DNA. | 5235-5591 |
| Gene of | P-Os.Act1 | Promoter from the rice actin gene act1. | 5609-7009 |
| interest plant expression cassette | L-Os.Act1 | Leader (first exon) from the rice actin 1 gene. | |
| | I-Os.Act1 | First intron and flanking UTR exon sequences from the rice actin 1 gene | |
| | insertion site | | |
| | T-St.Pis4 | The 3' non-translated region of the potato proteinase inhibitor II gene which functions to direct polyadenylation of the mRNA | 7084-8026 |
| Plant selectable marker expression cassette | P-CaMV.35S | CaMV 35S promoter | 8075-8398 |
| | L-CaMV.35S | 5' UTR from the 35S RNA of CaMV | |
| | CR-Ec.nptII-Tn5 | nptII selectable marker that confers resistance to neomycin and kanamycin | 8432-9226 |
| | T-AGRtu.nos | A 3' non-translated region of the nopaline synthase gene of Agrobacterium tumefaciens Ti plasmid which functions to direct polyadenylation of the mRNA. | 9255-9507 |

(continued)

| function | name | annotation | Coordinates of SEQ ID NO: 19249 |
|---|---|---|---|
| Agro transformation | B-AGRtu.left border | Agro left border sequence, essential for transfer of T-DNA. | 39-480 |
| | OR-Ec.oriV-RK2 | The vegetative origin of replication from plasmid RK2. | 567-963 |
| | CR-Ec.rop | Coding region for repressor of primer from the ColE1 plasmid. Expression of this gene product interferes with primer binding at the origin of replication, keeping plasmid copy number low. | 2472-2663 |
| Maintenance in E. coli | OR-Ec.ori-ColE1 | The minimal origin of replication from the E. coli plasmid ColE1. | 3091-3679 |
| | P-Ec.aadA-SPC/STR | promoter for Tn7 adenylyltransferase (AAD(3")) | 4210-4251 |
| | CR-Ec.aadA-SPC/STR | Coding region for Tn7 adenylyltransferase (AAD(3")) conferring spectinomycin and streptomycin resistance. | 4252-5040 |
| | T-Ec.aadA-SPC/STR | 3' UTR from the Tn7 adenylyltransferase (AAD(3")) gene of E. coli. | 5041-5098 |

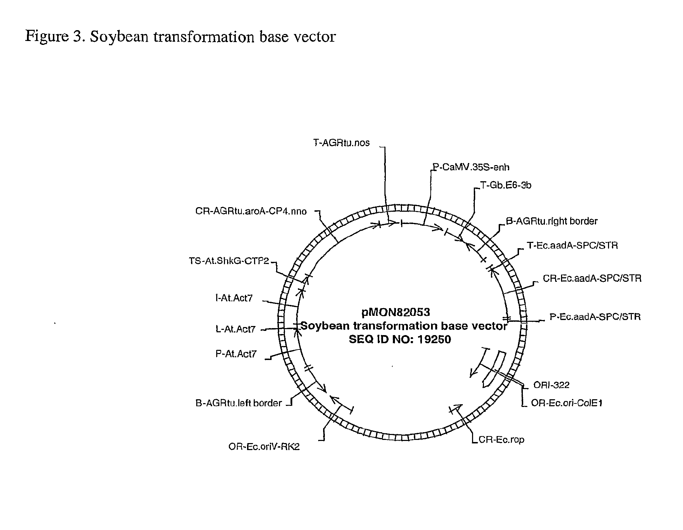

Plasmids for use in transformation of soybean are also prepared. Elements of an exemplary common expression vector plasmid pMON82053 are shown in Table 19 below. This exemplary soybean transformation base vector illustrated in Figure 3 was assembled using the technology known in the art. DNA of interest, i.e. each DNA identified in Table 1 and the DNA for the identified homologous genes, are cloned and amplified by PCR prior to insertion into the insertion site the base vector at the insertion site between the enhanced 35S CaMV promoter and the termination sequence of cotton E6 gene.

**Table 19. pMON82053**

| function | name | annotation | Coordinates of SEQ ID NO: 19250 |
|---|---|---|---|
| Agro transformation | B-AGRtu.left border | Agro left border sequence, essential for transfer of T-DNA. | 6144-6585 |

(continued)

| function | name | annotation | Coordinates of SEQ ID NO: 19250 |
|---|---|---|---|
| Plant selectable marker expression cassette | P-At.Act7 | Promoter from the arabidopsis actin 7 gene | 6624-7861 |
| | L-At.Act7 | 5'UTR of Arabidopsis Act7 gene | |
| | I-At.Act7 | Intron from the Arabidopsis actin7 gene | |
| | TS-At.ShkG-CTP2 | Transit peptide region of Arabidopsis EPSPS | 7864-8091 |
| | CR-AGRtu.aroA-CP4.nno_At | Synthetic CP4 coding region with dicot preferred codon usage. | 8092-9459 |
| | T-AGRtu.nos | A 3' non-translated region of the nopaline synthase gene of Agrobacterium tumefaciens Ti plasmid which functions to direct polyadenylation of the mRNA. | 9466-9718 |
| Gene of interest expression cassette | P-CaMV.35S-enh | Promoter for 35S RNA from CaMV containing a duplication of the -90 to -350 region. | 1-613 |
| | insertion site | | |
| | T-Gb.E6-3b | 3' untranslated region from the fiber protein E6 gene of sea-island cotton; | 688-1002 |
| Agro transformation | B-AGRtu.right border | Agro right border sequence, essential for transfer of T-DNA. | 1033-1389 |
| Maintenance in E. coli | OR-Ec.oriV-RK2 | The vegetative origin of replication from plasmid RK2. | 5661-6057 |
| | CR-Ec.rop | Coding region for repressor of primer from the ColE1 plasmid. Expression of this gene product interferes with primer binding at the origin of replication, keeping plasmid copy number low. | 3961-4152 |

(continued)

| function | name | annotation | Coordinates of SEQ ID NO: 19250 |
|---|---|---|---|
| | OR-Ec.ori-ColE1 | The minimal origin of replication from the E. coli plasmid ColE1. | 2945-3533 |
| | P-Ec.aadA-SPC/STR | romoter for Tn7 adenylyltransferase (AAD (3")) | 2373-2414 |
| | CR-Ec.aadA-SPC/STR | Coding region for Tn7 adenylyltransferase (AAD (3")) conferring spectinomycin and streptomycin resistance. | 1584-2372 |
| | T-Ec.aadA-SPC/STR | 3' UTR from the Tn7 adenylyltransferase (AAD (3 ")) gene of E. coli. | 1526-1583 |

## Example 5

This example illustrates monocot plant transformation useful in producing the transgenic plant cells of this invention by transformation of corn. Corn plants of a readily transformable line are grown in the greenhouse and ears harvested when the embryos are 1.5 to 2.0 mm in length. Ears are surface sterilized by spraying or soaking the ears in 80% ethanol, followed by air drying. Immature embryos are isolated from individual kernels on surface sterilized ears. Prior to inoculation of maize cells, *Agrobacterium* cells are grown overnight at room temperature. Immature maize embryos are inoculated with *Agrobacterium* shortly after excision, and incubated at room temperature with *Agrobacterium* for 5-20 minutes. Immature embryos are then co-cultured with *Agrobacterium* for 1 to 3 days at 23°C in the dark. Co-cultured embryos are transferred to selection media and cultured for approximately two weeks to allow embryogenic callus to develop. Embryogenic callus is transferred to culture medium containing 100 mg/L paromomycin and subcultured at about two week intervals. Transformants are recovered 6 to 8 weeks after initiation of selection.

Plasmid vectors are prepared essentially as described in Example 5 for transforming into corn each of the DNA of interest, i.e. each DNA identified in Table 1 and the DNA for the identified homologous genes, by *Agrobacterium*-mediated transformation.

For *Agrobacterium*-mediated transformation of maize callus, immature embryos are cultured for approximately 8-21 days after excision to allow callus to develop. Callus is then incubated for about 30 minutes at room temperature with the *Agrobacterium* suspension, followed by removal of the liquid by aspiration. The callus and *Agrobacterium* are co-cultured without selection for 3-6 days followed by selection on paromomycin for approximately 6 weeks, with biweekly transfers to fresh media, and paromomycin resistant callus identified as containing the recombinant DNA in an expression cassette.

To regenerate transgenic corn plants trangenic callus resulting from transformation is placed on media to initiate shoot development in plantlets which are transferred to potting soil for initial growth in a growth chamber at 26 degrees C followed by a mist bench before transplanting to 5 inch pots where plants are grown to maturity. The plants are self fertilized and seed is harvested for screening as seed, seedlings or progeny R2 plants or hybrids, e.g. for yield trials in the screens indicated above. Populations of transgenic plants and seeds produced form transgenic plant cells from each transgenic event are screened as described in Example 7 below to identify the members of the population having the enhanced trait.

## Example 6.

This example illustrates dicot plant transformation useful in producing the transgenic plant cells of this invention by transformation of soybean plants. For Agrobacterium mediated transformation, soybean seeds are germinated overnight and the meristem explants excised. The meristems and the explants are placed in a wounding vessel. Soybean explants and induced Agrobacterium cells from a strain containing plasmid DNA with the gene of interest cassette and a plant selectable marker cassette are mixed no later than 14 hours from the time of initiation of seed germination and wounded using sonication. Following wounding, explants are placed in co-culture for 2-5 days at which point they are transferred to selection media for 6-8 weeks to allow selection and growth of transgenic shoots. Trait positive shoots are harvested approximately 6-8 weeks post bombardment and placed into selective rooting media for 2-3 weeks. Shoots producing roots are transferred to the greenhouse and potted in soil. Shoots that remain healthy on selection, but do not produce roots are transferred to non-selective rooting media for an additional two weeks. Roots from any shoots that produce roots off selection are tested for expression of the plant selectable marker before they are transferred to the greenhouse and potted in soil. Populations of transgenic plants and seeds produced form transgenic plant cells from each transgenic event are screened as described in Example 7 below to identify the members of the population having the enhanced trait.

**Example 7.**

This example illustrates identification of plant cells of the invention by screening derived plants and seeds for enhanced trait. Many transgenic events which survive to fertile transgenic plants that produce seeds and progeny plants will not exhibit an enhanced agronomic trait. Populations of transgenic seed and plants prepared in Examples 5 and 6 are screened to identify those transgenic events providing transgenic plant cells with recombinant DNA imparting an enhanced trait. Each population is screened for nitrogen use efficiency, increased yield, enhanced water use efficiency, enhanced tolerance to cold and heat, enhanced level of oil and protein in seed using assays described below. Plant cells having recombinant DNA with each of the genes identified in Table 1 and the identified homologs are identified in plants and seeds with at least one of the enhanced traits.

**Selection for enhanced Nitrogen Use Efficiency**

The physiological efficacy of transgenic corn plants (tested as hybrids) can be tested for nitrogen use efficiency (NUE) traits in a high-throughput nitrogen (N) selection method. The collected data are compared to the measurements from wildtype controls using a statistical model to determine if the changes are due to the transgene. Raw data were analyzed by SAS software. Results shown herein are the comparison of transgenic plants relative to the wildtype controls.

(1) Media Preparation for Planting a NUE Protocol

Planting materials used: Metro Mix 200 (vendor: Hummert) Cat. # 10-0325, Scotts Micro Max Nutrients (vendor: Hummert) Cat. # 07-6330, OS 4 1/3" x 3 7/8" pots (vendor: Hummert) Cat. # 16-1415, OS trays (vendor: Hummert) Cat. # 16-1515, Hoagland's macronutrients solution, Plastic 5" stakes (vendor: Hummert) yellow Cat. # 49-1569, white Cat. # 49-1505, Labels with numbers indicating material contained in pots. Fill 500 pots to rim with Metro Mix 200 to a weight of ~140g/pot. Pots are filled uniformly by using a balancer. Add 0.4g of Micro Max nutrients to each pot. Stir ingredients with spatula to a depth of 3 inches while preventing material loss.

(2) Planting a NUE selection in the Greenhouse

(a) Seed Germination - Each pot is lightly atered twice using reverse osmosis purified water. The first watering is scheduled to occur just before planting; and the second watering, after the seed has been planted in the pot. Ten Seeds of each entry (1 seed per pot) are planted to select eight healthy uniform seedlings. Additional wild type controls are planted for use as border rows. Alternatively, 15 seeds of each entry (1 seed per pot) are planted to select 12 healthy uniform seedlings (this larger number of plantings is used for the second, or confirmation, planting). Place pots on each of the 12 shelves in the Conviron growth chamber for seven days. This is done to allow more uniform germination and early seedling growth. The following growth chamber settings are 25° C/day and 22° C/night, 14 hours light and ten hours dark, humidity ~80%, and light intensity ~350 $\mu$mol/m$^2$/s (at pot level). Watering is done via capillary matting similar to greenhouse benches with duration of ten minutes three times a day.

(b) Seedling transfer - After seven days, the best eight or 12 seedlings for the first or confirmation pass runs, respectively, are chosen and transferred to greenhouse benches. The pots are spaced eight inches apart (center to center) and are positioned on the benches using the spacing patterns printed on the capillary matting. The Vattex matting creates a 384-position grid, randomizing all range, row combinations. Additional pots of controls are placed along the outside of the experimental block to reduce border effects.

Plants are allowed to grow for 28 days under the low N run or for 23 days under the high N run. The macronutrients are dispensed in the form of a macronutrient solution (see composition below) containing precise amounts of N added (2mM $NH_4NO_3$ for limiting N selection and 20mM $NH_4NO_3$ for high N selection runs). Each pot is manually dispensed 100ml of nutrient solution three times a week on alternate days starting at eight and ten days after planting for high N and low N runs, respectively. On the day of nutrient application, two 20 min waterings at 05:00 and 13:00 are skipped. The vattex matting should be changed every third run to avoid N accumulation and buildup of root matter. Table 7 shows the amount of nutrients in the nutrient solution for either the low or high nitrogen selection.

Table 22

| Nutrient Stock | 2mM NH$_4$NO$_3$ (Low Nitrogen Growth Condition, Low N) | 20mM NH$_4$NO$_3$ (high Nitrogen Growth Condition, High N) |
|---|---|---|
| | mUL | mUL |
| 1 M NH$_4$NO$_3$ | 2 | 20 |
| 1 M KH$_2$PO$_4$ | 0.5 | 0.5 |
| 1 M MgSO$_4$.7H$_2$O | 2 | 2 |
| 1 M CaCl$_2$ | 2.5 | 2.5 |
| 1 M K$_2$SO$_4$ | 1 | 1 |
| Note: Adjust pH to 5.6 with HCl or KOH | | |

(c) Harvest Measurements and Data Collection - After 28 days of plant growth for low N runs and 23 days of plant growth for high N runs, the following measurements are taken (phenocodes in parentheses): total shoot fresh mass (g) (SFM) measured by Sartorius electronic balance, V6 leaf chlorophyll measured by Minolta SPAD meter (relative units) (LC), V6 leaf area (cm$^2$) (LA) measured by a Li-Cor leaf area meter, V6 leaf fresh mass (g) (LFM) measured by Sartorius electronic balance, and V6 leaf dry mass (g) (LDM) measured by Sartorius electronic balance. Raw data were analyzed by SAS software. Results shown are the comparison of transgenic plants relative to the wildtype controls.

To take a leaf reading, samples were excised from the V6 leaf. Since chlorophyll meter readings of corn leaves are affected by the part of the leaf and the position of the leaf on the plant that is sampled, SPAD meter readings were done on leaf six of the plants. Three measurements per leaf were taken, of which the first reading was taken from a point one-half the distance between the leaf tip and the collar and halfway from the leaf margin to the midrib while two were taken toward the leaf tip. The measurements were restricted in the area from 1/2 to 3/4 of the total length of the leaf (from the base) with approximately equal spacing between them. The average of the three measurements was taken from the SPAD machine.

Leaf fresh mass is recorded for an excised V6 leaf, the leaf is placed into a paper bag. The paper bags containing the leaves are then placed into a forced air oven at 80° C for 3 days. After 3 days, the paper bags are removed from the oven and the leaf dry mass measurements are taken.

From the collected data, two derived measurements are made: (1)Leaf chlorophyll area (LCA), which is a product of V6 relative chlorophyll content and its leaf area (relative units). Leaf chlorophyll area= leaf chlorophyll X leaf area. This parameter gives an indication of the spread of chlorophyll over the entire leaf area; (2)specific leaf area (LSA) is calculated as the ratio of V6 leaf area to its dry mass (cm$^2$/g dry mass), a parameter also recognized as a measure of NUE.

Nitrogen use field efficacy assay

Level I. Transgenic plants provided by the present invention are planted in field without any nitrogen source being applied. Transgenic plants and control plants are grouped by genotype and construct with controls arranged randomly within genotype blocks. Each type of transgenic plants are tested by 3 replications and across 5 locations. Nitrogen levels in the fields are analyzed in early April pre-planting by collecting 30 sample soil cores from 0-24" and 24 to 48" soil layer. Soil samples are analyzed for nitrate-nitrogen, phosphorus(P), Potassium(K), organic matter and pH to provide baseline values. P, K and micronutrients are applied based upon soil test recommendations.

Level II. Transgenic plants provided by the present invention are planted in field with three levels of nitrogen (N) fertilizer being applied, i.e. low level (0 N), medium level (80 lb/ac) and high level (180 lb/ac). Liquid 28% or 32% UAN (Urea, Ammonium Nitrogen) are used as the N source and apply by broadcast boom and incorporate with a field cultivator with rear rolling basket in the same direction as intended crop rows. Although there is no N applied to the 0 N treatment the soil should still be disturbed in the same fashion as the treated area. Transgenic plants and control plants are grouped by genotype and construct with controls arranged randomly within genotype blocks. Each type of transgenic plants is tested by 3 replications and across 4 locations. Nitrogen levels in the fields are analyzed in early April pre-planting by collecting 30 sample soil cores from 0-24" and 24 to 48" soil layer. Soil samples are analyzed for nitrate-nitrogen, phosphorus(P), Potassium(K), organic matter and pH to provide baseline values. P, K and micronutrients are applied based upon soil test recommendations.

**Selection for increased yield**

Many transgenic plants of this invention exhibit improved yield as compared to a control plant. Improved yield can result from enhanced seed sink potential, i.e. the number and size of endosperm cells or kernels and/or enhanced sink strength, i.e. the rate of starch biosynthesis. Sink potential can be established very early during kernel development, as endosperm cell number and size are determined within the first few days after pollination.

Much of the increase in corn yield of the past several decades has resulted from an increase in planting density. During that period, corn yield has been increasing at a rate of 2.1 bushels/acre/year, but the planting density has increased at a rate of 250 plants/acre/year. A characteristic of modem hybrid corn is the ability of these varieties to be planted at high density. Many studies have shown that a higher than current planting density should result in more biomass production, but current germplasm does not perform. well at these higher densities. One approach to increasing yield is to increase harvest index (HI), the proportion of biomass that is allocated to the kernel compared to total biomass, in high density plantings. Effective yield selection of enhanced yielding transgenic corn events uses hybrid progeny of the transgenic event over multiple locations with plants grown under optimal production management practices, and maximum pest control. A useful target for improved yield is a 5% to 10% increase in yield as compared to yield produced by plants grown from seed for a control plant. Selection methods may be applied in multiple and diverse geographic locations, for example up to 16 or more locations, over one or more planting seasons, for example at least two planting seasons to statistically distinguish yield improvement from natural environmental effects. It is to plant multiple transgenic plants, positive and negative control plants, and pollinator plants in standard plots, for example 2 row plots, 20 feet long by 5 feet wide with 30 inches distance between rows and a 3 foot alley between ranges. Transgenic events can be grouped by recombinant DNA constructs with groups randomly placed in the field. A pollinator plot of a high quality corn line is planted for every two plots to allow open pollination when using male sterile transgenic events. A useful planting density is about 30,000 plants/acre. High planting density is greater than 30,000 plants/acre, preferably about 40,000 plants/acre, more preferably about 42,000 plants/acre, most preferably about 45,000 plants/acre. Transgenic corn plants and soybean plants with each recombinant DNA construct prepared in Examples 5 and 6 are identified as exhibiting at least 5% yield increase as compared to control plants.

**Selection for enhanced water use efficiency (WUE)**

Described in this example is a high-throughput method for greenhouse selection of transgenic corn plants to wild type corn plants (tested as inbreds or hybrids) for water use efficiency. This selection process imposes 3 drought/re-water cycles on plants over a total period of 15 days after an initial stress free growth period of 11 days. Each cycle consists of 5 days, with no water being applied for the first four days and a water quenching on the 5th day of the cycle. The primary phenotypes analyzed by the selection method are the changes in plant growth rate as determined by height and biomass during a vegetative drought treatment. The hydration status of the shoot tissues following the drought is also measured. The plant height are measured at three time points. The first is taken just prior to the onset drought when the plant is 11 days old, which is the shoot initial height (SIH). The plant height is also measured halfway throughout the drought/re-water regimen, on day 18 after planting, to give rise to the shoot mid-drought height (SMH). Upon the completion of the final drought cycle on day 26 after planting, the shoot portion of the plant is harvested and measured for a final height, which is the shoot wilt height (SWH) and also measured for shoot wilted biomass (SWM). The shoot is placed in water at 40 degree Celsius in the dark. Three days later, the shoot is weighted to give rise to the shoot turgid weight (STM). After drying in an oven for four days, the shoots are weighted for shoot dry biomass (SDM). The shoot average height (SAH) is the mean plant height across the 3 height measurements. The procedure described above may be adjusted for +/- ~one day for each step given the situation.

To correct for slight differences between plants, a size corrected growth value is derived from SIH and SWH. This is the Relative Growth Rate (RGR). Relative Growth Rate (RGR) is calculated for each shoot using the formula [RGR% = (SWH-SIH)/((SWH+SIH)/2)* 100]. Relative water content (RWC) is a measurement of how much (%) of the plant was water at harvest. Water Content (RWC) is calculated for each shoot using the formula [RWC% = (SWM-SDM)/(STM-SDM)* 100]. Fully watered corn plants of this age run around 98% RWC.

**Selection for Growth Under Cold Stress**

(1) Cold germination assay - Three sets of seeds are used for the assay. The first set consists of positive transgenic events (F1 hybrid) where the genes of the present invention are expressed in the seed. The second seed set is nontransgenic, wild-type negative control made from the same genotype as the transgenic events. The third set consisted of two cold tolerant and one cold sensitive commercial check lines of corn. All seeds are treated with a fungicide " Captan" (MAESTRO® 80DF Fungicide, Arvesta Corporation, San Francisco, CA, USA). 0.43 mL Captan is applied per 45 g of corn seeds by mixing it well and drying the fungicide prior to the experiment.

Corn kernels are placed embryo side down on blotter paper within an individual cell (8.9 x 8.9 cm) of a germination tray (54 x 36 cm). Ten seeds from an event are placed into one cell of the germination tray. Each tray can hold 21 transgenic events and 3 replicates of wildtype (LH244SDms+LH59), which is randomized in a complete block design. For every event there are five replications (five trays). The trays are placed at 9.7C for 24 days (no light) in a Convrion growth chamber *(Conviron Model PGV36, Controlled Environments, Winnipeg, Canada).* Two hundred and fifty millilters of deionized water are added to each germination tray. Germination counts are taken 10th, 11th, 12th, 13th, 14th, 17th, 19th, 21st, and 24th day after start date of the experiment. Seeds are considered germinated if the emerged radicle size is 1 cm. From the germination counts germination index is calculated.

*The germination index is calculated as per:*

$$Germination\ index = (\Sigma\,([T+1-n_i]\,{}^{*}[P_i-P_{i-1}]))/T$$

Where T is the total number of days for which the germination assay is performed. The number of days after planting is defined by n. "i" indicated the number of times the germination had been counted, including the current day. P is the percentage of seeds germinated during any given rating. Statistical differences are calculated between transgenic events and wild type control. After statistical analysis, the events that show a statistical significance at the p level of less than 0.1 relative to wild-type controls will advance to a secondary cold selection. The secondary cold screen is conducted in the same manner of the primary selection only increasing the number of repetitions to ten. Statistical analysis of the data from the secondary selection is conducted to identify the events that show a statistical significance at the p level of less than 0.05 relative to wild-type controls.

(2) Cold Shock assay - The experimental set-up for the cold shock assay is the same as described in the above cold germination assay except seeds were grown in potted media for the cold shock assay.

The desired numbers of 2.5" square plastic pots are placed on flats (n=32, 4x8). Pots were filled with Metro Mix 200 soil-less media containing 19:6:12 fertilizer (6 lbs/cubic yard) (Metro Mix, Pots and Flat are obtained from Hummert International, Earth City, MO). After planting seeds, pots are placed in a growth chamber set at 23° C, relative humidity of 65% with 12 hour day and night photoperiod (300 uE/m2-min). Planted seeds are watered for 20 minute every other day by sub-irrigation and flats were rotated every third day in a growth chamber for growing corn seedlings.

On the 10th day after planting the transgenic positive and wild-type negative (WT) plants are positioned in flats in an alternating pattern. Chlorophyll fluorescence of plants is measured on the 10th day during the dark period of growth by using a PAM-2000 portable fluorometer as per the manufacturer's instructions (Walz, Germany). After chlorophyll measurements, leaf samples from each event are collected for confirming the expression of genes of the present invention. For expression analysis six V1 leaf tips from each selection are randomly harvested. The flats are moved to a growth chamber set at 5° C. All other conditions such as humidity, day/night cycle and light intensity are held constant in the growth chamber. The flats are subirrigated every day after transfer to the cold temperature. On the 4th day chlorophyll fluorescence is measured. Plants are transferred to normal growth conditions after six days of cold shock treatment and allowed to recover for the next three days. During this recovery period the length of the V3 leaf is measured on the 1st and 3rd days. After two days of recovery V2 leaf damage is determined visually by estimating percent of green V2 leaf.

Statistical differences in V3 leaf growth, V2 leaf necrosis and fluorescence during pre-shock and cold shock can be used for estimation of cold shock damage on corn plants. (3) Early seedling growth assay - Three sets of seeds are used for the experiment. The first set consists of positive transgenic events (F1 hybrid) where the genes of the present invention are expressed in the seed. The second seed set is nontransgenic, wild-type negative control made from the same genotype as the transgenic events. The third seed set consists of two cold tolerant and two cold sensitive commercial check lines of corn. All seeds are treated with a fungicide" Captan", (3a,4,7,a-tetrahydro-2-[(trichloromethly) thio]-1H-isoindole-1,3(2H)-dione, Drex Chemical Co. Memphis, TN). Captan (0.43 mL) was applied per 45 g of corn seeds by mixing it well and drying the fungicide prior to the experiment.

Seeds are grown in germination paper for the early seedling growth assay. Three 12"x18" pieces of germination paper (Anchor Paper #SD7606) are used for each entry in the test (three repetitions per transgenic event). The papers are wetted in a solution of 0.5% $KNO_3$ and 0.1% Thyram.

For each paper fifteen seeds are placed on the line evenly spaced down the length of the paper. The fifteen seeds are positioned on the paper such that the radical would grow downward, for example longer distance to the paper's edge. The wet paper is rolled up starting from one of the short ends. The paper is rolled evenly and tight enough to hold the seeds in place. The roll is secured into place with two large paper clips, one at the top and one at the bottom. The rolls are incubated in a growth chamber at 23° C for three days in a randomized complete block design within an appropriate container. The chamber is set for 65% humidity with no light cycle. For the cold stress treatment the rolls are then incubated in a growth chamber at 12° C for twelve days. The chamber is set for 65% humidity with no light cycle.

After the cold treatment the germination papers are unrolled and the seeds that did not germinate are discarded.

The lengths of the radicle and coleoptile for each seed are measured through an automated imaging program that automatically collects and processes the images. The imaging program automatically measures the shoot length, root length, and whole seedling length of every individual seedling and then calculates the average of each roll.

After statistical analysis, the events that show a statistical significance at the p level of less than 0.1 relative to wild-type controls will advance to a secondary cold selection. The secondary cold selection is conducted in the same manner of the primary selection only increasing the number of repetitions to five. Statistical analysis of the data from the secondary selection is conducted to identify the events that show a statistical significance at the p level of less than 0.05 relative to wild-type controls.

4. Cold field efficacy trial

This example sets forth a cold field efficacy trial to identify gene constructs that confer enhanced cold vigor at germination and early seedling growth under early spring planting field conditions in conventional-till and simulated no-till environments. Seeds are planted into the ground around two weeks before local farmers are beginning to plant corn so that a significant cold stress is exerted onto the crop, named as cold treatment. Seeds also are planted under local optimal planting conditions such that the crop has little or no exposure to cold condition, named as normal treatment. The cold field efficacy trials are carried out in five locations, including Glyndon MN, Mason MI, Monmouth IL, Dayton IA, Mystic CT. At each location, seeds are planted under both cold and normal conditions with 3 repetitions per treatment, 20 kernels per row and single row per plot. Seeds are planted 1.5 to 2 inch deep into soil to avoid muddy conditions. Two temperature monitors are set up at each location to monitor both air and soil temperature daily.

Seed emergence is defined as the point when the growing shoot breaks the soil surface. The number of emerged seedling in each plot is counted everyday from the day the earliest plot begins to emerge until no significant changes in emergence occur. In addition, for each planting date, the latest date when emergence is 0 in all plots is also recorded. Seedling vigor is also rated at V3-V4 stage before the average of corn plant height reaches 10 inches, with 1=excellent early growth, 5=Average growth and 9=poor growth. Days to 50% emergence, maximum percent emergence and seedling vigor are calculated using SAS software for the data within each location or across all locations.

**Screens for transgenic plant seeds with increased protein and/or oil levels**

This example sets forth a high-throughput selection for identifying plant seeds with improvement in seed composition using the Infratec 1200 series Grain Analyzer, which is a near-infrared transmittance spectrometer used to determine the composition of a bulk seed sample. Near infrared analysis is a non-destructive, high-throughput method that can analyze multiple traits in a single sample scan.. An NIR calibration for the analytes of interest is used to predict the values of an unknown sample. The NIR spectrum is obtained for the sample and compared to the calibration using a complex chemometric software package that provides a predicted values as well as information on how well the sample fits in the calibration.

Infratec Model 1221, 1225, or 1227 with transport module by Foss North America is used with cuvette, item # 1000-4033, Foss North America or for small samples with small cell cuvette, Foss standard cuvette modified by Leon Girard Co. Corn and soy check samples of varying composition maintained in check cell cuvettes are supplied by Leon Girard Co. NIT collection software is provided by Maximum Consulting Inc.Software. Calculations are performed automatically by the software. Seed samples are received in packets or containers with barcode labels from the customer. The seed is poured into the cuvettes and analyzed as received.

Table 26.

| Typical sample(s): | Whole grain corn and soybean seeds |
|---|---|
| Analytical time to run method: | Less than 0.75 min per sample |
| Total elapsed time per run: | 1.5 minute per sample |
| Typical and minimum sample size: | Corn typical: 50cc; minimum 30cc Soybean typical: 50cc; minimum 5cc |
| Typical analytical range: | Determined in part by the specific calibration. Corn - moisture 5-15%, oil 5-20%, protein 5-30%, starch 50-75%, and density 1.0-1.3%. Soybean - moisture 5-15%, oil 15-25%, and protein 35-50%. |

## Example 8.

This example describes recombinant DNA constructs of the invention, useful for suppressing the expression of a protein identified by Pfam, Catalase, Bromdomain, FTCD_N, MatE, DPBB_1, tRNA-synt_2b, Sugar_tr and MFS_1, DUF6 and DUF250, LEA_4, MIP or DUF231, in a corn or soybean plant, by expressing a sense and an anti-sense fragment of the native DNA encoding the protein, essentially as described in U.S. Patent Application Serial No. 11/303,745, incorporated herein by reference. Specific gene suppression constructs are targeted to the native gene in corn and soybean plants that are homologs of the genes encoding the protein with an amino acid sequence of SEQ ID NO:213, 215, 218, 222, 258, 269, 275, 334, 361, 368, and 407.

The constructs include a promoter operably linked to DNA that transcribes to RNA that forms a double stranded RNA in transgenic plant cells for suppressing expression of the protein to provided the enhanced trait in the corn and soybean plants. Populations of transgenic plants and seeds derived from the plant cells are screened to identify those plants exhibiting the enhanced traits associated with suppression of those genes.

A plant cell with stably integrated, recombinant DNA comprising a promoter that is functional in plant cells and that is operably linked to DNA from a plant, bacteria or yeast that encodes a protein having at least one domain of amino acids in a sequence that exceeds the Pfam gathering cutoff of 16.9 for amino acid sequence alignment with a protein domain family identified by Pfam name, RNA_pol_L; wherein said plant cell is selected from a population of plant cells with said recombinant DNA by screening plants that are regenerated from plant cells in said population and that express said protein for an enhanced trait as compared to control plants that do not have said recombinant DNA; and wherein said enhanced trait is selected from group of enhanced traits consisting of enhanced water use efficiency, enhanced cold tolerance, increased yield, enhanced nitrogen use efficiency, enhanced seed protein and enhanced seed oil. A plant cell with stably integrated, recombinant DNA comprising a promoter that is functional in plant cells and that is operably linked to DNA from a plant, bacteria or yeast that encodes a protein having at least one domain of amino acids in a sequence that exceeds the Pfam gathering cutoff for amino acid sequence alignment with a protein domain family identified by a Pfam name in the group of Pfam names consisting of 2-oxoacid_dh, ADH_N, ADH_zinc_N, AP2, AUX_IAA, Aa_trans, Abhydrolase_1, Acyl_transf_1, Aldedh, Aldo_ket_red, Alpha-amylase, Aminotran_1_2, Aminotran_3, Ammonium_transp, Arm, Asn_synthase, BAG, BSD, Beta_elim_lyase, Biotin_lipoyl, Brix, Bromodomain, C1_4, CTP_transf_2, Catalase, CcmH, Chal_sti_synt_C, Cyclin_C, Cyclin_N, Cys_Met_Meta_PP, DAO, DIM1, DPBB_1, DRMBL, DUF167, DUF231, DUF250, DUF6, DUF783, DUF962, E2F TDP, E3_binding, EBP, Enolase_C, Enolase_N, F420_oxidored, FAD_binding_2, FA_desaturase, FKBP_C, FTCD_N, Fe_bilin_red, Fer4, GAF, GATase_2, GIDA, GSHPx, Gpi16, HGTP_anticodon, HI0933_like, HLH, HMG_CoA_synt, HWE_HK, Hamlp_like, HhH-GPD, Homeobox, Hpt, Iso_dh, K-box, LEA_4, LRRNT_2, LRR_1, Ldh_1_C, Ldh_1_N, Lectin_legA, Lectin_legB, Lipase_GDSL, MFS_1, MIP, MatE, Metalloenzyme, Methyltransf_11, Methyltransf_12, Molybdop_Fe4S4, Molybdopterin, Molydop_binding, Mov34, MtN3_slv, Myb_DNA-binding, NAD_Gly3P_dh_N, NAD_binding_2, NIR_SIR, NIR_SIR_ferr, NPH3, NTP_transferase, Nuc_sug_transp, PA, PAR1, PFK, PGI, PGK, PGM_PMM_I, PGM_PMM_II, PGM_PMM_III, PGM_PMM_IV, PP2C, PTR2, Peptidase_C26, Phi_1, Phytochrome, Pkinase, Pkinase_Tyr, Pollen_allerg_1, Pribosyltran, Proteasome, Pyr_redox, Pyr_redox_2, Pyr_redox_dim, RNA_pol_L, RNA_pol_Rpb6, RRM_1, RRN3, Radical_SAM, Ras, Response_reg, Rhodanese, Ribosomal_S8e, Rieske, SAC3_GANP, SBDS, SET, SRF-TF, SURF5, Skp1, Skp1_POZ, Ssl1, Sterol_desat, Sugar_tr, TCP, ThiF, Transaldolase, UQ_con, Ubie_methyltran, WD40, WRKY, adh_short, bZIP_1, bZIP_2, cNMP_binding, iPGM_N, p450, tRNA-synt_2b, ubiquitin, zf-A20, zf-AN1, zf-B_box, zf-C2H2, zf-C3HC4, zf-CCCH wherein the Pfam gathering cuttoff for said protein domain families is stated in Table 16 ; wherein said plant cell is selected from a population of plant cells with said recombinant DNA by screening plants that are regenerated from plant cells in said population and that express said protein for an enhanced trait as compared to control plants that do not have said recombinant DNA; and wherein said enhanced trait is selected from group of enhanced traits consisting of enhanced water use efficiency, enhanced cold tolerance, enhanced heat tolerance, enhanced resistance to salt exposure, enhanced shade tolerance, increased yield, enhanced nitrogen use efficiency, enhanced seed protein and enhanced seed oil.

A plant cell as defined above, wherein said protein has an amino acid sequence with at least 90% identity to a consensus amino acid sequence in the group of consensus amino acid sequences consisting of the consensus amino acid sequence constructed for SEQ ID NO: 205 and homologs thereof listed in Table 2 through the consensus amino acid sequence constructed for SEQ ID NO:408 and homologs thereof listed in Table 2.

A plant cell as defined above, wherein said protein is selected from the group of proteins identified in Table 1.

A plant cell as defined above, further comprising DNA expressing a protein that provides tolerance from exposure to an herbicide applied at levels that are lethal to a wild type of said plant cell.

A plant cell as defined above, wherein the agent of said herbicide is a glyphosate, dicamba, or glufosinate compound.

A transgenic plant comprising a plurality of the plant cells of as defined above.

A transgenic plant as defined above, which is homozygous for said recombinant DNA.

A transgenic seed comprising a plurarility of the plant cell as defined above.

A transgenic seed as defined above from a corn, soybean, cotton, canola, alfalfa, wheat or rice plant.

Non-natural, transgenic corn seed as defined above, wherein said seed can produce corn plants that are resistant to disease from the Mal de Rio Cuarto virus or the Puccina sorghi fungus or both.

A transgenic pollen grain comprising a haploid derivative of a plant cell as defined above.

A method for manufacturing non-natural, transgenic seed that can be used to produce a crop of transgenic plants with an enhanced trait resulting from expression of stably-integrated, recombinant DNA comprising a promoter that is (a) functional in plant cells and (b) is operably linked to DNA from a plant, bacteria or yeast that encodes a protein having at least one domain of amino acids in a sequence that exceeds the Pfam gathering cutoff for amino acid sequence alignment with a protein domain family identified by a Pfam name in the group of Pfam names consisting of2-oxoacid_ dh, ADH_N, ADH_zinc_N, AP2, AUX_IAA, Aa_trans, Abhydrolase_1, Acyl_transf_1, Aldedh, Aldo_ket_red, Alpha-amylase, Aminotran_1_2, Aminotran_3, Ammonium_transp, Arm, Asn_synthase, BAG, BSD, Beta_elim_lyase, Biotin lipoyl, Brix, Bromodomain, C1_4, CTP_transf_2, Catalase, CcmH, Chal_sti_synt_C, Cyclin_C, Cyclin_N, Cys_Met_Meta_PP, DAO, DIM1, DPBB_1, DRMBL, DUF167, DUF231, DUF250, DUF6, DUF783, DUF962, E2F_TDP, E3_binding, EBP, Enolase_C, Enolase_N, F420_oxidored, FAD_binding_2, FA_desaturase, FKBP_C, FTCD_N, Fe_bilin_red, Fer4, GAF, GATase_2, GIDA, GSHPx, Gpi16, HGTP_anticodon, HI0933_like, HLH, HMG_CoA_synt, HWE_HK, Ham1p_like, HhH-GPD, Homeobox, Hpt, Iso_dh, K-box, LEA_4, LRRNT_2, LRR_1, Ldh_1_C, Ldh_1_N, Lectin_legA, Lectin_legB, Lipase_ GDSL, MFS_1, MIP, MatE, Metalloenzyme, Methyltransf_11, Methyltransf_12, Molybdop_Fe4S4, Molybdopterin, Molydop_binding, Mov34, MtN3_slv, Myb_DNA-binding, NAD_Gly3P_dh_N, NAD_binding_2, NIR_SIR, NIR_SIR_ferr, NPH3, NTP_transferase, Nuc_sug_transp, PA, PAR1, PFK, PGI, PGK, PGM_PMM_I, PGM_PMM_II, PGM_PMM_III, PGM_PMM_IV, PP2C, PTR2, Peptidase_C26, Phi_1, Phytochrome, Pkinase, Pkinase_Tyr, Pollen_allerg_1, Pribosyltran, Proteasome, Pyr_redox, Pyr_redox_2, Pyr_redox_dim, RNA_pol_L, RNA_pol_Rpb6, RRM_1, RRN3, Radical_ SAM, Ras, Response_reg, Rhodanese, Ribosomal_S8e, Rieske, SAC3_GANP, SBDS, SET, SRF-TF, SURF5, Skp1, Skp1_POZ, Ssl1, Sterol_desat, Sugar_tr, TCP, ThiF, Transaldolase, UQ_con, Ubie_methyltran, WD40, WRKY, adh_ short, bZIP_1, bZIP_2, cNMP_binding, iPGM_N, p450, tRNA-synt_2b, ubiquitin, zf-A20, zf-AN1, zf-B_box, zf-C2H2, zf-C3HC4, zf-CCCH; wherein the gathering cutoff for said protein domain families is stated in Table 16; and wherein said enhanced trait is selected from the group of enhanced traits consisting of enhanced traits consisting of enhanced water use efficiency, enhanced cold tolerance, enhanced heat tolerance, enhanced resistance to salt exposure, enhanced shade tolerance, increased yield, enhanced nitrogen use efficiency, enhanced seed protein and enhanced seed oil, said method for manufacturing said seed comprising:

(a) screening a population of plants for said enhanced trait and said recombinant DNA, wherein individual plants in said population can exhibit said trait at a level less than, essentially the same as or greater than the level that said trait is exhibited in control plants which do not express the recombinant DNA,
(b) selecting from said population one or more plants that exhibit the trait at a level greater than the level that said trait is exhibited in control plants,
(c) verifying that said recombinant DNA is stably integrated in said selected plants,
(d) analyzing tissue of a selected plant to determine the production of a protein having the function of a protein encoded by nucleotides in a sequence of one of SEQ ID NO:205-408; and
(e) collecting seed from a selected plant.

A method as defined above, wherein plants in said population further comprise DNA expressing a protein that provides tolerance to exposure to an herbicide applied at levels that are lethal to wild type plant cells, and wherein said selecting is effected by treating said population with said herbicide.

A method as defined above, wherein said herbicide comprises a glyphosate, dicamba, or glufosinate compound.

A method as defined above, wherein said selecting is effected by identifying plants with said enhanced trait.

A method as defined above, wherein said seed is corn, soybean, cotton, alfalfa, wheat or rice seed.

A method of producing hybrid corn seed comprising:

(a) acquiring hybrid corn seed from a herbicide tolerant corn plant which also has stably-integrated, recombinant DNA comprising a promoter that is (a) functional in plant cells and (b) is operably linked to DNA that encodes a protein having at least one domain of amino acids in a sequence that exceeds the Pfam gathering cutoff for amino acid sequence alignment with a protein domain family identified by a Pfam name in the group of Pfam names consisting of 2-oxoacid_dh, ADH_N, ADH_zinc_N, AP2, AUX_IAA, Aa_trans, Abhydrolase_1, Acyl_transf_1, Aldedh, Aldo_ket_red, Alpha-amylase, Aminotran_1_2, Aminotran_3, Ammonium_transp, Arm, Asn_synthase, BAG, BSD, Beta-elim-lyase, Biotin-lipoyl, Brix, Bromodomain, C1_4, CTP-transf 2, Catalase, CcmH, Chal_sti_synt_C, Cyclin_C, Cyclin_N, Cys_Met_Meta_PP, DAO, DIM1, DPBB_1, DRMBL, DUF167, DUF231, DUF250, DUF6, DUF783, DUF962, E2F_TDP, E3_binding, EBP, Enolase_C, Enolase_N, F420_oxidored, FAD_binding_2, FA_ desaturase, FKBP_C, FTCD_N, Fe_bilin_red, Fer4, GAF, GATase_2, GIDA, GSHPx, Gpi16, HGTP_anticodon, HI0933_like, HLH, HMG_CoA_synt, HWE_HK, Ham1p_like, HhH-GPD, Homeobox, Hpt, Iso_dh, K-box, LEA_4, LRRNT_2, LRR_1, Ldh_1_C, Ldh_1_N, Lectin_legA, Lectin_legB, Lipase_GDSL, MFS_1, MIP, MatE, Metalloenzyme, Methyltransf_11, Methyltransf_12, Molybdop_Fe4S4, Molybdopterin, Molydop_binding, Mov34, MtN3_slv, Myb_DNA-binding, NAD_Gly3P_dh_N, NAD_binding_2, NIR_SIR, NIR_SIR_ferr, NPH3, NTP_transferase, Nuc_sug_transp, PA, PAR1, PFK, PGI, PGK, PGM_PMM_I, PGM_PMM_II, PGM_PMM_III, PGM_PMM_IV, PP2C, PTR2, Peptidase_C26, Phi_1, Phytochrome, Pkinase, Pkinase_Tyr, Pollen_allerg_1, Pribosyltran, Proteasome, Pyr_redox, Pyr_redox_2, Pyr_redox_dim, RNA_pol_L, RNA_pol_Rpb6, RRM_1, RRN3, Radical_SAM, Ras, Response_reg, Rhodanese, Ribosomal_S8e, Rieske, SAC3_GANP, SBDS, SET, SRF-TF, SURF5, Skp1, Skp1_POZ, Ssl1, Sterol_desat, Sugar_tr, TCP, ThiF, Transaldolase, UQ_con, Ubie_methyltran, WD40, WRKY, adh_short, bZIP_1, bZIP_2, cNMP_binding, iPGM_N, p450, tRNA-synt_2b, ubiquitin, zf-A20, zf-AN1, zf-B_box, zf-C2H2, zf-C3HC4, zf-CCCH; wherein the gathering cutoff for said protein domain families is stated in Table 16;

(b) producing corn plants from said hybrid corn seed, wherein a fraction of the plants produced from said hybrid corn seed is homozygous for said recombinant DNA, a fraction of the plants produced from said hybrid corn seed is hemizygous for said recombinant DNA, and a fraction of the plants produced from said hybrid corn seed has none of said recombinant DNA;

(c) selecting corn plants which are homozygous and hemizygous for said recombinant DNA by treating with an herbicide;

(d) collecting seed from herbicide-treated-surviving corn plants and planting said seed to produce further progeny corn plants;

(e) repeating steps (c) and (d) at least once to produce an inbred corn line;

(f) crossing said inbred corn line with a second corn line to produce hybrid seed.

The method of selecting a plant comprising cells as defined above, wherein an immunoreactive antibody is used to detect the presence of said protein in seed or plant tissue.

Anti-counterfeit milled seed having, as an indication of origin, a plant cell as defined above,.

A method of growing a corn, cotton or soybean crop without irrigation water comprising planting seed having plant cells as defined above, which are selected for enhanced water use efficiency.

A method as defmed above comprising providing up to 300 millimeters of ground water during the production of said crop.

A method of growing a corn, cotton or soybean crop without added nitrogen fertilizer comprising planting seed having plant cells as defined above, which are selected for enhanced nitrogen use efficiency.

A plant cell with stably integrated, recombinant DNA that is to suppress the level of an endogenous protein having at least one domain of amino acids in a sequence that exceeds that Pfam gathering cutoff for amino acid sequence alignment with a protein domain family identified by Pfam name in the group of Pfam names consisting of Catalase, Bromodomain, FTCD_N, MatE, DPBB_1, Pollen_allerg_1, tRNA-synt_2b, HGTP_anticodon, Sugar_tr, MFS_1, DUF6, DUF250, LEA_4, MIP, and DUF231 wherein the Pfam gathering cutoff for said protein domain families is stated in Table 16; wherein said plant cells is selected from a population of plant cells with said recombinant DNA by screening plants that are regenerated from plant cells in said population and that the level of said protein is suppressed for an enhanced trait as compared to control plants that do not have said recombinant DNA; and wherein said enhanced trait is selected from the group of enhanced traits consisting of enhanced water use efficiency, enhanced cold tolerance, enhanced heat tolerance, enhanced resistance to salt exposure, enhanced shade tolerance, increased yield, enhanced nitrogen use efficiency, enhanced seed protein and enhanced seed oil.

**Claims**

1. A plant cell with stably integrated, recombinant DNA comprising a promoter that is functional in plant cells and that is operably linked to DNA from a plant, bacteria or yeast that encodes a protein having an amino acid sequence with at least 90% identity to a sequence of one of SEQ ID NOs: 331, 290, 319, 205-289, 291, 293-318, 320-330 and 332-408, wherein the plant cell is selected from a population of plant cells with the recombinant DNA by screening plants that are regenerated from plant cells in the population and that express the protein for an enhanced trait as compared to control plants that do not have the recombinant DNA, wherein the enhanced trait is selected from enhanced resistance to salt exposure, enhanced heat tolerance, enhanced cold tolerance, and increased yield.

2. The plant cell of claim 1 further comprising DNA expressing a protein that provides tolerance from exposure to an herbicide applied at levels that are lethal to a wild type of the plant cell, preferably the agent of the herbicide is a glyphosate, dicamba, or glufosinate compound.

**3.** A transgenic plant or a transgenic seed comprising a plurality of the plant cells of claim 1.

**4.** The transgenic plant of claim 3 which is homozygous for the recombinant DNA.

**5.** A transgenic seed of claim 3 which is from a corn, soybean, cotton, canola, alfalfa, wheat or rice plant.

**6.** Non-natural, transgenic corn seed of claim 7, wherein the seed can produce corn plants that are resistant to disease from the *Mal de Rio Cuarto* virus or the *Puccina sorghi* fungus or both.

**7.** A transgenic pollen grain comprising a haploid derivative of a plant cell of claim 1.

**8.** A method for manufacturing non-natural, transgenic seed that can be used to produce a crop of transgenic plants with an enhanced trait resulting from expression of stably-integrated, recombinant DNA comprising a promoter that is (a) functional in plant cells and (b) is operably linked to DNA from a plant, bacteria or yeast that encodes a protein having an amino acid sequence with at least 90% identity to a sequence of one of SEQ ID NOs: 331, 290, 319, 205-289, 291, 293-318, 320-330 and 332-408, and wherein the enhanced trait is enhanced cold tolerance, enhanced heat tolerance, enhanced resistance to salt exposure, and the method comprising:

(a) screening a population of plants for the enhanced trait and the recombinant DNA, wherein individual plants in the population can exhibit the trait at a level less than, essentially the same as or greater than the level that the trait is exhibited in control plants which do not express the recombinant DNA,
(b) selecting from the population one or more plants that exhibit the trait at a level greater than the level that the trait is exhibited in control plants,
(c) verifying that the recombinant DNA is stably integrated in the selected plants,
(d) analyzing tissue of a selected plant to determine the production of a protein having the function of a protein encoded by nucleotides in the sequence of one of SEQ ID NOs: 331, 290, 319, 205-289, 291, 293-318, 320-330 and 332-408; and
(e) collecting seed from a selected plant.

**9.** The method of claim 8, wherein

(i) plants in the population further comprise DNA expressing a protein that provides tolerance to exposure to an herbicide applied at levels that are lethal to wild type plant cells, and wherein the selecting is effected by treating the population with the herbicide, preferably the herbicide comprises a glyphosate, dicamba, or glufosinate compound, or the seed is corn, soybean, cotton, alfalfa, wheat or rice seed; or
(ii) the selecting is effected by identifying plants with the enhanced trait.

**10.** A method of producing hybrid corn seed comprising:

(a) acquiring hybrid corn seed from a herbicide tolerant corn plant which also has stably-integrated, recombinant DNA comprising a promoter that is (a) functional in plant cells and (b) is operably linked to DNA that encodes a protein having an amino acid sequence with at least 90% identity to a sequence of one of SEQ ID NOs: 331, 290, 319, 205-289, 291-318, 320-330 and 332-408;
(b) producing corn plants from the hybrid corn seed, wherein a fraction of the plants produced from the hybrid corn seed is homozygous for the recombinant DNA, a fraction of the plants produced from the hybrid corn seed is hemizygous for the recombinant DNA, and a fraction of the plants produced from the hybrid corn seed has none of the recombinant DNA;
(c) selecting corn plants which are homozygous and hemizygous for the recombinant DNA by treating with an herbicide;
(d) collecting seed from herbicide-treated-surviving corn plants and planting the seed to produce further progeny corn plants;
(e) repeating steps (c) and (d) at least once to produce an inbred corn line;
(f) crossing the inbred corn line with a second corn line to produce hybrid seed.

**11.** A method of selecting a plant comprising cells of claim 1 wherein an immunoreactive antibody is used to detect the presence of the protein in seed or plant tissue.

**12.** Anti-counterfeit milled seed having, as an indication of origin, a plant cell of claim 1.

13. A method of growing a corn, cotton or soybean crop without irrigation water comprising planting seed having plant cells of claim 1 which are selected for enhanced water use efficiency.

14. A method of claim 13 comprising providing up to 300 millimeters of ground water during the production of the crop.

15. A method of growing a corn, cotton or soybean crop without added nitrogen fertilizer comprising planting seed having plant cells of claim 1 which are selected for enhanced nitrogen use efficiency.

Figure 1

```
SEQ ID NO:
406       :------------------------------------MAKRQLLMLG----IRTSF
6984      :---------------------------------MAKAHQIVLGSNWGIRNIF
624       :------------------------------MAPEERMKQTEGSHKVVTAAGGKMAVGVRSIV
3333      :------------------------------MKQTEGSHKVVTAAGGKMAVGVRSIV
9382      :-------------------------------------------------------QQ
13337     :-----------------------------------------------------------
15475     :-----------------------------------------------------------
1464      :------------------------------MKTEASHKAIAAGGGKMTVLHSPVGVRSIV
7412      :------------------------------MKTEASHKAIAAGGGKMTVLHSPVGVRSIV
11818     :------------------------------MKTEASHKAIAAGGGKMTVLHSPVGVRSIV
11760     :----------------------------------------RWEPAWRRART
11490     :-----------------------------------------------------------
16367     :MWSALFSHLREVHKRSGVKEEKLIMKSPPAAGEAGCHKPQATATNKMTVLQSPLGLRTIL
10381     :--------------------------------MKPEQATHNKMTTAASSPSPVVGLRGVV
17305     :-----------------------------------------MTTTGSTPPRKNRSNV
8426      :-----------------------------------------------------------
4667      :--------------------------MMKPQHGGMAGHGGGRTRSPFLTSY
5355      :----------------------------------------MQRWKWNRKKPPIFPLL
consensus :---------------------------------xxxxxxxxxxxxxxxxxxxxxxxxxxxxx
```

```
HTIAAVLVAGLIFTAVFLSRNSLP-----------------------------------
QSLVAILTTILVVAAIYLTQEGEQ-----------------------------------
TSLVAFFILASSVVFLLLDREAG------------------------------------
TSLVAFFILASSVVFLLLDREAG------------------------------------
QQQVAFFILASSVVFLLLDREAG------------------------------------
-----------------------AG---------------------------------
-----------------------------------------------------------
TSLVAFFILASSIVFLLDRGQEEQVQVAVEHGRQEVQVKL-------------------
TSLVAFFILASSIVFLLDRGQEEQVQVAVEHGRQEVQVKL-------------------
TSLVAFFILASSIVFLLDRGQEEQVQVAVEHGRQEVQVKL-------------------
ARRCRWPRWCSRSSSSRRSSTTR------------------------------------
-----------------------------------------------------------
TSLVAFFIVVSSVSLLFDRGQDAQAQLAVEQHQHQEVLLKQKPASAAVGEQKSVVVDQSS
SSLVAFFIVVSSVSLLFDRGHESQVQLAVQHRHQEVKVAAAGRR---------------
TGGEGGSLEEYAWRAAGEAAAAKKATRAWGVSVSLRSHFSSLVLLLLLLLVALAVSATTK
-----------------------------------------------------------
ALTLAFITFVSVLYFKDFSSTLHQPFLTRPPPHRRQIARPRAP---------------
VLILLFFIAFSTLHSEHTIQRIHENPDHVHNHQEVSSATFVKPNLSGHLKQAPEVLDRFS
XXXXXXXXXXXXXXXXXXXXXXXXXXXXXXXXXXXXXXXXXX-----------------
```

```
-----------KENPQSHGVTDRGGDSGRECNLFEGKWVFDNVSYPLYKEEDCKFMSDQ
-----------WSNERNK------LHSLSKCNLFSGKWVFDNESYPLYKEHQCTFMSDQ
------------LQEEPAIRAGGG---DEECSWSRGRWVYDNVSRPLYDGLKCAFIFPE
------------LQEESAIRAGGGGGGDEECSWSRGRWVYDNVSRPLYDGLKCAFIFPE
------------LQEEPAIRAGGGGG-DEECSWSRGRWVYDNVSRPLYDGLKCAFIFPE
------------LQEQSPITAGGGGSCDEECRWSRGPRVRDNVSRPLYDGLKCAFIFPE
```

```
-------------------EPAAAAGDEECSWSRGRWVYDNVSRPLYDGLKCAFIFPE
---------ESGLQEPAIRGTTQEGDASNEECNWSRGRWVYDNVSRPLYSGLKCSFIFPE
---------ESGLQEPAIRGTTQEGDASNEECNWSRGRWVYDNVSRPLYSGLKCSFIFPE
---------ESGLQEPAIRGTTQEGDASNEECNWSRGRWVYDNVSRPLYSGLKCSFIFPE
--------------TSSPTAALRGVVSVPQTCDLYRGSWVYDEVSAPVYKEGECEFLTEQ
---------MLRRNSPSSSISTKNGFDGEEDCNWSLGRWVYDNASRPLYSGLKCSFIFDE
LRSQEAQVQWTSELQDVATDSGDGGFDGEEDCNWSLGRWVYDNASRPLYSGLKCSFIFDE
----------EAQVQWTDELMGEAVRGSGEECNWSXGRWVYDNASQPLYSGLNCSFSFDE
NGDPAETPHAPPLPPPASIKLPSSSSSGGGECDLFSGRWVYDEAAYPLYRESACRVMSEQ
-----------------SIKLPSSSSSGGGECDLFSGRWVYDEAAYPLYRESACRVMSEQ
---SHHHGGGSSSGGGDVVPPFAVGAAAAAGCDVGVGEWVYDEAARPWYEEEECPYIQPQ
RCNSTVEYSGRKIAWLGDSRHSGHWSARPESCDVFSGKWVFDNVSHPLYNESDCPYMSDQ
---------xxxxxxxxxxxxxxxxxxxxxxxCxxxxGxwVyDnxsxPlYxxxxCxfxxxx
```

```
LACEKFGRKDLSYKFWRWQPHT--CDLPRFNGTKLLERLRNKRMVYVGDSLNRGQWVSMV
LACEKFGRKDLSYQNWRWKPHQ--CDLPRFNATALLERLRNKRMVFVGDSLNRGQWVSMV
VACDKYGRKDVMYQHWRWQPHGHGCDLPRFDGIKLLEELRNKRMVFVGDSVNRNQWVSLV
VACDKYGRKDVMYQHWRWQPHGHGCDLPRFDGMKLLEELRNKRLVFVGDSVNRNQWVSLV
VACDKYGRKDVMYQHWRWQPHGHGCDLPRFDAMKLLEELRNKRLVFVGDSVNRNQWVSLV
VACDKYGRKDVMYQHWRWQPHGHGCDIPRFDGIKLLEELRNKRLVFVGDSVNRNQWVSLV
VACDKYGRKDVMYQHWRWQPHGHGCDLPRFDGIKLLEELRNKRLVFVGDSVNRNQWVSLV
VACDKYGRKDAMYQHWRWQPHGHGCDLPRFDAIKLLENLRNKRMVFVGDSVNRNQWVSLV
VACDKYGRKDAMYQHWRWQPHGHGCDLPRFDAIKLLENLRNKRMVFVGDSVNRNQWVSLV
VACDKYGRKDAMYQHWRWQPHGHGCDLPRFDAIKLLENLRNKRMVFVGDSVNRNQWVSLV
VTCMRNGRRDDSYQKWRWQPAG--CDLPRFDARLLLERLRNKRLMFVGDSLNRNQWESMV
VACDKYGRNDTKYQHWRWQPHG--CNLPRFNATKFLEKLRNKRLVFVGDSVNRNQWVSMV
VACDKYGRNDTKYQHWRWQPHG--CNLPRFNATKFLEKLRNKRLVFVGDSVNRNQWVSMV
VAXEKYGRNDTRYXYWRWQPDG--CDLSRFNATKLLEKLRNKRMVFVGDSINRNQWVSMV
SACEKYGRTDLRYQHWRWQPHG--CDLPRFDAEKFLGKLRNKRLVFVGDSLNRNQWASML
SACEKYGRTDLRYQHWRWQPHG--CDLPRFDAEKFLGKLRNKRLVFVGDSLNRNQWASML
LTCQAHGRPDTAYQHWRWQPRG--CSLPSFNATLMLEMLRGKRMMFVGDSLNRGQYVSLV
LACHKHGRSDLGYQYWRWQPHN--CNLKRWNVKEMWEKLRGKRLMFVGDSLNRGQWISMV
xacxkyGRxDxxYqhWRWqPhgxxCdlprfxxxkxlexLRnKRxvfVGDSxNRnQwvSxv
```

```
CMVSSVITN--PKAMYMHNNGSNLITFKALEYNATIDYYWAPLLVESNSDDPTNHRFPDR
CLVESSVPP--TLKSMRTIANGSLNIFKAEEYNATIEFYWAPLLVESNSDDPVXHRVAER
CMVEASIPD---DRLKIRTFNGSLISFKALEYNATIDFYWSPLLVESNSDNPIIHRVEYR
CMVEASIPD---DRLKMRIFNGSLISFKALEYNATIDFYWSPLLVESNSDNPIIHRVEYR
CMVEASIPD---DRLKMRIFNGSLISFKALEYNATIDFYWSPLLVESNSDNPIIHRVEYR
CMVEASIPD---DRLKMRIFNGSLISFKALEYNATIDFYWSPLLVESNSDNPIIHRVEYR
CMVEASIPD---DRLKRRIFNGSLISFKALEYNATIDFYWSPLLVESNSDNPIIHRVEYR
CMVEASIPD---DRLKIRTFNGSLISFKALEYNATIDFYWSPLLVESNSDNPIIHRVEYR
CMVEASIPD---DRLKIRTFNGSLISFKALEYNATIDFYWSPLLVESNSDNPIIHRVEYR
CMVEASIPD---DRLKIRTFNGSLISFKALEYNATIDFYWSPLLVESNSDNPIIHRVEYR
CLVQSVIPDRGQKTLTKFVNGGSSNVFYAHEYNATIDFYWSPLLVESNSDNPIIHRVEYR
CMVEHFIPD----GRKMRVYNGSLISFKAFEYNATIDFYWSPLLLESNSDNPIIHRVEYR
CMVEHFIPD----GRKMRVYNGSLISFKAFEYNATIDFYWSPLLLESNSDNPIIHRVEYR
CMVEASIPE----GQKMRVYNGSLISFTAFEYNATIDFYGPPLILESNSDNPIIHRVEYR
CLIDTGAPE----LHTSINSSRSLTTFKIHEYNASVDFYWSPLLVESNSDHPLRHRVADR
CLIDTGAPE----LHTSINSSRSLTTFKIHEYNASVDFYWSPLLVESNSDHPLRHRVADR
CLLHRSIPE----SSKSMETFDSLTVFRAKNYNATIEFYWAPFLAESNSDDAVVHRIADR
```

```
CLLQSVIPA----DKRSMSPNAHLTIFRAEEYNATVEFLWAPLLAESNSDDPVNHRLDER
Cxvxxxipx---xxxxxxxxngslxxFkaxeYNAtidfywsPllvESNSDxpxxHRvxxR
```

```
IVRIQSIEKHARHWTNSDIIVFNSYLWWRM----P----HIKSLWG---------SFEKL
TVRVQAIEKHARYWTDADILVFNTFLWWRR----R----AMNVLWG---------SFGDP
IIRADRIEKHASVWRDADIIVFNSYLWWRK----QKDDMRMKVMYG---------SFEDG
IIRADRIEKHASVWRDADVIVFNSYLWWRK----QKDDMRMKVMYG---------SFEDG
IIRADRIEKHASVWRDADVIVFNSYLWWRK----QKDDMRMKVMYG---------SFEDG
IIRADRIEKHASVWRDADVIVFNSYLWWRK----QKDDMRMKVMYG---------SFEDG
IIRADRIEKHASVWRDADVIVFNSYLWWRK----QKDDMRMKVMYG---------SFEDG
IIRADRIEKHASVWRDADIIVFNSYLWWRK----QKDDMRMKVMYG---------SFEDG
IIRADRIEKHASVWRDADIIVFNSYLWWRK----QKDDMRMKVMYG---------SFEDG
IIRADRIEKHASVWRDADIIVFNSYLWWRK----QKDDMRMKVMYG---------SFEDG
IIRADRIEKHASVWRDADVIVFNSYLWWRI----PP----CARRYG---------SFEDG
IIRADRIEKHANVWKDADFIVFNSYLWWR--------IIMQKCRYG---------SFEDG
IIRADRIEKHANVWKDADFIVFNSYLWWRK----QRDGMMMKVMYG---------SFEDG
IIRAEKIEKHARAWGNADVIVFNSYLWWRK----QKPDMKMKVMYG---------SFEDG
TVRAASINKHAAHWTNADVLVFNSYLWWQR----PAMKVLWGSFDNPAAVVAAAAEEGDE
TVRAASINKHAAHWTNADVLVFNSYLWWQR----PAMKVLX-------------EEGNE
IVRGTALEKHARFWKGADILVFNSYLWWMTGQKMKILQG----------------SFEDK
IIRPDTVLRHASLWENADILVFNTYLWWRQGPVKLLWT------------------HEE
iiRaxxiekHAxxWxxaDxiVFNsyLWWrx----xxxxxxxxxxxg---------sfedx
```

```
DGIYKEVEMVRVYEMALQTLSQWLEVHVNPNITKLFFMSMSPTHERAEEWGGILNQNCYG
NGISKRVGMVRVYEMALRTWSEWLEVHIKPNKTKLFFVSMSPTHQKA-------------
DAKLDEMEMVDGFEIAIKKLTEWLAENIDKNKTRIFFAGSSPTHSWASNWGGQDKNKCLN
DAKLDEMEMVDGFEIAIKKLTEWLAENIDKNKTRIFFAGSSPTHSWASNWGGQDKNKCLN
DAKLDEMEMADGFEIAIKKLTEWLAKNIDKNKTRIFFAGSSPTHSWASNWGGQDKNKCLN
DAKLDEMEMADGFEIAIKKLTEWLAENIDKNKTRIFFAGSSPTHSWASNWGGEDKNKCLN
DAKLDEMEMADGFEIAIKKLTEWLAENIDKNKTRIFFAGSSPTHSWASNWGGQDKNKCLN
DAKLDEMEMVDGFEIALKKLTEWLGENIDKNKTRIFFAGSSPTHSWASNWGGEDKNKCLN
DAKLDEMEMADGFEIAIKKLTEWLAENIDKNKTRIFFAGSSPTHSWASNWGGEDKNKCLN
DAKLDEMEMVDGFEIAIKKLTEWLAENIDKNKTRIFFAGSSPTHSWASNWGGQDKNKCLN
DAKLDEMEMADGFEIAIKKLTEWLAKNIDKNKTRIFFAGSSPTHSWASNWGGQDKNKCLN
DAKLDEVQMVDGYEIALKKLTEYLGANINKNKTRIFFAGSSPAHSWASNWGGDDNNKCLN
DAKLDEVQMVDGYEIALKKLTEYLGANINKNKTRIFFAGSSPAHSWASNWGGDDNNKCLN
DAKLDEVEMVEGFEIALKKLTEWVGANVN-NKTKIYFAGSSPTHTWASDWGGDDSNKCLN
YAVSKVIDSLRAYELAVRTWADWMEFHVDRARTQLFFMTMSPTHLRSDEWEDAAAAAAGG
YAVSKVIDSLRAYELAVRTWADWMEFHVDRARTQLFFMTMSPTHLRSDEWEDAAAAAAGG
SKDIVEMETEEAYGMVLNAVVRWVENNMNPRNSRVFFVTMSPTHTRSKDWGDDSDGNCYN
NGACEELDGHGAMELAMGAWADWVSSKVDPLKKRVFFVTMSPTHLWSREWKPGSEGNCYG
daxxxexxmxxxxexaxxxlxewlxxnxxxnktrxfFxxxSPtHxwaxxwggxxxxxcxn
```

```
EA---SLIDKEGY----TGRGSDPKMMRVLENVLDGLKNRGLNMQMINITQLSEYRKEGH
---------------------------------------------------------
ET---EPISYRPGGGYKAATTDYSLMAMARSYFRRTLEPRGIRVQILNITELSDYRKDGH
ET---EPISYRPGGGYKAATTDYSLMAMARSYFRRTLEPRGIRVQILNITELSDYRKDGH
ET---EPISYRPGGGYKAATTDYSLMAMARSYFRRTLEPRGIRVQILNITELSDYRKDGH
ET---EPIYR-PGGYYKAATTDYSLMAKARSYFQRTLEPRGIRVQILNITELSDYRKDGH
ET---EPISYRPGGGYKAATTDYSLMAMARSYFRRTLEPRGIRVQILNITELSDYRKDGH
ET---EPIYKTG---YKAATTDYSLMAKAKHYFR-TLEPKGIHVQILNITELSDYRKDGH
```

126

```
ET---EPIYRPGG--YKAATTDYSLMÄKARSYFRRTLEPRGIRVQILNITELSDYRKDGH
ET---EPISYRPGGGYKAATTDYSLMAMARSYFRRTLEPRGIRVQILNITELSDYRKDGH
ET---EPISYRPGGGYKAATTDYSLMAMARSYFRRTLEPRGIRVQILNITELSDYRKDGH
ET---EPIQIED---YRSATTDYGMMDKAKEIFG-TLEPKGIHVQILNITQLFEYRKDAH
ET---EPIQIED---YRSATTDYGMMDKAKEIFG-TLEPKGIHVQILNITQLSEYRKDAH
ES---EPIQKVG---YKGATTDYSMMDKAKQIFR-PLEQKGINVQILNFTQLTDYRIDAH
NHGCYGETEPIAAEEYRGTSGTDMAFARAVEAEARRLGERSVAVRLINVTRLSERRKDAH
NHGCYGETEPIAAEEYRGTSGTDMAFARAVEAEARRLGERSVAVRLINVTRLSERRKDAH
QT---------TPIRDLSYWGPGTSKGLMRVIGEVFSTSKVPVGIVNITQLSEYRKDAH
EK---------DPIDNEGYWGSGSDLPTMSTVEKILSNLSSKVSVINITQLSEYRKDGH
ex---xxixxxxxxyxxxxxxxxxmxxaxxxxxxxlxxxgxxvqixnitxlsxyrkdxh
```

```
PSIYRKQWGTVKENEISNPSSNADCIHWCLPGVPDVWNELLYAYILDHHSS*
---------------------------------------------------*
PTVFRRQFVPLTKEQIADPASYADCTHWCLPGVPDVWNEFLYGYLTQQRK-*
PTVFRRQFVPLTKEQIADPASYADCTHWCLPGVPDVWNEFLYGYLTQQRK-*
PTVFRRQFVPLTKEQIADPASYADCTHWCLPGVPDVWNEFLYGYLTQQSK-*
PTVFRRQFVPLTKEQIADPASYADCTHWCLPGVPDVWNEFLYGYLVHV---*
PTVFRRQFVPLTKEQIADPASYADCTHWCLPGVPDVWNEFLYGYLVHV---*
PTVYRKQFAPLTKEQIADPASYADCTHWCLPGVPDVWNEFLYGYLVQK---*
PTVFRRQFVPLTKEQIADPASYADCTHWCLPGVPDVWNEFLYGYLVQQRK-*
PTVFRRQFVPLTKEQIADPASYADCTHWCLPGVPDVWNEFLYGYLTQQRK-*
PTVFRRQFVPLTKEQIADPASYADCTHWCLPGVPDVWNEFLYGYLTQQSK-*
PTIFRRQYVPLTKEQIANPSIYADCTHWCLPGVPDVWNEFLYAYIMHK---*
PTIFRRQYVPLTKEQIANPSIYADCTHWCLPGVPDVWNEFLYAYIMHK---*
PTVFRRQFTPLTKEQIANPSSYADCTHWCLPGVPDVWNHFLYSYLVQK---*
PSVHRRYWDPVTDEQRRNPSSYADCIHWCLPGVPDVWNQLLYAHIVS----*
PSVHRRYWDPVTDEQRRNPSSYADCIHWCLPGVPDVWNQLLYAHIVS----*
TQIYKKQWNPLTPEQIANPKSYADCTHWCLPGLQDTWNELLYSKLFFP---*
PSIFRKFWEPLRPEQLSNPPSYSDCIHWCLPGVPDVWNELLFHFL------*
pxxxrrqxxpltxeqiaxpxsyadcthwclpgvpdvwnexlyxyxxxxxx-*
```

Figure 2. Corn transformation base vector

IG-St.Pis4

T-AGRtu.nos

B-AGRtu.left border

OR-Ec.oriV-RK2

CR-Ec.nptII-Tn5

L-CaMV.35S

P-CaMV.35S

**pMON82060**
**Corn transformation base v ector**
**SEQ ID NO: 19249**

CR-Ec.rop

T-St.Pis4

OR-Ec.ori-ColE1

P-Ec.aadA-SPC/STR

CR-Ec.aadA-SPC/STR

T-Ec.aadA-SPC/STR

I-Os.Act1

L-Os.Act1

P-Os.Act1

B-AGRtu.right border

Figure 3. Soybean transformation base vector

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 64271705 P
- US 20030101479 A
- US 5188642 A
- US 5728925 A
- US 4196265 A
- US 5858742 A
- US 5322938 A
- US 5378619 A
- US 6437217 B
- US 5641876 A
- US 6426446 B
- US 6429362 B
- US 6232526 B
- US 6177611 B
- US 6433252 B
- US 6429357 B
- US 5837848 A
- US 6084089 A
- US 6294714 B
- US 6140078 A
- US 6252138 B
- US 6175060 B
- US 20020192813 A1
- US 078972 A
- US 757089 A
- US 739565 A
- US 10739565 B
- US 5420034 A
- US 5107065 A, Shewmaker
- US 5759829 A, Shewmaker
- US 5283184 A, Jorgensen
- US 5231020 A, Jorgensen
- EP 0426195 A1
- WO 9401550 A, Agrawal
- WO 9805770 A
- US 20030175965 A1
- US 20020048814 A1
- US 20030018993 A1
- US 20030036197 A1
- US 6326193 B
- WO 9953050 A, Waterhouse
- WO 9949029 A, Graham
- US 465800 A, Fillatti
- US 6506559 B, Fire
- US 393347 A, Shewmaker
- US 6448473 B, Mitsky
- US 5015580 A, soybean
- US 5550318 A, corn
- US 5538880 A, corn
- US 5914451 A, soybean
- US 6160208 A, corn
- US 6399861 B, corn
- US 6153812 A, wheat
- US 5159135 A, cotton
- US 5824877 A, soybean
- US 5591616 A, corn
- US 6384301 B, soybean
- US 4959317 A
- US 5527695 A
- US 6194636 B
- US 5633435 A
- US 5780708 A
- US 6118047 A
- US 20030106096 A1
- US 20020112260 A1
- US 5034322 A
- US 5776760 A
- US 6107549 A
- US 6376754 A
- US 5250515 A
- US 5880275 A
- US 6506599 B
- US 5986175 A
- US 20030150017 A1
- US 303745 A

**Non-patent literature cited in the description**

- **S.R. EDDY.** *Bioinformatics,* 1998, vol. 14, 755-763
- **RUSSELL et al.** *Transgenic Res.,* 1997, vol. 6 (2), 157-166
- **BELANGER et al.** *Genetics,* 1991, vol. 129, 863-872
- **STACY et al.** *Plant Mol Biol.,* 1996, vol. 31 (6), 1205-1216
- **FISCHHOFF et al.** *Plant Mol Biol.,* 1992, vol. 20, 81-93
- **TANIGUCHI et al.** *Plant Cell Physiol.,* 2000, vol. 41 (1), 42-48

- **JORGENSEN et al.** *Mol. Gen. Genet.,* 1987, vol. 207, 471-477
- **STAM et al.** *The Plant Journal,* 1997, vol. 12 (1), 63-82
- **SIJEN et al.** *The Plant Cell,* 1996, vol. 8, 2277-2294
- **METTE et al.** *The EMBO Journal,* 1999, vol. 18 (1), 241-148
- **METTE et al.** *The EMBO Journal,* 2000, vol. 19 (19
- **PILON-SMITS et al.** *Plant Physiol.,* 1995, vol. 107, 125-130
- **BETHTOLD et al.** *Methods Mol. Biol.,* 1998, vol. 82, 259-66
- **BOYES et al.** *The Plant Cell,* 2001, vol. 13, 1499-1510
- **BOYES, D.C.** *The Plant Cell,* 2001, vol. 13, 1499-1510